(19) Europäisches Patentamt
European Patent Office
Office européen des brevets

(11) EP 4 725 948 A1

(12) EUROPEAN PATENT APPLICATION
published in accordance with Art. 153(4) EPC

(43) Date of publication:
**15.04.2026 Bulletin 2026/16**

(21) Application number: **24818793.2**

(22) Date of filing: **07.06.2024**

(51) International Patent Classification (IPC):
***C07D 487/04*** (2006.01) ***C07D 471/04*** (2006.01)
***C07D 519/00*** (2006.01) ***A61K 31/519*** (2006.01)
***A61P 9/00*** (2006.01) ***A61P 11/00*** (2006.01)

(52) Cooperative Patent Classification (CPC):
**A61K 31/519; A61P 9/00; A61P 11/00; C07D 471/04; C07D 487/04; C07D 519/00**

(86) International application number:
**PCT/CN2024/098179**

(87) International publication number:
**WO 2024/251267 (12.12.2024 Gazette 2024/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.06.2023 CN 202310680154**
**14.07.2023 CN 202310865500**
**04.08.2023 CN 202310977384**
**08.12.2023 CN 202311680092**
**27.03.2024 CN 202410358137**

(71) Applicant: **Haisco Pharmaceutical Group Co., Ltd.**
**Shannan, Tibet 856000 (CN)**

(72) Inventors:
- **ZHANG, Chen**
  **Chengdu, Sichuan 611130 (CN)**
- **WANG, Jianmin**
  **Chengdu, Sichuan 611130 (CN)**
- **QIAN, Guofei**
  **Chengdu, Sichuan 611130 (CN)**
- **HUANG, Zhenggang**
  **Chengdu, Sichuan 611130 (CN)**
- **CHEN, Tengfei**
  **Chengdu, Sichuan 611130 (CN)**
- **HUANG, Longbin**
  **Chengdu, Sichuan 611130 (CN)**
- **QIAN, Meilin**
  **Chengdu, Sichuan 611130 (CN)**
- **TANG, Pingming**
  **Chengdu, Sichuan 611130 (CN)**
- **CHEN, Quanlong**
  **Chengdu, Sichuan 611130 (CN)**
- **SHI, Ronghua**
  **Chengdu, Sichuan 611130 (CN)**
- **WANG, Ting**
  **Chengdu, Sichuan 611130 (CN)**
- **LI, Yao**
  **Chengdu, Sichuan 611130 (CN)**
- **YAN, Pangke**
  **Chengdu, Sichuan 611130 (CN)**

(74) Representative: **AWA Denmark A/S**
**Strandgade 56**
**1401 Copenhagen K (DK)**

(54) **PYRROLO[2,3-D]PYRIMIDIN-4-AMINE DERIVATIVE AND USE THEREOF IN MEDICINE**

(57) A pyrrolo[2,3-d]pyrimidin-4-amine derivative and a use thereof in medicine, specifically a compound represented by formula (I) or a stereoisomer, tautomer, deuterated compound, solvate, prodrug, metabolite, pharmaceutically acceptable salt or co-crystal thereof, and a use thereof in the preparation of a drug for treating sGC-related diseases.

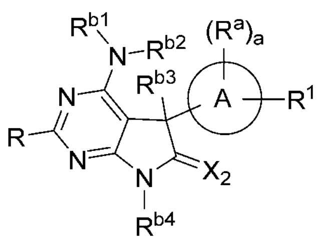

(I)

EP 4 725 948 A1

## Description

### Technical Field

The present invention belongs to the field of medicine, and specifically discloses a compound represented by formula (I) or a stereoisomer, tautomer, deuterated compound, solvate, prodrug, metabolite, pharmaceutically acceptable salt or co-crystal thereof, and the use thereof in the preparation of a drug for treating sGC-related diseases.

### Background Art

Nitric oxide (NO) signalling has pleiotropic effects in biology and plays a critical function in cardiovascular homeostasis. An increase in NO secretion occurs under the influence of mediators, such as norepinephrine (NA), angiotensin, adenosine triphosphate, or bradykinin. NO synthesis is also stimulated as a result of numerous physical factors (Int. J. Mol. Sci. 2021, 22, 6029; Molecules 2021, 26, 3418).

The mechanism of NO's intracellular action is primarily through the stimulation of the activity of soluble guanylyl cyclase (sGC). The sGC is heme-containing enzyme, which causes an increase in the level of cyclic 3'-5'-guanosine monophosphate (cGMP) in smooth muscle and subsequent vascular relaxation (Nat. Rev. Cardiol. 2018, 15,292-316). The NO/sGC/cGMP regulatory pathway plays a leading role in the homeostasis of the cardiovascular and pulmonary systems and organs (such as the kidney, brain, and liver). In addition to smooth muscle cells, cGMP influences the function of fibroblasts, cardiomyocytes, platelets, neurons, and immune cells, regulating the processes of fibrosis, inflammatory response, and neurotransmission (Molecules 2023, 28, 861).

sGC modulators and sGC agonists are a class of drugs that can stimulate cGMP formation, and provide tools for studying the regulatory mechanisms of sGC and its role in pathological mechanisms. The development of sGC modulators or agonists has made it possible to develop drugs that directly target diseased blood vessels, myocardium, kidneys, and other organs (Molecules 2023, 28, 861). Riociguat is the first approved sGC agonist. In 2013, Riociguat was approved for two indications: pulmonary arterial hypertension (PAH) and chronic thromboembolic pulmonary hypertension (TEPH) (J. Med. Chem. 2017, 60, 5146-5161). In the 12-week, multicentre, double-blind, randomised, placebo-controlled, pivotal PATENT-1 study, the most common AEs (occurring in ≥ 3% of patients) that were reported more frequently in the riociguat group than in the placebo group were headache (27% vs. 18%), dyspepsia/gastritis (21% vs. 8%), dizziness (20% vs. 13%), nausea (14% vs. 11%), diarrhoea (12% vs. 8%), hypotension (10% vs. 4%), vomiting (10% vs. 7%), anaemia (7% vs. 2%), gastrooesophageal reflux disease (5% vs. 2%), and constipation (5% vs. 1%). The development of novel sGC modulators or agonists to improve therapeutic effects or reduce toxic and side effects has good application prospects.

### Summary of the Invention

The objective of the present invention is to provide a compound having a novel structure and represented by general formula (I) or a stereoisomer, deuterated compound, solvate, prodrug, metabolite, pharmaceutically acceptable salt or co-crystal thereof, an intermediate thereof, a preparation method therefor, and the use thereof in the preparation of a drug for treating sGC-related diseases.

The compounds of the present invention have good stimulatory effects on cGMP production in LNCap cells and exhibit excellent lung pharmacokinetic properties, e.g., superior lung AUC and/or lung-to-plasma ratios compared to control 1, thereby conferring the advantages of lung targeting and reduced systemic side effects.

The present invention provides a compound represented by general formula (I) or a stereoisomer, tautomer, deuterated compound, solvate, prodrug, metabolite, pharmaceutically acceptable salt or co-crystal thereof,

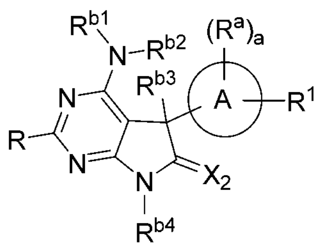

(I)

in some embodiments, the compound represented by general formula (I) is a compound represented by general formula (II),

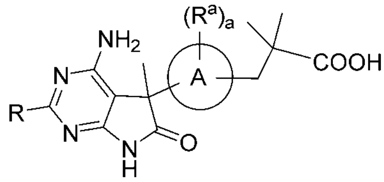

(II);

in some embodiments, the compound represented by general formula (I) or (II) is a compound represented by general formula (III-1) or (III-2),

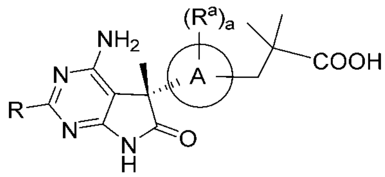

(III-1),

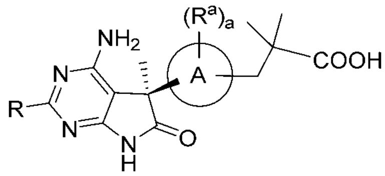

(III-2);

in some embodiments, R is selected from

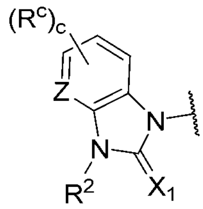
,
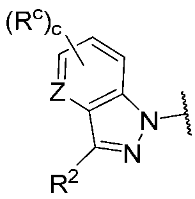
,

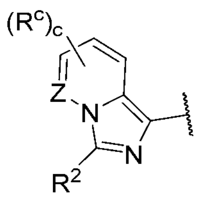
, or
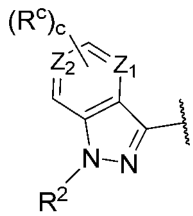
;

in some embodiments, R is selected from

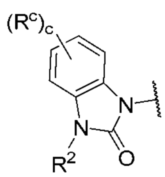
,
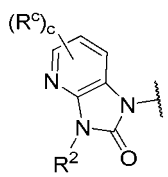
,

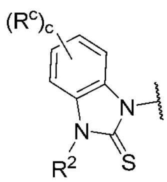
,
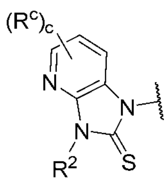
,
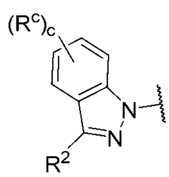
,
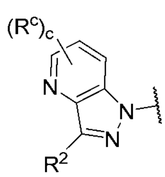
,
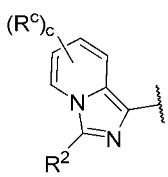
,
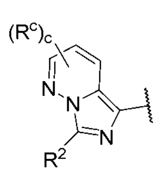
,

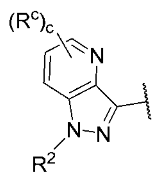, or 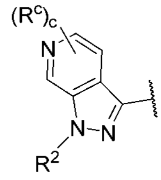;

in some embodiments, Z is selected from CH or N;
in some embodiments, $Z_1$ or $Z_2$ is each independently selected from CH or N, and at least one of $Z_1$ and $Z_2$ is N;
in some embodiments, $X_1$ or $X_2$ is each independently selected from O or S;
in some embodiments, A is selected from $C_{3-12}$ carbocyclyl or 4- to 12-membered heterocyclyl;
in some embodiments, A is selected from $C_{3-11}$ cycloalkyl, 4- to 11-membered heterocycloalkyl, $C_{6-10}$ aryl or 5- to 10-membered heteroaryl;
in some embodiments, A is selected from $C_{3-6}$ cycloalkyl, 4- to 6-membered heterocycloalkyl, $C_{6-10}$ aryl or 5- to 10-membered heteroaryl;
in some embodiments, A is selected from the following groups optionally substituted with 1 to 4 $R^a$: cyclopropyl, cyclobutyl, cyclopentyl, cyclohexyl, oxetanyl, azetidinyl, pyrrolidyl, piperidyl, piperazinyl, morpholinyl, tetrahydrofuryl, tetrahydropyranyl, phenyl, pyrrolyl, thienyl, furyl, pyrazolyl, imidazolyl, thiazolyl, isothiazolyl, oxazolyl, isoxazolyl, pyridyl, pyrimidyl, pyrazinyl, or pyridazinyl;
in some embodiments, A is selected from the following groups optionally substituted with 1 to 4 $R^a$: phenyl, thiazolyl, oxazolyl, pyridyl, pyrimidyl, pyrazinyl, or pyridazinyl;
in some embodiments, $R^1$ is $-M-(CR^{1a}R^{1b})_r-(CR^{1c}R^{1d})_s-COOH$;
in some embodiments, $R^1$ is selected from

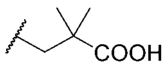, 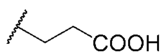,

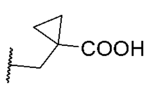, 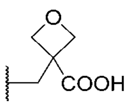, 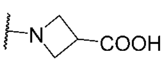, 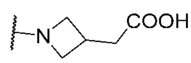, or 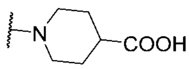;

in some embodiments, M is selected from a bond, $C_{3-12}$ carbocyclyl or 4- to 12-membered heterocyclyl, wherein the carbocyclyl or heterocyclyl is optionally substituted with 1 to 4 $R^m$;
in some embodiments, M is selected from a bond, $C_{3-11}$ cycloalkyl, 4- to 11-membered heterocycloalkyl, $C_{6-10}$ aryl or 5- to 10-membered heteroaryl, wherein the cycloalkyl, heterocycloalkyl, aryl, or heteroaryl is optionally substituted with 1 to 4 $R^m$;
in some embodiments, M is selected from a bond, $C_{3-6}$ cycloalkyl, 4- to 6-membered heterocycloalkyl, $C_{6-10}$ aryl or 5- to 10-membered heteroaryl, wherein the cycloalkyl, heterocycloalkyl, aryl, or heteroaryl is optionally substituted with 1 to 4 $R^m$;
in some embodiments, M is selected from a bond, or the following groups optionally substituted with 1 to 4 $R^m$: cyclopropyl, cyclobutyl, cyclopentyl, cyclohexyl, azetidinyl, pyrrolidyl, piperidyl, piperazinyl, phenyl, pyrrolyl, thienyl, furyl, pyrazolyl, imidazolyl, thiazolyl, isothiazolyl, oxazolyl, isoxazolyl, pyridyl, pyrimidyl, pyrazinyl, or pyridazinyl;
in some embodiments, $R^2$ is selected from $C_{1-6}$ alkyl or $C_{1-6}$ alkylene-Q, wherein the alkyl or alkylene is optionally substituted with 1 to 10 $R^k$;
in some embodiments, $R^2$ is selected from $C_{1-5}$ alkyl or $C_{1-4}$ alkylene-Q, wherein the alkyl or alkylene is optionally substituted with 1 to 8 $R^k$;
in some embodiments, $R^2$ is selected from $C_{1-4}$ alkyl or $C_{1-3}$ alkylene-Q, wherein the alkyl or alkylene is optionally substituted with 1 to 6 $R^k$;
in some embodiments, $R^2$ is selected from methyl, ethyl, propyl, butyl, - methylene-Q, -ethylene-Q, or -propylene-Q, wherein the methyl, ethyl, propyl, butyl, methylene, ethylene, or propylene is optionally substituted with 1 to 6 $R^k$;
in some embodiments, $R^2$ is selected from

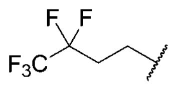, 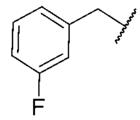, 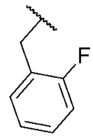,

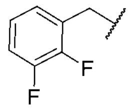, 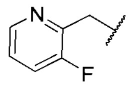, or 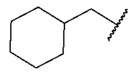;

in some embodiments, Q is selected from $C_{3-12}$ carbocyclyl or 4- to 12-membered heterocyclyl, wherein the carbocyclyl or heterocyclyl is optionally substituted with 1 to 4 $R^q$;

in some embodiments, Q is selected from $C_{3-11}$ cycloalkyl, 4- to 11-membered heterocycloalkyl, $C_{6-10}$ aryl or 5- to 10-membered heteroaryl, wherein the cycloalkyl, heterocycloalkyl, aryl, or heteroaryl is optionally substituted with 1 to 4 $R^q$;

in some embodiments, Q is selected from $C_{3-6}$ cycloalkyl, 4- to 6-membered heterocycloalkyl, $C_{6-10}$ aryl or 5- to 10-membered heteroaryl, wherein the cycloalkyl, heterocycloalkyl, aryl, or heteroaryl is optionally substituted with 1 to 4 $R^q$;

in some embodiments, Q is selected from the following groups optionally substituted with 1 to 4 $R^q$: cyclopropyl, cyclobutyl, cyclopentyl, cyclohexyl, oxetanyl, azetidinyl, pyrrolidyl, piperidyl, piperazinyl, morpholinyl, tetrahydrofuryl, tetrahydropyranyl, phenyl, pyrrolyl, thienyl, furyl, pyrazolyl, imidazolyl, thiazolyl, isothiazolyl, oxazolyl, isoxazolyl, pyridyl, pyrimidyl, pyrazinyl, or pyridazinyl;

in some embodiments, $R^a$, $R^c$, $R^m$, $R^q$, $R^{b3}$, $R^{1a}$, $R^{1b}$, $R^{1c}$, and $R^{1d}$ are each independently selected from H, deuterium, halogen, OH, CN, $NH_2$, $C_{1-6}$ alkyl, $OC_{1-6}$ alkyl, $SC_{1-6}$ alkyl, $C_{2-6}$ alkenyl, $C_{2-6}$ alkynyl, $NHC_{1-6}$ alkyl, $N(C_{1-6}$ alkyl$)_2$, -O-$C_{3-6}$ carbocyclyl, -O-3- to 7-membered heterocyclyl, -NH-$C_{3-6}$ carbocyclyl, -NH-3- to 7-membered heterocyclyl, -$C_{0-4}$ alkylene-$C_{3-6}$ carbocyclyl, or -$C_{0-4}$ alkylene-3- to 7-membered heterocyclyl, wherein the alkyl, alkylene, alkenyl, alkynyl, carbocyclyl or heterocyclyl is optionally substituted with 1 to 4 $R^k$;

in some embodiments, $R^a$, $R^c$, $R^m$, $R^q$ , $R^{b3}$ , $R^{1a}$, $R^{1b}$, $R^{1c}$, and $R^{1d}$ are each independently selected from H, deuterium, halogen, OH, CN, $NH_2$, $C_{1-4}$ alkyl, $OC_{1-4}$ alkyl, $SC_{1-4}$ alkyl, $C_{2-4}$ alkenyl, $C_{2-4}$ alkynyl, $NHC_{1-4}$ alkyl, $N(C_{1-4}$ alkyl$)_2$, -O-$C_{3-6}$ carbocyclyl, -O-3- to 6-membered heterocyclyl, -NH-$C_{3-6}$ carbocyclyl, -NH-3- to 6-membered heterocyclyl, -$C_{0-2}$ alkylene-$C_{3-6}$ carbocyclyl, or -$C_{0-2}$ alkylene-3- to 6-membered heterocyclyl, wherein the alkyl, alkylene, alkenyl, alkynyl, carbocyclyl or heterocyclyl is optionally substituted with 1 to 4 $R^k$;

in some embodiments, $R^a$, $R^c$, $R^m$, $R^q$ , $R^{b3}$ , $R^{1a}$, $R^{1b}$, $R^{1c}$, and $R^{1d}$ are each independently selected from H, deuterium, F, Cl, Br, I, OH, CN, $NH_2$, $NHCH_3$, $N(CH_3)_2$, or the following groups optionally substituted with 1 to 4 $R^k$: methyl, ethyl, propyl, isopropyl, methoxy, ethoxy, isopropoxy, ethenyl, ethynyl, methylthio, cyclopropyl, cyclobutyl, cyclopentyl, cyclohexyl, azetidinyl, oxetanyl, pyrrolidyl, piperidyl, morpholinyl, or phenyl;

in some embodiments, $R^a$, $R^c$, $R^m$, $R^q$, $R^{b3}$ , $R^{1a}$ , $R^{1b}$, $R^{1c}$, and $R^{1d}$ are each independently selected from H, deuterium, F, Cl, Br, I, OH, CN, $NH_2$, $NHCH_3$, $N(CH_3)_2$, or methyl, ethyl, methoxy or cyclopropyl optionally substituted with 1 to 4 $R^k$;

in some embodiments, $R^a$, $R^c$, and $R^{b3}$ are each independently selected from H, deuterium, F, Cl, Br, I, OH, CN, $NH_2$, $NHCH_3$, $N(CH_3)_2$, $CF_3$, $CHF_2$, $CH_2F$, methyl, ethyl, methoxy, or cyclopropyl;

in some embodiments, $R^{1a}$ and $R^{1b}$ or $R^{1c}$ and $R^{1d}$ respectively taken together with the carbon atom to which they are attached form $C_{3-12}$ carbocyclyl or 4- to 12-membered heterocyclyl, wherein the carbocyclyl or heterocyclyl is optionally substituted with 1 to 4 $R^k$;

in some embodiments, $R^{1a}$ and $R^{1b}$ or $R^{1c}$ and $R^{1d}$ respectively taken together with the carbon atom to which they are attached form $C_{3-11}$ cycloalkyl or 4- to 11-membered heterocycloalkyl, wherein the cycloalkyl or heterocycloalkyl is optionally substituted with 1 to 4 $R^k$;

in some embodiments, $R^{1a}$ and $R^{1b}$ or $R^{1c}$ and $R^{1d}$ respectively taken together with the carbon atom to which they are attached form $C_{3-7}$ cycloalkyl or 4- to 7-membered heterocycloalkyl, wherein the cycloalkyl or heterocycloalkyl is optionally substituted with 1 to 4 $R^k$;

in some embodiments, $R^{1a}$ and $R^{1b}$ or $R^{1c}$ and $R^{1d}$ respectively taken together with the carbon atom to which they are attached form the following groups optionally substituted with 1 to 4 $R^k$: cyclopropyl, cyclobutyl, cyclopentyl, cyclohexyl, oxetanyl, azetidinyl, pyrrolidyl, piperidyl, piperazinyl, morpholinyl, tetrahydrofuryl, or tetrahydropyranyl;

in some embodiments, $R^{b1}$, $R^{b2}$, and $R^{b4}$ are each independently selected from H, deuterium, OH, CN, $NH_2$, $C_{1-6}$ alkyl, $C_{2-6}$ alkenyl, $C_{2-6}$ alkynyl, $NHC_{1-6}$ alkyl, $N(C_{1-6}$ alkyl$)_2$, -NH-$C_{3-6}$ carbocyclyl, -NH-3- to 7-membered heterocyclyl, -$C_{0-4}$ alkylene-$C_{3-6}$ carbocyclyl, or -$C_{0-4}$ alkylene-3- to 7-membered heterocyclyl, wherein the alkyl, alkylene, alkenyl, alkynyl, carbocyclyl or heterocyclyl is optionally substituted with 1 to 4 $R^k$;

in some embodiments, $R^{b1}$, $R^{b2}$, and $R^{b4}$ are each independently selected from H, deuterium, OH, CN, $NH_2$, $C_{1-4}$ alkyl, $C_{2-4}$ alkenyl, $C_{2-4}$ alkynyl, $NHC_{1-4}$ alkyl, $N(C_{1-4}$ alkyl$)_2$, -NH-$C_{3-6}$ carbocyclyl, -NH-3- to 6-membered heterocyclyl, -$C_{0-2}$ alkylene-$C_{3-6}$ carbocyclyl, or -$C_{0-2}$ alkylene-3- to 6-membered heterocyclyl, wherein the alkyl, alkylene, alkenyl, alkynyl, carbocyclyl or heterocyclyl is optionally substituted with 1 to 4 $R^k$;

in some embodiments, $R^{b1}$, $R^{b2}$, and $R^{b4}$ are each independently selected from H, deuterium, OH, CN, $NH_2$, or the following groups optionally substituted with 1 to 4 $R^k$: methyl, ethyl, propyl, isopropyl, ethenyl, ethynyl, cyclopropyl, cyclobutyl, cyclopentyl, cyclohexyl, azetidinyl, oxetanyl, pyrrolidyl, piperidyl, morpholinyl, or phenyl;

in some embodiments, $R^{b1}$, $R^{b2}$, and $R^{b4}$ are each independently selected from H, deuterium, OH, CN, $NH_2$, or methyl, ethyl, methoxy or cyclopropyl optionally substituted with 1 to 4 $R^k$;

in some embodiments, $R^{b1}$, $R^{b2}$, and $R^{b4}$ are each independently selected from H, deuterium, OH, CN, $NH_2$, methyl, ethyl, methoxy, or cyclopropyl;

in some embodiments, each $R^k$ is independently selected from H, deuterium, halogen, OH, =O, CN, $NH_2$, COOH, $CONH_2$, $C_{1-6}$ alkyl, $OC_{1-6}$ alkyl, $SC_{1-6}$ alkyl, $C_{2-6}$ alkenyl, $C_{2-6}$ alkynyl, $NHC_{1-6}$ alkyl, $N(C_{1-6}$ alkyl$)_2$, -O-$C_{3-6}$ carbocyclyl, -O-3-to 7-membered heterocyclyl, -NH-$C_{3-6}$ carbocyclyl, -NH-3- to 7-membered heterocyclyl, -$C_{0-4}$ alkylene-$C_{3-6}$ carbocyclyl, or -$C_{0-4}$ alkylene-3- to 7-membered heterocyclyl, wherein the alkyl, alkylene, alkenyl, alkynyl, carbocyclyl or heterocyclyl is optionally substituted with 1 to 4 substituents selected from deuterium, halogen, =O, CN, OH, $NH_2$, $C_{1-6}$ alkyl, or $C_{1-6}$ alkoxy;

in some embodiments, each $R^k$ is independently selected from H, deuterium, halogen, OH, =O, CN, $NH_2$, COOH, $CONH_2$, $C_{1-4}$ alkyl, $OC_{1-4}$ alkyl, $SC_{1-4}$ alkyl, $C_{2-4}$ alkenyl, $C_{2-4}$ alkynyl, $NHC_{1-4}$ alkyl, $N(C_{1-4}$ alkyl$)_2$, -O-$C_{3-6}$ carbocyclyl, -O-3-to 6-membered heterocyclyl, -NH-$C_{3-6}$ carbocyclyl, -NH-3- to 6-membered heterocyclyl, -$C_{0-2}$ alkylene-$C_{3-6}$ carbocyclyl, or -$C_{0-2}$ alkylene-3- to 6-membered heterocyclyl, wherein the alkyl, alkylene, alkenyl, alkynyl, carbocyclyl or heterocyclyl is optionally substituted with 1 to 4 substituents selected from deuterium, halogen, =O, CN, OH, $NH_2$, $C_{1-4}$ alkyl, or $C_{1-4}$ alkoxy;

in some embodiments, each $R^k$ is independently selected from deuterium, F, Cl, Br, I, OH, =O, CN, $NH_2$, COOH, $CONH_2$, $NHCH_3$, $N(CH_3)_2$, methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl, wherein the methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl is optionally substituted with 1 to 4 substituents selected from deuterium, F, Cl, Br, I, =O, CN, OH, $NH_2$, $C_{1-4}$ alkyl, or $C_{1-4}$ alkoxy;

in some embodiments, each $R^k$ is independently selected from deuterium, F, Cl, Br, I, OH, =O, CN, $NH_2$, COOH, $CONH_2$, $NHCH_3$, $N(CH_3)_2$, methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl, wherein the methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl is optionally substituted with 1 to 4 substituents selected from deuterium, F, Cl, Br, I, =O, CN, OH, $NH_2$, methyl, ethyl, methoxy, or ethoxy;

in some embodiments, a, c, r, and s are each independently selected from 0, 1, 2, 3, or 4;

in some embodiments, a and c are each independently selected from 0, 1 or 2;

in some embodiments, c is selected from 0, 1 or 2.

A first embodiment of the present invention provides the above-described compound represented by general formula (I) or the stereoisomer, tautomer, deuterated compound, solvate, prodrug, metabolite, pharmaceutically acceptable salt or co-crystal thereof, wherein

R is selected from

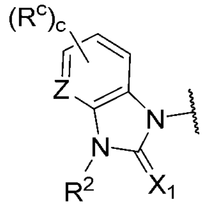 , 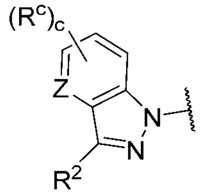 , 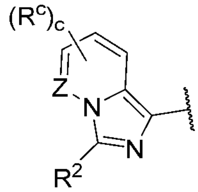 ,

or

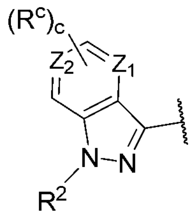

;

Z is selected from CH or N;

$Z_1$ or $Z_2$ is each independently selected from CH or N, and at least one of $Z_1$ and $Z_2$ is N;

$X_1$ or $X_2$ is each independently selected from O or S;

A is selected from $C_{3-12}$ carbocyclyl or 4- to 12-membered heterocyclyl;

$R^1$ is -M-$(CR^{1a}R^{1b})_r$-$(CR^{1c}R^{1d})_s$-COOH;

M is selected from a bond, $C_{3-12}$ carbocyclyl or 4- to 12-membered heterocyclyl, wherein the carbocyclyl or heterocyclyl is optionally substituted with 1 to 4 $R^m$;

$R^2$ is selected from $C_{1-6}$ alkyl or $C_{1-6}$ alkylene-Q, wherein the alkyl or alkylene is optionally substituted with 1 to 10 $R^k$;

Q is selected from $C_{3-12}$ carbocyclyl or 4- to 12-membered heterocyclyl, wherein the carbocyclyl or heterocyclyl is optionally substituted with 1 to 4 $R^q$;

$R^a$, $R^c$, $R^m$, $R^q$, $R^{b3}$, $R^{1a}$, $R^{1b}$, $R^{1c}$, and $R^{1d}$ are each independently selected from H, deuterium, halogen, OH, CN, $NH_2$, $C_{1-6}$ alkyl, $OC_{1-6}$ alkyl, $SC_{1-6}$ alkyl, $C_{2-6}$ alkenyl, $C_{2-6}$ alkynyl, $NHC_{1-6}$ alkyl, $N(C_{1-6}$ alkyl$)_2$, -O-$C_{3-6}$ carbocyclyl, -O-3- to 7-membered heterocyclyl, -NH-$C_{3-6}$ carbocyclyl, -NH-3- to 7-membered heterocyclyl, -$C_{0-4}$ alkylene-$C_{3-6}$ carbocyclyl, or -$C_{0-4}$ alkylene-3- to 7-membered heterocyclyl, wherein the alkyl, alkylene, alkenyl, alkynyl, carbocyclyl or heterocyclyl is optionally substituted with 1 to 4 $R^k$;

alternatively, $R^{1a}$ and $R^{1b}$ or $R^{1c}$ and $R^{1d}$ respectively taken together with the carbon atom to which they are attached form $C_{3-12}$ carbocyclyl or 4- to 12-membered heterocyclyl, wherein the carbocyclyl or heterocyclyl is optionally substituted with 1 to 4 $R^k$;

$R^{b1}$, $R^{b2}$, and $R^{b4}$ are each independently selected from H, deuterium, OH, CN, $NH_2$, $C_{1-6}$ alkyl, $C_{2-6}$ alkenyl, $C_{2-6}$ alkynyl, $NHC_{1-6}$ alkyl, $N(C_{1-6}$ alkyl$)_2$, -NH-$C_{3-6}$ carbocyclyl, -NH-3- to 7-membered heterocyclyl, -$C_{0-4}$ alkylene-$C_{3-6}$ carbocyclyl, or -$C_{0-4}$ alkylene-3- to 7-membered heterocyclyl, wherein the alkyl, alkylene, alkenyl, alkynyl, carbocyclyl or heterocyclyl is optionally substituted with 1 to 4 $R^k$;

each $R^k$ is independently selected from H, deuterium, halogen, OH, =O, CN, $NH_2$, COOH, $CONH_2$, $C_{1-6}$ alkyl, $OC_{1-6}$ alkyl, $SC_{1-6}$ alkyl, $C_{2-6}$ alkenyl, $C_{2-6}$ alkynyl, $NHC_{1-6}$ alkyl, $N(C_{1-6}$ alkyl$)_2$, -O-$C_{3-6}$ carbocyclyl, -O-3- to 7-membered heterocyclyl, -NH-$C_{3-6}$ carbocyclyl, -NH-3- to 7-membered heterocyclyl, -$C_{0-4}$ alkylene-$C_{3-6}$ carbocyclyl, or -$C_{0-4}$ alkylene-3- to 7-membered heterocyclyl, wherein the alkyl, alkylene, alkenyl, alkynyl, carbocyclyl or heterocyclyl is optionally substituted with 1 to 4 substituents selected from deuterium, halogen, =O, CN, OH, $NH_2$, $C_{1-6}$ alkyl, or $C_{1-6}$ alkoxy;

a, c, r, and s are each independently selected from 0, 1, 2, 3, or 4.

A second embodiment of the present invention provides the above-described compound represented by general formula (I) or the stereoisomer, tautomer, deuterated compound, solvate, prodrug, metabolite, pharmaceutically acceptable salt or co-crystal thereof, wherein

A is selected from $C_{3-11}$ cycloalkyl, 4- to 11-membered heterocycloalkyl, $C_{6-10}$ aryl or 5- to 10-membered heteroaryl;

M is selected from a bond, $C_{3-11}$ cycloalkyl, 4- to 11-membered heterocycloalkyl, $C_{6-10}$ aryl or 5- to 10-membered heteroaryl, wherein the cycloalkyl, heterocycloalkyl, aryl, or heteroaryl is optionally substituted with 1 to 4 $R^m$;

$R^2$ is selected from $C_{1-5}$ alkyl or $C_{1-4}$ alkylene-Q, wherein the alkyl or alkylene is optionally substituted with 1 to 8 $R^k$;

Q is selected from $C_{3-11}$ cycloalkyl, 4- to 11-membered heterocycloalkyl, $C_{6-10}$ aryl or 5- to 10-membered heteroaryl, wherein the cycloalkyl, heterocycloalkyl, aryl, or heteroaryl is optionally substituted with 1 to 4 $R^q$;

$R^a$, $R^c$, $R^m$, $R^q$, $R^{b3}$, $R^{1a}$, $R^{1b}$, $R^{1c}$, and $R^{1d}$ are each independently selected from H, deuterium, halogen, OH, CN, $NH_2$, $C_{1-4}$ alkyl, $OC_{1-4}$ alkyl, $SC_{1-4}$ alkyl, $C_{2-4}$ alkenyl, $C_{2-4}$ alkynyl, $NHC_{1-4}$ alkyl, $N(C_{1-4}$ alkyl$)_2$, -O-$C_{3-6}$ carbocyclyl, -O-3- to 6-membered heterocyclyl, -NH-$C_{3-6}$ carbocyclyl, -NH-3- to 6-membered heterocyclyl, -$C_{0-2}$ alkylene-$C_{3-6}$ carbocyclyl, or -$C_{0-2}$ alkylene-3- to 6-membered heterocyclyl, wherein the alkyl, alkylene, alkenyl, alkynyl, carbocyclyl or heterocyclyl is optionally substituted with 1 to 4 $R^k$;

alternatively, $R^{1a}$ and $R^{1b}$ or $R^{1c}$ and $R^{1d}$ respectively taken together with the carbon atom to which they are attached form $C_{3-11}$ cycloalkyl or 4- to 11-membered heterocycloalkyl, wherein the cycloalkyl or heterocycloalkyl is optionally substituted with 1 to 4 $R^k$;

$R^{b1}$, $R^{b2}$, and $R^{b4}$ are each independently selected from H, deuterium, OH, CN, $NH_2$, $C_{1-4}$ alkyl, $C_{2-4}$ alkenyl, $C_{2-4}$ alkynyl, $NHC_{1-4}$ alkyl, $N(C_{1-4}$ alkyl$)_2$, -NH-$C_{3-6}$ carbocyclyl, -NH-3- to 6-membered heterocyclyl, -$C_{0-2}$ alkylene-$C_{3-6}$

carbocyclyl, or -$C_{0-2}$ alkylene-3- to 6-membered heterocyclyl, wherein the alkyl, alkylene, alkenyl, alkynyl, carbocyclyl or heterocyclyl is optionally substituted with 1 to 4 $R^k$;

each $R^k$ is independently selected from H, deuterium, halogen, OH, =O, CN, $NH_2$, COOH, $CONH_2$, $C_{1-4}$ alkyl, $OC_{1-4}$ alkyl, $SC_{1-4}$ alkyl, $C_{2-4}$ alkenyl, $C_{2-4}$ alkynyl, $NHC_{1-4}$ alkyl, $N(C_{1-4}$ alkyl$)_2$, -O-$C_{3-6}$ carbocyclyl, -O-3- to 6-membered heterocyclyl, -NH-$C_{3-6}$ carbocyclyl, -NH-3- to 6-membered heterocyclyl, -$C_{0-2}$ alkylene-$C_{3-6}$ carbocyclyl, or -$C_{0-2}$ alkylene-3- to 6-membered heterocyclyl, wherein the alkyl, alkylene, alkenyl, alkynyl, carbocyclyl or heterocyclyl is optionally substituted with 1 to 4 substituents selected from deuterium, halogen, =O, CN, OH, $NH_2$, $C_{1-4}$ alkyl, or $C_{1-4}$ alkoxy;

the remaining definitions are the same as those in the first embodiment of the present invention.

A third embodiment of the present invention provides the above-described compound represented by general formula (I) or the stereoisomer, tautomer, deuterated compound, solvate, prodrug, metabolite, pharmaceutically acceptable salt or co-crystal thereof, wherein

A is selected from $C_{3-6}$ cycloalkyl, 4- to 6-membered heterocycloalkyl, $C_{6-10}$ aryl or 5- to 10-membered heteroaryl;

M is selected from a bond, $C_{3-6}$ cycloalkyl, 4- to 6-membered heterocycloalkyl, $C_{6-10}$ aryl or 5- to 10-membered heteroaryl, wherein the cycloalkyl, heterocycloalkyl, aryl, or heteroaryl is optionally substituted with 1 to 4 $R^m$;

$R^2$ is selected from $C_{1-4}$ alkyl or $C_{1-3}$ alkylene-Q, wherein the alkyl or alkylene is optionally substituted with 1 to 6 $R^k$;

Q is selected from $C_{3-6}$ cycloalkyl, 4- to 6-membered heterocycloalkyl, $C_{6-10}$ aryl or 5- to 10-membered heteroaryl, wherein the cycloalkyl, heterocycloalkyl, aryl, or heteroaryl is optionally substituted with 1 to 4 $R^q$;

$R^a$, $R^c$, $R^m$, $R^q$, $R^{b3}$ , $R^{1a}$, $R^{1b}$, $R^{1c}$, and $R^{1d}$ are each independently selected from H, deuterium, F, Cl, Br, I, OH, CN, $NH_2$, $NHCH_3$, $N(CH_3)_2$, or the following groups optionally substituted with 1 to 4 $R^k$: methyl, ethyl, propyl, isopropyl, methoxy, ethoxy, isopropoxy, ethenyl, ethynyl, methylthio, cyclopropyl, cyclobutyl, cyclopentyl, cyclohexyl, azetidinyl, oxetanyl, pyrrolidyl, piperidyl, morpholinyl, or phenyl;

alternatively, $R^{1a}$ and $R^{1b}$ or $R^{1c}$ and $R^{1d}$ respectively taken together with the carbon atom to which they are attached form $C_{3-7}$ cycloalkyl or 4- to 7-membered heterocycloalkyl, wherein the cycloalkyl or heterocycloalkyl is optionally substituted with 1 to 4 $R^k$;

$R^{b1}$, $R^{b2}$, and $R^{b4}$ are each independently selected from H, deuterium, OH, CN, $NH_2$, or the following groups optionally substituted with 1 to 4 $R^k$: methyl, ethyl, propyl, isopropyl, ethenyl, ethynyl, cyclopropyl, cyclobutyl, cyclopentyl, cyclohexyl, azetidinyl, oxetanyl, pyrrolidyl, piperidyl, morpholinyl, or phenyl;

each $R^k$ is independently selected from deuterium, F, Cl, Br, I, OH, =O, CN, $NH_2$, COOH, $CONH_2$, $NHCH_3$, $N(CH_3)_2$, methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl, wherein the methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl is optionally substituted with 1 to 4 substituents selected from deuterium, F, Cl, Br, I, =O, CN, OH, $NH_2$, $C_{1-4}$ alkyl, or $C_{1-4}$ alkoxy;

the remaining definitions are the same as those in the first or second embodiment of the present invention.

A fourth embodiment of the present invention provides the above-described compound represented by general formula (I) or the stereoisomer, tautomer, deuterated compound, solvate, prodrug, metabolite, pharmaceutically acceptable salt or co-crystal thereof, wherein

A is selected from the following groups optionally substituted with 1 to 4 $R^a$: cyclopropyl, cyclobutyl, cyclopentyl, cyclohexyl, oxetanyl, azetidinyl, pyrrolidyl, piperidyl, piperazinyl, morpholinyl, tetrahydrofuryl, tetrahydropyranyl, phenyl, pyrrolyl, thienyl, furyl, pyrazolyl, imidazolyl, thiazolyl, isothiazolyl, oxazolyl, isoxazolyl, pyridyl, pyrimidyl, pyrazinyl, or pyridazinyl;

M is selected from a bond, or the following groups optionally substituted with 1 to 4 $R^m$: cyclopropyl, cyclobutyl, cyclopentyl, cyclohexyl, azetidinyl, pyrrolidyl, piperidyl, piperazinyl, phenyl, pyrrolyl, thienyl, furyl, pyrazolyl, imidazolyl, thiazolyl, isothiazolyl, oxazolyl, isoxazolyl, pyridyl, pyrimidyl, pyrazinyl, or pyridazinyl;

$R^2$ is selected from methyl, ethyl, propyl, butyl, -methylene-Q, -ethylene-Q, or -propylene-Q, wherein the methyl, ethyl, propyl, butyl, methylene, ethylene, or propylene is optionally substituted with 1 to 6 $R^k$;

Q is selected from the following groups optionally substituted with 1 to 4 $R^q$: cyclopropyl, cyclobutyl, cyclopentyl, cyclohexyl, oxetanyl, azetidinyl, pyrrolidyl, piperidyl, piperazinyl, morpholinyl, tetrahydrofuryl, tetrahydropyranyl, phenyl, pyrrolyl, thienyl, furyl, pyrazolyl, imidazolyl, thiazolyl, isothiazolyl, oxazolyl, isoxazolyl, pyridyl, pyrimidyl, pyrazinyl, or pyridazinyl;

$R^a$, $R^c$, $R^m$, $R^q$, $R^{b3}$, $R^{1a}$, $R^{1b}$, $R^{1c}$, and $R^{1d}$ are each independently selected from H, deuterium, F, Cl, Br, I, OH, CN, $NH_2$, $NHCH_3$, $N(CH_3)_2$, or methyl, ethyl, methoxy or cyclopropyl optionally substituted with 1 to 4 $R^k$;

alternatively, $R^{1a}$ and $R^{1b}$ or $R^{1c}$ and $R^{1d}$ respectively taken together with the carbon atom to which they are attached

form the following groups optionally substituted with 1 to 4 $R^k$: cyclopropyl, cyclobutyl, cyclopentyl, cyclohexyl, oxetanyl, azetidinyl, pyrrolidyl, piperidyl, piperazinyl, morpholinyl, tetrahydrofuryl, or tetrahydropyranyl;

$R^{b1}$, $R^{b2}$, and $R^{b4}$ are each independently selected from H, deuterium, OH, CN, $NH_2$, or methyl, ethyl, methoxy or cyclopropyl optionally substituted with 1 to 4 $R^k$;

each $R^k$ is independently selected from deuterium, F, Cl, Br, I, OH, =O, CN, $NH_2$, COOH, $CONH_2$, $NHCH_3$, $N(CH_3)_2$, methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl, wherein the methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl is optionally substituted with 1 to 4 substituents selected from deuterium, F, Cl, Br, I, =O, CN, OH, $NH_2$, methyl, ethyl, methoxy, or ethoxy;

the remaining definitions are the same as those in the first, second, or third embodiment of the present invention.

A fifth embodiment of the present invention provides the above-described compound represented by general formula (I) or the stereoisomer, tautomer, deuterated compound, solvate, prodrug, metabolite, pharmaceutically acceptable salt or co-crystal thereof, wherein

A is selected from the following groups optionally substituted with 1 to 4 $R^a$: phenyl, thiazolyl, oxazolyl, pyridyl, pyrimidyl, pyrazinyl, or pyridazinyl;

$R^1$ is selected from

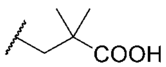, 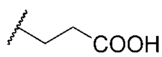, 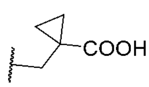, 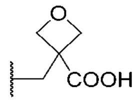,

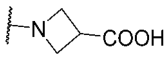, 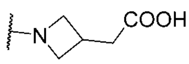, or 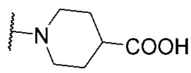;

R is selected from

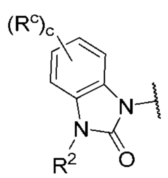, 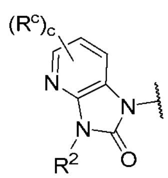, 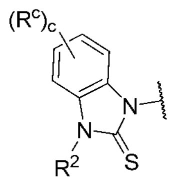, 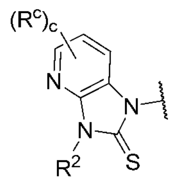,

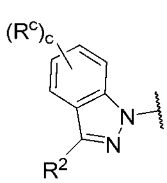, 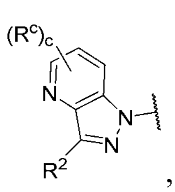, 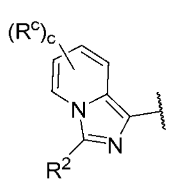, 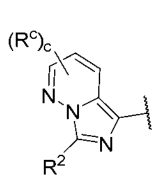, 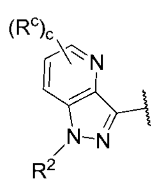, or 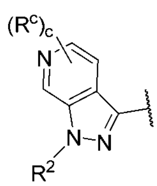;

$R^2$ is selected from

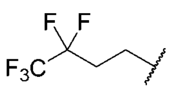, 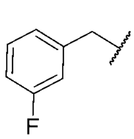, 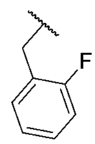, 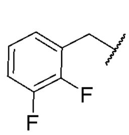, 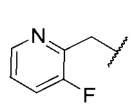,

or

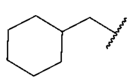;

$R^a$, $R^c$, and $R^{b3}$ are each independently selected from H, deuterium, F, Cl, Br, I, OH, CN, $NH_2$, $NHCH_3$, $N(CH_3)_2$, $CF_3$, $CHF_2$, $CH_2F$, methyl, ethyl, methoxy, or cyclopropyl;
$R^{b1}$, $R^{b2}$, and $R^{b4}$ are each independently selected from H, deuterium, OH, CN, $NH_2$, methyl, ethyl, methoxy, or cyclopropyl;
c is selected from 0, 1 or 2;
the remaining definitions are the same as those in the first, second, third, or fourth embodiment of the present invention.

A sixth embodiment of the present invention provides the above-described compound represented by general formula (I) or the stereoisomer, tautomer, deuterated compound, solvate, prodrug, metabolite, pharmaceutically acceptable salt or co-crystal thereof, wherein the compound represented by general formula (I) is a compound represented by general formula (II), wherein

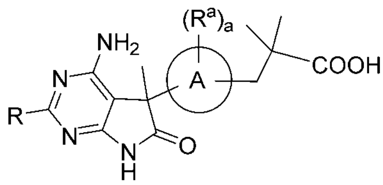
(II);

A is selected from the following groups: phenyl, thiazolyl, oxazolyl, pyridyl, pyrimidyl, pyrazinyl, or pyridazinyl;
R is selected from

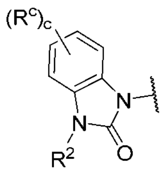 , 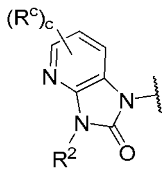 , 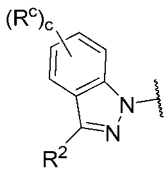 , 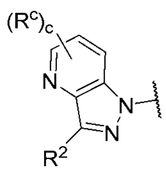 ,

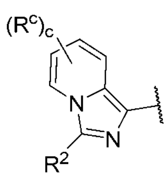 , 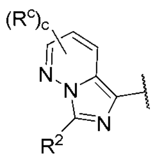 , 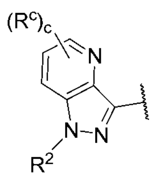 , or 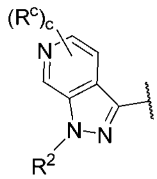 ;

$R^2$ is selected from

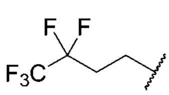 , 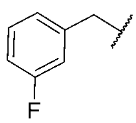 , 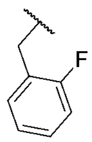 , 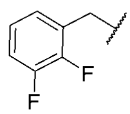 , 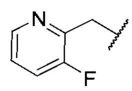 ,

or

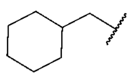 ;

$R^a$ and $R^c$ are each independently selected from H, deuterium, F, Cl, Br, I, OH, CN, $NH_2$, $NHCH_3$, $N(CH_3)_2$, $CF_3$, $CHF_2$, $CH_2F$, methyl, ethyl, methoxy, or cyclopropyl;
a and c are each independently selected from 0, 1 or 2.

A seventh embodiment of the present invention provides the above-described compound represented by general formula (I) or (II), or the stereoisomer, tautomer, deuterated compound, solvate, prodrug, metabolite, pharmaceutically acceptable salt or co-crystal thereof, wherein the compound represented by general formula (I) or (II) is a

compound represented by general formula (III-1) or (III-2), wherein

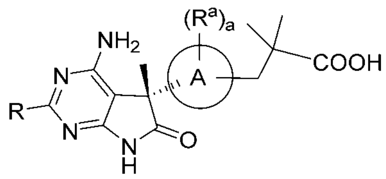

(III-1),

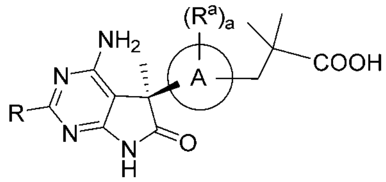

(III-2);

the remaining definitions are the same as the corresponding definitions in any one of the first to sixth embodiments of the present invention.

The present invention relates to a compound as described below or a stereoisomer, tautomer, deuterated compound, solvate, prodrug, metabolite, pharmaceutically acceptable salt or co-crystal thereof, wherein the compound has a structure selected from one of those in Table E-1 below:

Table E-1

| | | |
|---|---|---|
| 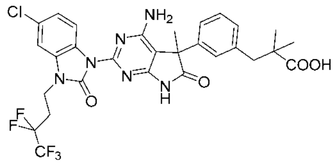 | 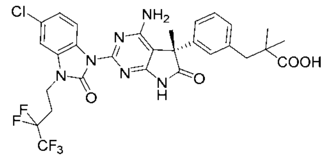 | 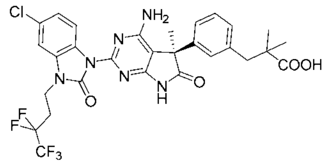 |
| 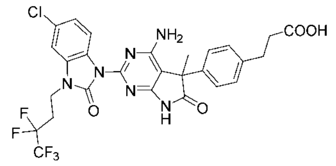 | 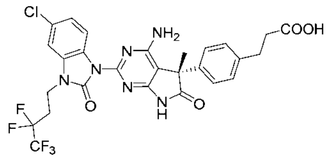 | 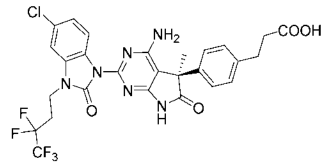 |
| 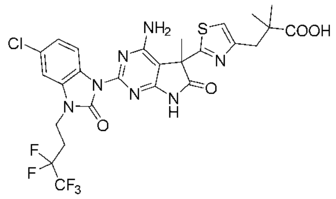 | 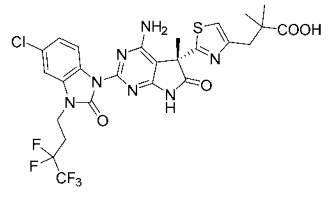 | 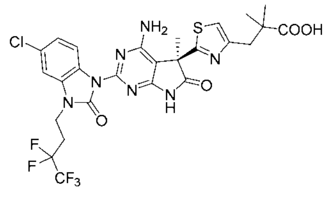 |
| 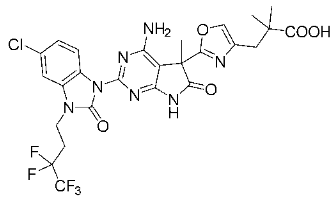 | 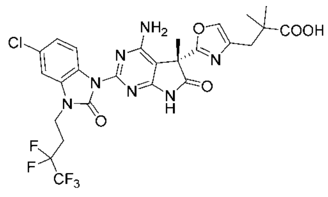 | 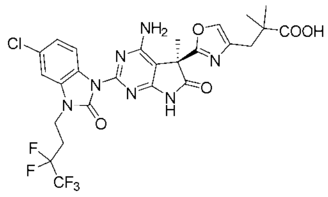 |
| 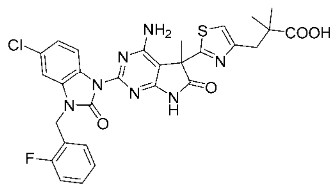 | 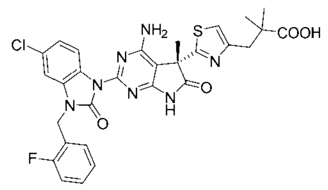 | 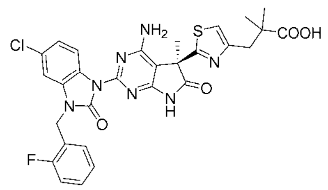 |

| | | |
|---|---|---|
| 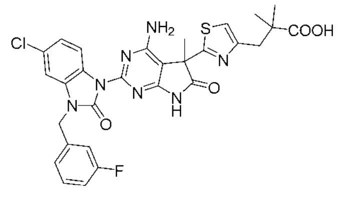 | 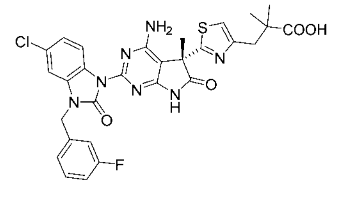 | 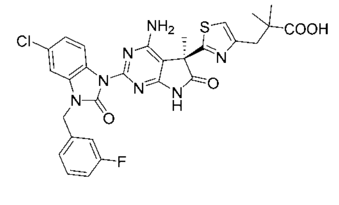 |
| 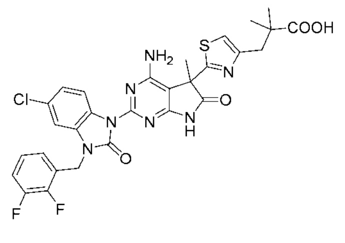 | 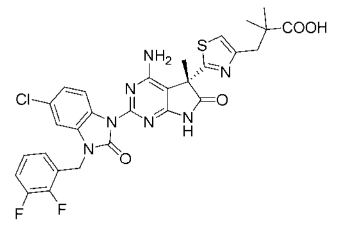 | 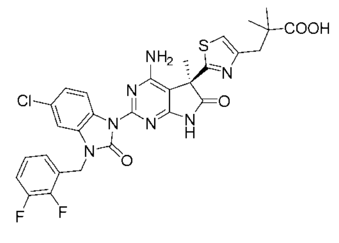 |
| 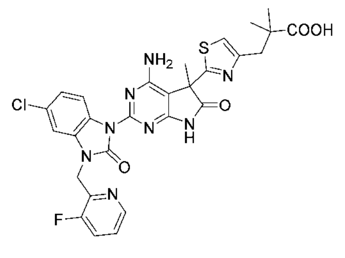 | 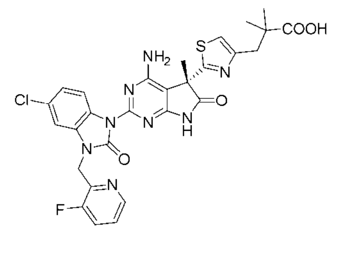 | 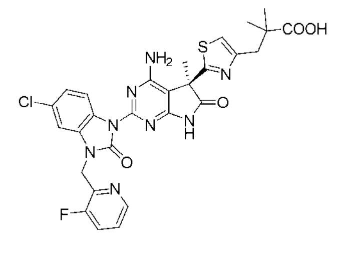 |
| 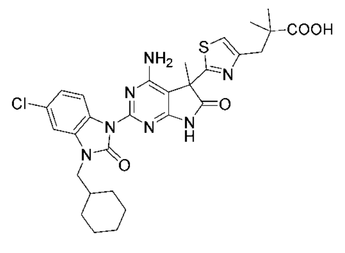 | 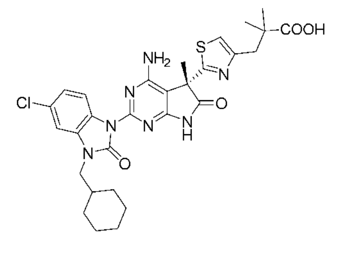 | 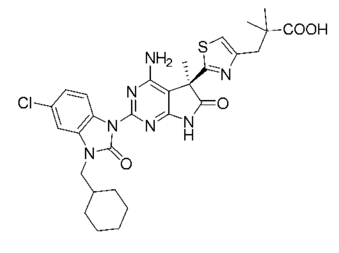 |
| 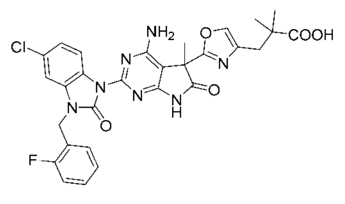 | 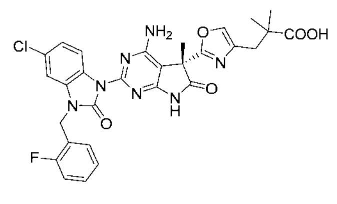 | 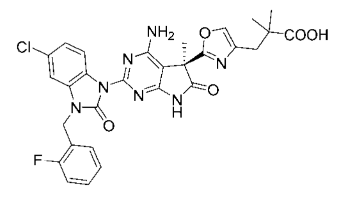 |
| 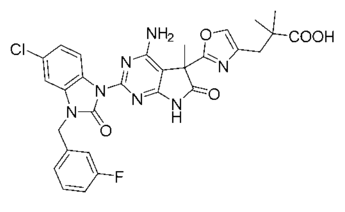 | 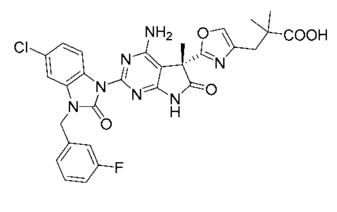 | 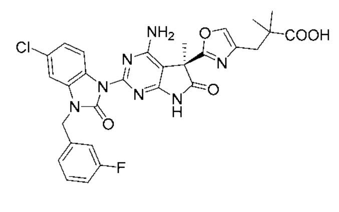 |
| 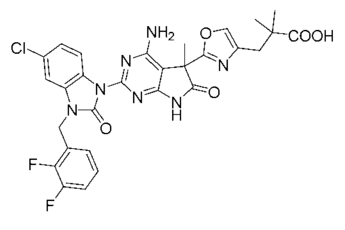 | 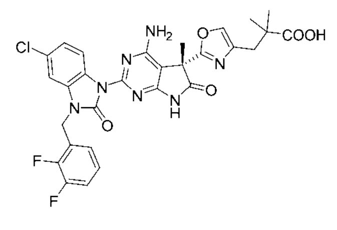 | 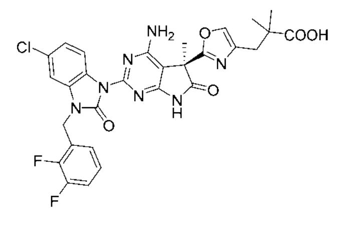 |

| | | |
|---|---|---|
| 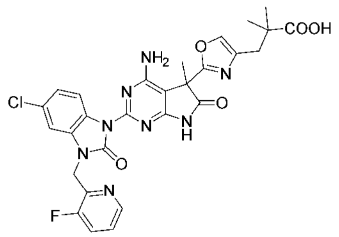 | 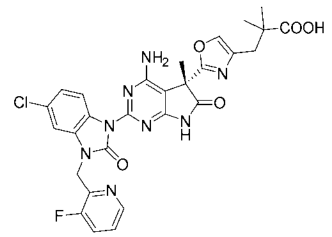 | 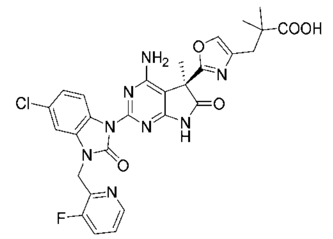 |
| 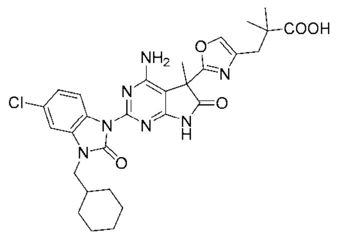 | 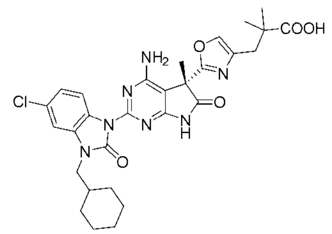 | 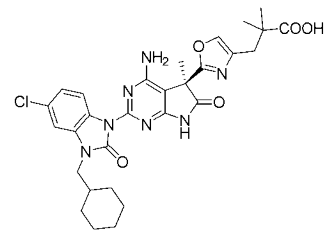 |
| 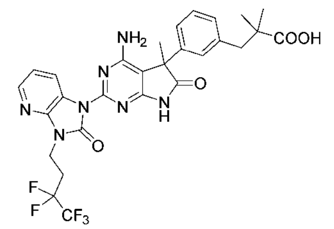 | 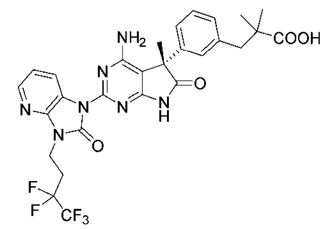 | 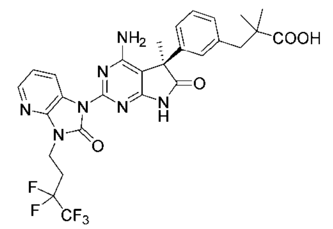 |
| 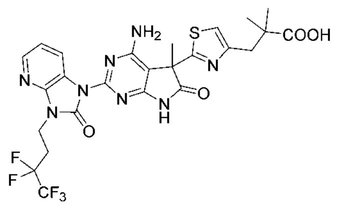 | 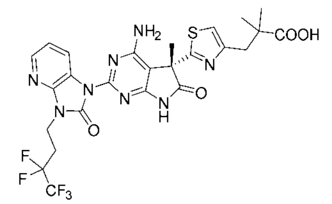 | 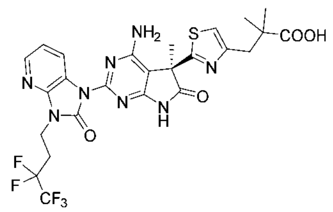 |
| 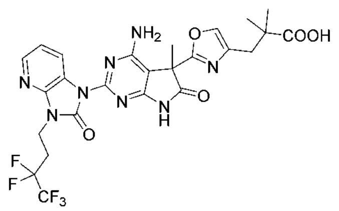 | 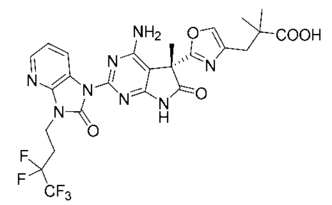 | 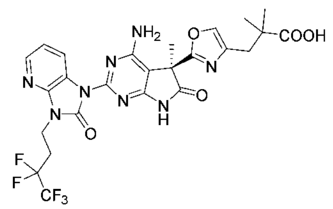 |
| 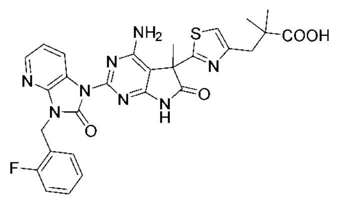 | 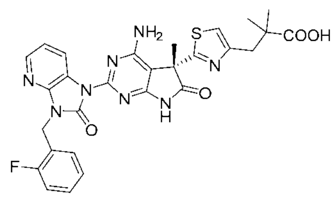 | 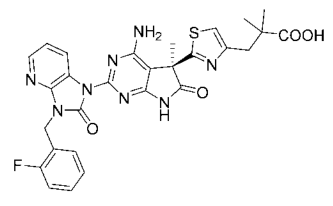 |
| 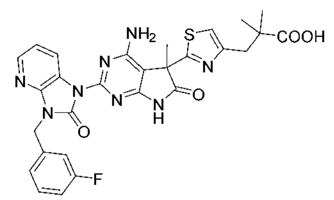 | 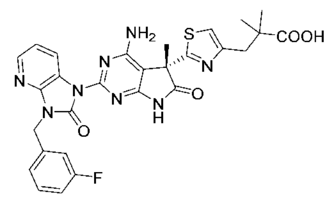 | 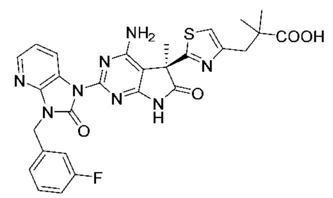 |

| | | |
|---|---|---|
| 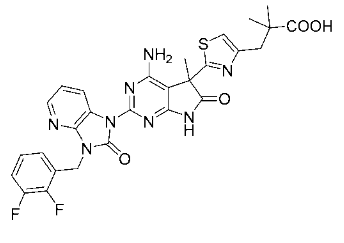 | 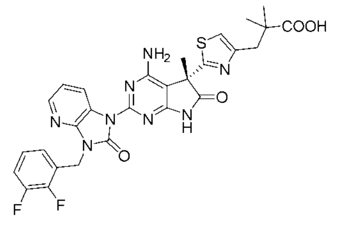 | 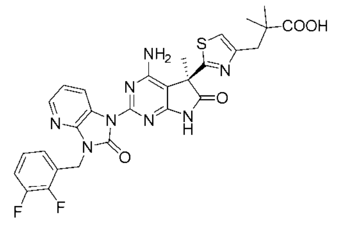 |
| 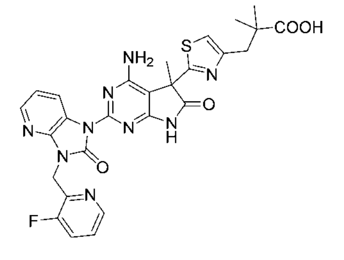 | 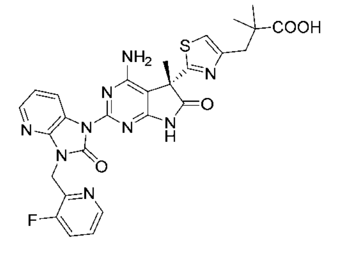 | 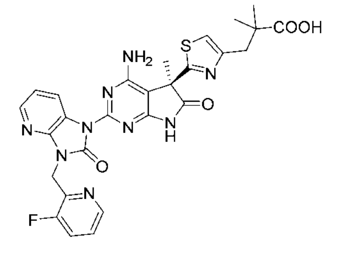 |
| 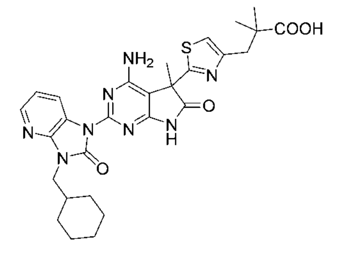 | 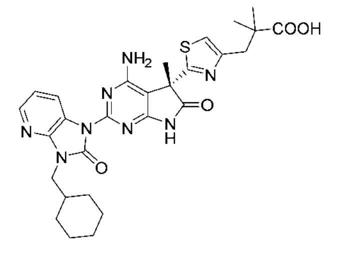 | 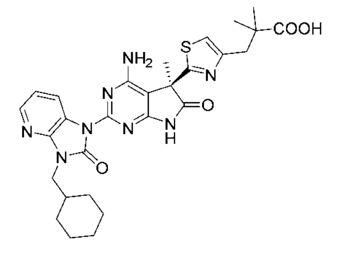 |
| 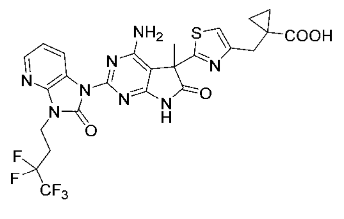 | 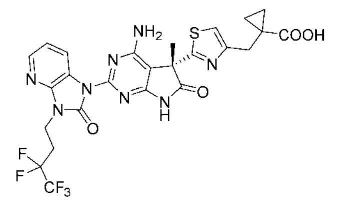 | 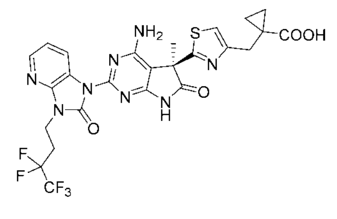 |
| 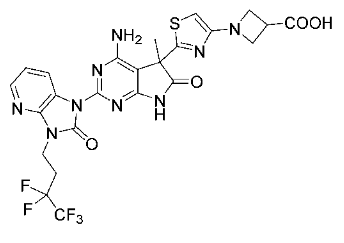 | 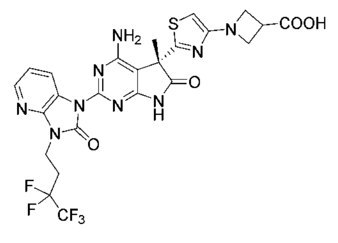 | 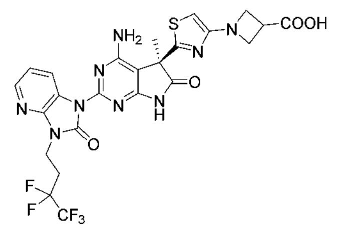 |
| 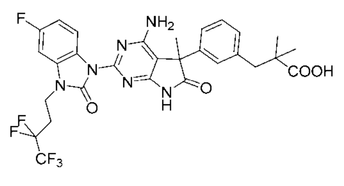 | 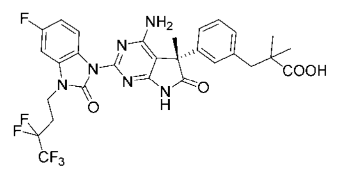 | 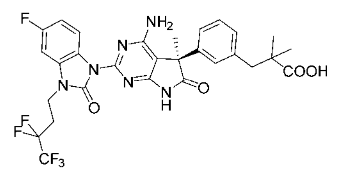 |
| 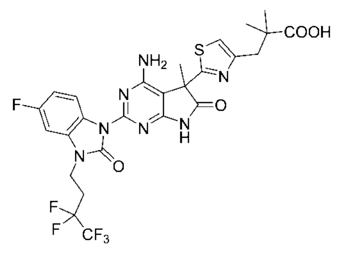 | 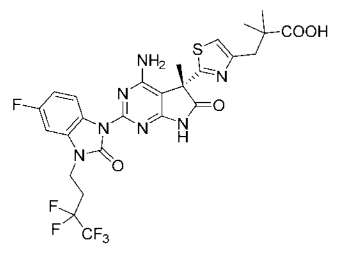 | 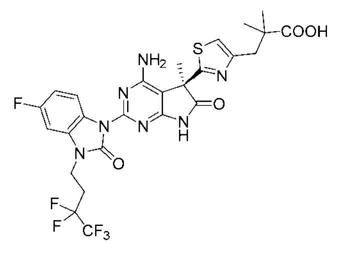 |

| | | |
|---|---|---|
| 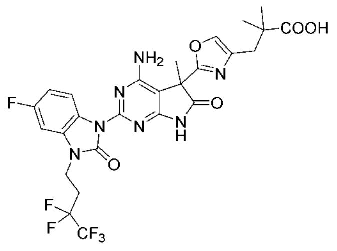 | 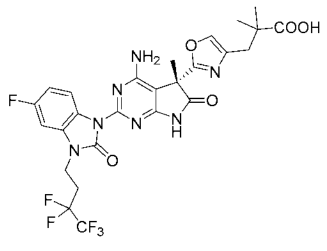 | 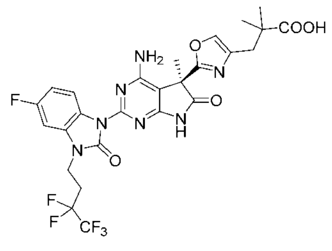 |
| 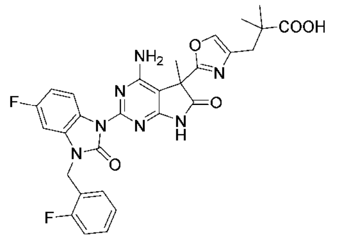 | 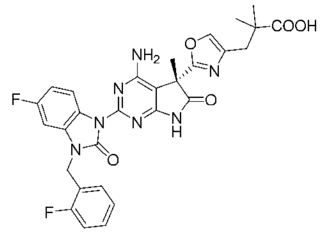 | 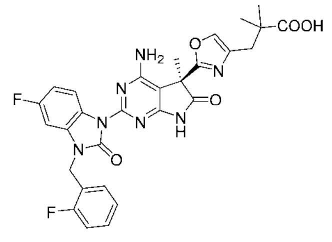 |
| 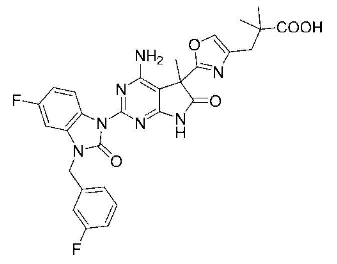 | 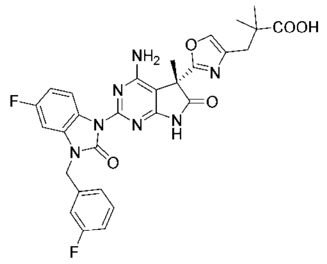 | 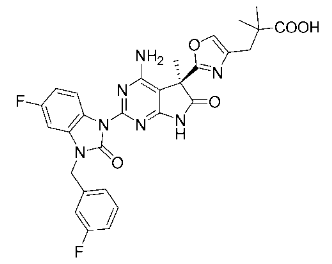 |
| 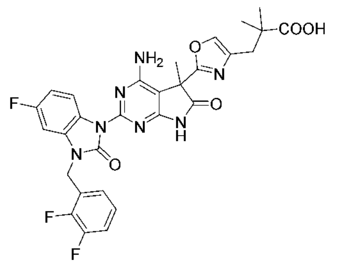 | 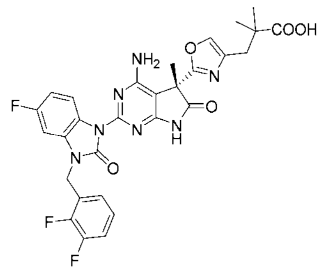 | 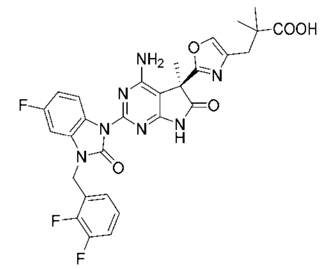 |
| 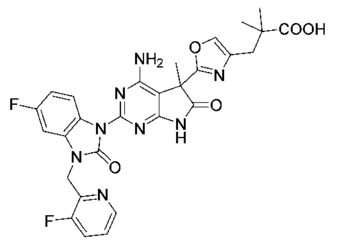 | 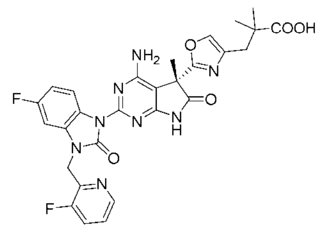 | 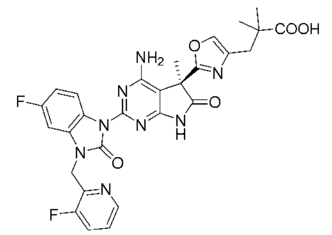 |
| 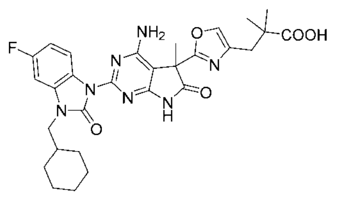 | 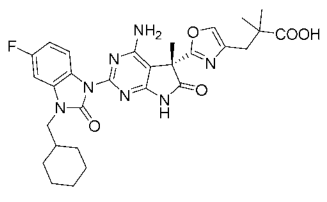 | 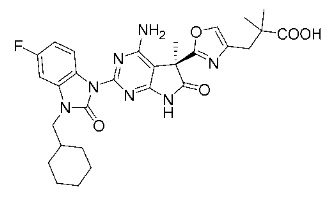 |

| | | |
|---|---|---|
| 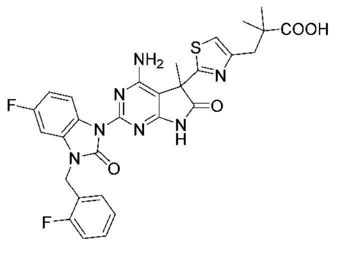 | 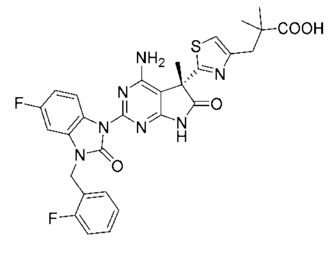 | 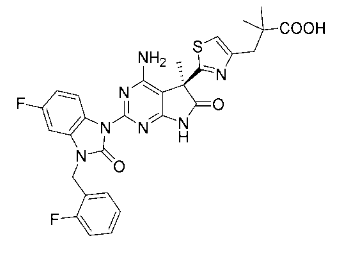 |
| 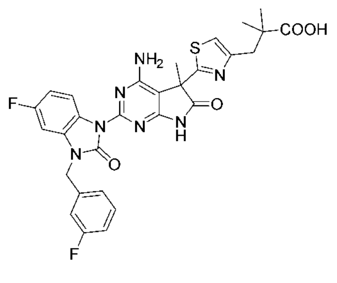 | 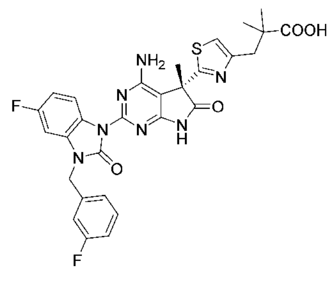 | 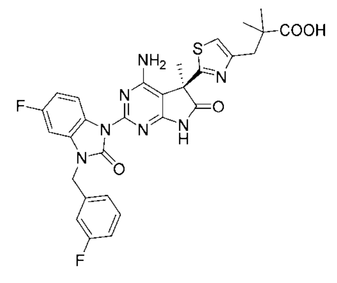 |
| 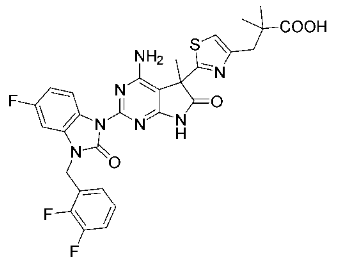 | 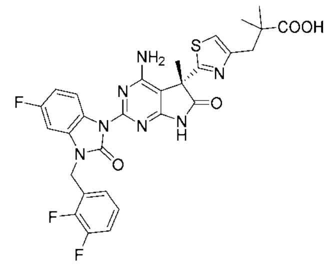 | 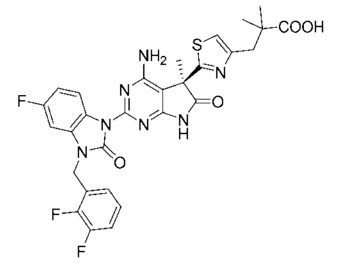 |
| 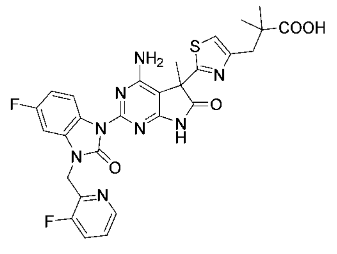 | 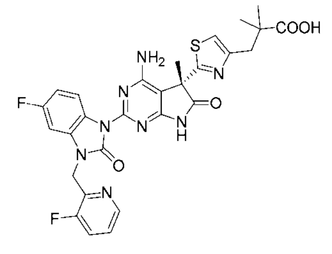 | 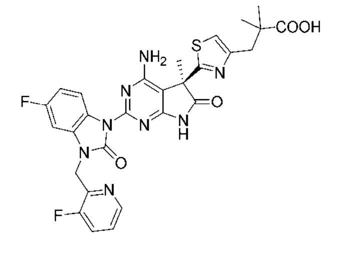 |
| 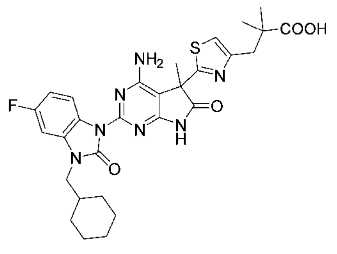 | 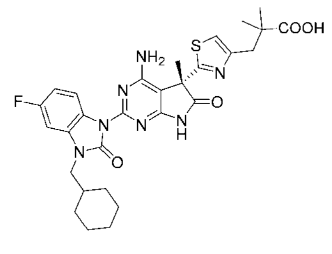 | 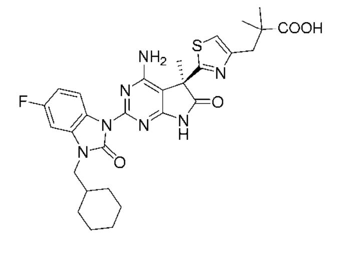 |
| 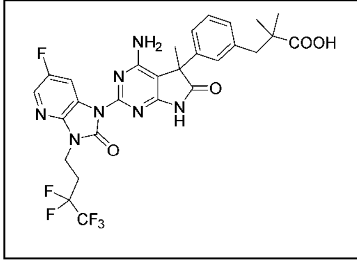 | 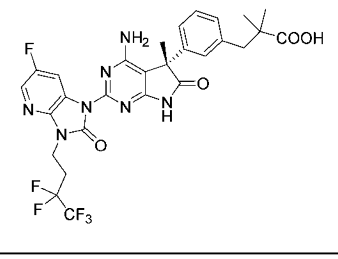 | 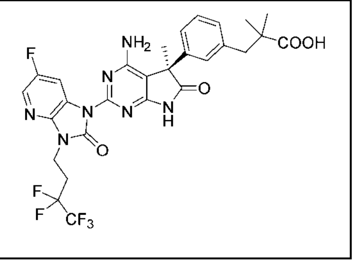 |

| | | |
|---|---|---|
| 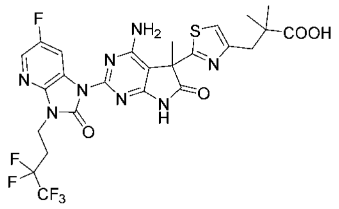 | 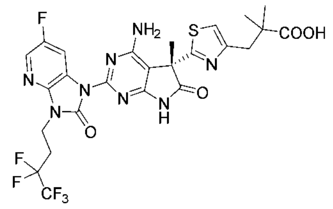 | 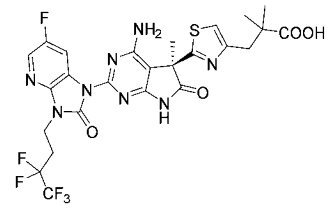 |
| 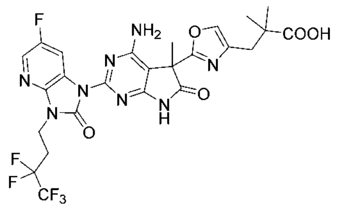 | 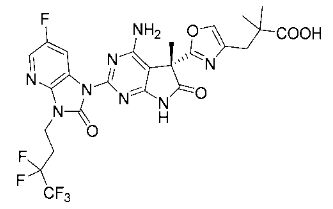 | 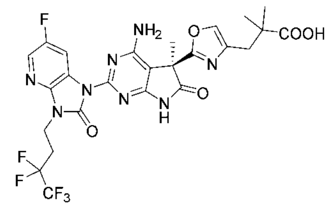 |
| 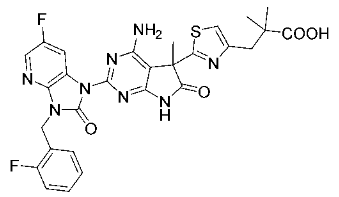 | 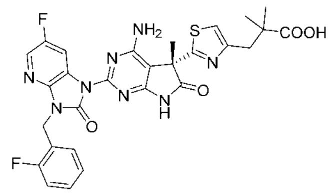 | 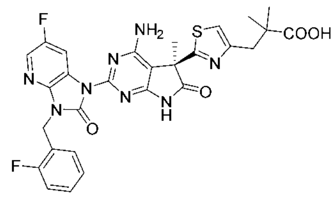 |
| 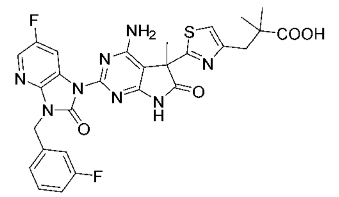 | 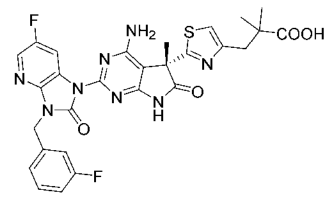 | 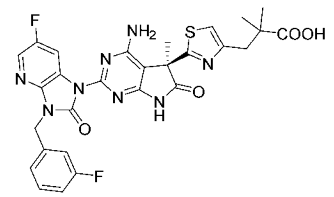 |
| 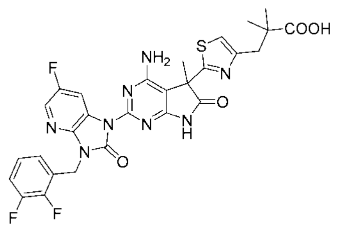 | 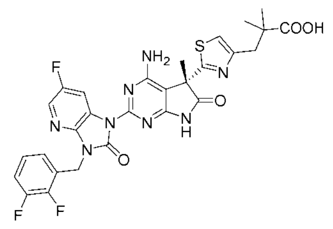 | 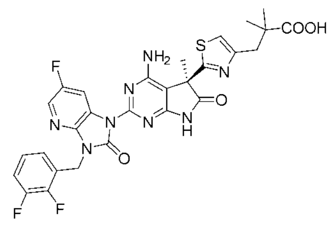 |
| 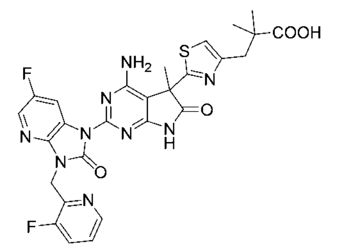 | 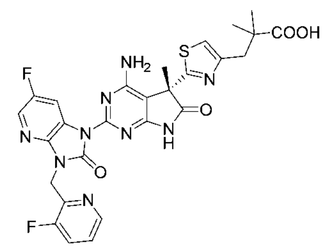 | 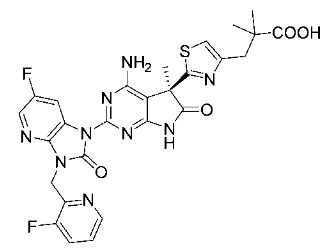 |
| 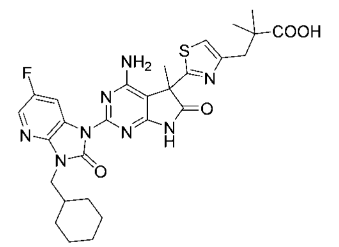 | 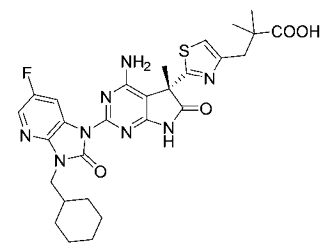 | 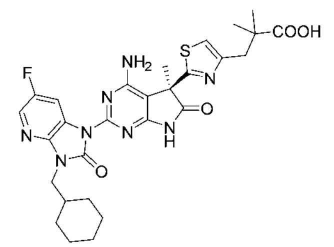 |

| | | |
|---|---|---|
| 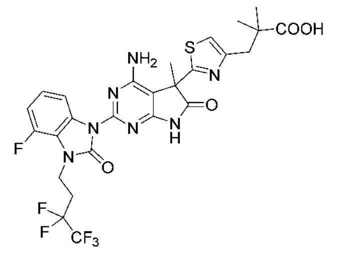 | 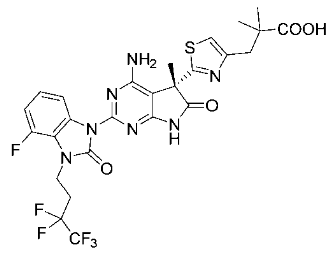 | 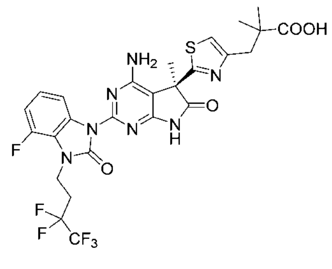 |
| 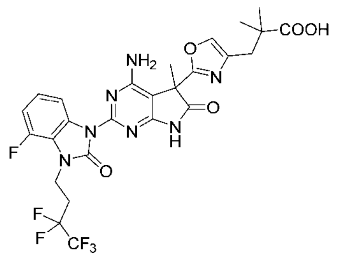 | 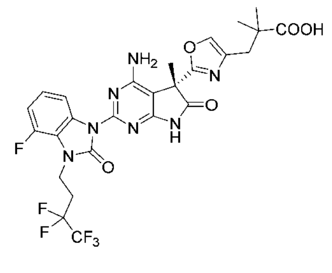 | 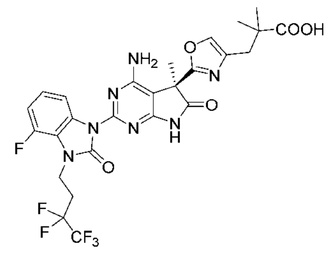 |
| 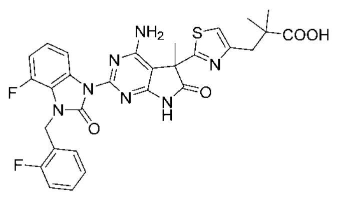 | 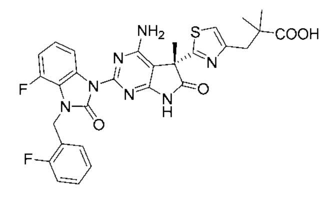 | 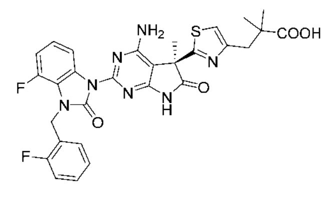 |
| 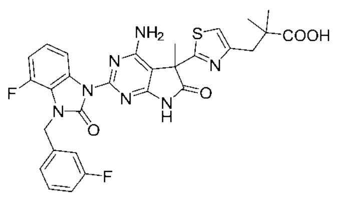 | 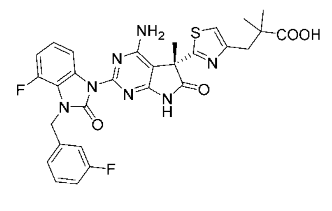 | 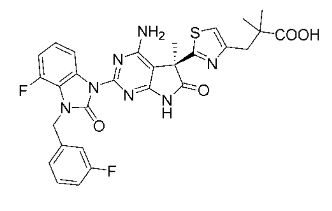 |
| 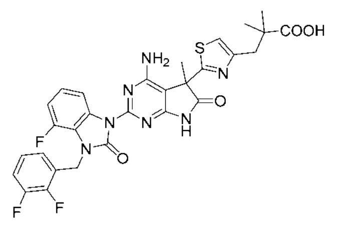 | 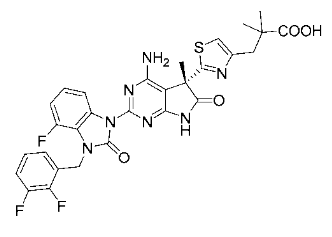 | 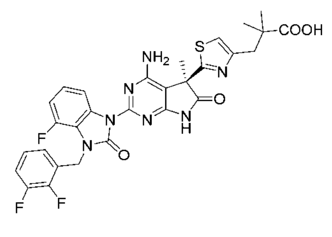 |
| 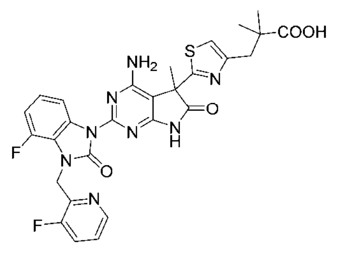 | 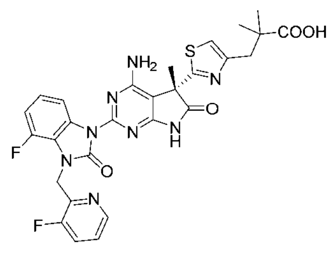 | 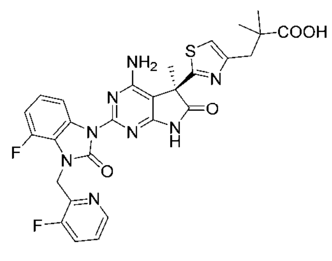 |
| 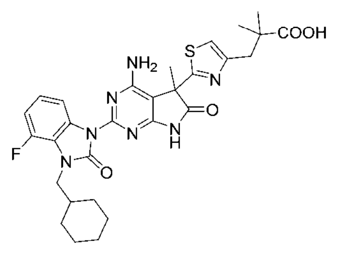 | 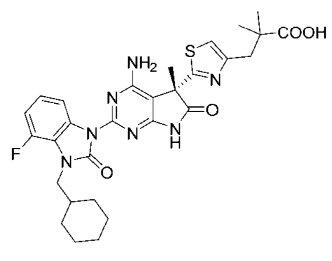 | 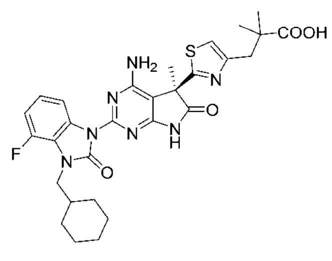 |

| | | |
|---|---|---|
| 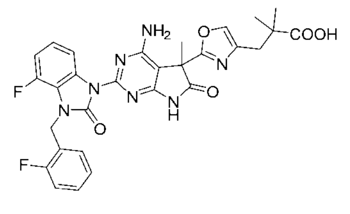 | 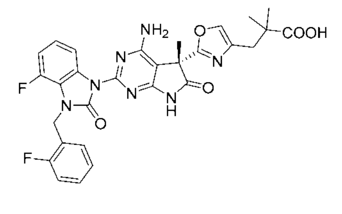 | 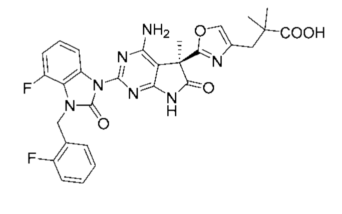 |
| 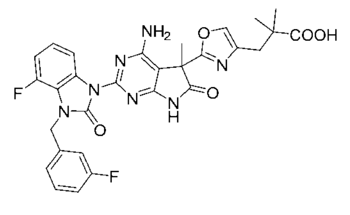 | 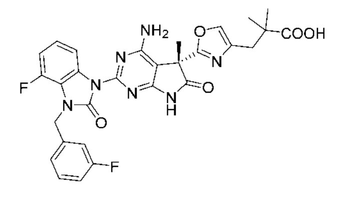 | 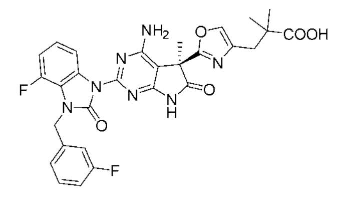 |
| 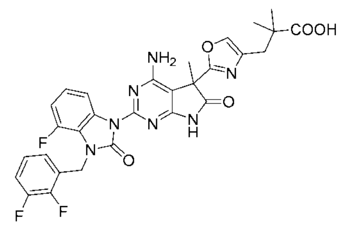 | 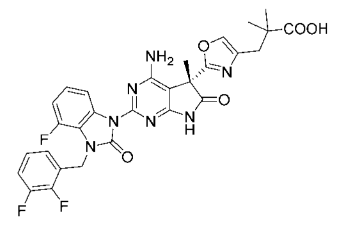 | 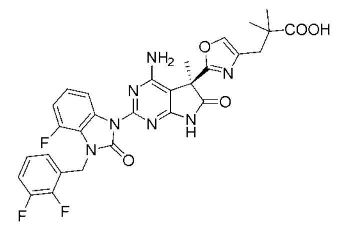 |
| 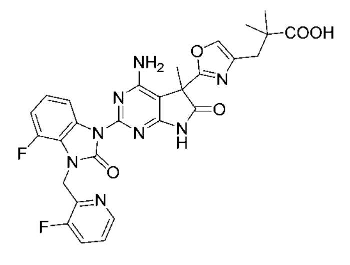 | 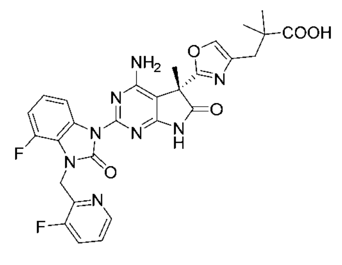 | 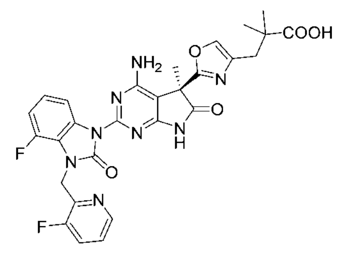 |
| 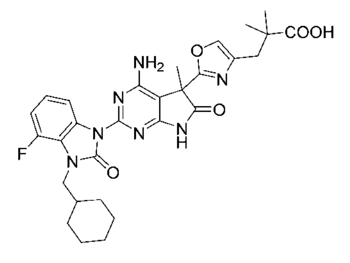 | 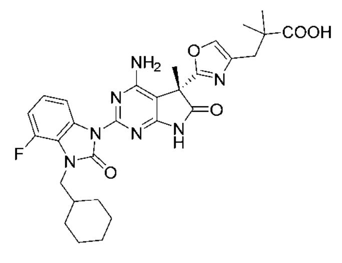 | 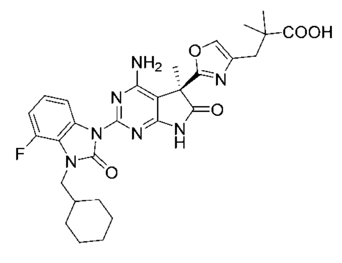 |
| 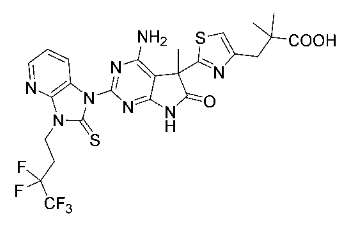 | 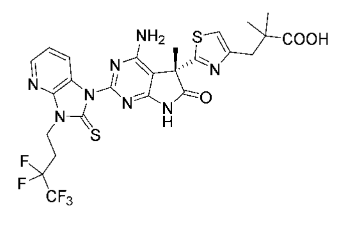 | 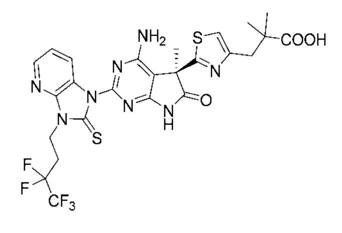 |
| 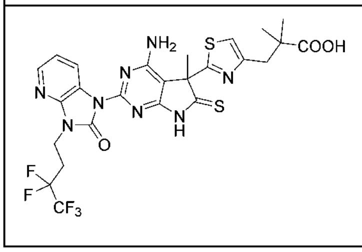 | 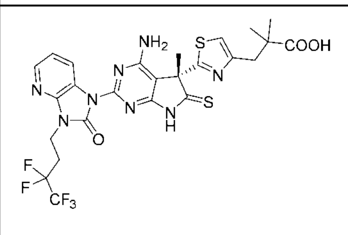 | 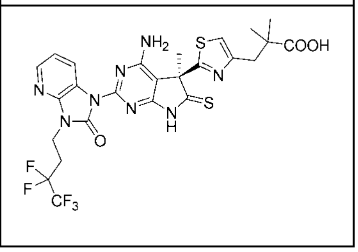 |

| | | |
|---|---|---|
| 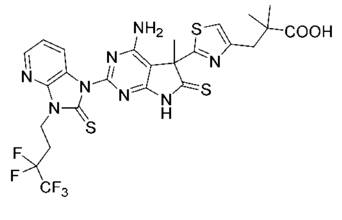 | 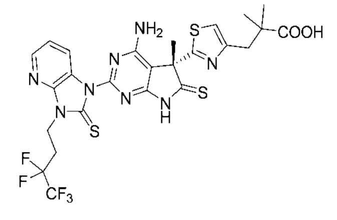 | 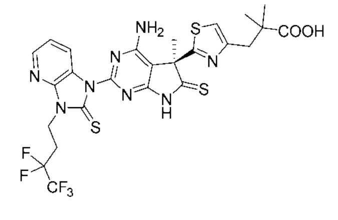 |
| 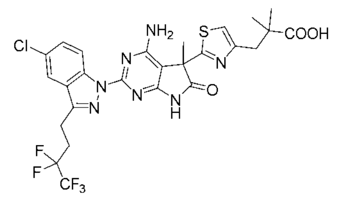 | 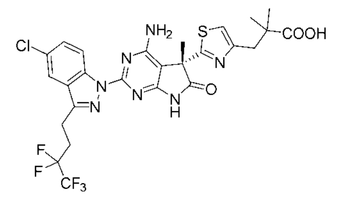 | 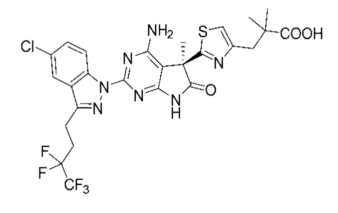 |
| 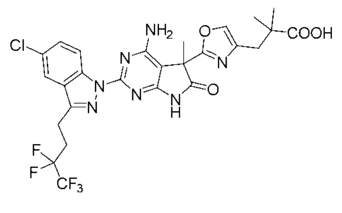 | 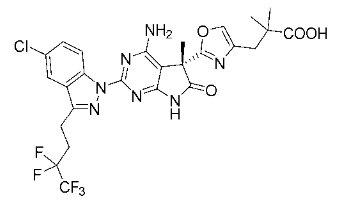 | 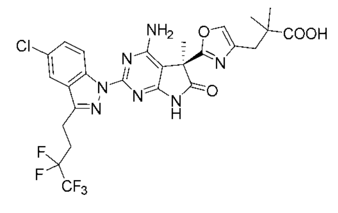 |
| 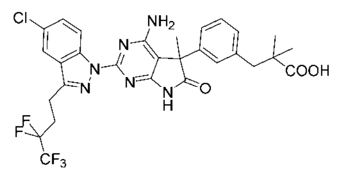 | 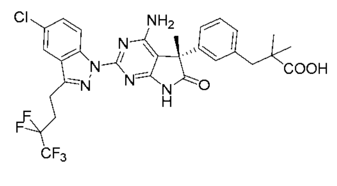 | 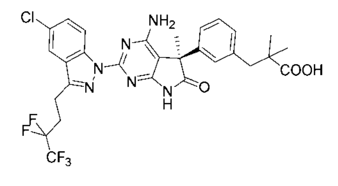 |
| 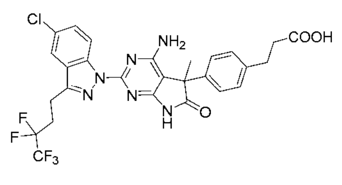 | 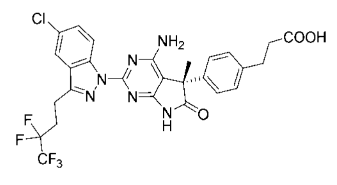 | 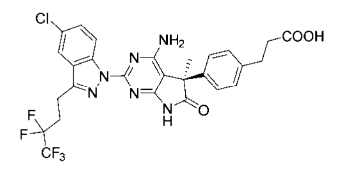 |
| 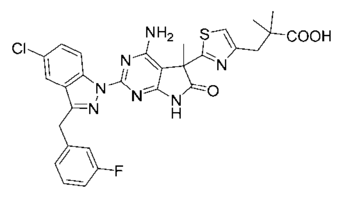 | 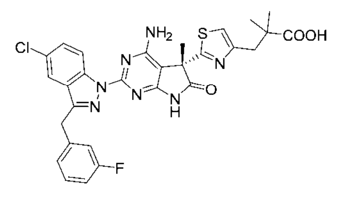 | 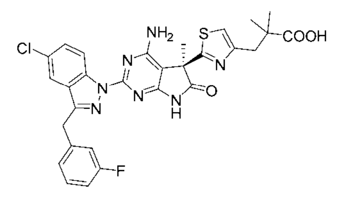 |
| 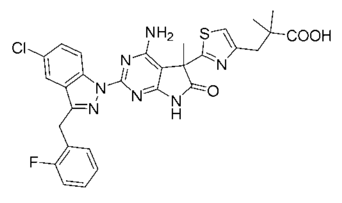 | 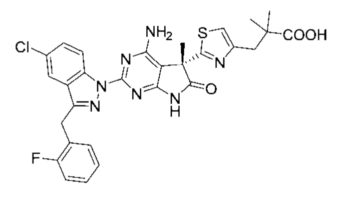 | 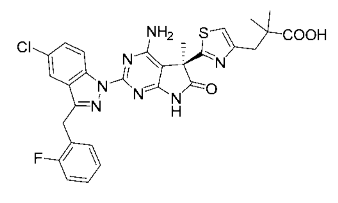 |
| 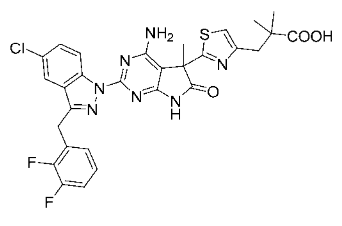 | 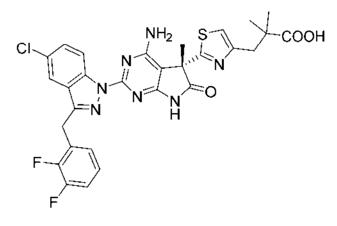 | 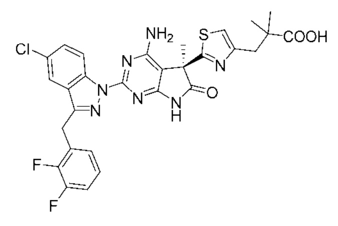 |

| | | |
|---|---|---|
| 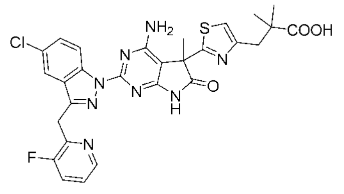 | 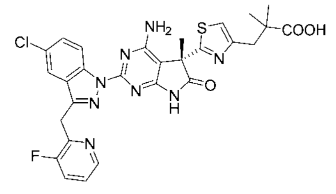 | 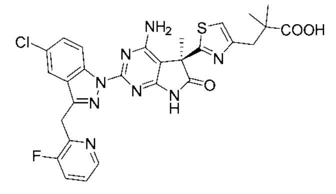 |
| 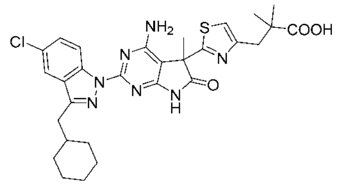 | 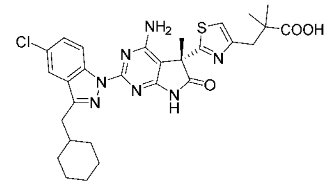 | 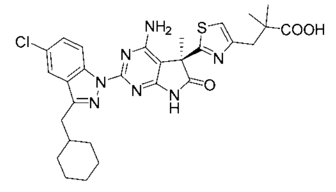 |
| 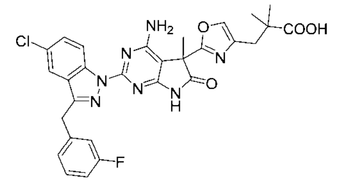 | 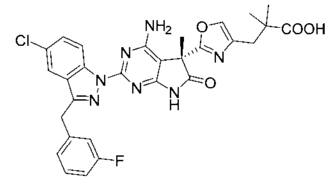 | 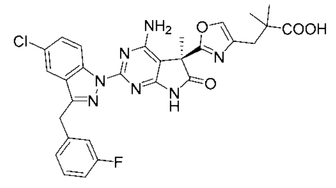 |
| 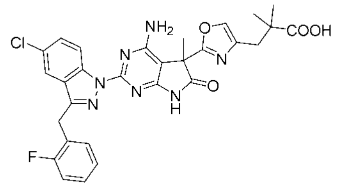 | 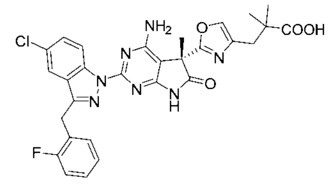 | 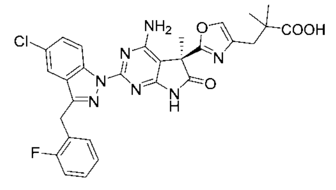 |
| 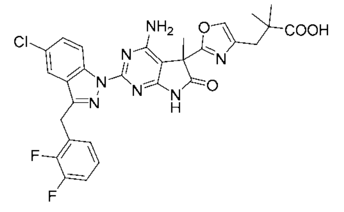 | 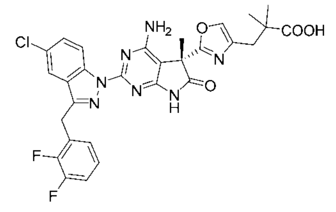 | 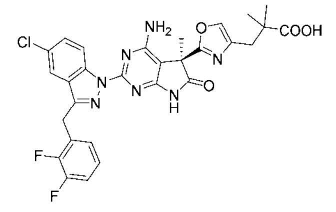 |
| 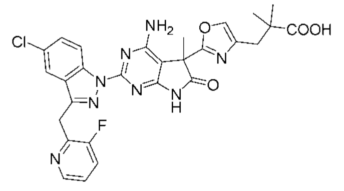 | 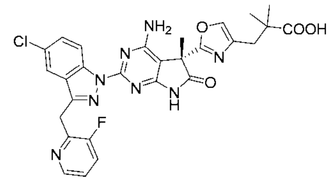 | 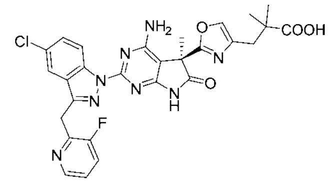 |
| 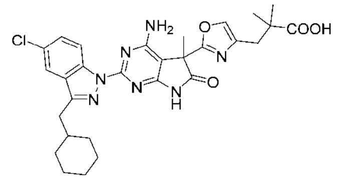 | 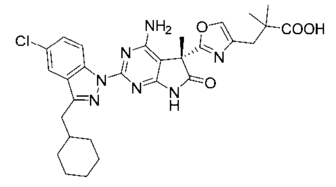 | 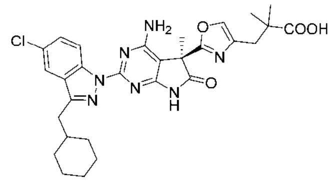 |
| 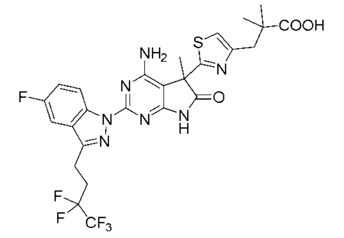 | 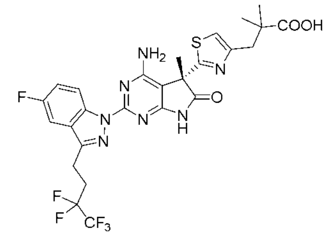 | 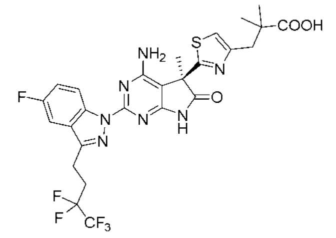 |

| | | |
|---|---|---|
| 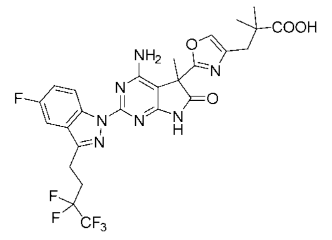 | 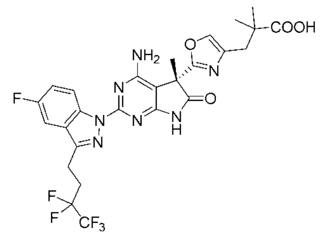 | 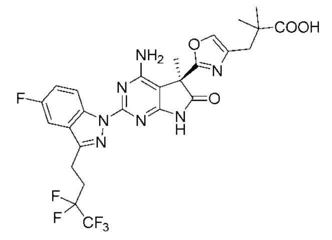 |
| 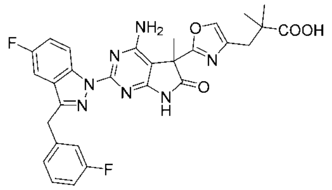 | 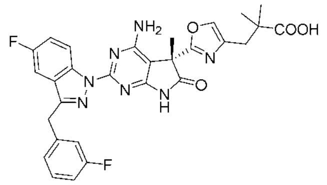 | 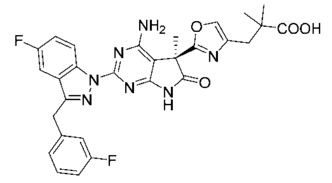 |
| 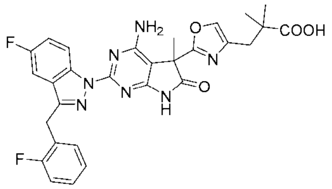 | 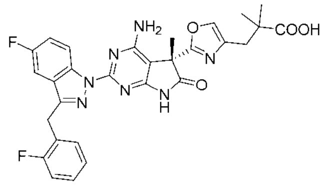 | 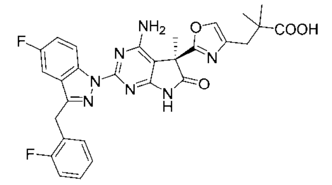 |
| 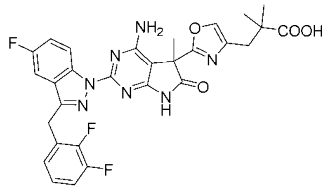 | 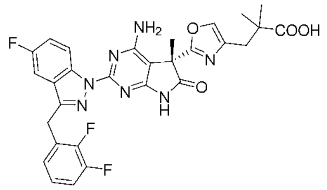 | 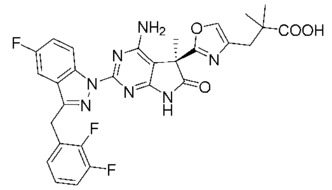 |
| 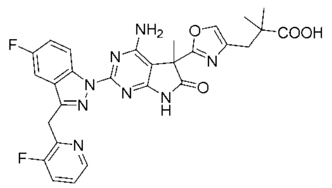 | 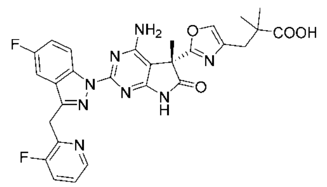 | 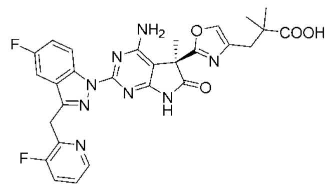 |
| 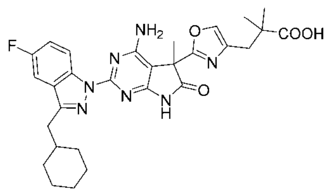 | 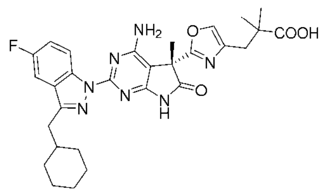 | 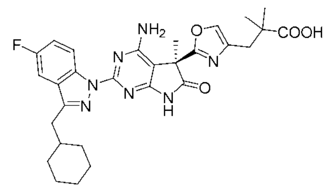 |
| 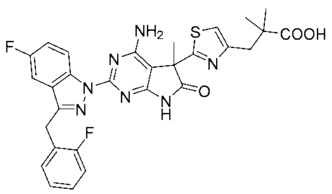 | 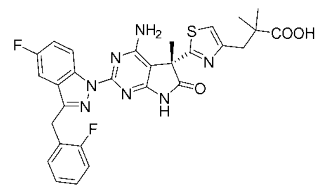 | 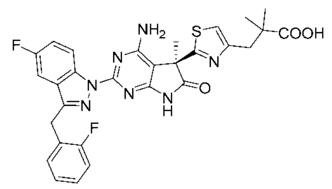 |
| 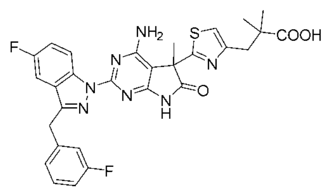 | 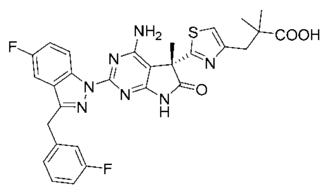 | 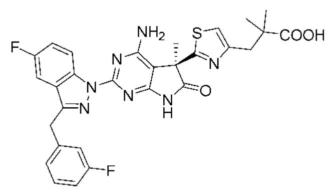 |

| | | |
|---|---|---|
| 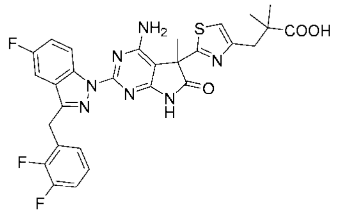 | 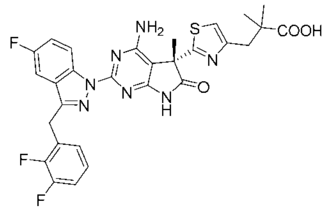 | 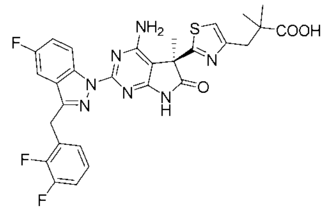 |
| 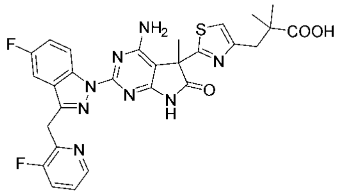 | 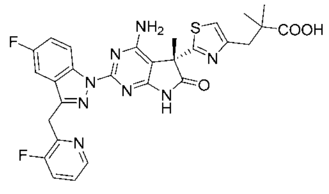 | 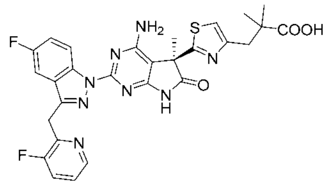 |
| 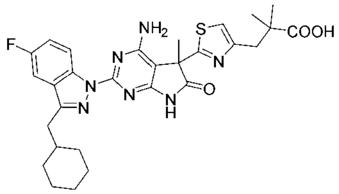 | 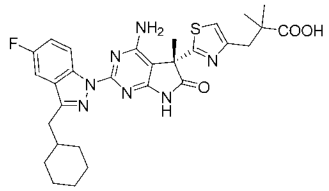 | 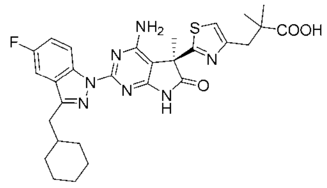 |
| 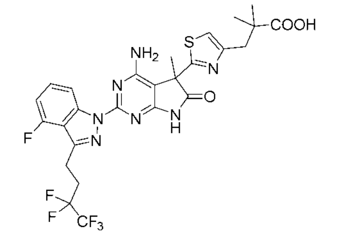 | 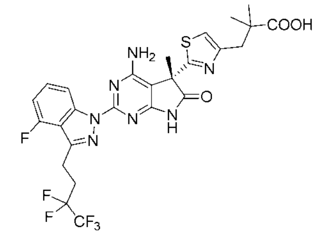 | 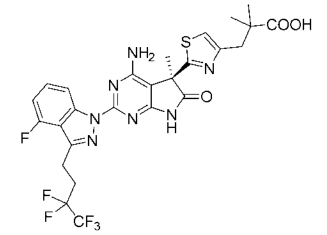 |
| 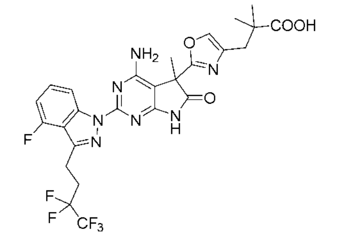 | 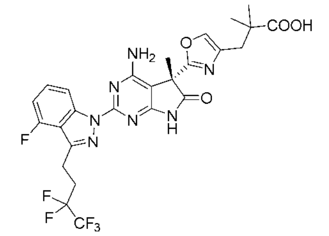 | 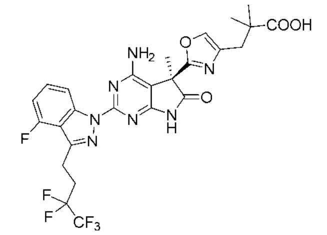 |
| 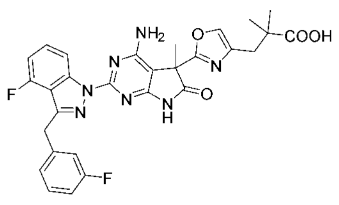 | 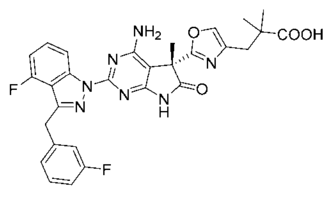 | 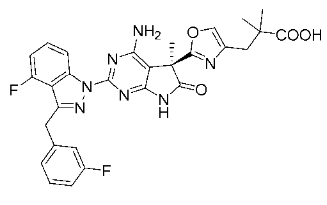 |
| 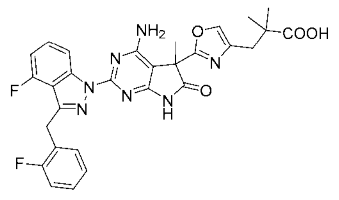 | 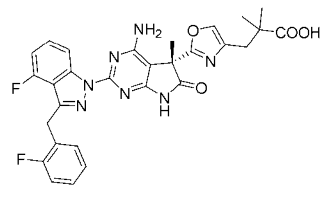 | 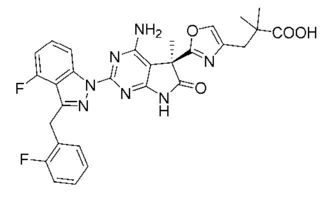 |

| | | |
|---|---|---|
| 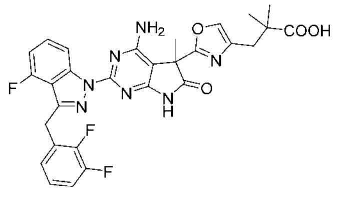 | 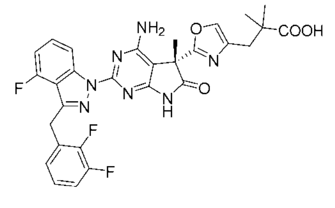 | 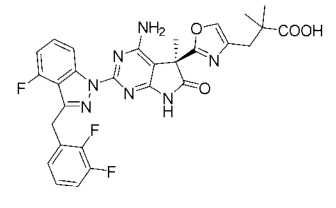 |
| 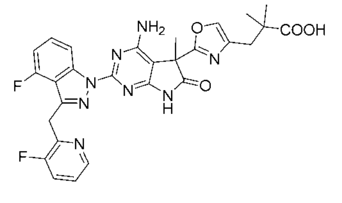 | 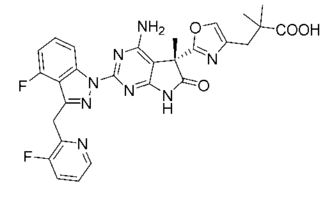 | 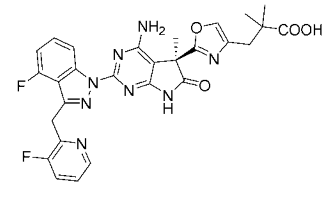 |
| 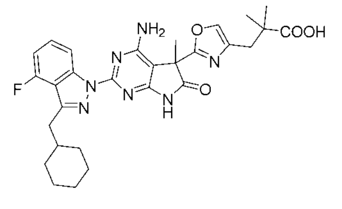 | 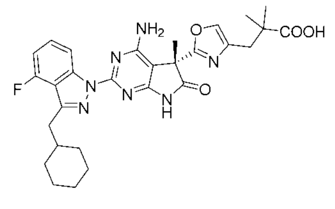 | 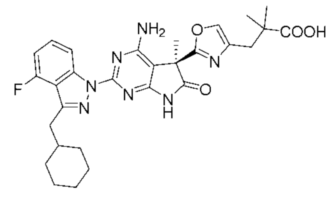 |
| 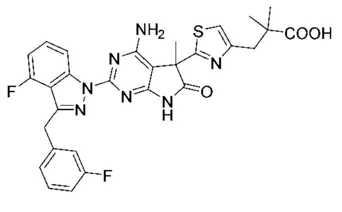 | 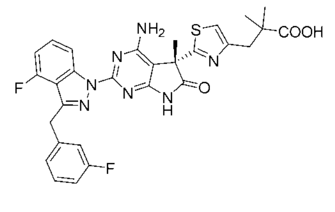 | 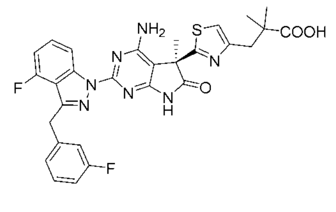 |
| 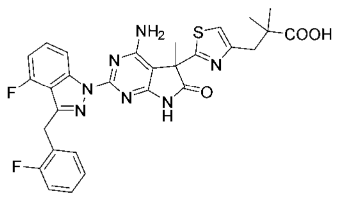 | 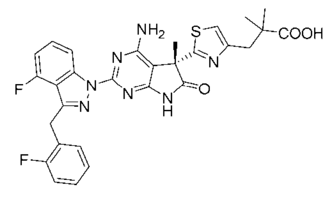 | 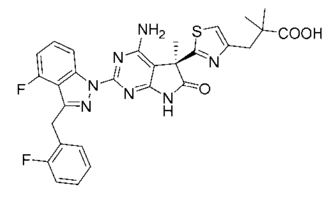 |
| 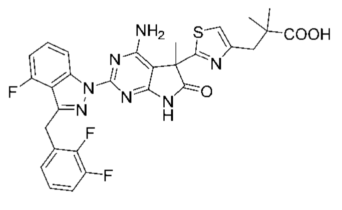 | 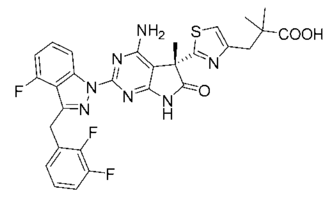 | 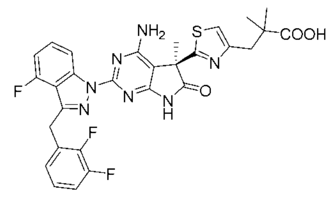 |
| 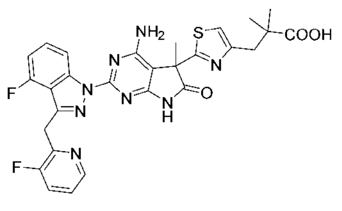 | 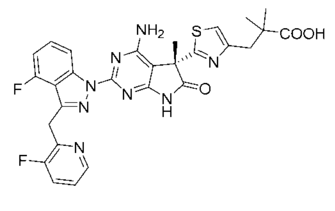 | 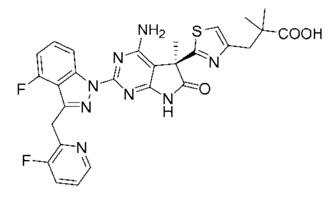 |
| 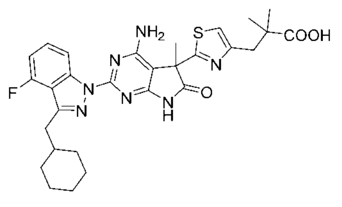 | 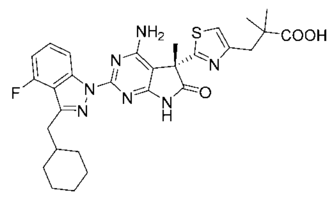 | 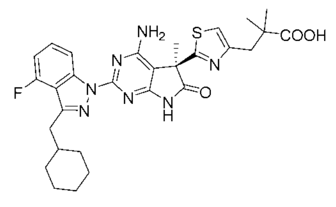 |

| | | |
|---|---|---|
| 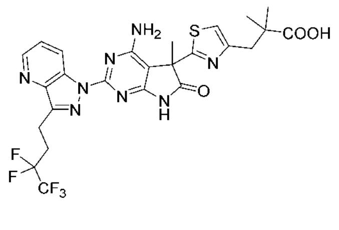 | 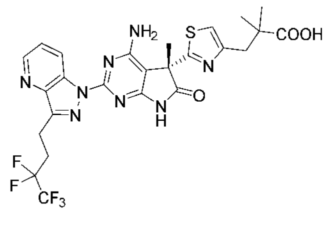 | 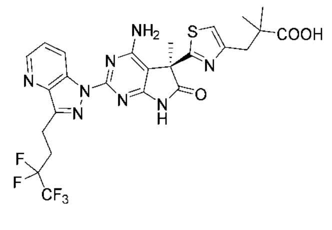 |
| 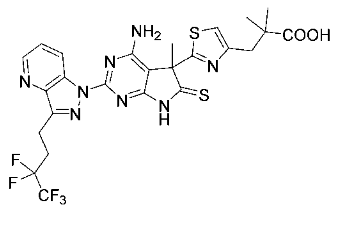 | 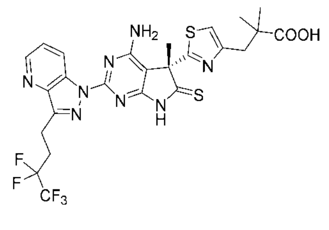 | 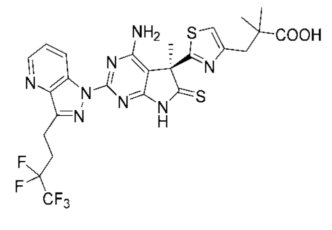 |
| 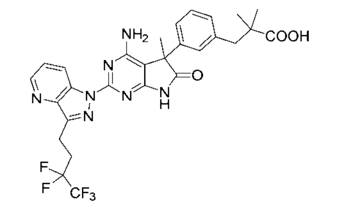 | 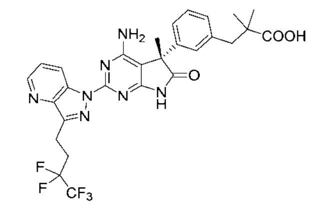 | 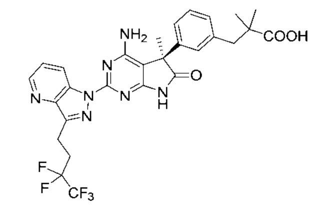 |
| 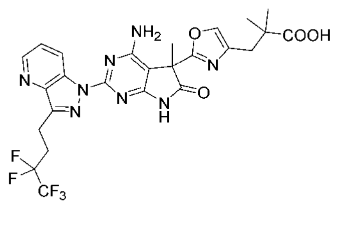 | 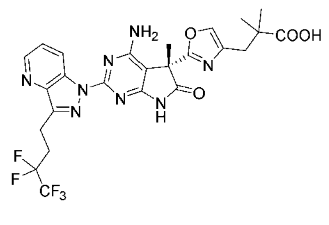 | 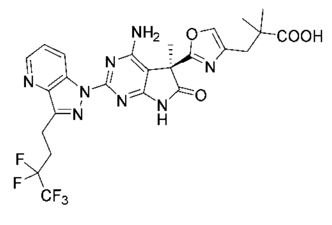 |
| 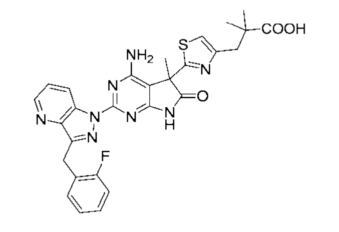 | 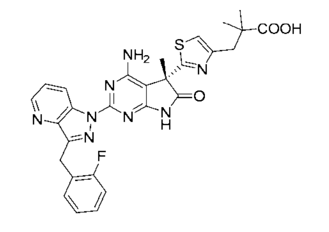 | 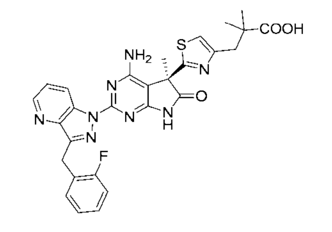 |
| 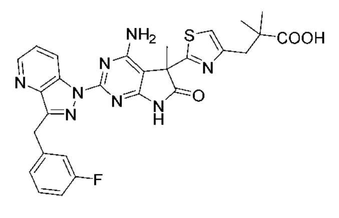 | 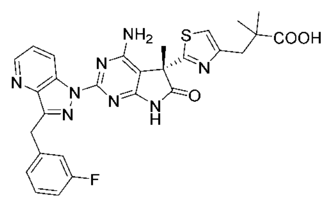 | 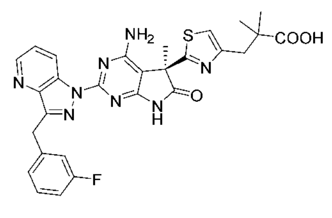 |
| 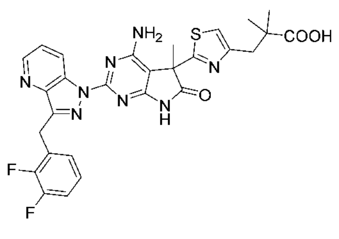 | 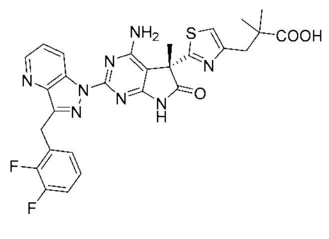 | 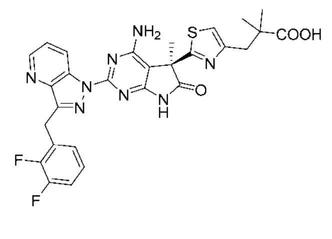 |

| | | |
|---|---|---|
| 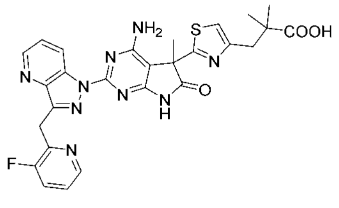 | 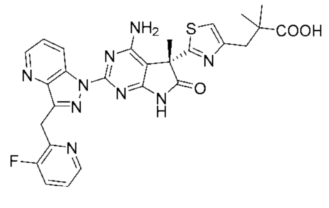 | 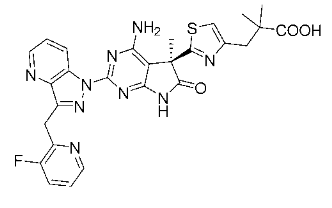 |
| 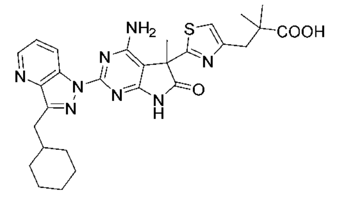 | 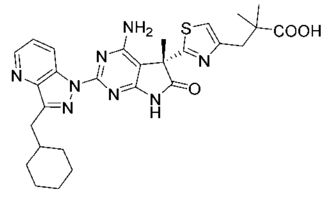 | 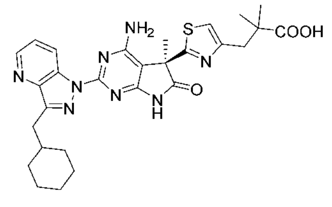 |
| 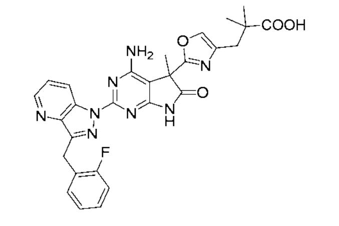 | 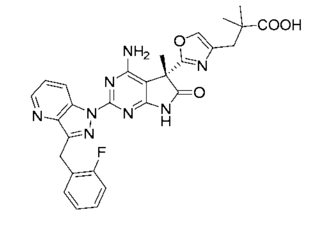 | 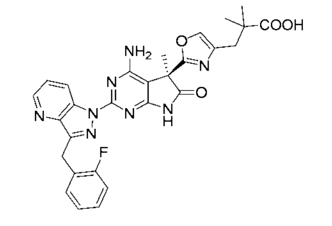 |
| 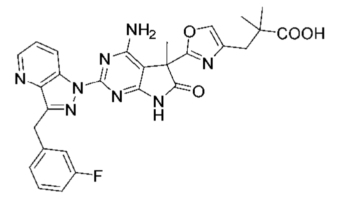 | 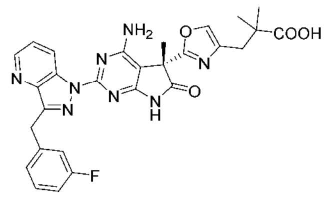 | 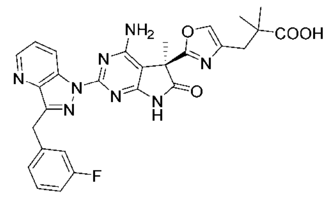 |
| 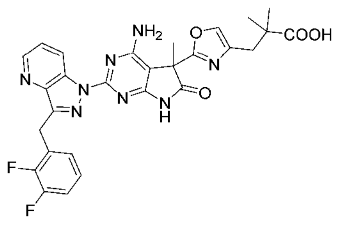 | 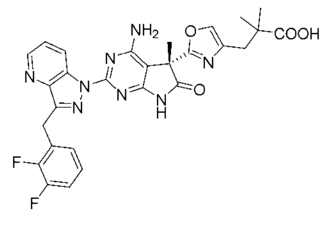 | 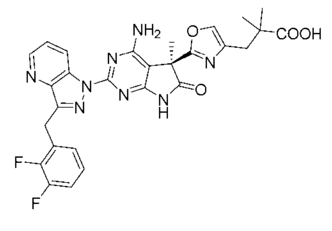 |
| 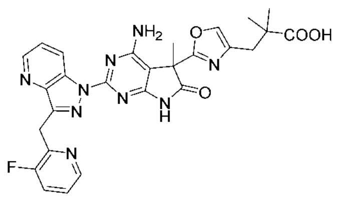 | 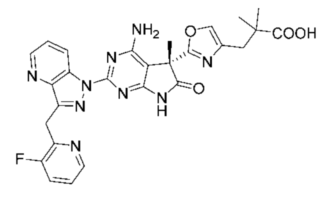 | 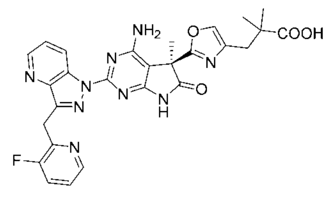 |
| 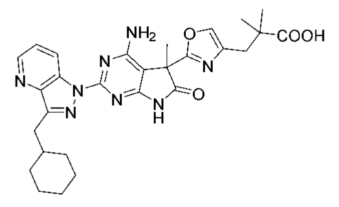 | 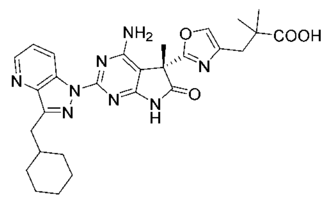 | 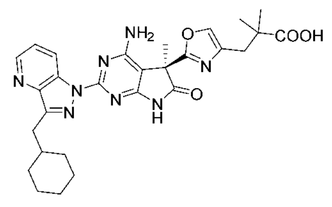 |

| | | |
|---|---|---|
| 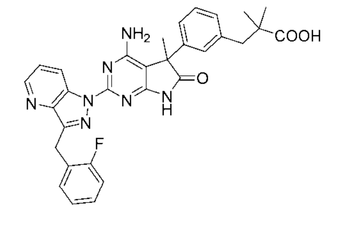 | 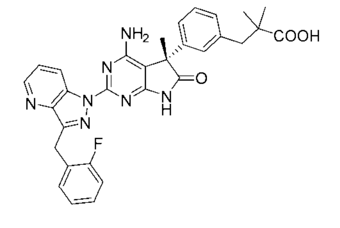 | 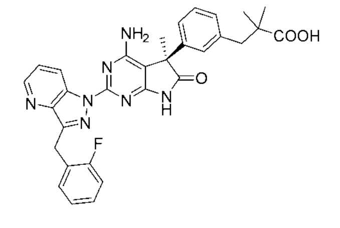 |
| 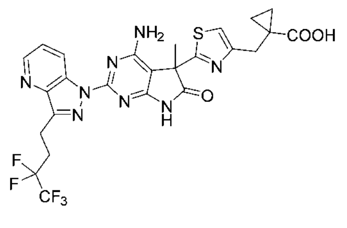 | 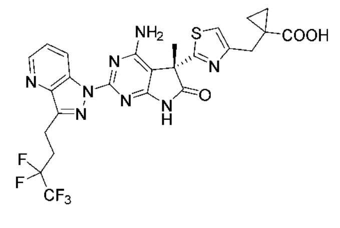 | 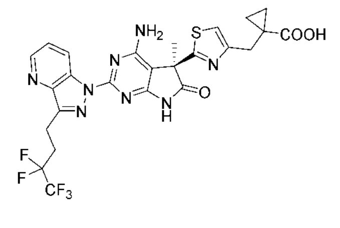 |
| 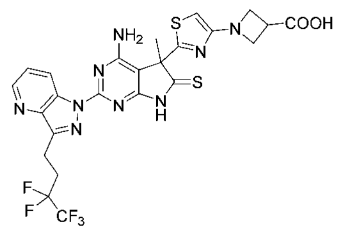 | 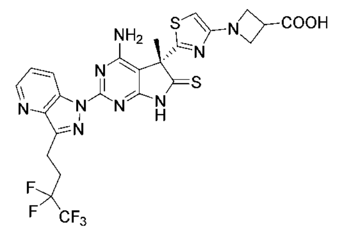 | 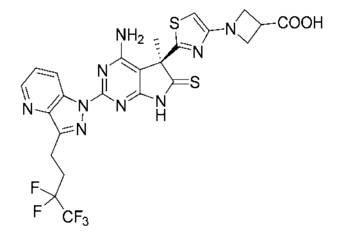 |
| 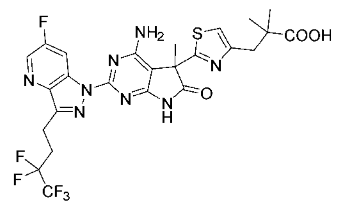 | 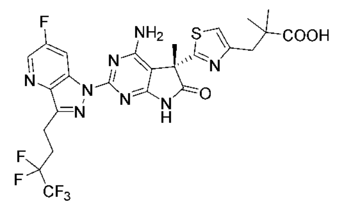 | 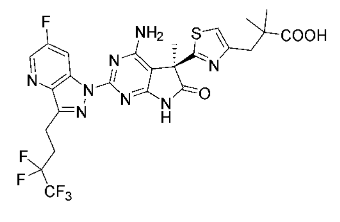 |
| 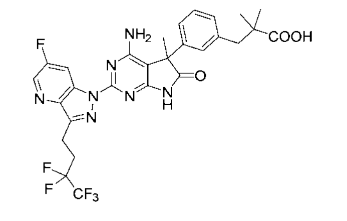 | 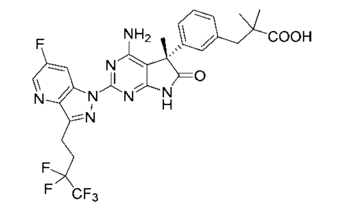 | 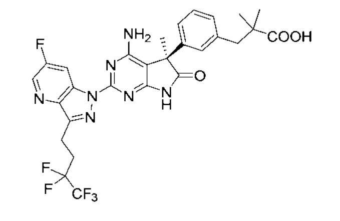 |
| 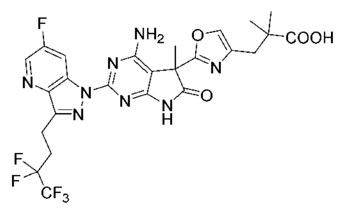 | 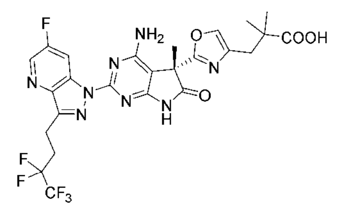 | 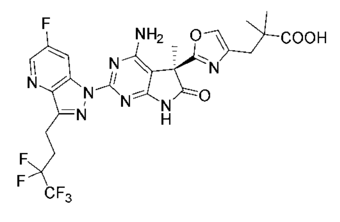 |
| 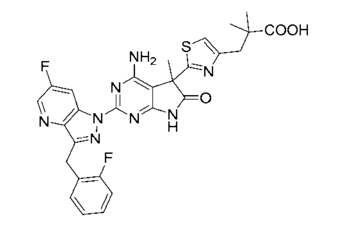 | 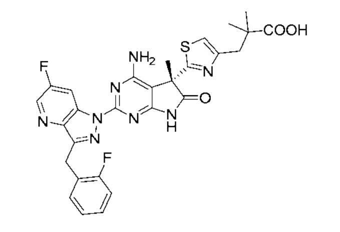 | 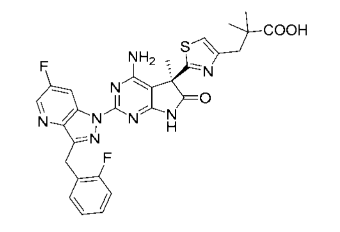 |

| | | |
|---|---|---|
| 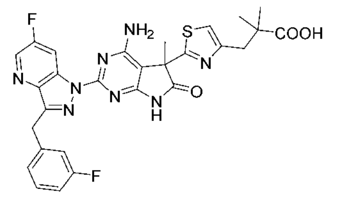 | 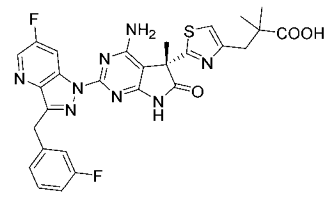 | 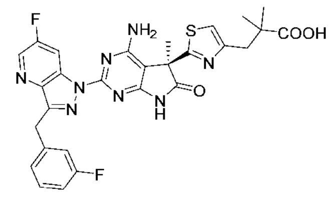 |
| 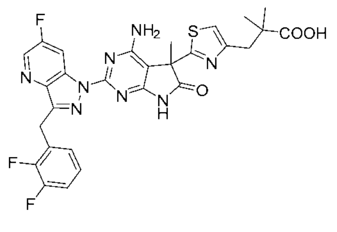 | 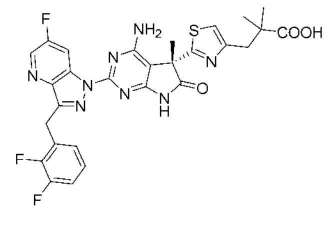 | 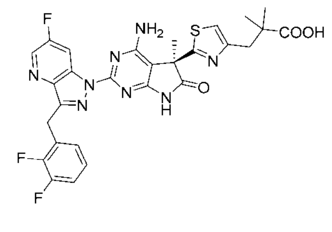 |
| 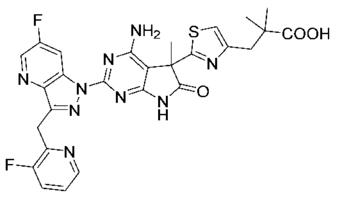 | 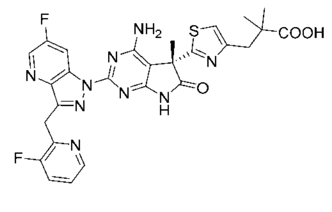 | 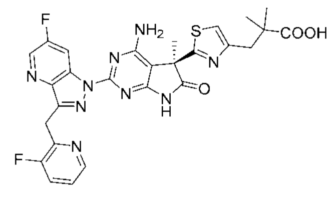 |
| 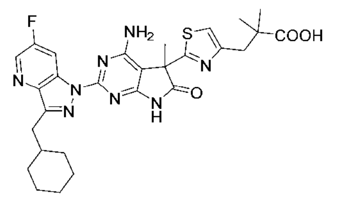 | 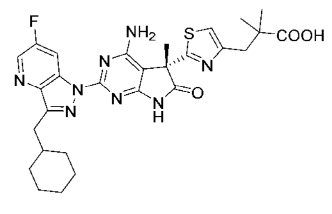 | 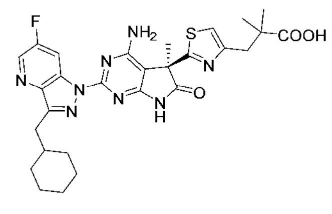 |
| 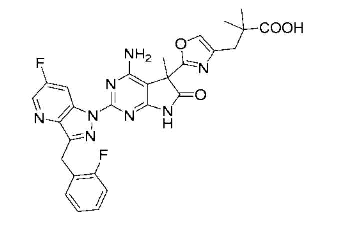 | 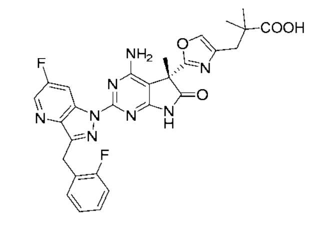 | 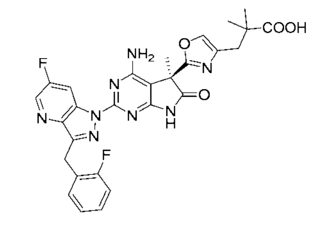 |
| 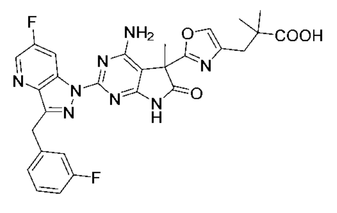 | 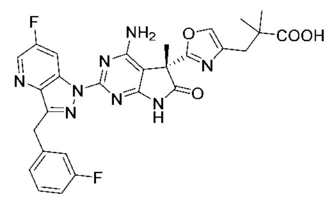 | 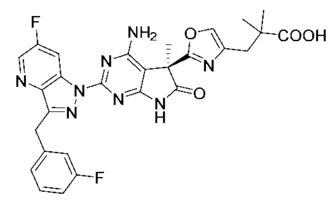 |
| 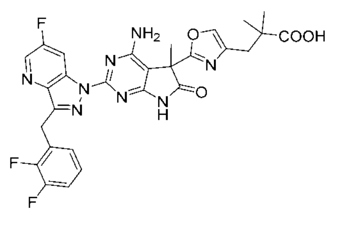 | 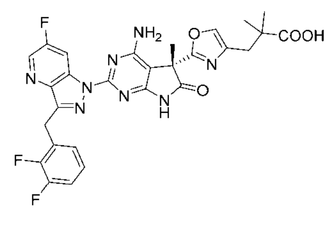 | 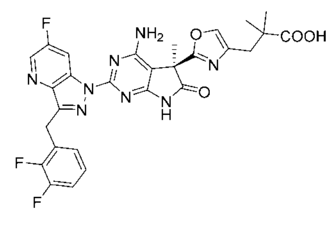 |

| | | |
|---|---|---|
| 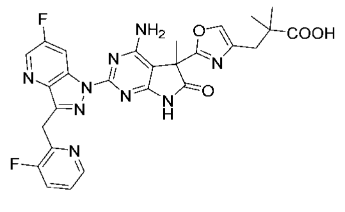 | 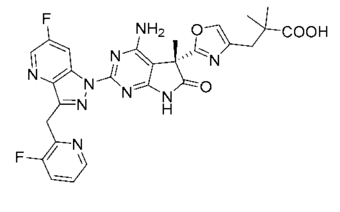 | 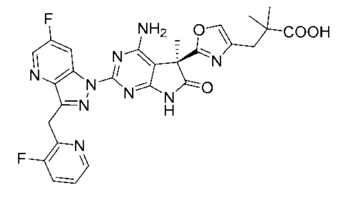 |
| 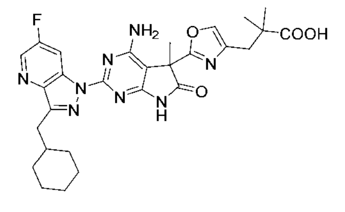 | 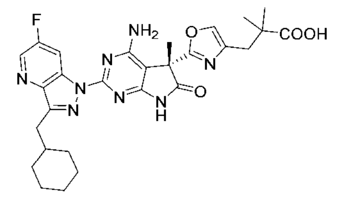 | 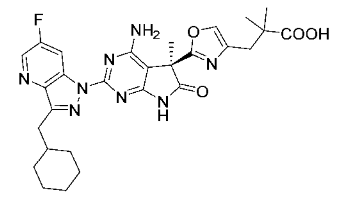 |
| 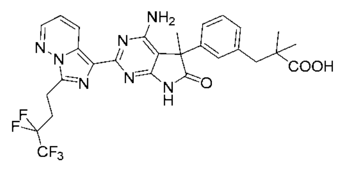 | 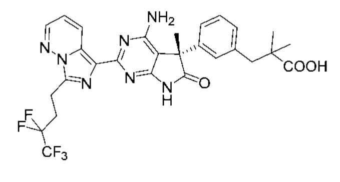 | 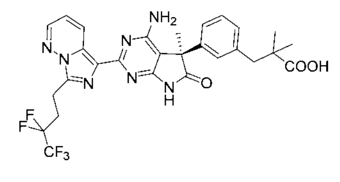 |
| 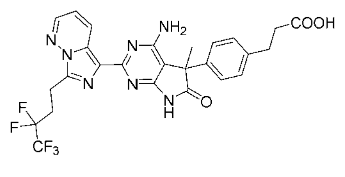 | 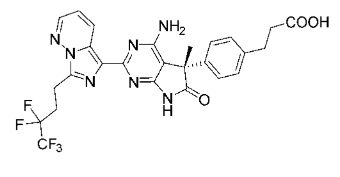 | 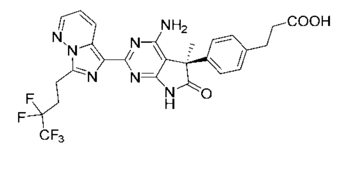 |
| 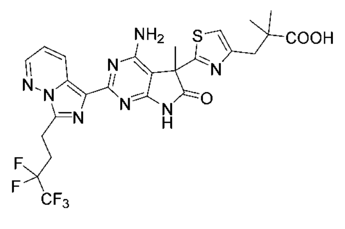 | 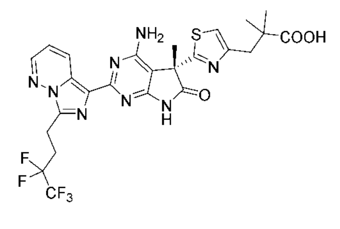 | 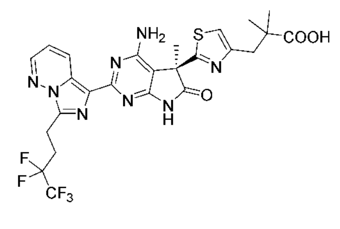 |
| 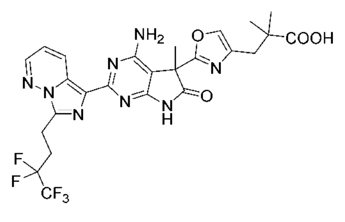 | 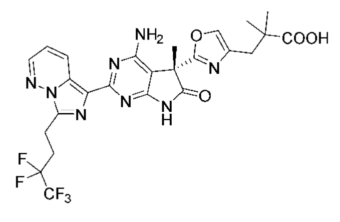 | 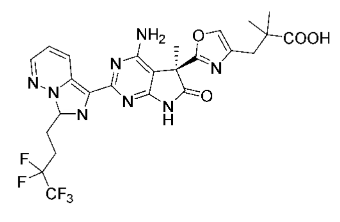 |
| 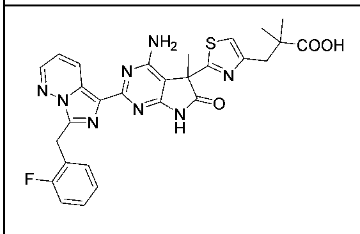 | 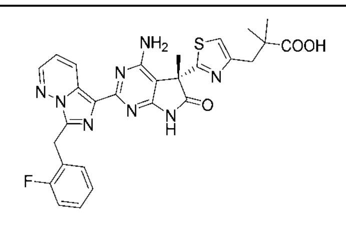 | 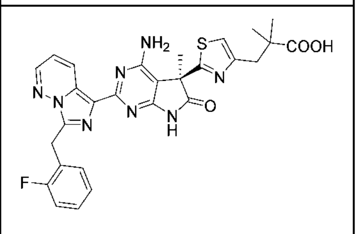 |
| 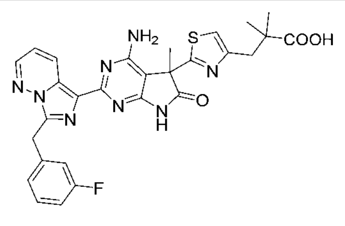 | 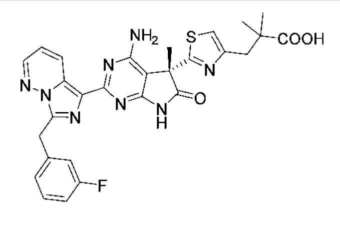 | 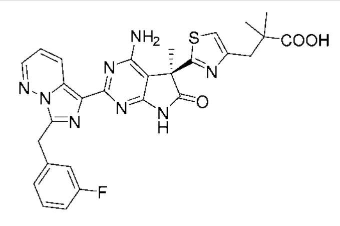 |

| | | |
|---|---|---|
| 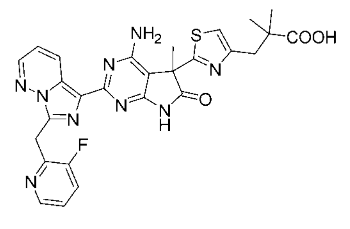 | 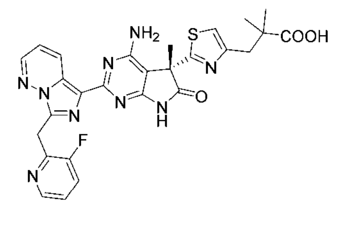 | 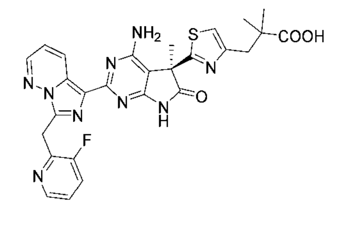 |
| 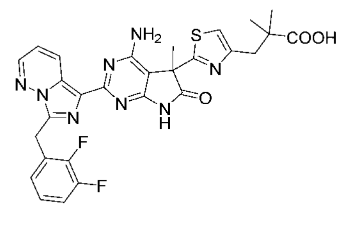 | 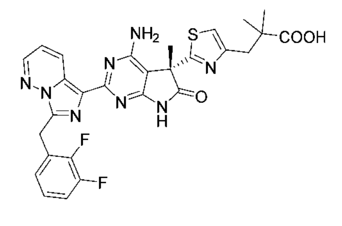 | 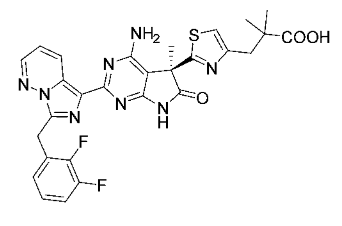 |
| 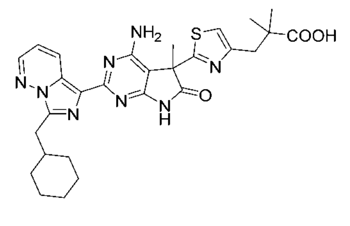 | 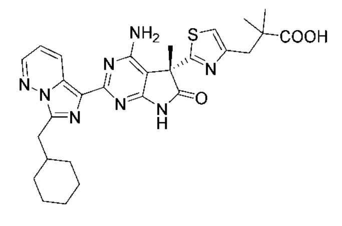 | 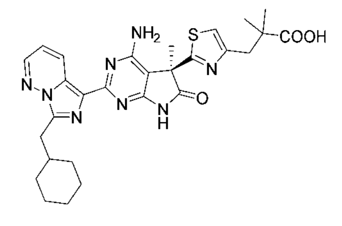 |
| 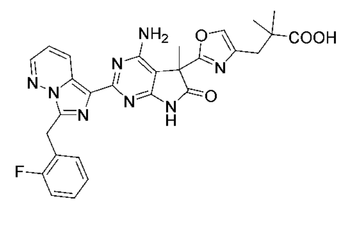 | 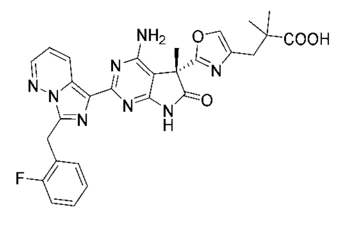 | 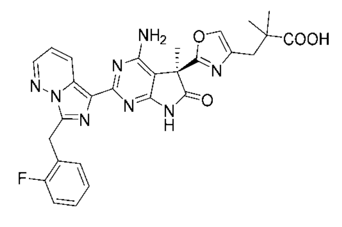 |
| 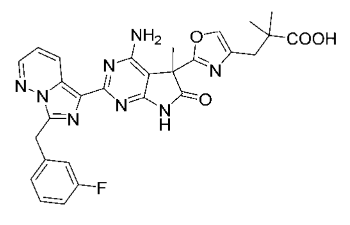 | 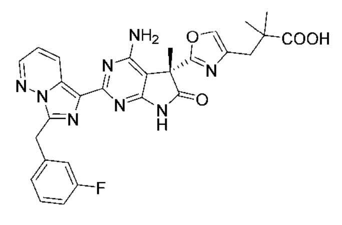 | 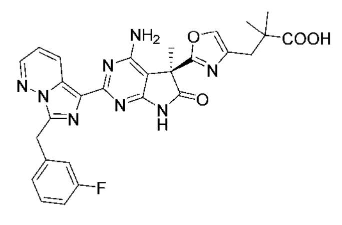 |
| 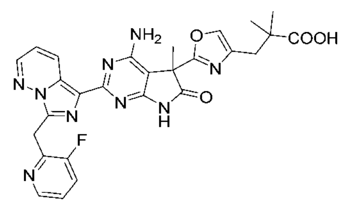 | 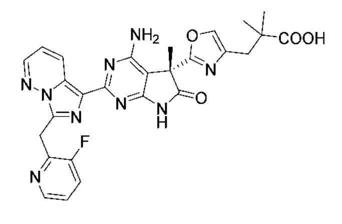 | 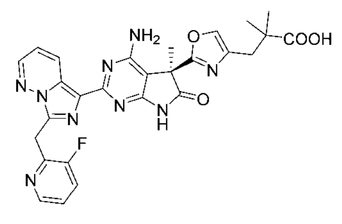 |
| 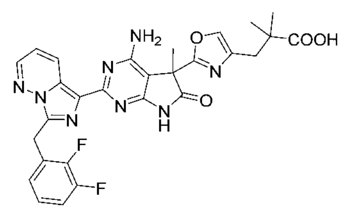 | 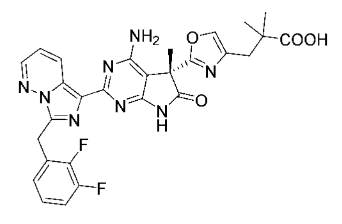 | 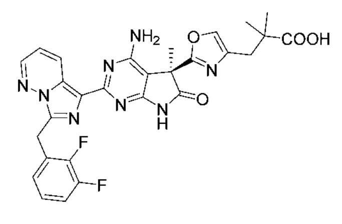 |

| | | |
|---|---|---|
| 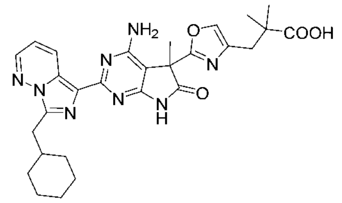 | 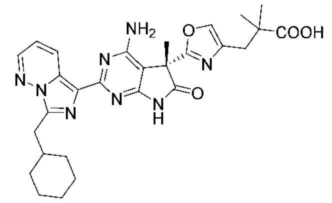 | 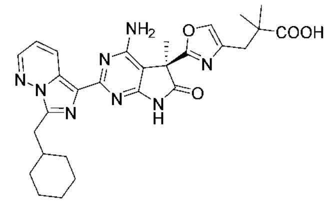 |
| 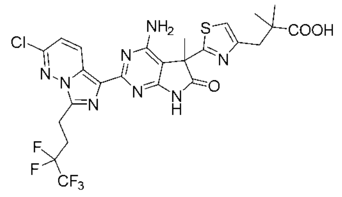 | 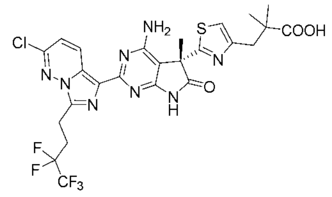 | 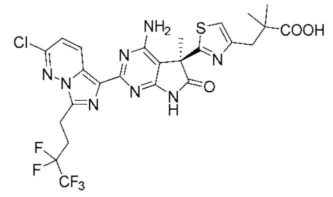 |
| 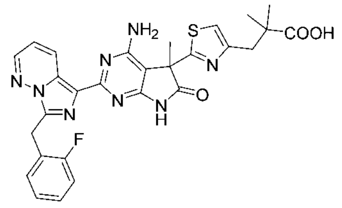 | 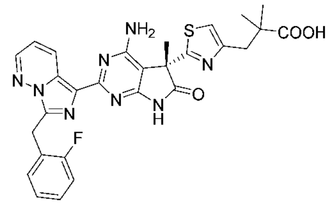 | 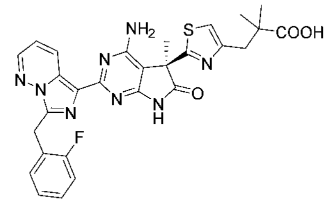 |
| 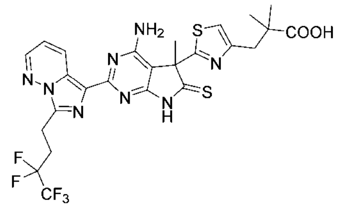 | 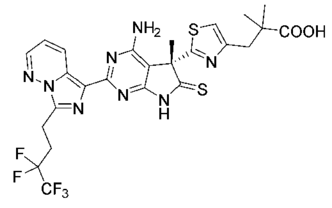 | 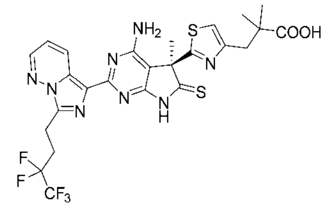 |
| 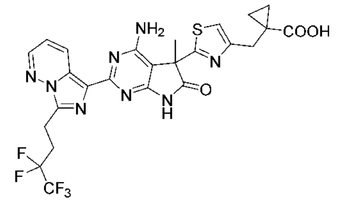 | 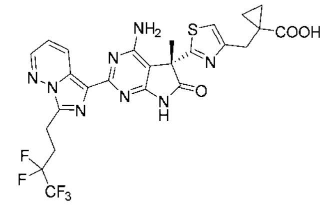 | 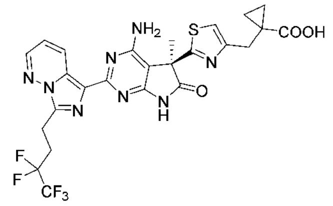 |
| 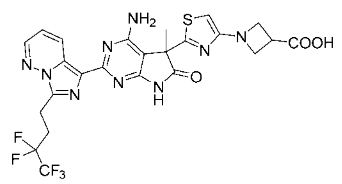 | 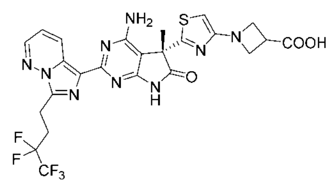 | 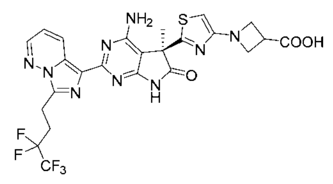 |
| 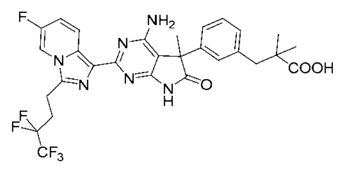 | 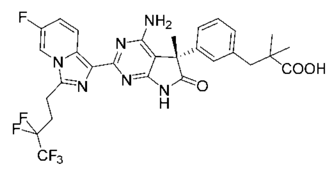 | 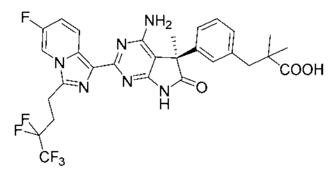 |
| 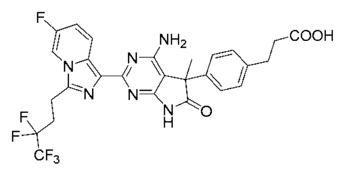 | 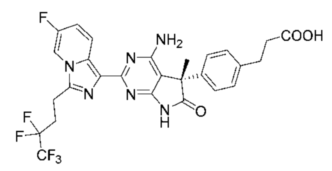 | 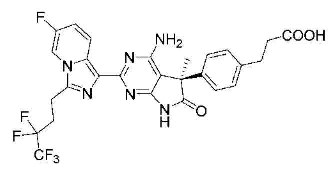 |

| | | |
|---|---|---|
| 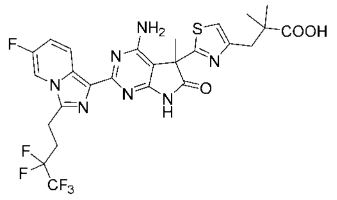 | 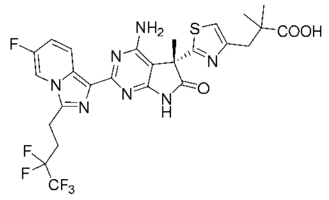 | 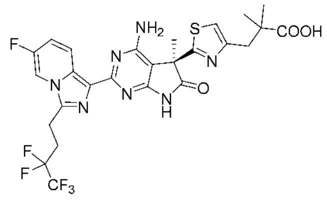 |
| 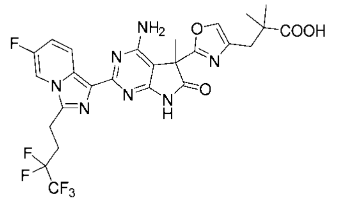 | 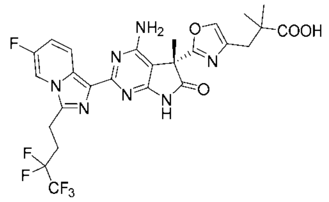 | 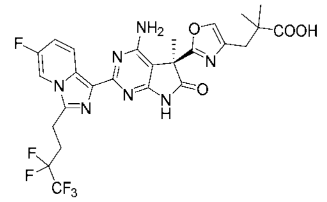 |
| 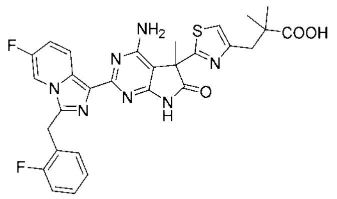 | 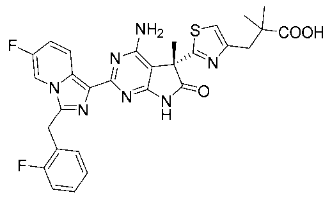 | 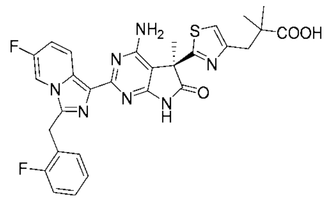 |
| 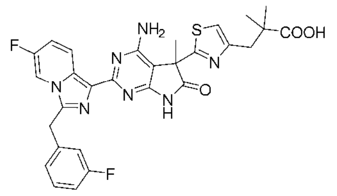 | 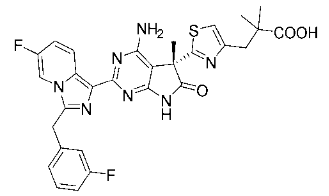 | 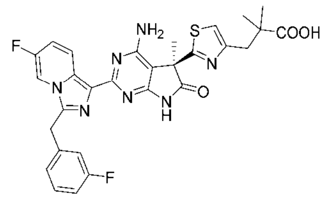 |
| 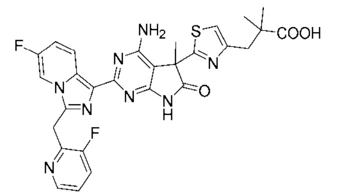 | 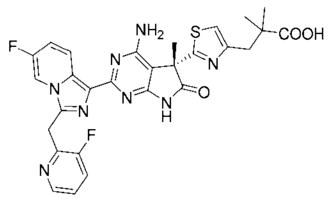 | 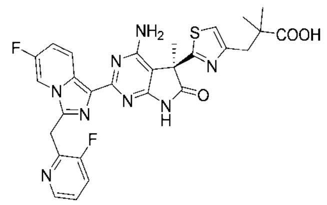 |
| 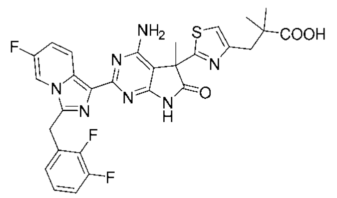 | 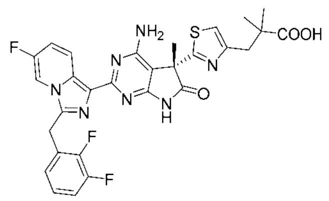 | 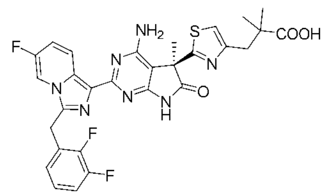 |
| 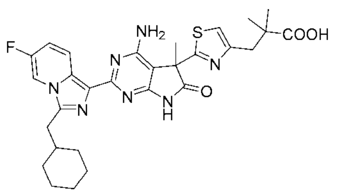 | 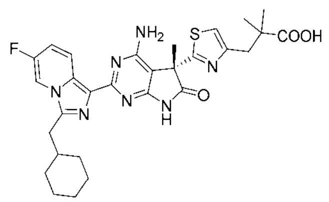 | 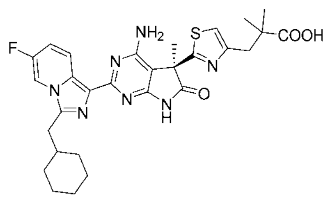 |

| | | |
|---|---|---|
| 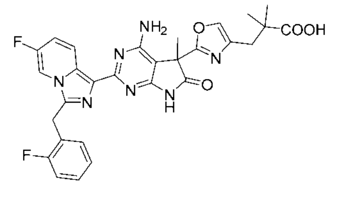 | 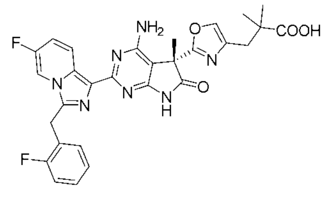 | 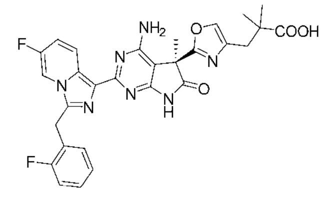 |
| 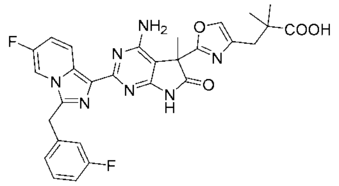 | 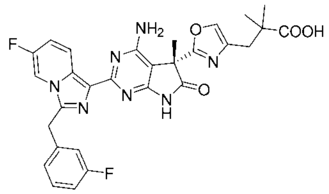 | 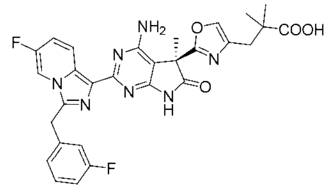 |
| 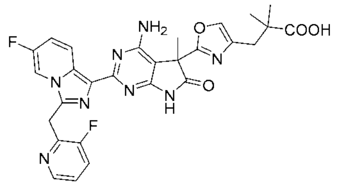 | 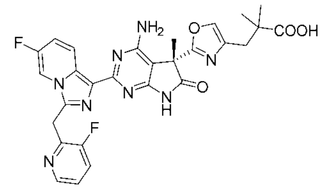 | 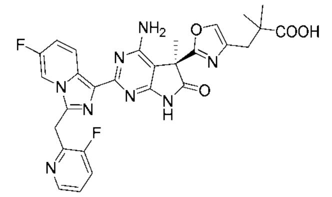 |
| 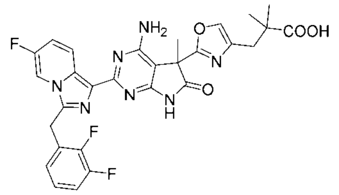 | 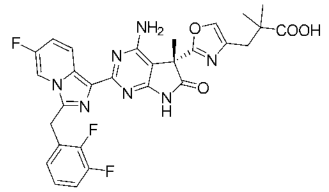 | 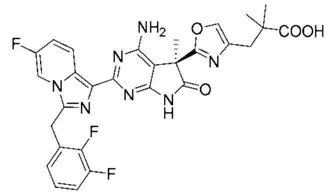 |
| 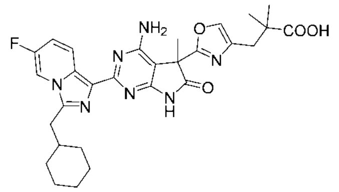 | 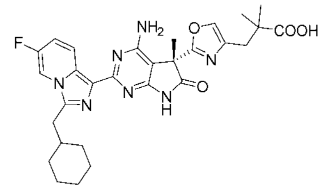 | 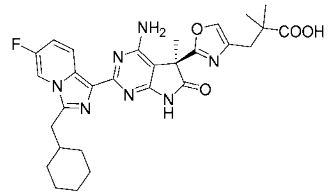 |
| 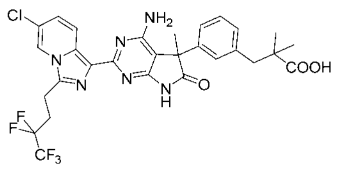 | 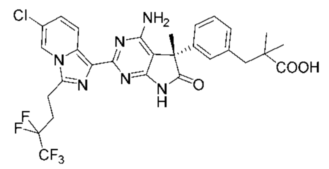 | 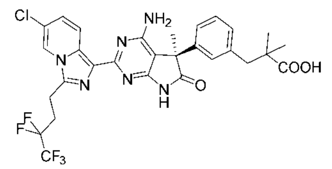 |
| 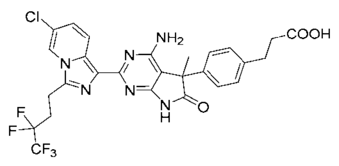 | 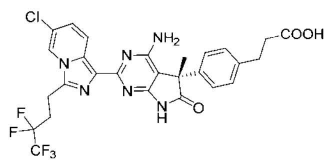 | 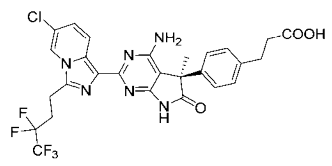 |
| 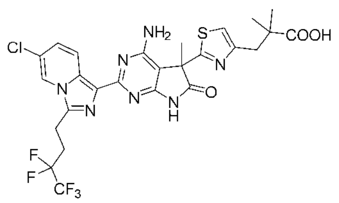 | 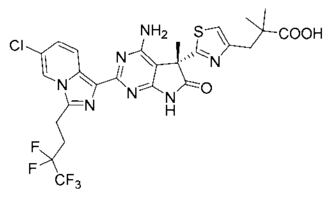 | 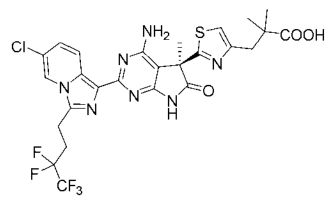 |

| | | |
|---|---|---|
| 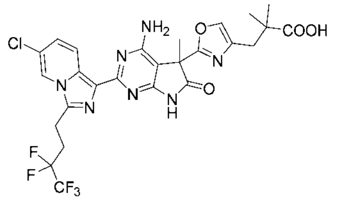 | 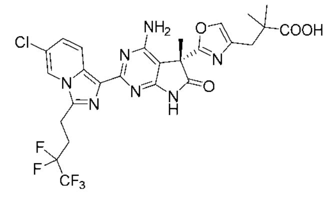 | 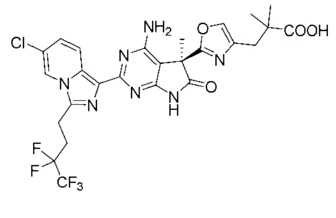 |
| 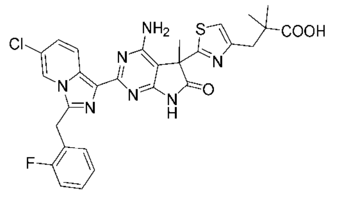 | 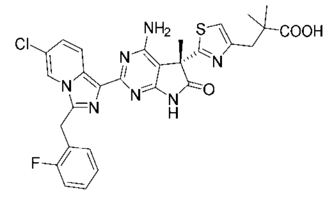 | 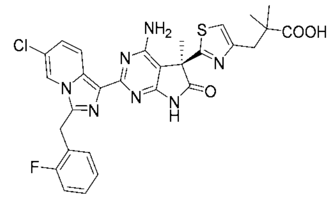 |
| 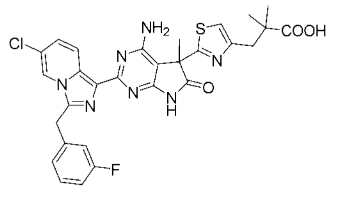 | 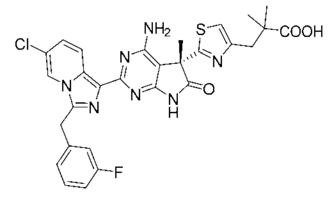 | 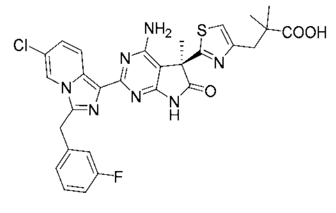 |
| 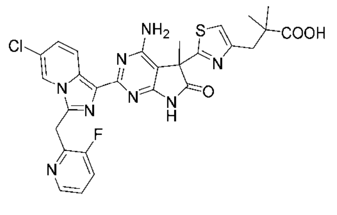 | 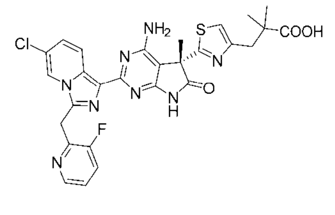 | 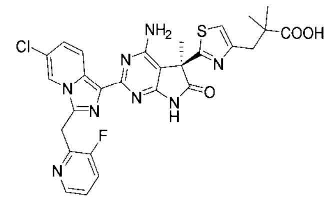 |
| 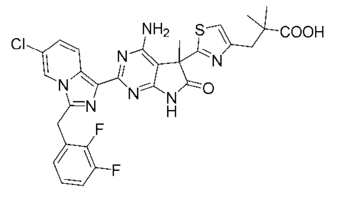 | 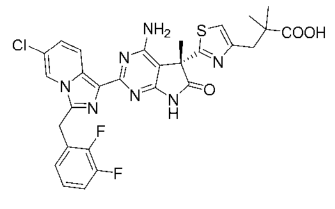 | 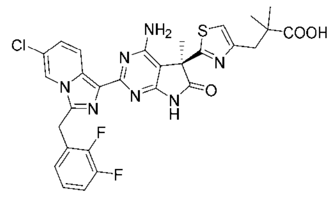 |
| 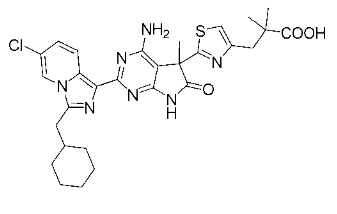 | 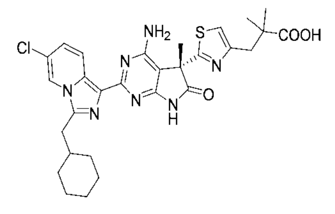 | 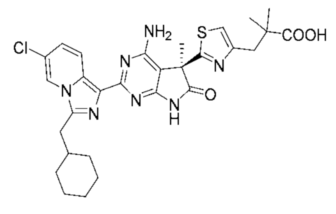 |
| 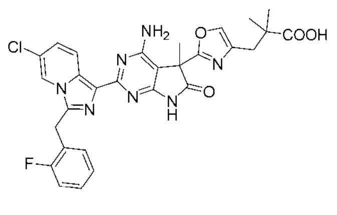 | 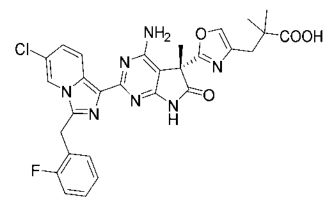 | 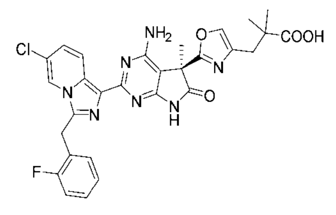 |

| | | |
|---|---|---|
| 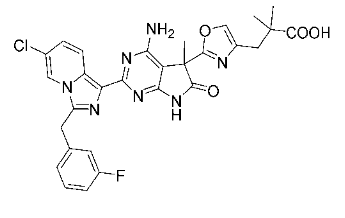 | 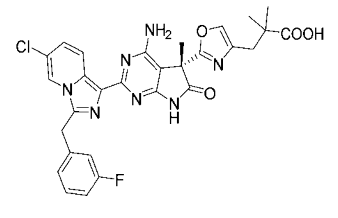 | 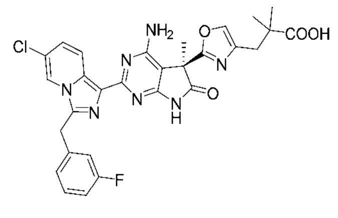 |
| 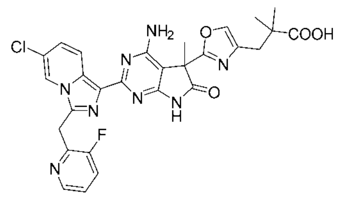 | 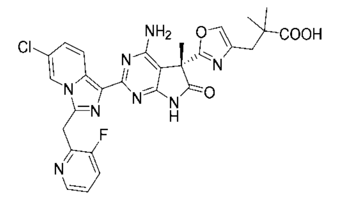 | 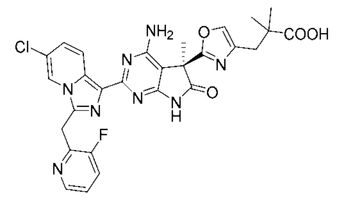 |
| 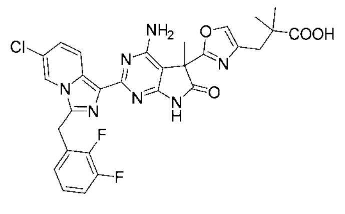 | 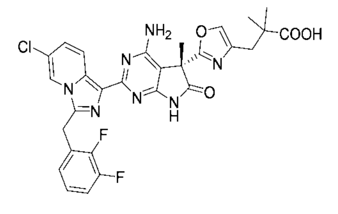 | 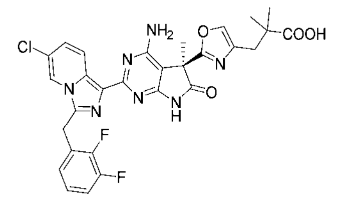 |
| 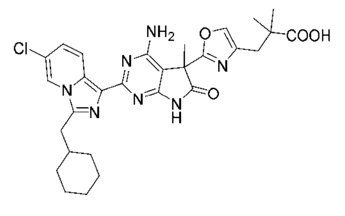 | 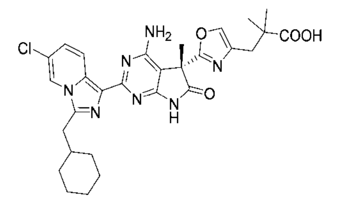 | 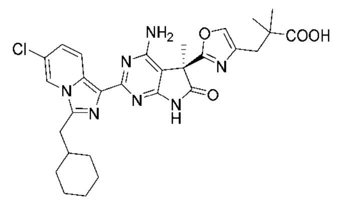 |
| 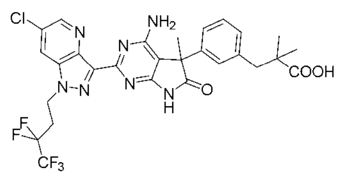 | 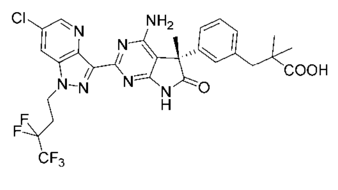 | 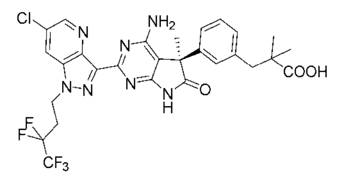 |
| 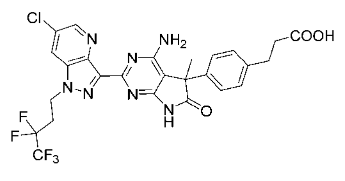 | 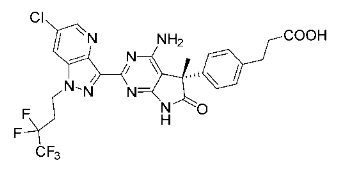 | 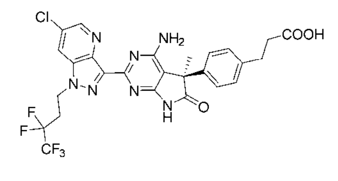 |
| 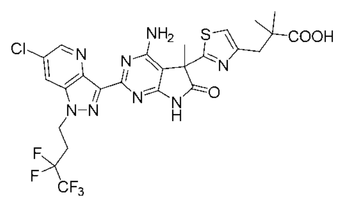 | 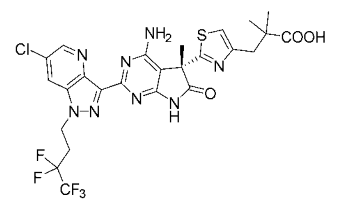 | 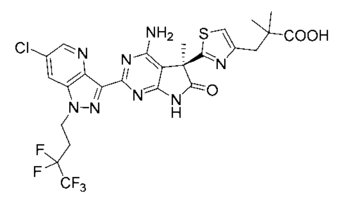 |
| 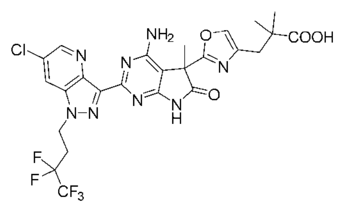 | 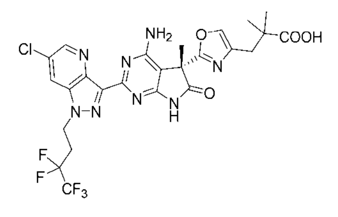 | 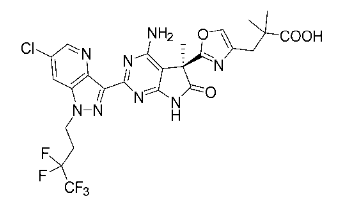 |

| | | |
|---|---|---|
| 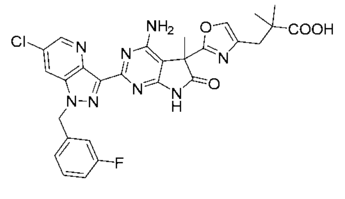 | 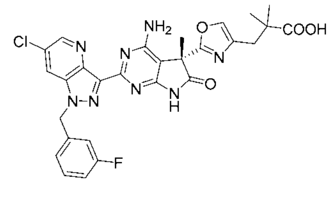 | 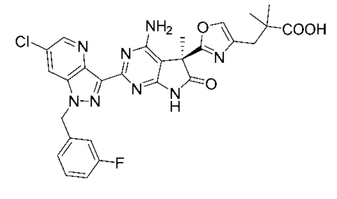 |
| 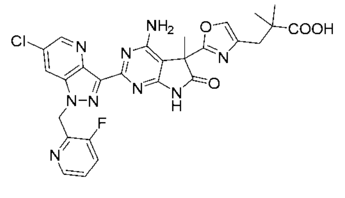 | 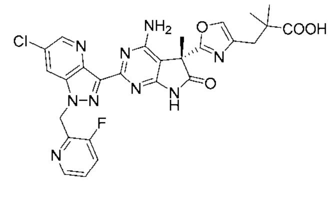 | 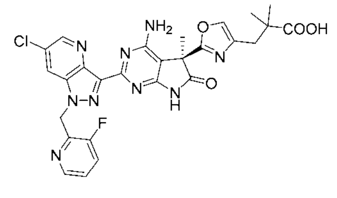 |
| 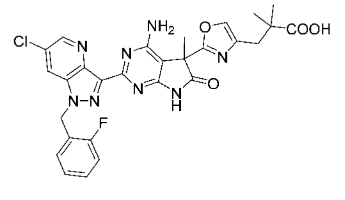 | 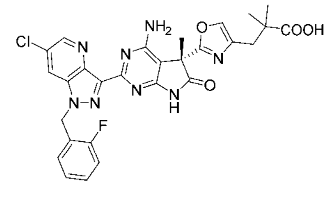 | 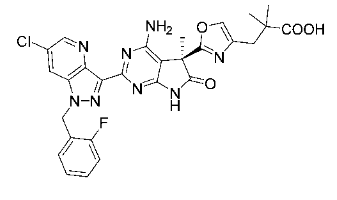 |
| 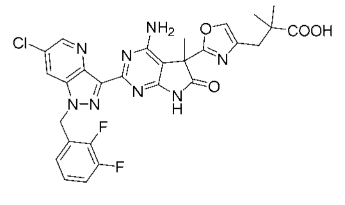 | 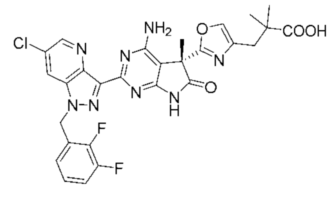 | 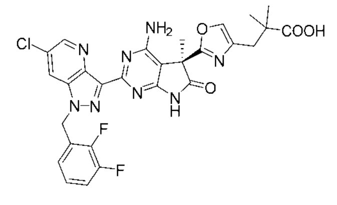 |
| 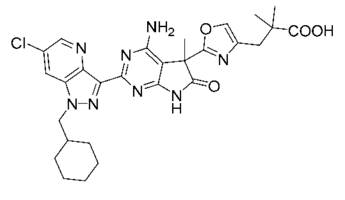 | 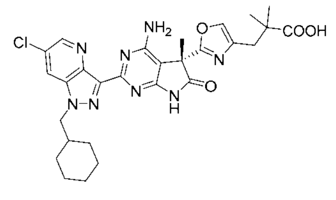 | 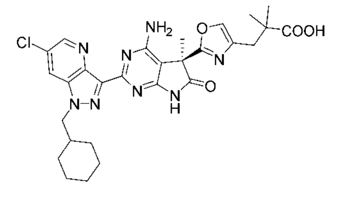 |
| 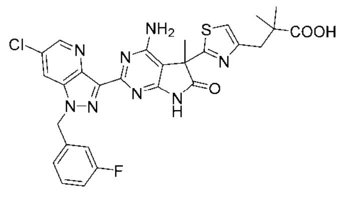 | 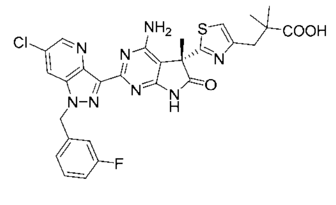 | 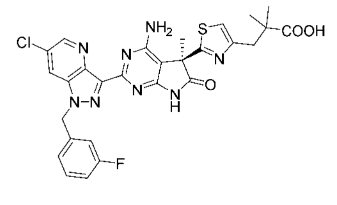 |
| 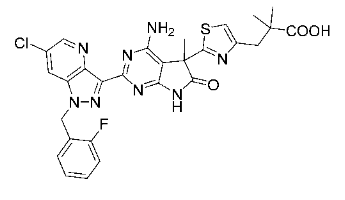 | 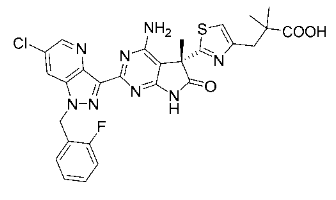 | 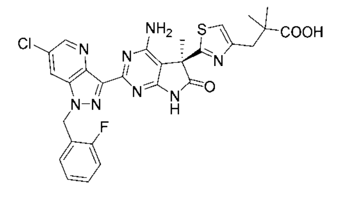 |
| 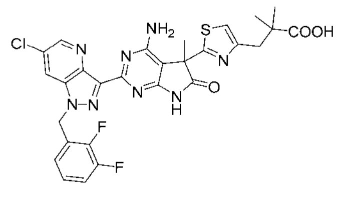 | 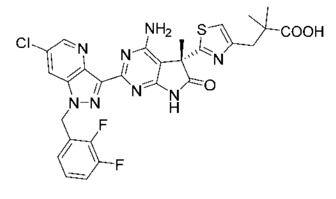 | 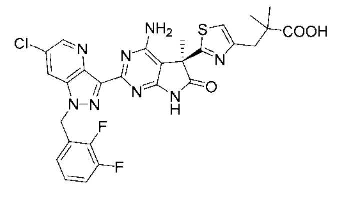 |

| | | |
|---|---|---|
| 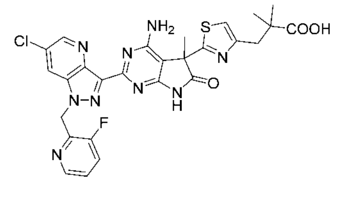 | 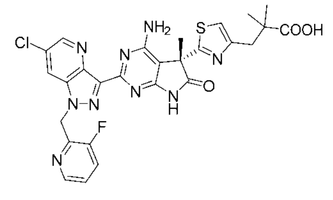 | 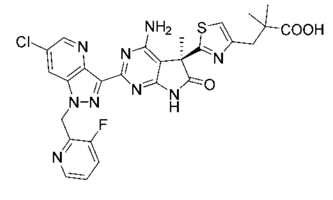 |
| 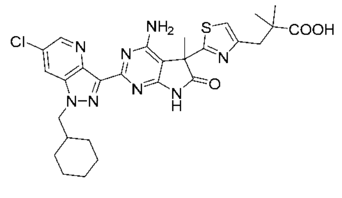 | 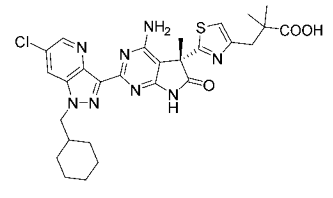 | 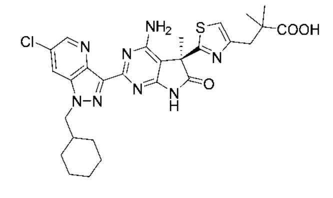 |
| 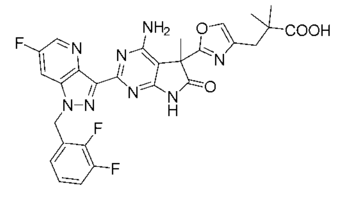 | 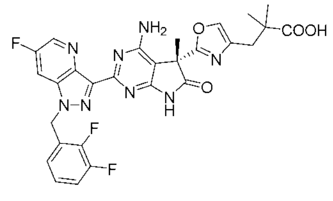 | 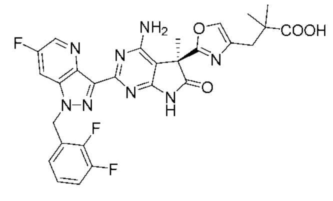 |
| 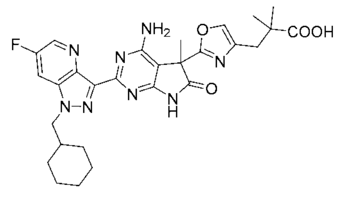 | 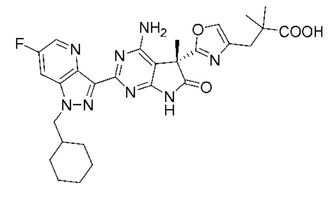 | 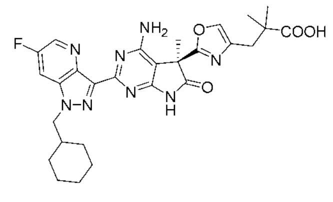 |
| 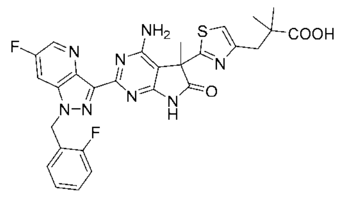 | 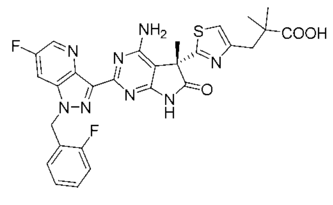 | 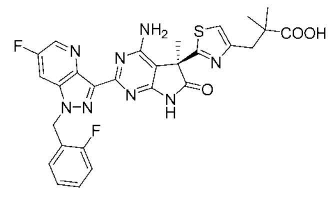 |
| 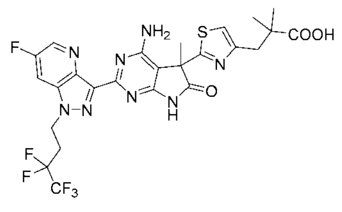 | 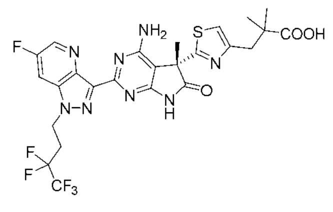 | 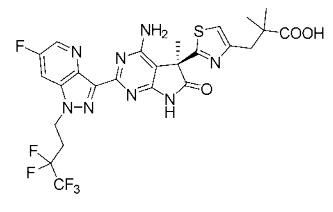 |
| 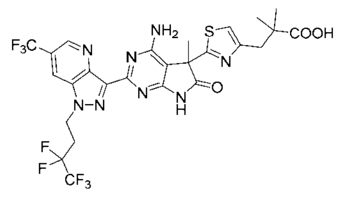 | 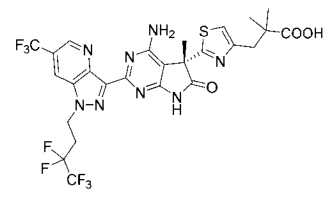 | 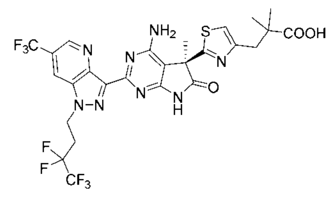 |
| 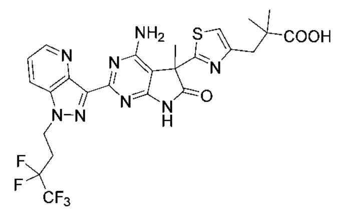 | 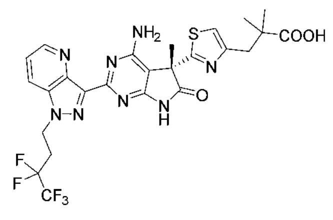 | 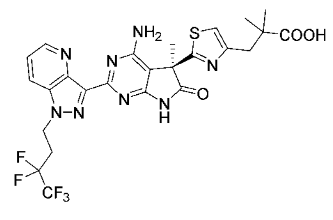 |

| | | |
|---|---|---|
| 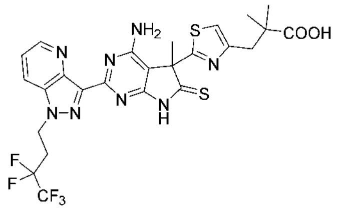 | 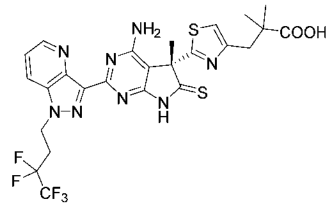 | 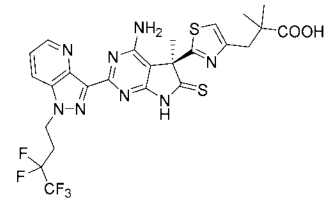 |
| 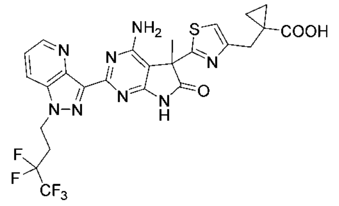 | 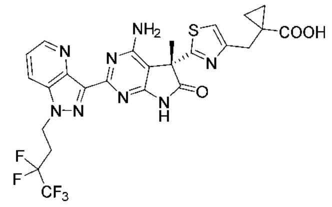 | 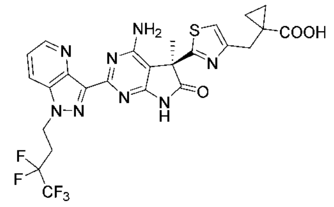 |
| 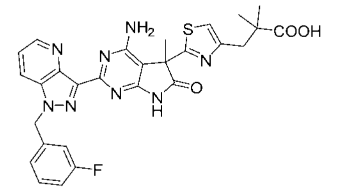 | 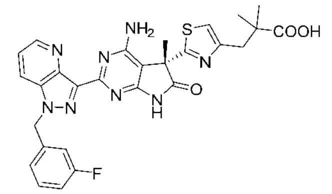 | 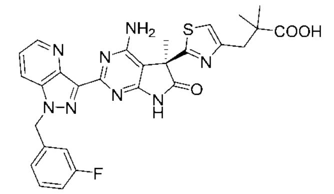 |
| 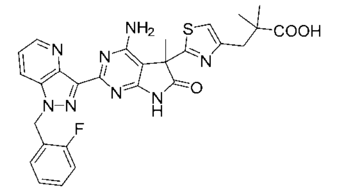 | 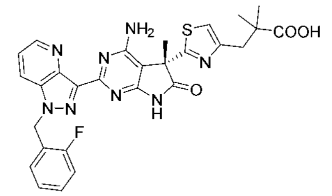 | 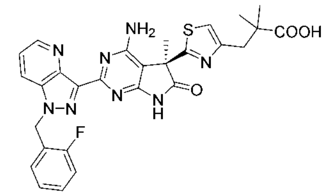 |
| 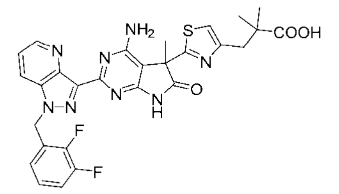 | 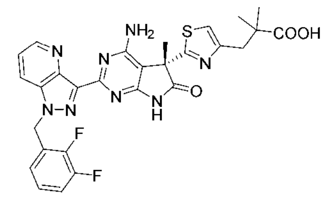 | 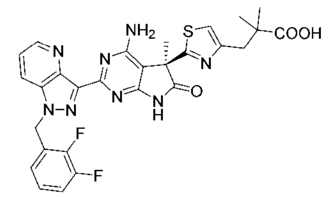 |
| 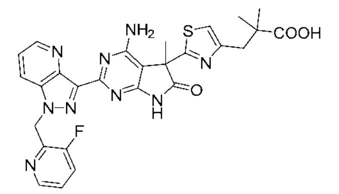 | 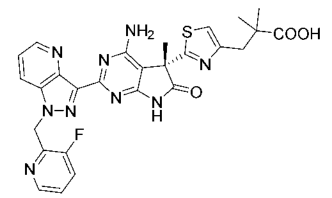 | 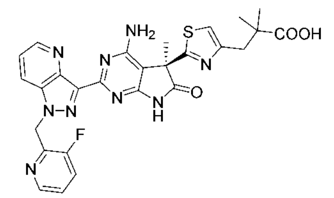 |
| 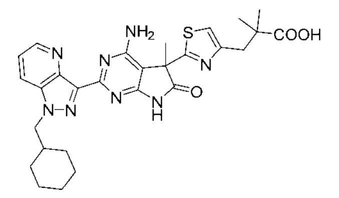 | 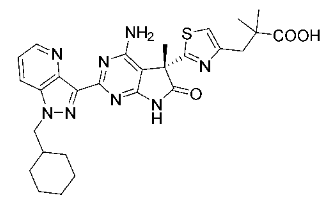 | 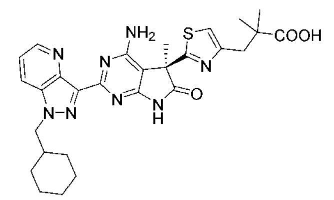 |

| | | |
|---|---|---|
| 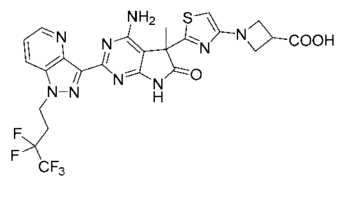 | 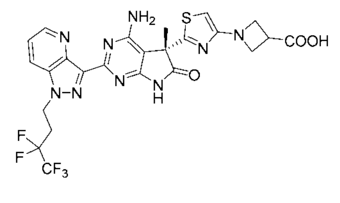 | 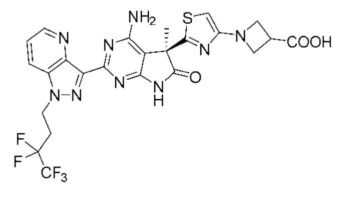 |
| 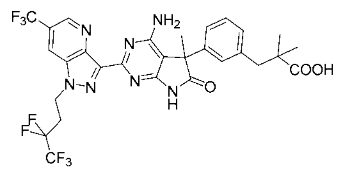 | 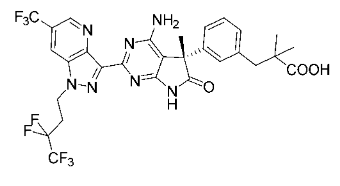 | 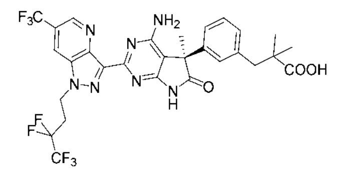 |
| 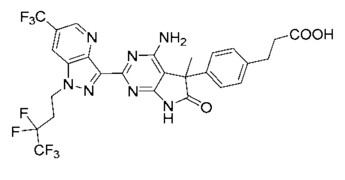 | 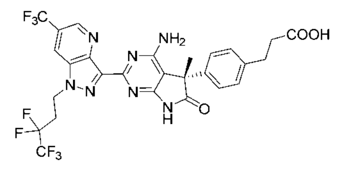 | 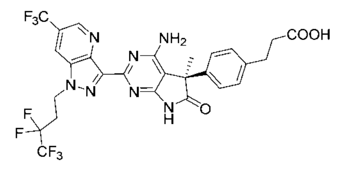 |
| 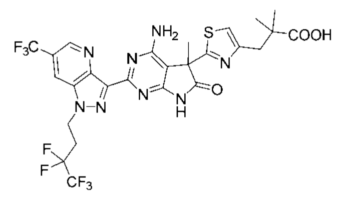 | 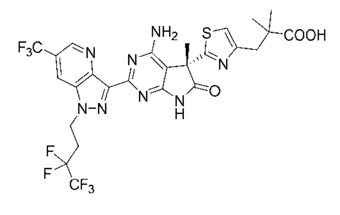 | 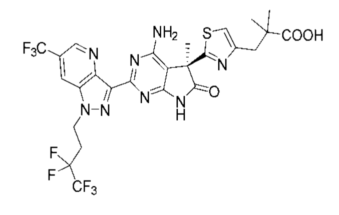 |
| 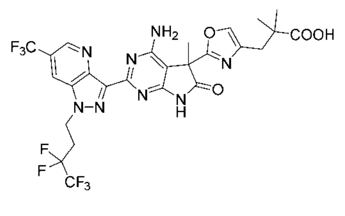 | 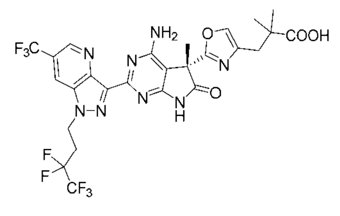 | 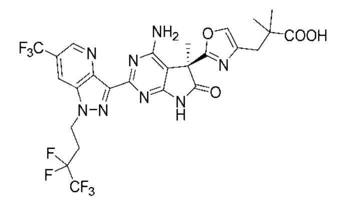 |
| 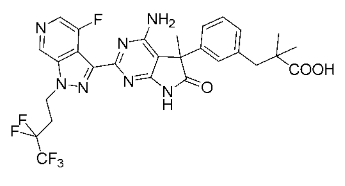 | 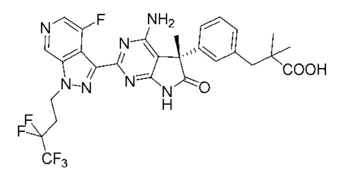 | 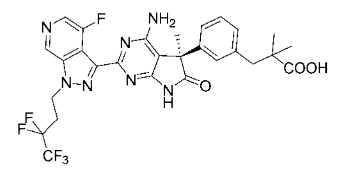 |
| 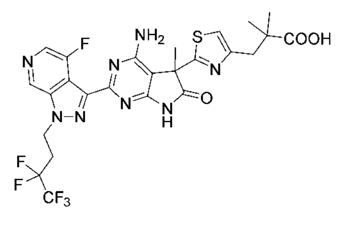 | 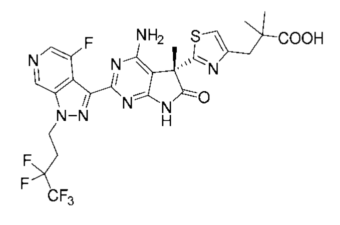 | 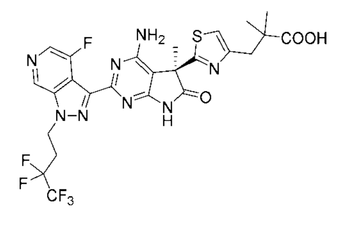 |
| 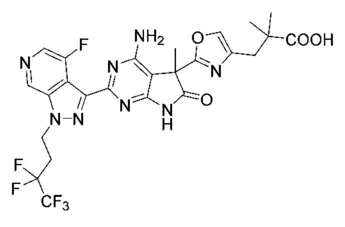 | 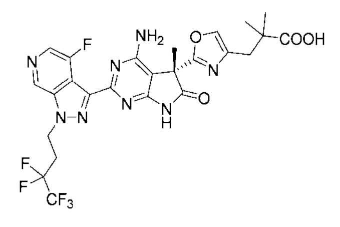 | 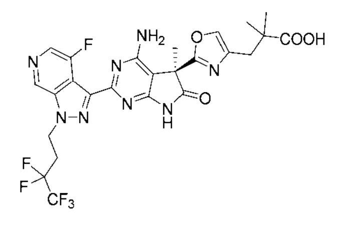 |

| | | |
|---|---|---|
| 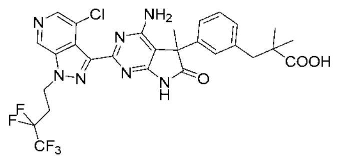 | 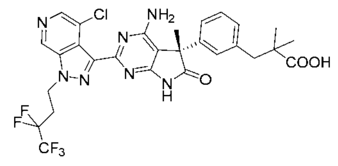 | 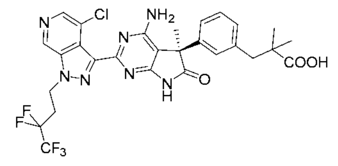 |
| 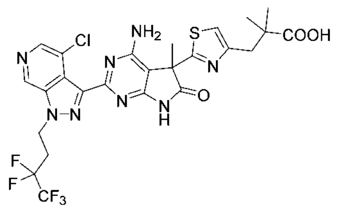 | 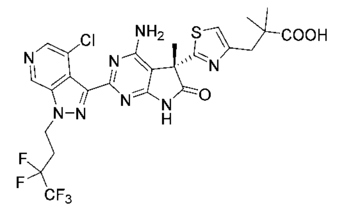 | 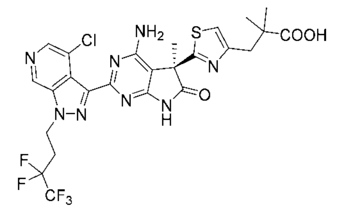 |
| 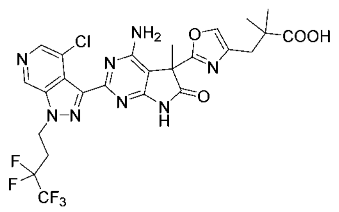 | 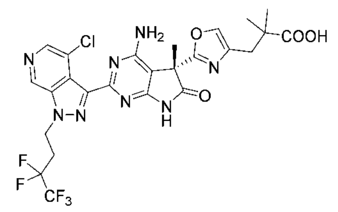 | 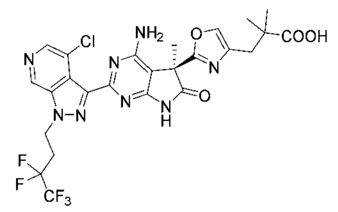 |
| 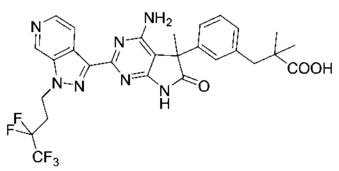 | 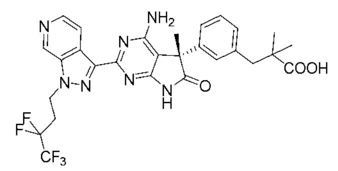 | 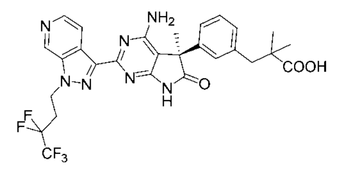 |
| 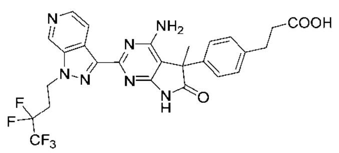 | 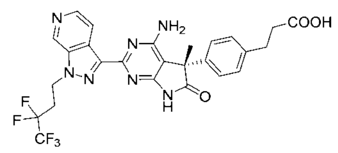 | 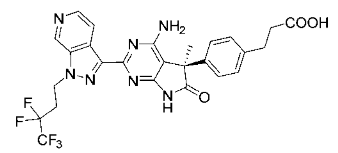 |
| 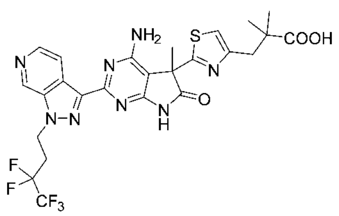 | 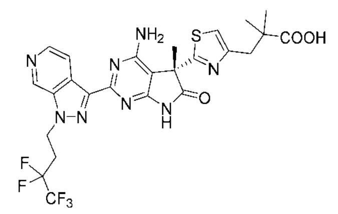 | 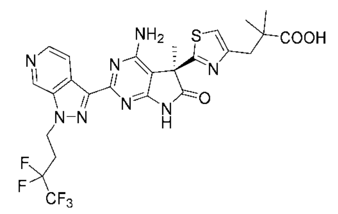 |
| 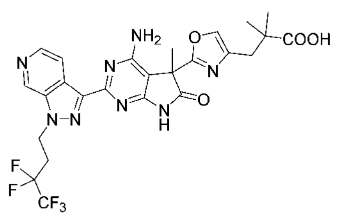 | 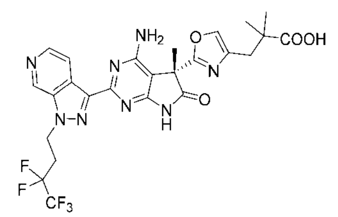 | 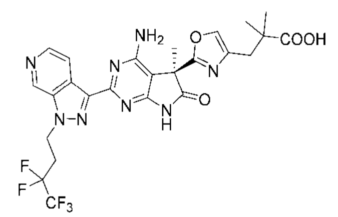 |
| 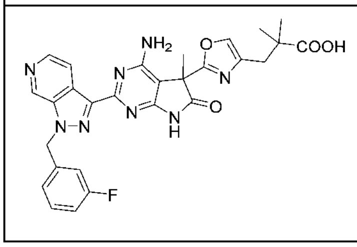 | 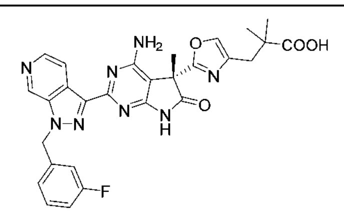 | 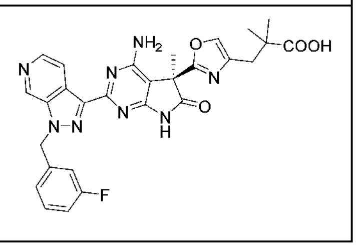 |

| | | |
|---|---|---|
| 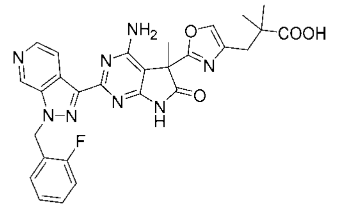 | 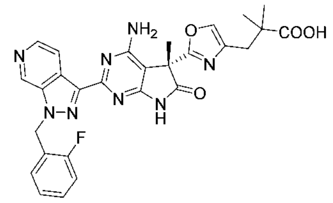 | 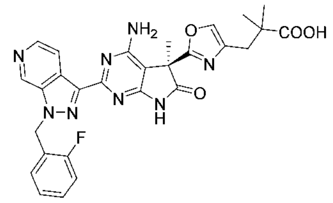 |
| 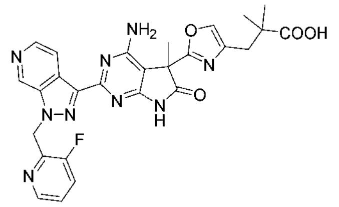 | 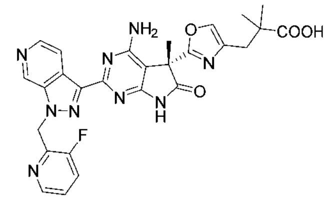 | 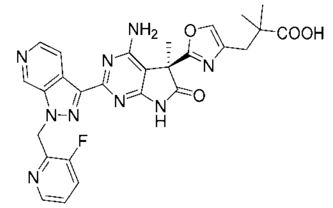 |
| 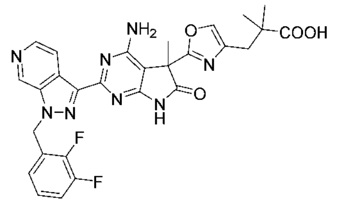 | 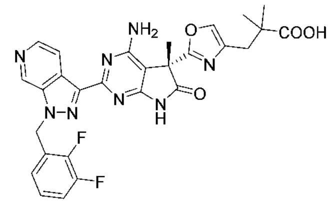 | 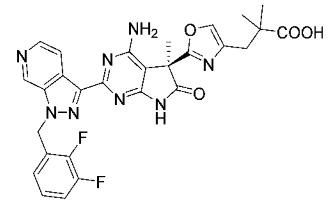 |
| 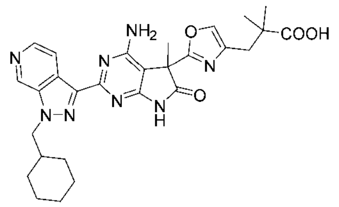 | 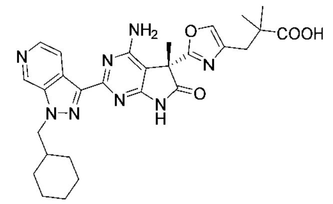 | 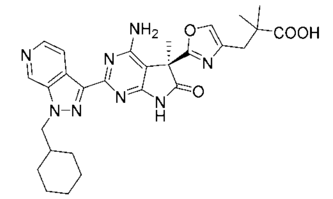 |
| 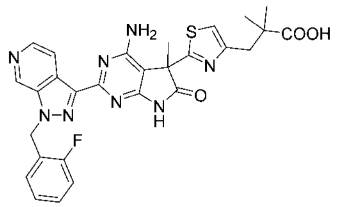 | 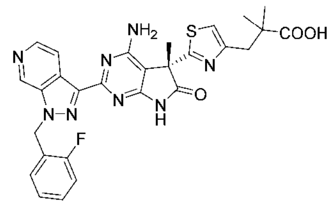 | 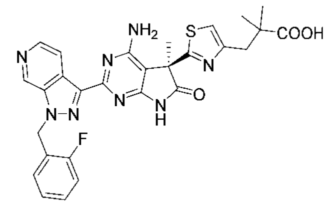 |
| 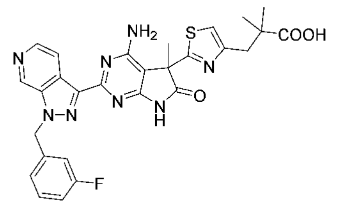 | 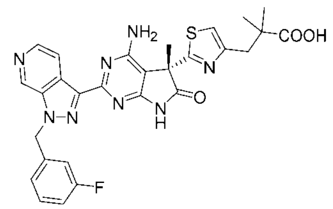 | 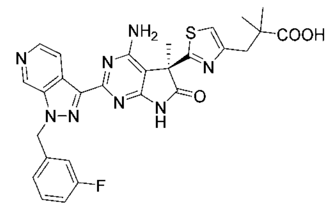 |
| 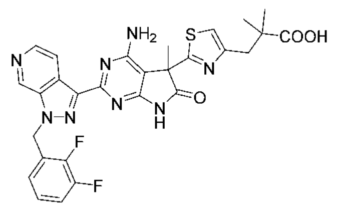 | 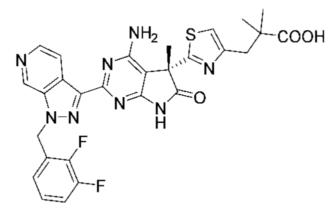 | 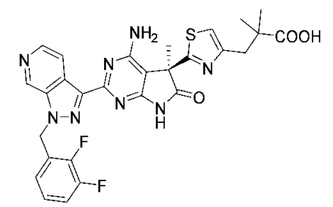 |

| | | |
|---|---|---|
| 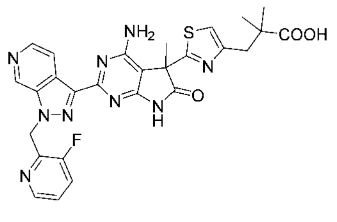 | 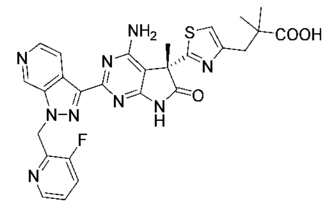 | 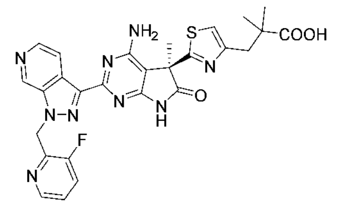 |
| 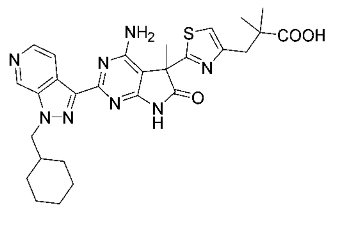 | 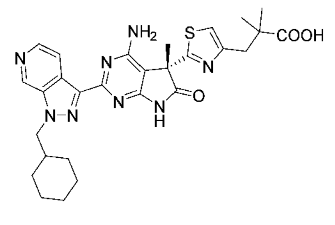 | 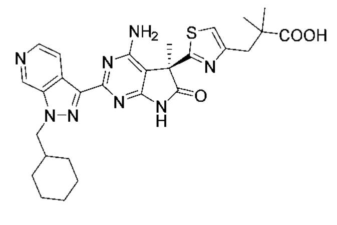 |
| 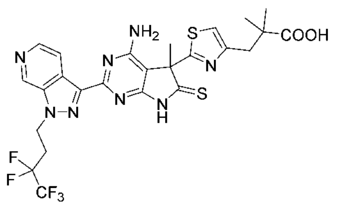 | 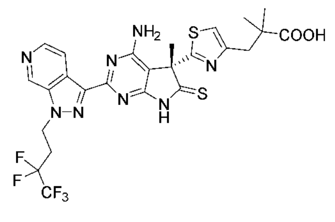 | 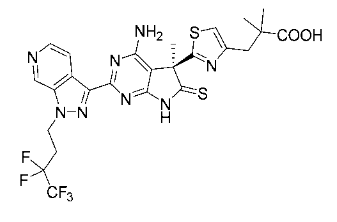 |
| 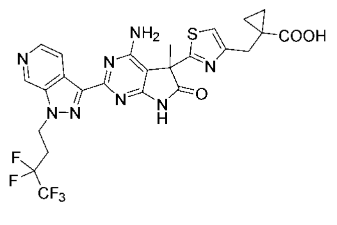 | 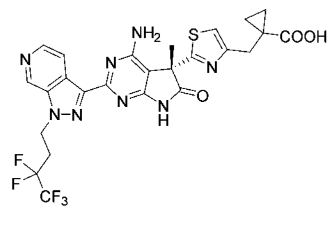 | 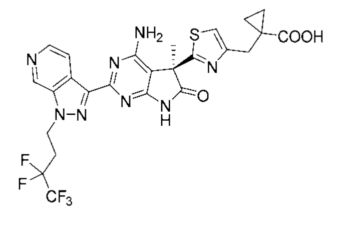 |
| 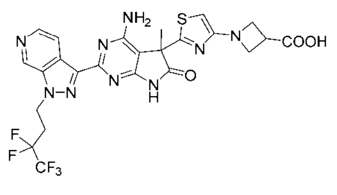 | 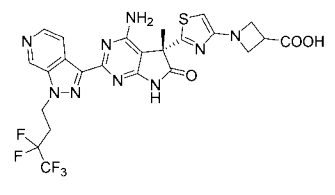 | 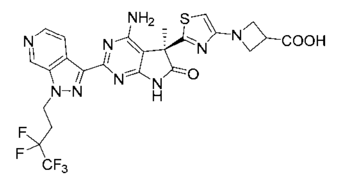 |

The present invention relates to a pharmaceutical composition comprising the above-described compound or the stereoisomer, tautomer, deuterated compound, solvate, prodrug, metabolite, pharmaceutically acceptable salt or co-crystal thereof, and a pharmaceutically acceptable carrier.

The present invention relates to the use of the above-described compound or the stereoisomer, tautomer, deuterated compound, solvate, prodrug, metabolite, pharmaceutically acceptable salt or co-crystal thereof or the above-described pharmaceutical composition in the preparation of a drug for treating sGC-related diseases.

The present invention relates to the use of the above-described compound or the stereoisomer, tautomer, deuterated compound, solvate, prodrug, metabolite, pharmaceutically acceptable salt or co-crystal thereof or the above-described pharmaceutical composition in the preparation of a drug for treating cardiovascular diseases, kidney diseases or respiratory diseases, preferably pulmonary arterial hypertension, pulmonary hypertension or chronic obstructive pulmonary disease.

The present invention relates to a pharmaceutical composition or pharmaceutical preparation, comprising a therapeutically effective amount of the compound or the stereoisomer, deuterated compound, solvate, prodrug, metabolite, pharmaceutically acceptable salt or co-crystal thereof according to the present invention, and a pharmaceutically acceptable excipient. The pharmaceutical composition can be in a unit dosage form (the amount of the active pharmaceutical ingredient per unit dosage form is also referred to as the "dosage strength").

The present invention also provides a method for treating a disease in a mammal, wherein the method comprises administering to the mammal a therapeutically effective amount of the compound or the stereoisomer, deuterated compound, solvate, prodrug, metabolite, pharmaceutically acceptable salt or co-crystal thereof or the pharmaceutical composition according to the present invention. In some embodiments, the mammal described in the present invention

comprises human.

The term "effective amount" or "therapeutically effective amount" in the present application refers to a sufficient amount of the compound disclosed in the present application that is administered to ameliorate, to some extent, one or more symptoms of a disease or condition being treated (e.g., a cardiovascular disease). In some embodiments, the outcome is the reduction and/or remission of signs, symptoms or causes of the disease, or any other desired change in the biological system. For example, an "effective amount" in terms of the therapeutic use is an amount of the included compound disclosed in the present application that is required to provide clinically significant reduction of the symptoms of the disease. Examples of the therapeutically effective amount include, but are not limited to 0.01-1500 mg, 0.01-1000 mg, 0.01-800 mg, 0.01-600 mg, 0.1-1500 mg, 0.1-1000 mg, 0.1-800 mg, 0.1-600 mg, 1-1500 mg, 1-1000 mg, 1-800 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 0.01-100 mg, 0.01-50 mg, 0.01-10 mg, 0.01-5 mg, 0.05-10 mg, 0.05-5 mg, 0.1-5 mg, 1-5 mg, 0.1-1 mg, or 0.1-5 mg;

in some embodiments, the pharmaceutical composition comprises, but not limited to 0.01-1500 mg, 1-1500 mg, 1-1000 mg, 1-800 mg, 1-600 mg, 20-400 mg, 25-200 mg, 0.01 mg, 0.05 mg, 0.1 mg, 0.15 mg, 0.2 mg, 0.3 mg, 0.5 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, or 300 mg of the compound or the stereoisomer, deuterated compound, solvate, prodrug, metabolite, pharmaceutically acceptable salt or co-crystal thereof according to the present invention.

A method for treating a disease in a mammal, wherein the method comprises administering to a subject a therapeutically effective amount of the compound or the stereoisomer, deuterated compound, solvate, prodrug, metabolite, pharmaceutically acceptable salt or co-crystal thereof according to the present invention, wherein the therapeutically effective amount is preferably 0.01-1500 mg, and the disease is preferably a cardiovascular disease.

A method for treating a disease in a mammal, wherein the method comprises administering to a subject the compound or the stereoisomer, deuterated compound, solvate, prodrug, metabolite, pharmaceutically acceptable salt or co-crystal thereof according to the present invention at a daily dose of 0.01-1500 mg/day, wherein the daily dose can be a single dose or divided doses. In some embodiments, the daily dose includes but is not limited to 0.01-1500 mg/day, 10-1500 mg/day, 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, 200-400 mg/day, in some embodiments, daily doses include but are not limited to 0.01 mg/day, 0.05 mg/day, 0.1 mg/day, 0.15 mg/day, 0.2 mg/day, 0.3 mg/day, 0.5 mg/day, 1 mg/day, 2 mg/day, 5 mg/day, 10 mg/day, 20 mg/day, 25 mg/day, 50 mg/day, 100 mg/day, 125 mg/day, 150 mg/day, 200 mg/day, 400 mg/day, 600 mg/day, or 800 mg/day.

The present invention relates to a kit which can comprise a composition in single-dose or multi-dose form, wherein the kit comprises the compound or the stereoisomer, deuterated compound, solvate, prodrug, metabolite, pharmaceutically acceptable salt or co-crystal thereof according to the present invention, and the amount of the compound or the stereoisomer, deuterated compound, solvate, prodrug, metabolite, pharmaceutically acceptable salt or co-crystal thereof according to the present invention is the same as the amount in the above-described pharmaceutical composition.

In the present invention, the amount of the compound or the stereoisomer, deuterated compound, solvate, prodrug, metabolite, pharmaceutically acceptable salt or co-crystal thereof according to the present invention is calculated in the form of a free base in each case.

The term "dosage strength" refers to the weight of the active pharmaceutical ingredient per unit dosage form, such as a vial or a tablet.

Synthesis method I:

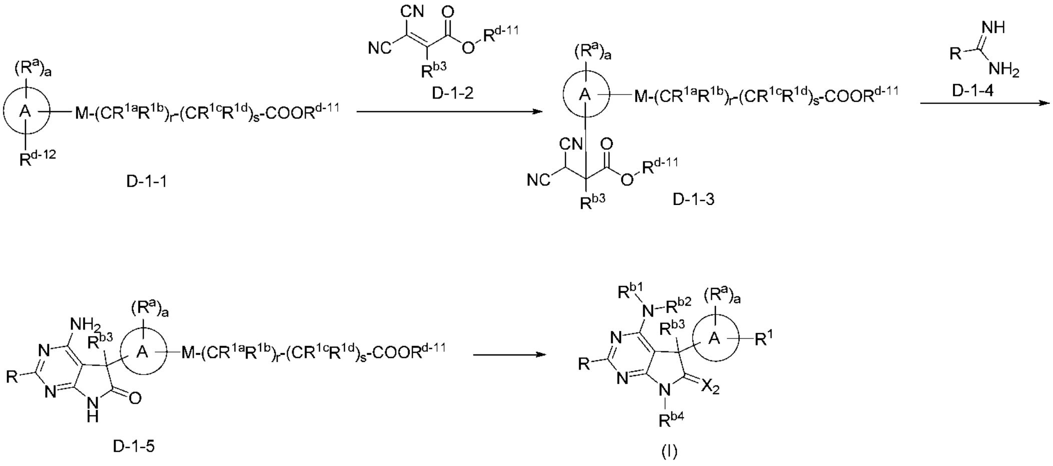


each $R^{d-11}$ is independently selected from $C_{1-6}$ alkyl;
$R^{d-12}$ is selected from halogen, preferably I or Br;
the definitions of the remaining groups are consistent with those in the description of the present invention;
a compound of general formula (D-1-1) and a compound of general formula (D-1-2) are subjected to an addition reaction to obtain a compound of general formula (D-1-3);
the compound of general formula (D-1-3) and a compound of general formula (D-1-4) are subjected to a cyclisation reaction to obtain a compound of general formula (D-1-5);
the compound of general formula (D-1-5) is subjected to a hydrolysis reaction to obtain a compound of general formula (I).

Unless stated to the contrary, the terms used in the description and claims of the present application have the following meanings.

The carbon, hydrogen, oxygen, sulphur, nitrogen, F, Cl, Br and I involved in the groups and compounds of the present invention all comprise their isotopes, and the carbon, hydrogen, oxygen, sulphur or nitrogen involved in the groups and compounds of the present invention is optionally substituted with one or more of their corresponding isotopes, wherein the isotopes of carbon comprise $^{12}C$, $^{13}C$ and $^{14}C$, the isotopes of hydrogen comprise protium (H), deuterium (D, also known as heavy hydrogen) and tritium (T, also known as superheavy hydrogen), the isotopes of oxygen comprise $^{16}O$, $^{17}O$ and $^{18}O$, the isotopes of sulphur comprise $^{32}S$, $^{33}S$, $^{34}S$ and $^{36}S$, the isotopes of nitrogen comprise $^{14}N$ and $^{15}N$, the isotopes of fluorine comprise $^{17}F$ and $^{19}F$, the isotopes of chlorine comprise $^{35}Cl$ and $^{37}Cl$, and the isotopes of bromine comprise $^{79}Br$ and $^{81}Br$.

"Halogen" refers to F, Cl, Br or I.

"Halogen-substituted" refers to a substitution with F, Cl, Br, or I, including but not limited to a substitution with 1 to 10 substituents selected from F, Cl, Br, or I, or a substitution with 1 to 6 substituents selected from F, Cl, Br, or I, or a substitution with 1 to 4 substituents selected from F, Cl, Br, or I. "Halogen-substituted" is referred to simply as "halo".

"Alkyl" refers to a substituted or unsubstituted linear or branched saturated aliphatic hydrocarbon group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl and various branched isomers thereof. The definition of the "alkyl" herein is consistent with this definition. The alkyl can be monovalent, divalent, trivalent or tetravalent.

"Alkylene" refers to a substituted or unsubstituted linear or branched divalent saturated hydrocarbon group, including $-(CH_2)_v-$ (v is an integer from 1 to 10), and examples of alkylene include, but are not limited to, methylene, ethylene, propylene, butylene, etc.

"Cycloalkyl" refers to a substituted or unsubstituted saturated carbocyclic hydrocarbon group, typically having 3 to 10 carbon atoms, and non-limiting examples include cyclopropyl, cyclobutyl, cyclopentyl, cyclohexyl, cycloheptyl, etc. The "cycloalkyl" herein is as defined above. The cycloalkyl can be monovalent, divalent, trivalent or tetravalent.

"Heterocycloalkyl" refers to a substituted or unsubstituted saturated heteroatom-containing cyclic hydrocarbon group, including but not limited to 3 to 10 atoms, 3 to 8 atoms, or 1 to 3 heteroatoms selected from N, O or S. The selectively substituted N and S in the ring of the heterocycloalkyl can be oxidised to various oxidation states. Heterocycloalkyl can be connected to a heteroatom or a carbon atom; heterocycloalkyl can be connected to an aromatic ring or a non-aromatic ring; and heterocycloalkyl can be connected to a bridged ring or a spiro ring. Non-limiting examples include oxiranyl, aziridinyl, oxetanyl, azetidinyl, tetrahydrofuryl, tetrahydro-2H-pyranyl, dioxolanyl, dioxanyl, pyrrolidyl, piperidyl, imidazolidinyl,

oxazolidinyl, oxazinanyl, morpholinyl, hexahydropyrimidyl or piperazinyl. Heterocycloalkyl can be monovalent, divalent, trivalent or tetravalent.

"Alkenyl" refers to a substituted or unsubstituted linear or branched unsaturated hydrocarbon group, having at least 1, usually 1, 2 or 3 carbon-carbon double bonds, with a main chain including but not limited to 2 to 10, 2 to 6, or 2 to 4 carbon atoms. Examples of alkenyl include, but are not limited to, ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. The definition of the alkenyl herein is consistent with this definition. The alkenyl can be monovalent, divalent, trivalent or tetravalent.

"Alkynyl" refers to a substituted or unsubstituted linear or branched unsaturated hydrocarbon group, having at least 1, usually 1, 2 or 3 carbon-carbon triple bonds, with a main chain including 2 to 10 carbon atoms, including but not limited to a main chain including 2 to 6 carbon atoms, or a main chain including 2 to 4 carbon atoms. Examples of alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, etc. The alkynyl can be monovalent, divalent, trivalent or tetravalent.

"Propynyl" refers to 1-propynyl or 2-propynyl.

"Alkoxy" refers to substituted or unsubstituted -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocyclic ring" refers to a substituted or unsubstituted saturated or unsaturated aromatic ring or non-aromatic ring, wherein the aromatic ring or non-aromatic ring can be a 3- to 8-membered monocyclic ring, 4- to 12-membered bicyclic ring, or 10- to 15-membered tricyclic ring system. Carbocyclyl can be connected to an aromatic ring or non-aromatic ring, wherein the aromatic ring or non-aromatic ring is optionally a monocyclic ring, a bridged ring, or a spiro ring. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring,

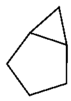, 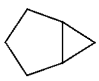, 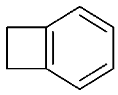,

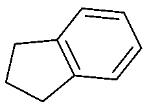 or 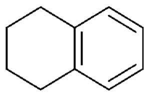.

The "carbocyclyl" or "carbocyclic ring" can be monovalent, divalent, trivalent or tetravalent.

"Heterocyclyl" or "heterocyclic ring" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system, and contains one or more (including but not limited to 2, 3, 4 or 5) heteroatoms selected from N, O or S. The selectively substituted N and S in the ring of the heterocyclyl can be oxidised to various oxidation states. Heterocyclyl can be connected to a heteroatom or a carbon atom; heterocyclyl can be connected to an aromatic ring or a non-aromatic ring; and heterocyclyl can be connected to a bridged ring or a spiro ring. Non-limiting examples include oxiranyl, aziridinyl, oxetanyl, azetidinyl, 1,3-dioxolanyl, 1,4-dioxolanyl, 1,3-dioxanyl, azepanyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzoimidazolyl, benzothiazolyl, benzoxazolyl, benzopyridyl, benzopyrimidyl, benzopyrazinyl, piperazinyl, azabicyclo[3.2.1]octanyl, azabicyclo [5.2.0]nonanyl, oxatricyclo[5.3.1.1]dodecyl, azaadamantyl, oxaspiro[3.3]heptanyl,

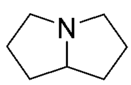,

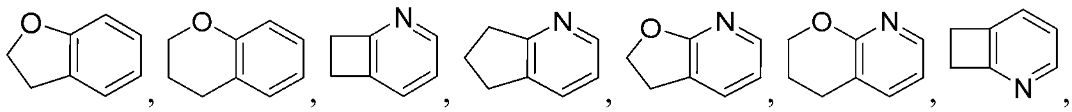

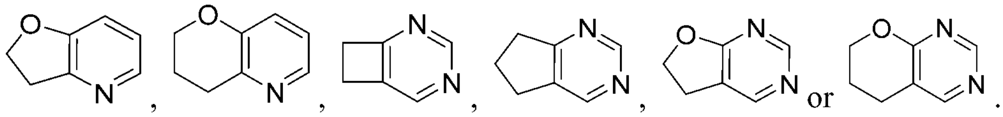

The "heterocyclyl" or "heterocyclic ring" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and may optionally contain 1 to 5 heteroatoms selected from N, O or $S(=O)_n$.

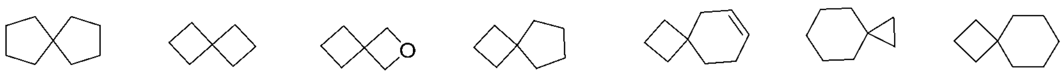

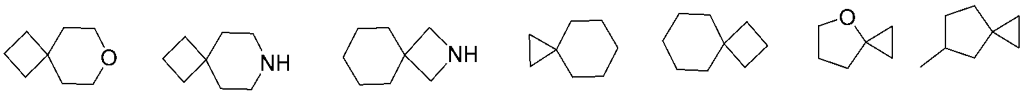

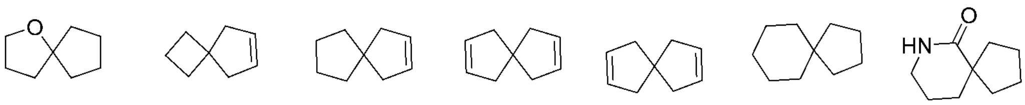

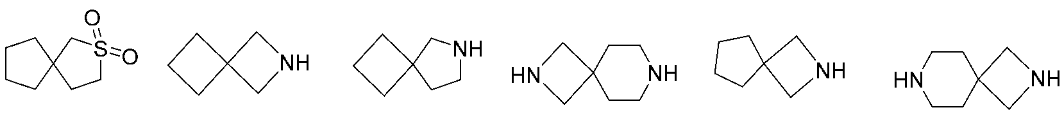

The "spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, $S(=O)_n$ or O, wherein n is 0, 1 or 2). The number of ring atoms in the fused ring system includes, but is not limited to 5 to 20, 5 to 14, 5 to 12, or 5 to 10. Non-limiting examples include:

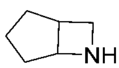 ,

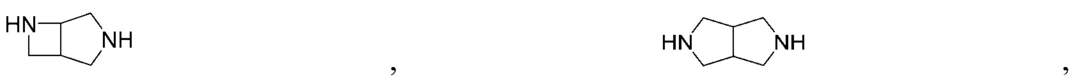

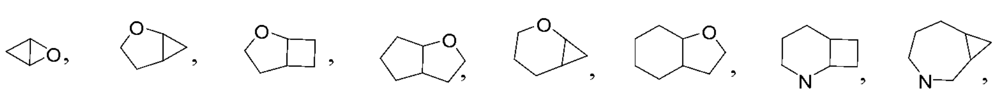

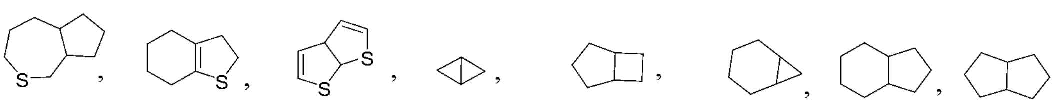

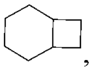, 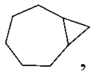, 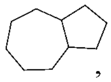, 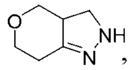, 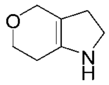, 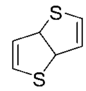 or 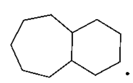.

The "fused ring" or "fused ring group" can be monovalent, divalent, trivalent or tetravalent.

"Bridged ring" or "bridged ring group" refers to a substituted or unsubstituted polycyclic group containing any two rings sharing two atoms that are not directly connected, and may contain 0 or more double bonds. Any ring in the bridged ring system may contain 0 to 5 groups selected from heteroatoms or groups containing heteroatoms (including but not limited to N, S(=O)n or O, wherein n is 0, 1 or 2). The number of ring atoms includes, but is not limited to 5 to 20, 5 to 14, 5 to 12, or 5 to 10. Non-limiting examples include

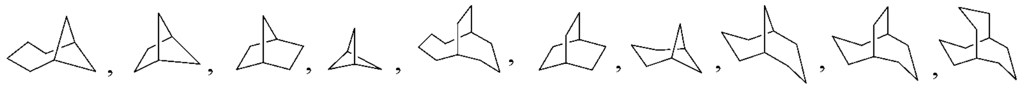

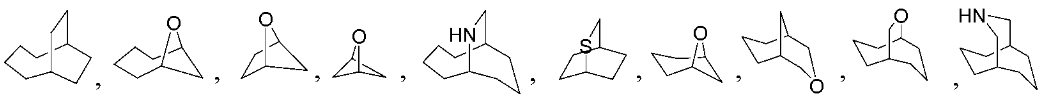

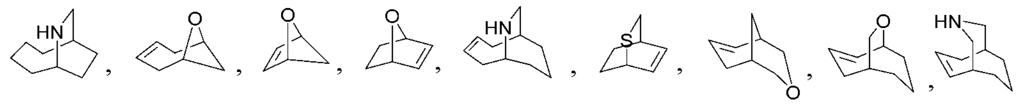

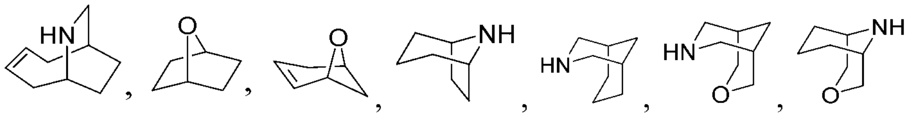

cubane and adamantane. The "bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms.

"Carbo-fused ring", "fused ring carbocyclyl", "fused carbocyclyl" or "carbo-fused ring group" refers to a "fused ring" with a ring system consisting only of carbon atoms.

"Carbo-bridged ring", "bridged ring carbocyclyl", "bridged carbocyclyl" or "carbo-bridged ring group" refers to a "bridged ring" with a ring system consisting only of carbon atoms.

"Mono-heterocyclic ring", "monocyclic heterocyclyl" or "mono-heterocyclyl" refers to "heterocyclyl" or "heterocyclic ring" with a monocyclic system.

"Fused-heterocyclic ring", "fused-heterocyclic ring group", "fused ring heterocyclyl" or "fused-heterocyclic ring group" refers to a "fused ring" containing a heteroatom.

"Spiro-heterocyclic ring", "spiro-heterocyclyl", "spiro ring heterocyclyl" or "spiro-heterocyclic ring group" refers to a "spiro ring" containing a heteroatom.

"Bridged-heterocyclic ring", "bridged-heterocyclyl", "bridged ring heterocyclyl" or "bridged-heterocyclic ring group" refers to a "bridged ring" containing a heteroatom.

"Aryl" or "aromatic ring" refers to a substituted or unsubstituted aromatic hydrocarbon group with a monocyclic ring or a fused ring, wherein the number of ring atoms in the aromatic ring includes, but is not limited to 6 to 18, 6 to 12, or 6 to 10 carbon atoms. The aryl ring can be fused to a saturated or unsaturated carbocyclic ring or heterocyclic ring, wherein the ring connected to the parent structure is the aryl ring. Non-limiting examples include a benzene ring, a naphthalene ring, or

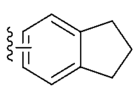, 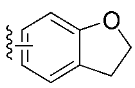, 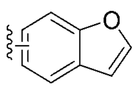, 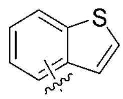, 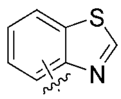,

and "aryl" or "aromatic ring" can be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbon group containing 1 to 5 heteroatoms or groups containing heteroatoms (including but not limited to N, O or S(=O)n, wherein n is 0, 1 or 2), wherein the number of ring atoms in the heteroaromatic ring includes, but is not limited to, 5 to 15, 5 to 10, or 5

to 6. Non-limiting examples of heteroaryl include, but are not limited to pyridyl, furyl, thienyl, pyridyl, pyranyl, N-alkylpyrrolyl, pyrimidyl, pyrazinyl, pyridazinyl, imidazolyl, benzopyrazole, benzoimidazole, benzopyridine, pyrrolopyridine, etc. The heteroaryl ring can be fused to a saturated or unsaturated carbocyclic ring or heterocyclic ring, wherein the ring connected to the parent structure is the heteroaryl ring. Non-limiting examples include

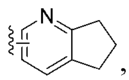, 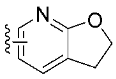, 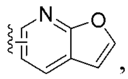, 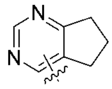, 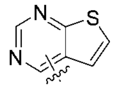, 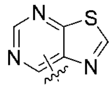, 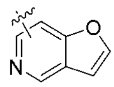

and

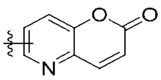.

The definition of the heteroaryl herein is consistent with this definition. The heteroaryl can be monovalent, divalent, trivalent or tetravalent. When divalent, trivalent or tetravalent, the point of connection is on the heteroaryl ring.

"Substitution" or "substituted" refers to a substitution with 1 or more (including, but not limited to 2, 3, 4 or 5) substituents including, but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, bridged ring group, spiro ring group, fused ring group, hydroxyalkyl, =O, carbonyl, aldehyde, carboxylic acid, carboxylate, $-(CH_2)_m-C(=O)-R^a$, $-O-(CH_2)_m-C(=O)-R^a$, $-(CH2)_m-C(=O)-NR^bR^c$, $-(CH_2)_mS(=O)_nR^a$, $-(CH2)_m$-alkenyl-$R^a$, $OR^d$ or $-(CH_2)_m$-alkynyl-$R^a$ (wherein m and n are 0, 1 or 2), arylthio, thiocarbonyl, silyl, $-NR^bR^c$, etc., wherein $R^b$ and $R^c$ are independently selected from H, hydroxyl, amino, carbonyl, alkyl, alkoxy, cycloalkyl, heterocyclyl, aryl, heteroaryl, sulphonyl, or trifluoromethylsulphonyl. Alternatively, $R^b$ and $R^c$ can form a five- or six-membered cycloalkyl or heterocyclyl. $R^a$ and $R^d$ are each independently selected from aryl, heteroaryl, alkyl, alkoxy, cycloalkyl, heterocyclyl, carbonyl, ester group, bridged ring group, spiro ring group or bicyclic ring group.

"Containing 1 to 5 heteroatoms selected from O, S and N" means containing 1, 2, 3, 4 or 5 heteroatoms selected from O, S and N.

"Substituted with 1 to X substituents selected from ..." refers to a substitution with 1, 2, 3 ... X substituents, wherein X is any integer from 1 to 10. For example, "substituted with 1 to 4 $R^k$" refers to a substitution with 1, 2, 3 or 4 $R^k$. For example, "substituted with 1 to 5 substituents selected from ..." refers to a substitution with 1, 2, 3, 4 or 5 substituents selected from ... For example, "bridged-heterocyclic ring is optionally substituted with 1 to 4 substituents selected from D or F" means that the bridged-heterocyclic ring is optionally substituted with 1, 2, 3 or 4 substituents selected from D or F.

An X- to Y-membered ring ($3 \leq X < Y$, and Y is selected from any integer between 4 and 12) includes X, X+1-, X+2-, X+3-, X+4-,...to Y-membered rings. Rings include a heterocyclic ring, a carbocyclic ring, an aromatic ring, aryl, heteroaryl, cycloalkyl, a mono-heterocyclic ring, a fused-heterocyclic ring, a spiro-heterocyclic ring, or a bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound of the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Carrier" refers to a material that does not cause significant irritation to an organism and does not eliminate the biological activity and characteristics of a compound administered.

"Co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

"Animal" is meant to include mammals, such as humans, companion animals, zoo animals, and domestic animals, preferably humans, horses, or dogs.

"Stereoisomer" refers to an isomer produced as a result of different spatial arrangement of atoms in molecules, including cis-trans isomers, enantiomers, diastereomers and conformational isomers.

"Tautomer" refers to a functional group isomer produced by the rapid movement of an atom in two positions in a molecule, such as keto-enol isomerisation and amide-imino alcohol isomerisation.

Detailed Description of Embodiments

The technical solutions of the present invention will be described in detail by the following examples, but the scope of protection of the present invention includes but is not limited thereto.

The structures of the compounds are determined by nuclear magnetic resonance (NMR) or (and) mass spectrometry (MS). The NMR shift (δ) is given in the unit of $10^{-6}$ (ppm). NMR is determined with NMR instruments (Bruker Avance III 400 and Bruker Avance 300); the solvent for determination is deuterated dimethylsulphoxide (DMSO-$d_6$), deuterated chloroform ($CDCl_3$) or deuterated methanol ($CD_3OD$); and the internal standard is tetramethylsilane (TMS).

MS is determined with Agilent 6120B (ESI) and Agilent 6120B (APCI).

HPLC is determined with Agilent 1260DAD high pressure liquid chromatograph (Zorbax SB-C18 100 × 4.6 mm, 3.5 μM).

Yantai Huanghai $HSGF_{254}$ or Qingdao $GF_{254}$ silica gel plate is used as a thin layer chromatography silica plate; and the silica gel plate for the thin layer chromatography (TLC) is of the specification of 0.15 mm-0.20 mm, and the specification when separating and purifying a product by thin layer chromatography is 0.4 mm-0.5 mm.

Yantai Huanghai silica gel of 200-300 mesh is generally used as a carrier in column chromatography.

**Example 1**:

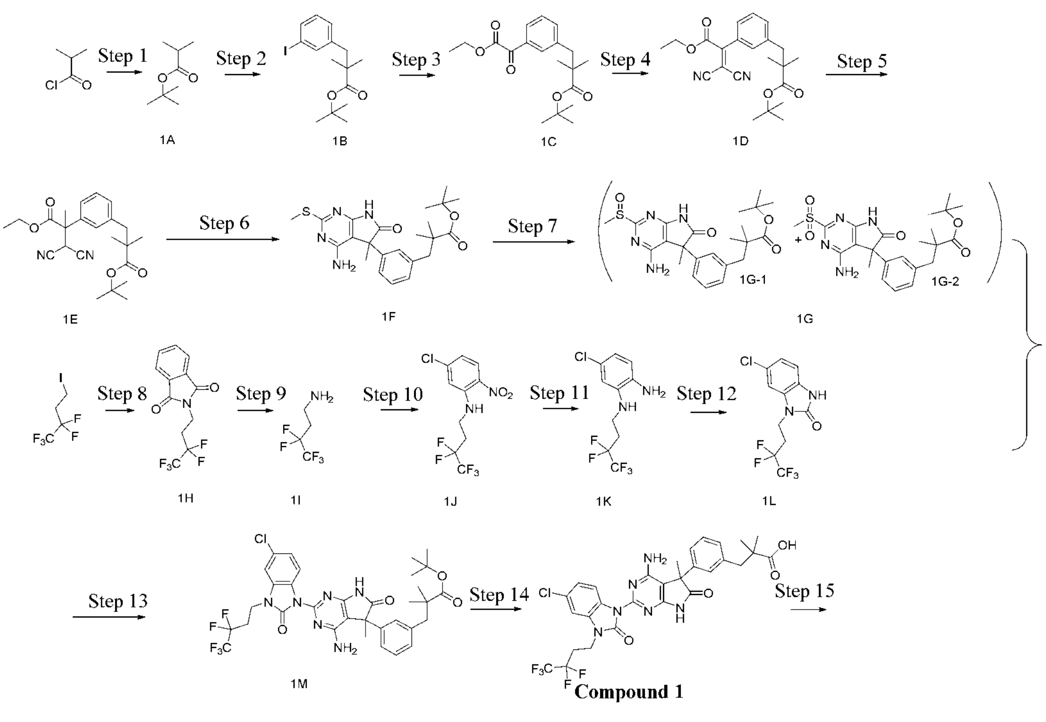


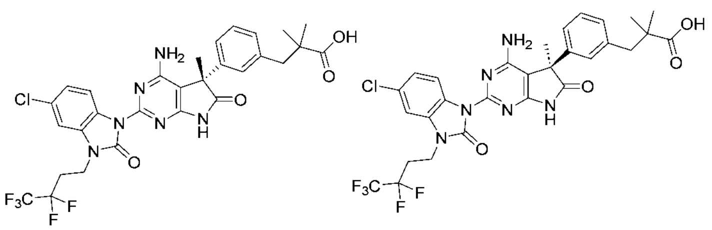

**Compound 1-1 and 1-2**

Step 1: Preparation of **1A**

Compound isobutyryl chloride (7 g, 65.67 mmol) was dissolved in THF (40 mL), and the mixture was cooled to -70°C. A solution of potassium tert-butoxide in tetrahydrofuran (72 mL, 1 *N*) was slowly added. After the addition, the mixture was naturally warmed to room temperature and stirred for 10 min, and then 100 mL of water and 50 mL of methyl tert-butyl ether were added sequentially. The organic layer was separated, washed with a saturated aqueous sodium chloride solution (50 mL), dried over anhydrous sodium sulphate and concentrated under reduced pressure. The residue was directly used in the next step.

Step 2: Preparation of **1B**

Compound **1A** (7.77 g, 53.89 mmol) was dissolved in THF (80 mL), and the mixture was purged three times with nitrogen and cooled to -70°C. A solution of LDA in tetrahydrofuran (80 mL, 1 N) was slowly added dropwise. After the addition, the mixture was further stirred for 30 min, warmed to 0°C and then stirred for 10 min. The mixture was cooled to -70°C, and a solution of 3-iodobenzyl bromide (16 g, 53.89 mmol) in tetrahydrofuran (20 mL) was slowly added. After the addition, the mixture was further stirred for 1 h and naturally warmed to room temperature. The reaction was quenched with 20 mL of a saturated aqueous ammonium chloride solution. 100 mL of water and 100 mL of methyl tert-butyl ether were added, and the mixture was stirred for layer separation. The organic layer was dried over anhydrous sodium sulphate and then concentrated under reduced pressure. The residue was purified by column chromatography (ethyl acetate/petroleum ether (V/V)=0/100-5/100) to obtain compound **1B** (6.3 g, yield: 32%).

$^1H$ NMR (400 MHz, $CDCl_3$) δ 7.56-7.51 (m, 2H), 7.14-7.09 (m, 1H), 7.02 - 6.95 (m, 1H), 2.75 (s, 2H), 1.45 (s, 9H), 1.13 (s, 6H).

Step 3: Preparation of **1C**

Compound **1B** (5.8 g, 16.10 mmol) was dissolved in THF (20 mL), and the mixture was purged three times with nitrogen and cooled to -70°C. A solution of isopropyl magnesium chloride-lithium chloride in tetrahydrofuran (24 mL, 1 *N*) was slowly added dropwise. After the addition, the mixture was warmed to 0°C, stirred for 10 min and then cooled to -70°C. Diethyl oxalate (3.53 g, 24.15 mmol) was slowly added dropwise. After the addition, the mixture was naturally warmed to room temperature and stirred for 10 min. The reaction was quenched with 10 mL of a saturated aqueous ammonium chloride solution and extracted with 50 mL of methyl tert-butyl ether and 50 mL of water. The organic layer was washed with a saturated aqueous sodium chloride solution (50 mL x 1), dried over anhydrous sodium sulphate and concentrated under reduced pressure. The residue was purified by column chromatography (ethyl acetate/petroleum ether (V/V) = 0/100-8/100) to obtain compound **1C** (4.2 g, yield: 78%).

Step 4: Preparation of **1D**

Compound **1C** (2.4 g, 7.18 mmol) was dissolved in a mixed solvent of ethanol (30 mL) and water (1 mL). Malononitrile (1.90 g, 28.79 mmol) and β-alanine (0.064 g, 0.72 mmol) were added sequentially, and the mixture was stirred overnight at room temperature and concentrated under reduced pressure. The residue was directly purified by column chromatography (ethyl acetate/petroleum ether (V/V) = 0/100-10/100) to obtain compound **1D** (2.56 g, yield: 93%).

Step 5: Preparation of **1E**

Compound **1D** (2.56 g, 6.69 mmol) was dissolved in THF (15 mL). Under nitrogen atmosphere, the mixture was cooled to -70°C, and a solution of methylmagnesium chloride in tetrahydrofuran (2.7 mL, 3 *N*) was slowly added dropwise. After the addition, the mixture was further stirred at this temperature for 10 min. The reaction was quenched with 10 ml of 1 *N* hydrochloric acid. 30 mL of ethyl acetate and 20 mL of water were added, and the mixture was stirred for layer separation. The organic phase was washed with a saturated aqueous sodium chloride solution (20 mL x 1), dried over anhydrous sodium sulphate and concentrated under reduced pressure. The residue was purified by column chromatography (ethyl acetate/petroleum ether (V/V) = 0/100-10/100) to obtain compound **1E** (2.13 g, yield: 79%).

Step 6: Preparation of **1F**

Compound **1E** (0.54 g, 1.36 mmol), S-methylisothiourea sulphate (0.26 g, 1.36 mmol, CAS: 867-44-7) and potassium bicarbonate (0.54 g, 5.39 mmol) were mixed and dissolved in tert-butanol (5 mL). The mixture was warmed to 80°C, stirred overnight and cooled to room temperature. 20 mL of ethyl acetate was added, and the mixture was washed with 10 mL of a saturated aqueous sodium chloride solution. The organic layer was dried over anhydrous sodium sulphate,

filtered and concentrated. The residue was purified by column chromatography (ethyl acetate/petroleum ether (V/V) = 1/5-1/2) to obtain compound **1F** (0.45 g, yield: 75%).

LCMS m/z = 443.2 $[M+H]^+$

Step 7: Preparation of **1G**

Compound **1F** (0.44 g, 0.99 mmol) was dissolved in THF (5 mL). m-Chloroperoxybenzoic acid (0.34 g, 1.97 mmol, wt% = 85%) was added at room temperature. After the addition, the mixture was further stirred for 2 h. 10 mL of a saturated aqueous sodium thiosulphate solution was added, and the mixture was stirred for 20 min. 10 mL of a saturated aqueous sodium bicarbonate solution was added, and the mixture was stirred for 10 min and extracted with 20 mL of ethyl acetate. The organic layer was washed with 10 mL of a saturated aqueous sodium chloride solution and then dried over anhydrous sodium sulphate, filtered and concentrated to obtain **1G (a mixture of 1G-1 and 1G-2),** which was directly used in the next step.

LCMS m/z = 457.2 $[M-H]^-$ and LCMS m/z = 473.2 $[M-H]^-$

Step 8: Preparation of **1H**

Compound 1, 1,1,2,2-pentafluoro-4-iodobutane (2 g, 7.29 mmol), phthalimide (1.07 g, 7.29 mmol) and potassium carbonate (1.51 g, 10.94 mmol) were mixed and dissolved in DMF (20 mL). The mixture was warmed to 80°C, stirred for 3 h and cooled to room temperature. 40 mL of MTBE and 40 mL of water were added, and the mixture was stirred for layer separation. The organic layer was washed with a saturated aqueous sodium chloride solution (20 mL x 4), dried over anhydrous sodium sulphate and concentrated under reduced pressure. The residue was purified by silica gel column chromatography (ethyl acetate/petroleum ether (V/V) = 0/10-1/5) to obtain compound **1H** (0.5 g, yield: 23%).

Step 9: Preparation of **1I hydrochloride**

Compound **1H** (0.5 g, 1.71 mmol) was dissolved in ethanol (5 mL). Hydrazine hydrate (0.12 g, 1.98 mmol, wt% = 80%) was added, and the mixture was warmed to 78°C, stirred for 2 h and cooled to room temperature. 10 mL of ethyl acetate was added, and the mixture was filtered. The filter cake was washed with 5 mL of ethyl acetate. The filtrates were combined. A solution of hydrogen chloride in ethyl acetate (1 mL, 4 N) was added, and the mixture was concentrated under reduced pressure to obtain the hydrochloride of compound **1I,** which was directly used in the next step.

LCMS m/z = 164.10 $[M+H]^+$

Step 10: Preparation of **1J**

Compound 4-chloro-2-fluoronitrobenzene (0.33 g, 1.87 mmol) and compound **1I** hydrochloride (0.37 g, 1.87 mmol) were dissolved in DMSO (5 mL). Diisopropylethylamine (0.73 g, 5.61 mmol) was added, and the mixture was warmed to 60°C, stirred for 4 h and cooled to room temperature. 30 mL of MTBE and 30 mL of water were added, and the mixture was stirred for layer separation. The organic layer was washed with a saturated aqueous sodium chloride solution (20 mL x 3), dried over anhydrous sodium sulphate and concentrated under reduced pressure. The residue was purified by silica gel column chromatography (ethyl acetate/petroleum ether (V/V) = 0/10-1/10) to obtain compound **1J** (0.43 g, yield: 72%).

LCMS m/z = 319.1 $[M+H]^+$

Step 11: Preparation of **1K**

Compound **1J** (0.43 g, 1.35 mmol) was dissolved in a mixed solvent of ethanol (6 mL) and water (2 mL). Ammonium chloride (0.73 g, 13.64 mmol) and iron powder (0.76 g, 13.54 mmol) were added sequentially. After the addition, the mixture was warmed to 75°C, stirred for 1.5 h, cooled to room temperature and filtered. The filter cake was washed with 20 mL of dichloromethane. The filtrates were combined and washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 0/10-3/10) to obtain compound **1K** (0.3 g, yield: 77%).

LCMS m/z = 289.10 $[M+H]^+$

Step 12: Preparation of **1L**

Compound **1K** (0.3 g, 1.04 mmol) was dissolved in DCM (5 mL), and diisopropylethylamine (0.41 g, 3.14 mmol)

was added. In an ice bath, triphosgene solids (0.12 g, 0.42 mmol) were added in portions. After the addition, the mixture was naturally warmed to room temperature and stirred for 2 h. 10 mL of a saturated aqueous sodium bicarbonate solution was added, and the mixture was stirred for 10 min and extracted with 20 mL of dichloromethane. The organic layer was washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 1/10-1/2) to obtain compound **1L** (0.2 g, yield: 61%).

LCMS m/z = 315.0 $[M+H]^+$

Step 13: Preparation of **1M**

Compound **1L** (0.2 g, 0.64 mmol), **1G** (0.46 g) and potassium carbonate (0.18 g, 1.29 mmol) were mixed and dissolved in DMF (3 mL). The mixture was warmed to 100°C and stirred for 16 h, further warmed to 120°C and stirred for 24 h, and cooled to room temperature. 20 mL of ethyl acetate and 20 mL of water were added, and the mixture was stirred for layer separation. The organic layer was washed with a saturated aqueous sodium chloride solution (10 mL x 3), dried over anhydrous sodium sulphate and concentrated under reduced pressure. The residue was purified by silica gel column chromatography (dichloromethane/methanol (V/V) = 100/0-100/3). The resulting residue was further purified by reversed-phase column chromatography (acetonitrile/water (containing 0.1% trifluoroacetic acid) (V/V) = 0/100-60/40) to obtain compound **1M** (55 mg, yield: 12%).

LCMS m/z = 709.70 $[M+H]^+$

Step 14: Preparation of **compound 1**

Compound **1M** (0.052 g, 0.073 mmol) was placed in a 50 mL single-necked flask. TFA (2 mL) was added, and the mixture was stirred at room temperature for 2 h and concentrated under reduced pressure. The residue was purified by column chromatography (dichloromethane/methanol (V/V) = 100/0-100/4) to obtain **compound 1** (34 mg, yield: 71%).

Step 15: **Compounds 1-1 and 1-2**

**Compound 1** (30 mg) was subjected to chiral separation and purification. Preparative conditions: instrument: Waters 150 ap; chromatographic column: ChoralPak IC 19*250 5 um; column temperature: 35°C; mobile phase A: $CO_2$, and mobile phase B: isopropanol; gradient: B 24%; back pressure: 95 bar; cycle: 3.6 min; detection wavelength: 210 nm; flow rate: 40 ml/min.

Analysis conditions: instrument: Shimadzu LC-20AD; chromatographic column: CHIRALPAK AD-H 4.6*250 mm 5 um; mobile phase A: n-hexane, and mobile phase B: ethanol; flow rate: 1 ml/min; column temperature: 35°C; detection wavelength: 210 nm; injection volume: 10 μL; run time: 20 min; isocratic elution: n-hexane: ethanol = (80 : 20).

After preparative separation, the components with the same retention time were combined and concentrated under reduced pressure to obtain **compound 1-1** (10 mg) and **compound 1-2** (10 mg).

**Compound 1-1:** retention time under analysis conditions: 5.1 min, LCMS m/z = 653.1 $[M+H]^+$

$^1H$ NMR (400 MHz, DMSO-$d_6$) δ 7.83 (d, 1H), 7.53 (d, 1H), 7.28 - 7.21 (m, 1H), 7.16 - 7.03 (m, 4H), 6.78 - 6.48 (m, 2H), 4.24 (t, 2H), 2.85 - 2.65 (m, 4H), 1.78 (s, 3H), 1.07 (s, 3H), 1.03 (s, 3H).

**Compound 1-2:** retention time under analysis conditions: 7.5 min, LCMS m/z = 653.1 $[M+H]^+$

$^1H$ NMR (400 MHz, DMSO-$d_6$) δ 7.83 (d, 1H), 7.53 (d, 1H), 7.28 - 7.21 (m, 1H), 7.16 - 7.03 (m, 4H), 6.78 - 6.48 (m, 2H), 4.24 (t, 2H), 2.85 - 2.65 (m, 4H), 1.78 (s, 3H), 1.07 (s, 3H), 1.03 (s, 3H).

**Example 2:**

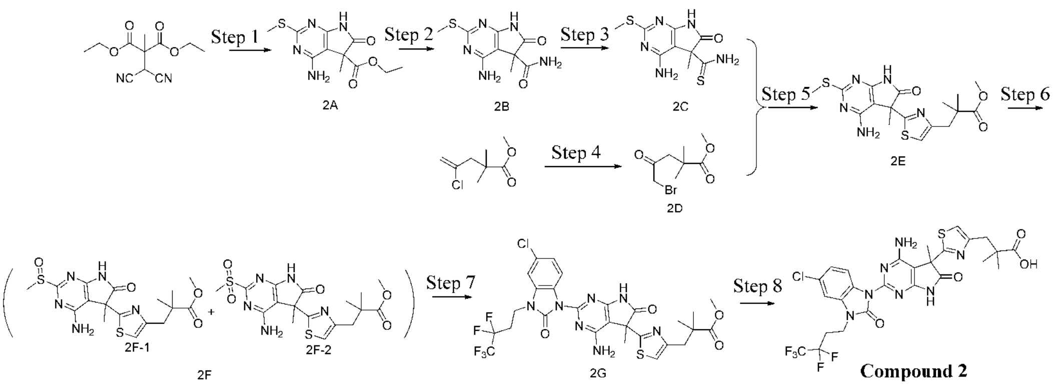


2A 2B 2C 2D 2E 2F-1 2F-2 2F 2G Compound 2

Step 1: Preparation of **2A**

Compound diethyl 2-(dicyanomethyl)-2-methylmalonate (CAS: 1350855-72-9) (1 g, 4.20 mmol), S-methylisothiourea sulphate (0.79 g, 4.2 mmol) and potassium bicarbonate (1.68 g, 16.78 mmol) were mixed and dissolved in tert-butanol (10 mL). The mixture was warmed to 80°C, stirred overnight and cooled to room temperature. 30 mL of ethyl acetate was added, and the mixture was washed with 20 mL of a saturated aqueous sodium chloride solution. The organic layer was dried over anhydrous sodium sulphate, filtered and concentrated. The residue was purified by column chromatography (ethyl acetate/petroleum ether (V/V) = 1/5-1/2) to obtain compound **2A** (0.9 g, yield: 76%).

LCMS m/z = 283.20 $[M+H]^+$

Step 2: Preparation of **2B**

Compound **2A** (0.9 g, 3.19 mmol) was dissolved in methanol (5 mL). A solution of ammonia in methanol (10 mL, 7 *N*) was added, and the mixture was warmed to 40°C, stirred overnight, cooled to room temperature and concentrated under reduced pressure to obtain compound **2B,** which was directly used in the next step.

LCMS m/z = 254.1 $[M+H]^+$

Step 3: Preparation of **2C**

Compound **2B** (0.85 g, 3.36 mmol) and Lawson's reagent (1.49 g, 3.70 mmol) were mixed and dissolved in toluene (15 mL). After the addition, the mixture was purged three times with nitrogen, warmed to 100°C and stirred overnight. The resulting mixture was cooled to room temperature and concentrated under reduced pressure. The residue was purified by silica gel column chromatography (ethyl acetate/petroleum ether (V/V) = 1/5-1/1) to obtain compound 2C (0.52 g, yield: 57%).

Step 4: Preparation of **2D**

Compound methyl 4-chloro-2,2-dimethylpent-4-enoate (2 g, 11.29 mmol, CAS: 86799-85-1) was dissolved in a mixed solvent of ethanol (10 mL) and water (10 mL). In an ice bath, NBS (2.21 g, 12.42 mmol) was added in portions. After the addition, the mixture was naturally warmed to room temperature and stirred for 1 h. The reaction was quenched by slowly adding 10 mL of a saturated aqueous sodium bicarbonate solution and extracted with 20 mL of MTBE. The organic phase was further washed with a saturated aqueous sodium bicarbonate solution (10 mL x 2), dried over anhydrous sodium sulphate and concentrated under reduced pressure. The residue was directly used in the next step.

Step 5: Preparation of **2E**

2C (0.5 g, 1.86 mmol) and **2D** (0.66 g, 2.79 mmol) were mixed and dissolved in ethanol (10 mL). The mixture was warmed to 60°C, stirred overnight and cooled to room temperature. 30 mL of ethyl acetate and 20 mL of a saturated aqueous sodium bicarbonate solution were added, and the mixture was stirred for 10 min. The organic layer was separated, washed with 10 mL of a saturated aqueous sodium chloride solution, dried over anhydrous sodium sulphate and concentrated under reduced pressure. The residue was purified by silica gel column chromatography (ethyl acetate/petroleum ether (V/V) = 1/10-1/2) to obtain compound **2E** (0.55 g, yield: 73%).

LCMS m/z = 408.70 $[M+H]^+$

Step 6: Preparation of **2F**

Compound **2E** (0.55 g, 1.35 mmol) was dissolved in tetrahydrofuran (5 mL), and m-chloroperoxybenzoic acid (0.47 g, 2.72 mmol) was added at room temperature. After the addition, the mixture was further stirred for 2 h. 10 mL of a saturated aqueous sodium thiosulphate solution was added, and the mixture was stirred for 20 min. 10 mL of a saturated aqueous sodium bicarbonate solution was added, and the mixture was stirred for 10 min. 20 mL of ethyl acetate was added, and the mixture was stirred for layer separation. The organic layer was washed with 10 mL of a saturated aqueous sodium chloride solution and then dried over anhydrous sodium sulphate, filtered and concentrated to obtain **2F (a mixture of 2F-1 and 2F-2),** which was directly used in the next step.

LCMS m/z = 424.1 $[M+H]^+$ and LCMS m/z = 440.1 $[M+H]^+$

Step 7: Preparation of**2G**

Compound **1L** (0.12 g, 0.38 mmol), **2F** (0.19 g) and potassium carbonate (0.10 g, 0.76 mmol) were mixed and dissolved in DMF (3 mL). The mixture was warmed to 100°C, stirred for 16 h and cooled to room temperature. 20 mL of ethyl acetate and 20 mL of water were added, and the mixture was stirred for layer separation. The organic layer was washed with a saturated aqueous sodium chloride solution (10 mL x 3), dried over anhydrous sodium sulphate and concentrated under reduced pressure. The residue was purified by silica gel column chromatography (dichloromethane/methanol (V/V) = 100/0-100/3) to obtain compound **2G** (54 mg, yield: 21%).

LCMS m/z = 674.1 $[M+H]^+$

Step 8: Preparation of **compound 2**

Compound **2G** (0.054 g, 0.080 mmol) was dissolved in a mixed solvent of 1,4-dioxane (3 mL) and water (1.5 mL). Lithium hydroxide (0.019 g, 0.79 mmol) was added. The mixture was warmed to 60°C, stirred for 1 h and cooled to room temperature. The reaction solution was adjusted to pH = 3-4 by adding 1 *N* hydrochloric acid. 20 mL of ethyl acetate was added, and the organic phase was washed with 10 mL of a saturated aqueous sodium chloride solution, dried over anhydrous sodium sulphate and concentrated under reduced pressure. The residue was purified by column chromatography (acetonitrile/water (containing 0.1% trifluoroacetic acid) (V/V) = 0/100-60/40) to obtain the trifluoroacetate of **compound 2** (31 mg).

LCMS m/z = 660.0 $[M+H]^+$

$^1$H NMR (400 MHz, DMSO-$d_6$) δ 11.56 (s, 1H), 7.81 (d, 1H), 7.52 (d, 1H), 7.25 (s, 1H), 7.13 (dd, 1H), 7.09 - 6.96 (m, 2H), 4.22 (t, 2H), 2.93 (s, 2H), 2.81 - 2.65 (m, 2H), 1.81 (s, 3H), 1.14 - 1.04 (m, 6H).

Preparation of **compound 2-1 and compound 2-2:**

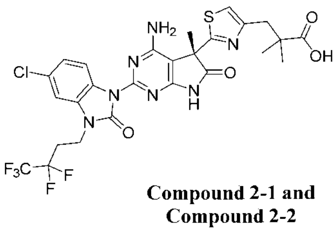
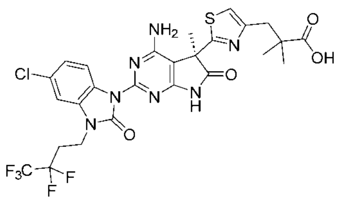

**Compound 2-1 and Compound 2-2**

The trifluoroacetate of **compound 2** (31 mg) was subjected to chiral separation and purification. Preparative conditions: instrument: SFC Prep 150 AP; chromatographic column: IG (19 mm×250 mm). The sample was dissolved in DMF and filtered with a 0.45 μm filter to prepare a sample solution. Preparative chromatography conditions: mobile phase A: $CO_2$, and mobile phase B: methanol (containing 0.5% aqueous ammonia); isocratic elution, mobile phase B: 30%; flow rate: 52 ml/min.

Analysis conditions: instrument: UPC2; chromatographic column: IG (3 mm×50 mm). The sample was dissolved in methanol and filtered with a 0.45 μm filter to prepare a sample solution. Preparative chromatography conditions: mobile phase A: $CO_2$, and mobile phase B: methanol (containing 0.5% aqueous ammonia); gradient elution: mobile phase B: 10%-40%; time: 5 min; isocratic elution, mobile phase B: 40%; flow rate: 1.5 ml/min.

After preparative separation, the components with the same retention time were combined and concentrated under reduced pressure to obtain **compound 2-1** (10 mg) and **compound 2-2** (15 mg).

**Compound 2-1:** retention time under analysis conditions: 2.3 min, LCMS m/z = 660.0 [M+H]$^+$

**Compound 2-2:** retention time under analysis conditions: 2.7 min, LCMS m/z = 660.0 [M+H]$^+$

**Example 3:**

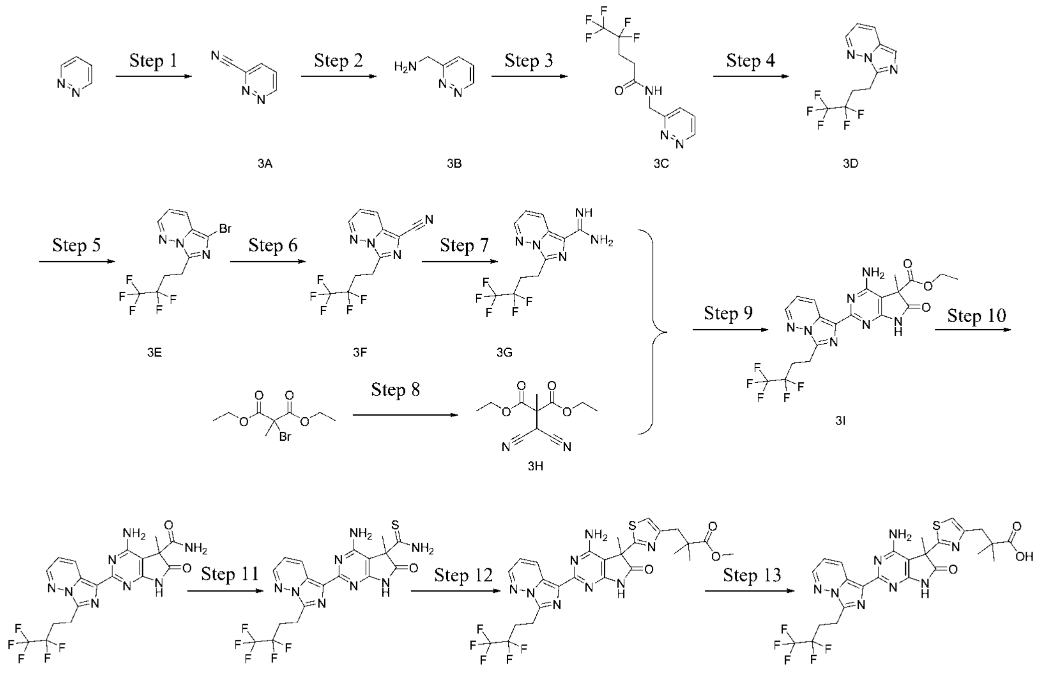


3A 3B 3C 3D

3E 3F 3G

3H

3I

3J 3K 3L **Compound 3**

Step 1: Preparation of**3A**

Pyridazine (3 g, 37.50 mmol) was dissolved in chloroform (60 mL). Trifluoromethanesulphonic anhydride (12.59 g, 44.63 mmol) was slowly added, and the mixture was stirred at room temperature for 1 h. Trimethylsilyl cyanide (15.81 g, 159.26 mmol) was added, and the mixture was warmed to 60°C, reacted for 3 h and cooled. Then, N-methyl morpholine (4.93 g, 48.75 mmol) was added, and the mixture was warmed to 60°C again, reacted overnight and cooled to room temperature. The reaction was quenched with a saturated aqueous sodium bicarbonate solution and extracted twice with DCM. The organic phases were combined, dried over anhydrous sodium sulphate and concentrated under reduced pressure. The residue was purified by column chromatography (ethyl acetate/petroleum ether (V/V) = 0/1-1/13) to obtain compound **3A** (2.5 g, yield: 63%).

LCMS m/z = 106.20 [M+H]$^+$

Step 2: Preparation of **3B hydrochloride**

Compound **3A** (2 g, 19.03 mmol) was dissolved in methanol (10 mL). 10 mL of 6 N hydrochloric acid and palladium on carbon (2.03 g, 1.90 mmol) were added, and the mixture was purged three times with hydrogen and reacted overnight under hydrogen atmosphere. The reaction solution was filtered over celite and washed 3 times with methanol. The filtrate was concentrated under reduced pressure to obtain compound **3B hydrochloride,** which was directly used in the next step.

Step 3: Preparation of**3C**

Compound **3B hydrochloride** (1.1 g, 7.52 mmol) and 4,4,5,5,5-pentafluoropentanoic acid (1.73 g, 9.02 mmol) were dissolved in DMF (20 mL). HATU (5.72 g, 15.04 mmol) was added, and the mixture was stirred at room temperature for 30 min. DIPEA (2.92 g, 22.56 mmol) was added, and the mixture was reacted at room temperature for 1 h and extracted with water and ethyl acetate. The organic phase was washed with a saturated aqueous sodium chloride solution, dried

over anhydrous sodium sulphate and concentrated under reduced pressure. The residue was purified by column chromatography (dichloromethane/methanol (V/V) = 100/0-100/10) to obtain compound **3C** (700 mg, yield: 32%).

Step 4: Preparation of **3D**

Compound **3C** (700 mg, 2.47 mmol) was dissolved in DCE (100 mL). Phosphorus oxychloride (1.31 mL, 14.35 mmol) was added, and the mixture was warmed to reflux, reacted overnight, cooled to room temperature and concentrated under reduced pressure. The residue was carefully and slowly added to water, and the mixture was stirred for 5 min. Ethyl acetate was added, and the mixture was stirred for layer separation. The aqueous layer was treated with a saturated aqueous sodium bicarbonate solution and then extracted three times with ethyl acetate. The organic layers were combined, washed with a saturated aqueous sodium chloride solution, dried over anhydrous sodium sulphate and concentrated under reduced pressure. The residue was directly purified by column chromatography (ethyl acetate/petroleum ether (V/V) = 0/100-20/100) to obtain compound **3D** (350 mg, yield: 53%).

LCMS m/z = 266.30 $[M+H]^+$

Step 5: Preparation of**3E**

Compound **3D** (350 mg, 1.32 mmol) was dissolved in DCM (10 mL). NBS (246.68 mg, 1.39 mmol) was added, and the mixture was reacted at room temperature for 30 min. Water was added, and the mixture was stirred for layer separation. The organic layer was washed with a saturated aqueous sodium chloride solution, dried over anhydrous sodium sulphate and concentrated under reduced pressure. The residue was purified by column chromatography (ethyl acetate/petroleum ether (V/V) = 0/100-50/100) to obtain compound **3E** (430 mg, yield: 94%).

LCMS m/z = 344.30 $[M+H]^+$

Step 6: Preparation of **3F**

Compound **3E** (400 mg, 1.16 mmol) and zinc cyanide (272.46 mg, 2.32 mmol) were added to a 50 mL single-necked flask, and then zinc powder (151.75 mg, 2.32 mmol), 1,1'-bis(diphenylphosphino)ferrocene (385.85 mg, 0.70 mmol), tris(dibenzylidene-BASE acetone)dipalladium (318.67 mg, 0.35 mmol) and N,N-dimethylacetamide (10 mL) were added. Under nitrogen atmosphere, the mixture was warmed to 120°C and stirred for 2 h. The reaction solution was diluted in 50 mL of ethyl acetate, washed 3 times with water and washed once with saturated sodium chloride. The organic phase was dried over anhydrous sodium sulphate and then concentrated under reduced pressure. The residue was subjected to flash column chromatography (mobile phase: petroleum ether/ethyl acetate (V/V) = 2/1) to obtain compound **3F** (320 mg, yield: 95%).

LCMS m/z = 291.1 $[M+H]^+$

Step 7: Preparation of**3G**

In an ice bath, ammonium chloride (275.47 mg, 5.15 mmol) was added to a 50 mL single-necked flask. Toluene (10 mL) was added, and then trimethylaluminium (2.58 mL, 2 M in toluene) was slowly added dropwise. The mixture was warmed to room temperature, stirred and reacted for 3 h. Compound 3F (300 mg, 1.03 mmol) was dissolved in toluene and added dropwise to the reaction solution. The mixture was warmed to 110°C, stirred overnight and cooled to room temperature. In an ice bath, silica gel and 20 mL of methanol were added. The mixture was further stirred for 30 min and subjected to suction filtration. The filter cake was washed 3 times with methanol. The organic phases were combined and concentrated under reduced pressure. The residue was purified by column chromatography (mobile phase: dichloromethane/methanol (V/V) = 8/1) to obtain compound **3G** (190 mg, yield: 60%).

LCMS m/z = 308.50 $[M+H]^+$

Step 8: Preparation of**3H**

Diethyl 2-bromo-2-methylmalonate (20.00 g, 79.02 mmol) and malononitrile (5.22 g, 79.02 mmol) were dissolved in tetrahydrofuran (120 mL). In an ice bath, potassium tert-butoxide (8.87 g, 79.02 mmol) was added in portions. After the addition, the mixture was warmed to 85°C and stirred for 16 h. The reaction solution was cooled to room temperature and subjected to suction filtration to remove the solid. The filter cake was washed with dichloromethane. The organic phases were combined and concentrated under reduced pressure. The residue was purified by flash column chromatography (mobile phase: petroleum ether/ethyl acetate (V/V) = 4/1) to obtain compound **3H** (4.94 g, yield: 26%).

LCMS m/z = 239.1 $[M+H]^+$

Step 9: Preparation of **3I**

Compound **3G** (190 mg, 0.62 mmol), **3H** (177.25 mg, 0.74 mmol) and potassium bicarbonate (124.15 mg, 1.24 mmol) were mixed and dissolved in tert-butanol (5 mL). The mixture was warmed to 80°C, stirred overnight, cooled to room temperature, and directly concentrated under reduced pressure. The residue was purified by column chromatography (mobile phase: dichloromethane/methanol (V/V) = 15/1) to obtain compound **3I** (250 mg, yield: 80%).

Step 10: Preparation of **3J**

Compound **3I** (250 mg, 0.50 mmol) was dissolved in methanol (5 mL). A solution of ammonia in methanol (5 mL, 7 *N*) was added, and the mixture was reacted overnight at 40°C and concentrated under reduced pressure to obtain compound **3J** (230 mg, yield: 97%).
LCMS m/z = 471.1 $[M+H]^+$

Step 11: Preparation of **3K**

Compound **3J** (230 mg, 0.49 mmol) was dissolved in toluene (5 mL). Lawesson's Reagent (237.83 mg, 0.59 mmol) was added. After the addition, the mixture was warmed to 80°C, stirred overnight, cooled to room temperature and concentrated under reduced pressure. The residue was purified by silica gel column chromatography (mobile phase: dichloromethane/methanol (V/V) = 1/0-5/1) to obtain compound **3K** (200 mg, yield: 83%).
LCMS m/z = 487.0 $[M+H]^+$

Step 12: Preparation of**3L**

Compound **3K** (200 mg, 0.41 mmol) was dissolved in ethanol (5 mL). **2D** (145.81 mg, 0.61 mmol) was added. After the addition, the mixture was warmed to 80°C, stirred overnight, cooled to room temperature and concentrated under reduced pressure. The residue was purified by silica gel column chromatography (mobile phase: dichloromethane/-methanol (V/V) = 1/0-5/1) to obtain compound **3L** (200 mg, yield: 78%).
LCMS m/z = 625.1 $[M+H]^+$

Step 13: Preparation of **compound 3**

Compound **3L** (200 mg, 0.32 mmol) was dissolved in a mixed solvent of 1,4-dioxane (3 mL) and water (3 mL). Lithium hydroxide hydrate (134.27 mg, 3.2 mmol) was added. The mixture was warmed to 60°C, stirred for 3 h and cooled to room temperature. In an ice bath, the mixture was acidified by adding dilute hydrochloric acid, with the solids precipitated. The resulting mixture was extracted 3 times with dichloromethane. The organic phases were combined, dried over anhydrous sodium sulphate and concentrated under reduced pressure. The residue was purified by reverse-phase column chromatography (mobile phase: acetonitrile/0.1% TFA in water (V/V) = 5/95-50/50). The resulting product was treated with a saturated aqueous sodium bicarbonate solution, extracted with dichloromethane and concentrated under reduced pressure. An appropriate amount of water and acetonitrile was added, and the mixture was subjected to lyophilisation to obtain compound **3** (100 mg, yield: 51%)
LCMS m/z = 611.2 $[M+H]^+$
$^1H$ NMR (400 MHz, DMSO-$d_6$) δ 12.38 - 11.89 (m, 1H), 11.33 (s, 1H), 9.00 (dd, 1H), 8.45 (dd, 1H), 7.24 (s, 1H), 6.99 (dd, 1H), 6.78 (*br.*s, 1H), 3.42-3.34 (m, 2H), 2.96 - 2.76 (m, 4H), 1.79 (s, 3H), 1.11 (s, 6H).

Preparation of **compound 3-1** and **compound 3-2:**

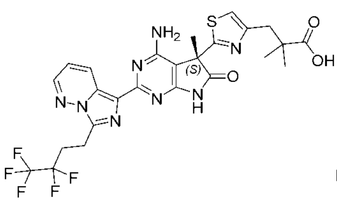

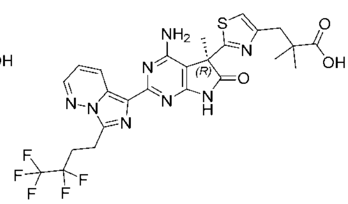

**Compound 3-1 and Compound 3-2**

**Compound 3** (100 mg) was subjected to chiral resolution, and the chiral resolution method was as follows:

1. Instrument: SFC Prep 150 AP; chromatographic column: IG (19 mm × 250 mm).
2. The sample was dissolved in methanol and filtered with a 0.45 μm filter to prepare a sample solution.
3. Preparative chromatography conditions: a. the mobile phase consists of systems A and B: mobile phase A: $CO_2$; mobile phase B: methanol (aqueous ammonia 0.05%); b. isocratic elution, mobile phase B: 30%; c. flow rate: 54 mL/min.

After preparative separation, the components with the same retention time were combined and concentrated under reduced pressure to obtain **compound 3-1** (35 mg) and **compound 3-2** (35 mg).

**Compound 3-1:** retention time under chiral preparative conditions: 3.33 min, LCMS m/z = 611.2 $[M+1]^+$

**Compound 3-2:** retention time under chiral preparative conditions: 8.60 min, LCMS m/z = 611.2 $[M+1]^+$

**Example 4:**

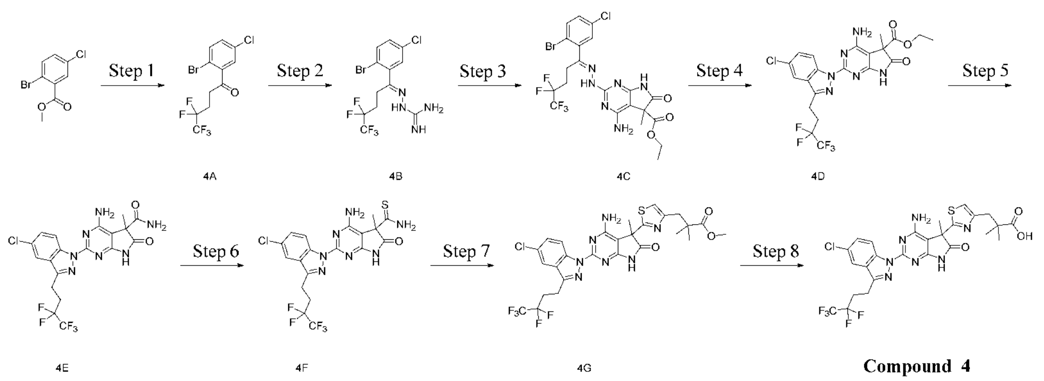


Step 1: Preparation of **4A**

Compound methyl 2-bromo-5-chlorobenzoate (2 g, 8.02 mmol) and 4,4,5,5,5-pentafluoropentanoic acid (1.69 g, 8.82 mmol) were dissolved in THF (40 mL). At -78°C, sodium bis(trimethylsilyl)amide (12 mL, 2 M, 24.06 mmol) was added, and the mixture was stirred and reacted at this temperature for 15 min, and then warmed to 0°C and reacted for 2 h. The reaction was quenched with 1 *N* hydrochloric acid (60 mL), and the mixture was stirred overnight at room temperature and extracted with ethyl acetate. The organic phase was washed twice with a saturated sodium bicarbonate solution, washed once with a saturated aqueous sodium chloride solution, dried over anhydrous sodium sulphate and concentrated under reduced pressure. The residue was purified by column chromatography (ethyl acetate/petroleum ether (V/V) = 0/1-1/20) to obtain compound **4A** (1.6 g, yield: 54%).

Step 2: Preparation of **4B**

Compound **4A** (1.6 g, 4.38 mmol) was dissolved in methanol (10 mL). Aminoguanidine hydrochloride (0.72 g, 6.54 mmol) and boron trifluoride diethyl etherate (1.1 mL, 8.91 mmol) were added. The tube was sealed, warmed to 100°C, reacted for 3 h and cooled to room temperature. A 1 *N* aqueous sodium hydroxide solution and ethyl acetate were added, and the mixture was stirred for layer separation. The organic phase was washed with a saturated aqueous sodium chloride solution, dried over anhydrous sodium sulphate and then concentrated under reduced pressure to obtain compound **4B,** which was directly used in the next step.

LCMS m/z = 421.0 $[M+H]^+$

Step 3: Preparation of **4C**

Compound **4B** (1.6 g, 3.80 mmol), **3H** (1.81 g, 7.6 mmol) and potassium tert-butoxide (0.43 g, 3.8 mmol) were mixed and dissolved in tert-butanol (15 mL). The tube was sealed, warmed to 130°C, stirred for 3 h and cooled to room temperature. A 1 N aqueous sodium hydroxide solution and ethyl acetate were added, and the mixture was stirred for layer

separation. The organic phase was washed with a saturated aqueous sodium chloride solution, dried over anhydrous sodium sulphate and concentrated under reduced pressure. The residue was purified by column chromatography (mobile phase: dichloromethane/methanol (V/V) = 1/0-10/1) to obtain compound **4C** (550 mg, yield: 23%).

LCMS m/z = 613.0 $[M+H]^+$

Step 4: Preparation of **4D**

Compound **4C** (550 mg, 0.90 mmol) was dissolved in DMF (10 mL). N,N-dimethylethylenediamine (119 mg, 1.35 mmol) and cuprous iodide (34.28 mg, 0.18 mmol) were added, and the mixture was purged three times with nitrogen. Under nitrogen atmosphere, the mixture was reacted for 2 h. Ethyl acetate and water were added, and the mixture was stirred for layer separation. The organic layer was washed three times with a saturated aqueous sodium chloride solution, dried over anhydrous sodium sulphate and concentrated under reduced pressure. The residue was purified by column chromatography (dichloromethane/methanol (V/V) = 1/0-10/1) to obtain compound **4D** (170 mg, yield: 35%).

LCMS m/z = 533.30 $[M+H]^+$

Step 5: Preparation of **4E**

Compound **4D** (170 mg, 0.32 mmol) was dissolved in methanol (3 mL). A solution of ammonia in methanol (3 mL, 7 M) was added, and the mixture was reacted overnight at 40°C and concentrated under reduced pressure to obtain compound **4E** (160 mg).

LCMS m/z = 504.1 $[M+H]^+$

Step 6: Preparation of **4F**

Compound **4E** (160 mg, 0.32 mmol) was dissolved in toluene (5 mL). Lawesson's Reagent (155.32 mg, 0.38 mmol) was added. After the addition, the mixture was warmed to 80°C, stirred overnight, cooled to room temperature and concentrated under reduced pressure. The residue was purified by silica gel column chromatography (mobile phase: dichloromethane/methanol (V/V) = 1/0-5/1) to obtain compound **4F** (160 mg, yield: 96%).

LCMS m/z = 520.50 $[M+H]^+$

Step 7: Preparation of **4G**

Compound **4F** (160 mg, 0.31 mmol) was dissolved in ethanol (5 mL). **2D** (110.25 mg, 0.46 mmol) was added. After the addition, the mixture was warmed to 80°C, stirred overnight, cooled to room temperature and concentrated under reduced pressure. The residue was purified by silica gel column chromatography (mobile phase: dichloromethane/-methanol (V/V) = 1/0-5/1) to obtain compound **4G** (110 mg, yield: 53%).

Step 8: Preparation of **compound 4**

Compound **4G** (110 mg, 0.17 mmol) was dissolved in a mixed solvent of 1,4-dioxane (3 mL) and water (3 mL). Lithium hydroxide hydrate (71.33 mg, 1.7 mmol) was added. The mixture was warmed to 60°C, stirred for 3 h and cooled to room temperature. In an ice bath, the mixture was acidified by adding dilute hydrochloric acid, with the solids precipitated. The resulting mixture was extracted 3 times with dichloromethane. The organic phases were combined, dried over anhydrous sodium sulphate and concentrated under reduced pressure. The residue was purified by reverse-phase column chromatography (mobile phase: acetonitrile/0.1% TFA in water (V/V) = 5/95-50/50) to obtain the trifluoroacetate of **compound 4** (90 mg).

LCMS m/z = 644.0 $[M+H]^+$

$^1$H NMR (400 MHz, DMSO-$d_6$) δ 11.53 (s, 1H), 8.85 (d, 1H), 8.10 (d, 1H), 7.55 (dd, 1H), 7.24 (s, 1H), 7.21 - 7.01 (m, 2H), 3.37 - 3.24 (m, 2H), 2.93 (s, 2H), 2.87 - 2.70 (m, 2H), 1.81 (s, 3H), 1.11 (s, 6H).

Preparation of **compound 4-1** and **compound 4-2:**

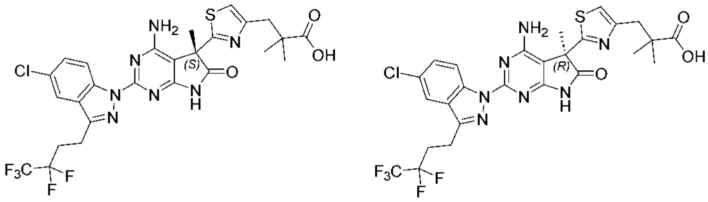

**Compound 4-1 and Compound 4-2**

**Compound 4** (90 mg) was subjected to chiral resolution, and the chiral resolution method was as follows:

1. Instrument: SFC Prep 150 AP; chromatographic column: IG (19 mm × 250 mm).
2. The sample was dissolved in methanol and filtered with a 0.45 μm filter to prepare a sample solution.
3. Preparative chromatography conditions: a. the mobile phase consists of systems A and B: mobile phase A: $CO_2$; mobile phase B: methanol (aqueous ammonia 0.05%); b. isocratic elution, mobile phase B: 25%; c. flow rate: 50 mL/min.

After preparative separation, the components with the same retention time were combined and concentrated under reduced pressure to obtain **compound 4-1** (20 mg) and **compound 4-2** (20 mg).

**Compound 4-1:** retention time under chiral preparative conditions: 3.80 min, LCMS m/z = 644.1 $[M+1]^+$

**Compound 4-2:** retention time under chiral preparative conditions: 10.10 min, LCMS m/z = 644.0 $[M+1]^+$

**Example 5:**

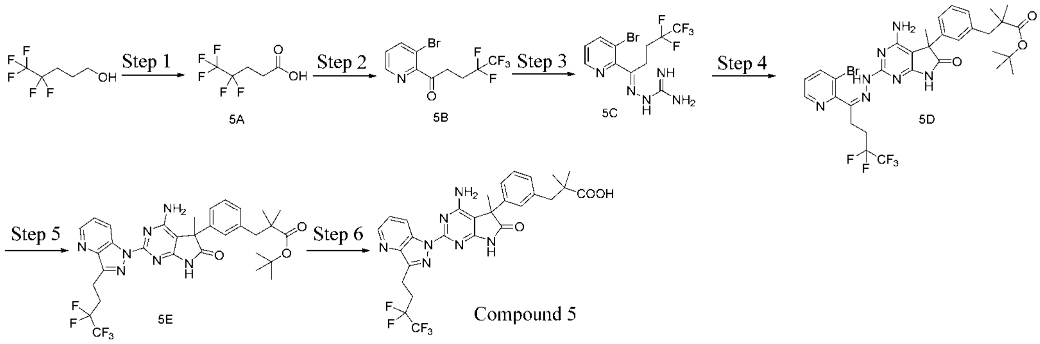


Compound 5

With reference to the synthesis for **Example 1** and **Example 4**, the trifluoroacetate of **compound 5** (15 mg) was obtained.

LCMS m/z = 604.3$[M+H]^+$

$^1$H NMR (400 MHz, DMSO-$d_6$) δ 11.21 (s, 1H), 9.19 (dd, 1H), 8.67 (dd, 1H), 7.58 (dd, 1H), 7.25 (t, 1H), 7.19 - 7.03 (m, 3H), 6.99 - 6.39 (m, 2H), 3.44 - 3.33 (m, 2H), 3.01 - 2.84 (m, 2H), 2.84 - 2.69 (m, 2H), 1.79 (s, 3H), 1.06 (s, 3H), 1.03 (s, 3H).

**Example 6:**

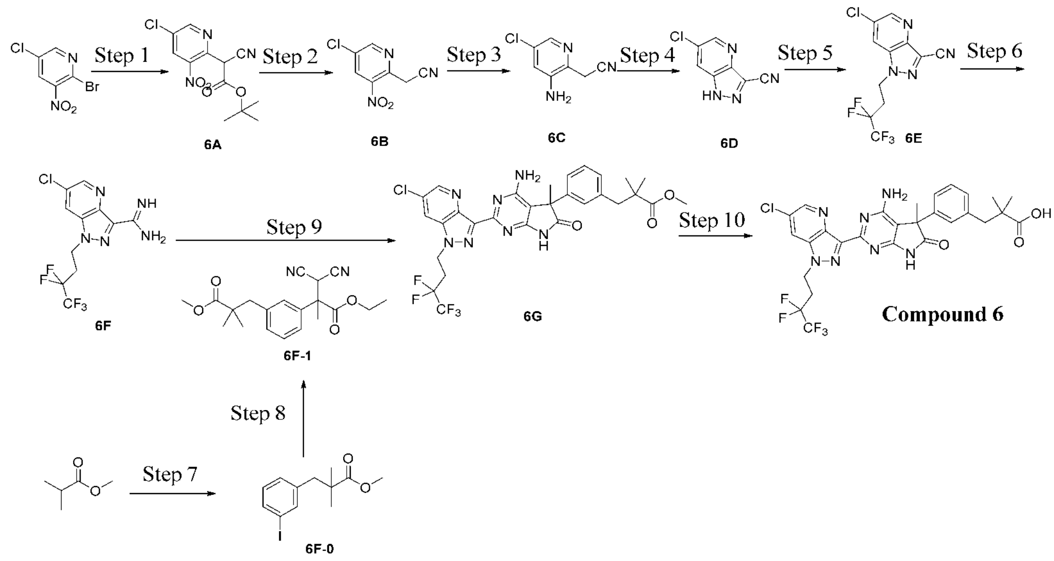


Step 1: Preparation of **6A**

5-Chloro-2-bromo-3-nitropyridine (5.00 g, 21.08 mmol) and tert-butyl cyanoacetate (4.32 g, 30.57 mmol) were dissolved in N,N-dimethylformamide (100 mL). Potassium carbonate (7.87 g, 56.92 mmol) was added. After the addition, the mixture was warmed to 100°C and stirred for 1 h. The reaction solution was cooled to room temperature, and water was added to dissolve potassium carbonate. Then, the mixture was adjusted to pH = 2 with dilute hydrochloric acid, with light-yellow solids precipitated. The resulting mixture was subjected to suction filtration. The filter cake was washed 3 times with water, redissolved in dichloromethane, dried over anhydrous sodium sulphate and concentrated under reduced pressure. The residue was purified by flash column chromatography (mobile phase: petroleum ether/ethyl acetate (V/V) = 4/1) to obtain compound **6A** (3.04 g, yield: 48%).

Step 2: Preparation of **6B**

In a 100 mL reaction flask, 26 mL of concentrated hydrochloric acid was added to 20 mL of water. **6A** (3.04 g, 10.21 mmol) was added, forming a suspension. Then, glacial acetic acid (24 mL) was added. After the addition, the mixture was warmed to 80°C and stirred for 30 min. The reaction solution was cooled to room temperature. The reaction was quenched with water. 500 mL of ethyl acetate was added, and the mixture was stirred for layer separation. The organic phase was washed 3 times with water, dried over anhydrous sodium sulphate and concentrated under reduced pressure. The residue was subjected to flash column chromatography (mobile phase: petroleum ether/ethyl acetate (V/V) = 1/1) to obtain compound **6B** (1.4 g, yield: 69%).

LCMS m/z = 198.1[M+H]$^+$

Step 3: Preparation of **6C**

Compound **6B** (1.20 g, 6.07 mmol) was dissolved in tetrahydrofuran (30 mL). Zinc powder (1.99 g, 30.35 mmol) was added, and then an aqueous ammonium chloride solution (1.62 g, 30.35 mmol, 5 mL) was added dropwise. At room temperature, the mixture was stirred and reacted for 0.5 h. To the reaction solution was added 1 mL of aqueous ammonia and 10 mL of water. The mixture was extracted 3 times with dichloromethane. The organic phases were combined, dried over anhydrous sodium sulphate and then concentrated under reduced pressure. The residue was subjected to flash column chromatography (mobile phase: petroleum ether/ethyl acetate (V/V) = 1/1) to obtain compound **6C** (0.91 g, yield: 89%).

LCMS m/z = 168.1 [M+H]$^+$

Step 4: Preparation of **6D**

Compound **6C** (0.61 g, 3.64 mmol) was dissolved in concentrated hydrochloric acid (3 mL) and water (15 mL). In an ice bath, sodium nitrite (0.41 g, 5.93 mmol) was added, and the mixture was stirred at room temperature for 16 h, with yellow solids precipitated. The resulting mixture was subjected to suction filtration to obtain solids, which were dissolved in glacial acetic acid (6 mL). The mixture was warmed to 100°C, reacted for 2 h and concentrated under reduced pressure.

The residue was directly purified by column chromatography (mobile phase: petroleum ether/ethyl acetate (V/V) = 3/1) to obtain compound **6D** (0.24 g, yield: 37%).

LCMS m/z = 179.10 $[M+H]^+$

Step 5: Preparation of **6E**

Compound **6D** (0.34 g, 1.89 mmol) and 1,1,1,2,2-pentafluoro-4-iodobutane (1.14 g, 4.16 mmol) were dissolved in acetonitrile (15 mL). Potassium carbonate (1.31 g, 9.45 mmol) was added. The mixture was warmed to 100°C, stirred and reacted for 16 h. The reaction solution was cooled to room temperature and subjected to suction filtration to remove solids. The filter cake was washed 3 times with dichloromethane. The organic phases were combined and concentrated under reduced pressure. The residue was purified by column chromatography (mobile phase: petroleum ether/ethyl acetate (V/V) = 5/1) to obtain compound **6E** (0.44 g, yield: 71%).

LCMS m/z = 325.00 $[M+H]^+$

Step 6: Preparation of **6F**

In an ice bath, ammonium chloride (0.36 g, 6.80 mmol) was added to a 500 mL single-necked flask. Toluene (30 mL) was added, and then trimethylaluminium (3.9 mL, 2 M in toluene) was slowly added dropwise. The mixture was warmed to room temperature, stirred and reacted for 3 h. Compound **6E** (0.44 g, 1.36 mmol) was dissolved in toluene and added dropwise to the reaction solution. The mixture was warmed to 110°C, stirred for 6 h and cooled to room temperature. In an ice bath, silica gel and 100 mL of methanol were added. The mixture was further stirred for 30 min and subjected to suction filtration. The filter cake was washed 3 times with methanol. The organic phases were combined and concentrated under reduced pressure. The residue was purified by column chromatography (mobile phase: dichloromethane/methanol (V/V) = 8/1) to obtain compound **6F** (0.46 g, yield: 99%).

LCMS m/z = 342.0 $[M+H]^+$

Step 7: Preparation of compound **6F-0**

At -78°C, lithium diisopropylamide (60 mL, 2 M in THF) was added dropwise to a solution of methyl isobutyrate (10.18 g, 99.72 mmol) in tetrahydrofuran (500 mL). At -78°C, the mixture was stirred for 0.5 h, and then 3-iodobenzyl bromide (29.6 g, 99.69 mmol) was added. The mixture was slowly warmed to room temperature and stirred for 1 h. The reaction was quenched with a saturated aqueous ammonium chloride solution (400 mL) and extracted with ethyl acetate (100 mL×3). The organic phases were combined, dried over anhydrous sodium sulphate and then concentrated under reduced pressure. The residue was purified by silica gel column chromatography (ethyl acetate/petroleum ether (v/v) = 1/100-1/10) to obtain compound **6F-0** (18 g, yield: 57%).

$^1H$ NMR (400 MHz, $CDCl_3$) δ 7.57 - 7.44 (m, 2H), 7.10 - 7.03 (m, 1H), 6.99 (t, 1H), 3.66 (s, 3H), 2.78 (s, 2H), 1.18 (s, 6H).

Step 8: Preparation of compound **6F-1**

At -78°C, an isopropyl magnesium chloride-lithium chloride complex (35 mL, 1.3 M in THF) was added dropwise to a solution of compound **6F-0** (10 g, 31.4 mmol) in tetrahydrofuran (200 mL), the mixture was stirred for 30 min, and then ethyl 3,3-dicyano-2-methylacrylate (synthesised with reference to *synthesis,* **1974:** 9,669) (5.16 g, 31.43 mmol) was slowly added. After the addition, the mixture was stirred at room temperature for 1 h. The reaction was quenched with a saturated aqueous ammonium chloride solution (100 mL) and extracted with ethyl acetate (80 mL×2). The organic phases were combined, dried over anhydrous sodium sulphate and then concentrated under reduced pressure. The residue was purified by silica gel column chromatography (ethyl acetate/petroleum ether (v/v) = 1/100-1/10) to obtain compound **6F-1** (5 g, yield: 45%).

Step 9: Preparation of **6G**

Compound **6F** (0.10 g, 0.29 mmol), **6F-1** (0.10 g, 0.29 mmol) and potassium bicarbonate (0.09 g, 0.87 mmol) were mixed and dissolved in tert-butanol (5 mL). The mixture was warmed to 80°C, stirred overnight, cooled to room temperature, and directly concentrated under reduced pressure. The residue was purified by column chromatography (mobile phase: dichloromethane/methanol (V/V) = 15/1) to obtain compound **6G** (0.16 g).

LCMS m/z = 652.2 $[M+H]^+$

Step 10: Preparation of compound **6**

Compound **6G** (0.16 g, 0.25 mmol) was dissolved in a mixed solvent of 1,4-dioxane (5 mL) and water (5 mL). Lithium hydroxide hydrate (0.10 g, 2.50 mmol) was added. The mixture was warmed to 60°C, stirred for 3 h and cooled to room temperature. In an ice bath, the mixture was acidified by adding dilute hydrochloric acid, with white solids precipitated. The resulting mixture was extracted 3 times with dichloromethane. The organic phases were combined, dried over anhydrous sodium sulphate and concentrated under reduced pressure. The residue was purified by reverse-phase column chromatography (mobile phase: acetonitrile/0.1% TFA in water (V/V) = 5/95-50/50) to obtain compound **6** (0.14 g, yield: 87%).
LCMS m/z = 638.1 $[M+H]^+$
$^1H$ NMR (400 MHz, DMSO-$d_6$) δ 11.20 (s, 1H), 8.68 - 8.57 (m, 2H), 7.25 (t, 1H), 7.16 - 7.04 (m, 3H), 6.46 (s, 2H), 4.86 (t, 2H), 3.05 - 2.88 (m, 2H), 2.84 - 2.70 (m, 2H), 1.79 (s, 3H), 1.07 (s, 3H), 1.03 (s, 3H).

Preparation of **compound 6-1** and **compound 6-2:**

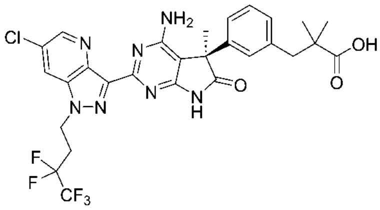 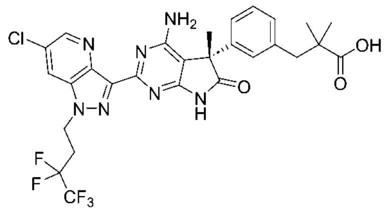

**Compound 6-1 and 6-2**

**Compound 6** (140 mg) was subjected to chiral separation and purification. Preparative conditions: instrument: SFC Prep 150 AP; chromatographic column: IG (19 mm*250 mm); The sample was dissolved in methanol and filtered with a 0.45 μm filter to prepare a sample solution. Preparative chromatography conditions: a. composition of mobile phases A and B: mobile phase A: $CO_2$, and mobile phase B: methanol (0.05% aqueous ammonia); b. isocratic elution, mobile phase B: 30%; c. flow rate: 54 ml/min.
Analytical method: 1. instrument: UPC2; chromatographic column: IG (3 mm×50 mm); 2. the sample was dissolved in methanol and filtered with a 0.45 μm filter to prepare a sample solution. 3. Preparative chromatography conditions: a. composition of mobile phases A and B: mobile phase A: $CO_2$, and mobile phase B: methanol (containing 0.5% aqueous ammonia); b. gradient elution, mobile phase B: 10%-40%, time: 5 min; c. flow rate: 1.5 ml/min.
After preparative separation, the components with the same retention time were combined and concentrated under reduced pressure to obtain **compound 6-1** (55 mg) and **compound 6-2** (40 mg).

**Compound 6-1:** retention time under analysis conditions: 2.824 min, LCMS m/z = 638.1 $[M+H]^+$

**Compound 6-2:** retention time under analysis conditions: 4.098 min, LCMS m/z = 638.1 $[M+H]^+$

**Example 7:**

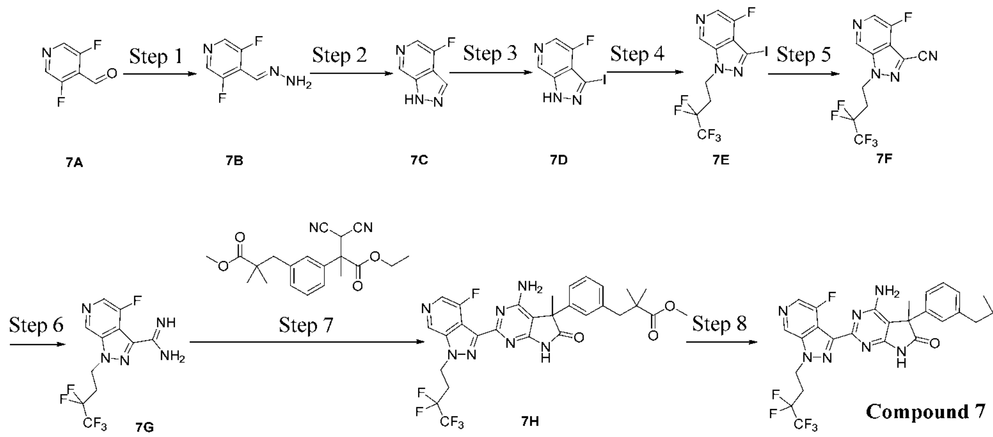


7A 7B 7C 7D 7E 7F 7G 7H **Compound 7**

With reference to the synthesis for **Example 6, compound 7** (0.073 g) was obtained.

LCMS m/z = 622.2 $[M+H]^+$

$^1$H NMR (400 MHz, DMSO-$d_6$) δ 11.12 (s, 1H), 9.20 (d, 1H), 8.28 (d, 1H), 7.33 - 7.20 (m, 1H), 7.14 - 7.03 (m, 3H), 6.52 - 6.30 (m, 2H), 5.00 (t, 2H), 3.10 - 2.93 (m, 2H), 2.86 - 2.68 (m, 2H), 1.80 (s, 3H), 1.07 (s, 3H), 1.03 (s, 3H).

**Example 8:**

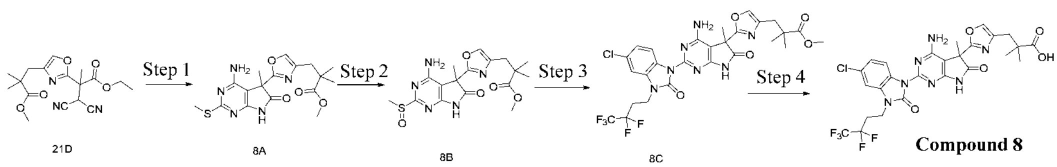


21D 8A 8B 8C **Compound 8**

Step 1: Preparation of **8A**

Compound **21D** (0.5 g, 1.44 mmol), S-methylisothiourea sulphate (0.27 g, 1.44 mmol) and potassium bicarbonate (0.58 g, 5.76 mmol) were mixed and dissolved in tert-butanol (5 mL). The mixture was warmed to 80°C, stirred overnight and cooled to room temperature. 20 mL of ethyl acetate was added, and the mixture was washed with 10 mL of a saturated aqueous sodium chloride solution. The organic layer was dried over anhydrous sodium sulphate, filtered and concentrated. The residue was slurried with 15 mL of methyl tert-butyl ether/petroleum ether (V/V = 1/4) to obtain compound **8A** (0.4 g).

LCMS m/z = 392.1 $[M+H]^+$

Step 2: Preparation of**8B**

Compound **8A** (0.52 g, 1.33 mmol) was dissolved in THF (5 mL), and m-chloroperoxybenzoic acid (0.46 g, 2.66 mmol) was added at room temperature. After the addition, the mixture was further stirred for 2 h. 10 mL of a saturated aqueous sodium thiosulphate solution was added, and the mixture was stirred for 20 min. 10 mL of a saturated aqueous sodium bicarbonate solution was added, and the mixture was stirred for 10 min and extracted with 20 mL of ethyl acetate. The organic layer was washed with 10 mL of a saturated aqueous sodium chloride solution and then dried over anhydrous sodium sulphate, filtered and concentrated to obtain compound **8B,** which was directly used in the next step.

Step 3: Preparation of**8C**

Compound **1L** (0.12 g, 0.38 mmol), **8B** (0.23 g, 0.57 mmol) and potassium carbonate (0.10 g, 0.76 mmol) were mixed and dissolved in DMF (5 mL). The mixture was warmed to 100°C, stirred for 16 h, cooled to room temperature and extracted with 20 mL of ethyl acetate and 20 mL of water. The organic layer was washed with a saturated aqueous sodium chloride solution (10 mL x 3), dried over anhydrous sodium sulphate and concentrated under reduced pressure. The residue was purified by silica gel column chromatography (ethyl acetate/petroleum ether (V/V) = 1/5-1/1) to obtain compound **8C** (54 mg, yield: 21%).

LCMS m/z = 658.2 $[M+H]^+$

Step 4: Preparation of **compound 8**

Compound **8C** (0.054 g, 0.082 mmol) was dissolved in a mixed solvent of 1,4-dioxane (3 mL) and water (1.5 mL). Lithium hydroxide (0.020 g, 0.83 mmol) was added. The mixture was warmed to 60°C, stirred for 1 h and cooled to room temperature. The reaction solution was adjusted to pH = 3-4 by adding a 1 *N* aqueous hydrochloric acid solution. 20 mL of ethyl acetate was added, and the organic phase was washed with 10 mL of a saturated aqueous sodium chloride solution, dried over anhydrous sodium sulphate and concentrated under reduced pressure. The residue was purified by column chromatography (acetonitrile/water (containing 0.1% trifluoroacetic acid) (V/V) = 0/100-60/40) to obtain **compound 8** (30 mg, yield: 56%).
LCMS m/z = 644.2 $[M+H]^+$
$^1H$ NMR (400 MHz, DMSO-$d_6$) δ 11.50 (s, 1H), 7.80 (d, 1H), 7.75 (s, 1H), 7.53 (d, 1H), 7.15-7.10 (m, 1H), 6.91-6.67 (m, 2H), 4.23 (t, 2H), 2.83 - 2.62 (m, 4H), 1.81 (s, 3H), 1.11 (s, 3H), 1.09 (s, 3H).

**Example 9:**

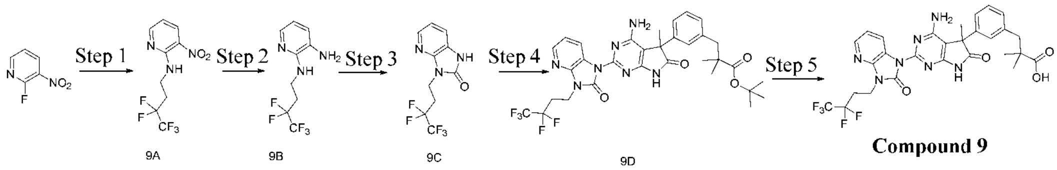

9A 9B 9C 9D **Compound 9**

Step 1: Preparation of**9A**

Compound **1I** hydrochloride (0.5 g, 2.51 mmol) and 2-fluoro-3-nitropyridine (0.36 g, 2.51 mmol) were dissolved in DMSO (5 mL). DIPEA (0.97 g, 7.53 mmol) was added, and the mixture was stirred at room temperature for 4 h and extracted with 30 mL of methyl tert-butyl ether and 30 mL of water. The organic layer was washed with a saturated aqueous sodium chloride solution (20 mL x 3), dried over anhydrous sodium sulphate and concentrated under reduced pressure. The residue was purified by silica gel column chromatography (ethyl acetate/petroleum ether (V/V) = 0/10-1/10) to obtain compound **9A** (0.62 g, yield: 86%).
LCMS m/z = 286.30 $[M+H]^+$

Step 2: Preparation of**9B**

Compound **9A** (0.62 g, 2.17 mmol) was dissolved in a mixed solvent of ethanol (10 mL) and water (3 mL). Ammonium chloride (1.16 g, 21.69 mmol) and iron powder (1.21 g, 21.66 mmol) were added sequentially. After the addition, the mixture was warmed to 75°C, stirred for 1.5 h, cooled to room temperature and filtered. The filter cake was washed with 20 mL of dichloromethane. The filtrates were combined and washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 0/10-3/10) to obtain compound **9B** (0.48 g, yield: 86%).

Step 3: Preparation of**9C**

Compound **9B** (0.48 g, 1.88 mmol) was dissolved in THF (10 mL). N,N'-carbonyldiimidazole (0.46 g, 2.84 mmol) was added. After the addition, the mixture was stirred at room temperature for 16 h. 20 mL of dichloromethane was added. The organic layer was washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 1/10-1/2) to obtain compound **9C** (0.32 g, yield: 60%).
LCMS m/z = 282.1$[M+H]^+$

Step 4: Preparation of**9D**

Compound **9C** (0.12 g, 0.43 mmol), **1G** (0.30 g) and potassium carbonate (0.12 g, 0.87 mmol) were mixed and

dissolved in DMF (5 mL). The mixture was warmed to 100°C, stirred for 16 h, cooled to room temperature and extracted with 20 mL of ethyl acetate and 20 mL of water. The organic layer was washed with a saturated aqueous sodium chloride solution (10 mL x 3), dried over anhydrous sodium sulphate and concentrated under reduced pressure. The residue was purified by silica gel column chromatography (ethyl acetate/petroleum ether (V/V) = 1/5-1/1) to obtain compound **9D** (0.2 g, yield: 68%).

LCMS m/z = 676.3 $[M+H]^+$

Step 5: Preparation of **compound 9**

Compound **9D** (0.20 g, 0.30 mmol) was placed in a 50 mL single-necked flask. Trifluoroacetic acid (2 mL) was added, and the mixture was stirred at room temperature for 2 h and concentrated under reduced pressure. The residue was purified by column chromatography (dichloromethane/methanol (V/V) = 100/0-100/5). The resulting crude was further purified by column chromatography (acetonitrile/water (containing 0.1% trifluoroacetic acid) (V/V) = 0/100-60/40) to obtain **compound 9** (80 mg, yield: 43%).

LCMS m/z = 620.3$[M+H]^+$

$^1$H NMR (400 MHz, DMSO-$d_6$) δ 12.50-11.90 (m, 1H), 11.22 (s, 1H), 8.29-8.23 (m, 1H), 8.15-8.09 (m, 1H), 7.28-7.21 (m, 1H), 7.18 - 7.03 (m, 4H), 6.80-6.44 (m, 2H), 4.25 (t, 2H), 2.92 - 2.70 (m, 4H), 1.78 (s, 3H), 1.07 (s, 3H), 1.03 (s, 3H).

Preparation of **compound 9-1** and **compound 9-2:**

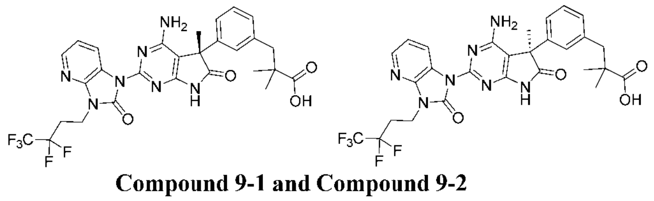

**Compound 9-1 and Compound 9-2**

**Compound 9** (80 mg) was subjected to chiral separation and purification. Preparative conditions: instrument: SFC Prep 150 AP; chromatographic column: IG (19 mm×250 mm). The sample was dissolved in DMF and filtered with a 0.45 μm filter to prepare a sample solution. Preparative chromatography conditions: mobile phase A: $CO_2$, and mobile phase B: methanol (containing 0.5% aqueous ammonia); isocratic elution, mobile phase B: 25%; flow rate: 44 ml/min.

Analysis conditions: instrument: UPC2; chromatographic column: IG (3 mm×50 mm). The sample was dissolved in methanol and filtered with a 0.45 μm filter to prepare a sample solution. Preparative chromatography conditions: mobile phase A: $CO_2$, and mobile phase B: methanol (containing 0.5% aqueous ammonia); gradient elution, mobile phase B: 10%-40%, time: 5 min; isocratic elution, mobile phase B: 40%; flow rate: 1.5 ml/min.

After preparative separation, the components with the same retention time were combined and concentrated under reduced pressure to obtain **compound 9-1** (25 mg) and **compound 9-2** (35 mg).

**Compound 9-1:** retention time under analysis conditions: 1.9 min, LCMS m/z = 620.2 $[M+H]^+$

**Compound 9-2:** retention time under analysis conditions: 2.2 min, LCMS m/z = 620.2 $[M+H]^+$

**Example 10:**

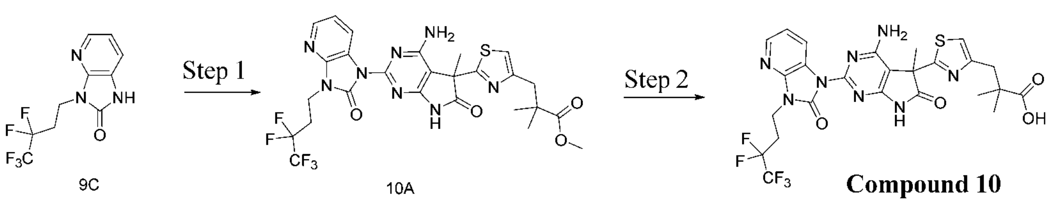


9C

10A

**Compound 10**

With reference to the synthesis for **Example 9** and **Example 2,** the trifluoroacetate of **compound 10** (15 mg) was

obtained.

LCMS m/z = 627.2 [M+H]+

$^1$H NMR (400 MHz, DMSO-$d_6$) 11.57 (s, 1H), 8.28-8.22 (m, 1H), 8.15-8.09 (m, 1H), 7.26 (s, 1H), 7.18-7.12 (m, 1H), 7.12-7.02 (m, 2H), 4.24 (t, 2H), 2.93 (s, 2H), 2.89-2.73 (m, 2H), 1.81 (s, 3H), 1.14-1.05(m, 6H).

**Example 11:**

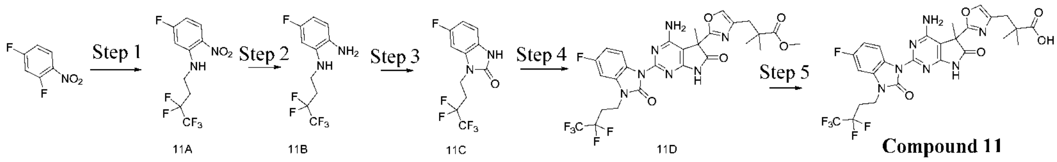


Step 1: Preparation of **11A**

Compound **1I** hydrochloride (0.5 g, 2.51 mmol) and 2,4-difluoronitrobenzene (0.40 g, 2.51 mmol) were dissolved in DMSO (5 mL). DIPEA (0.98 g, 7.58 mmol) was added, and the mixture was stirred at room temperature for 4 h and extracted with 30 mL of methyl tert-butyl ether and 30 mL of water. The organic layer was washed with a saturated aqueous sodium chloride solution (20 mL x 3), dried over anhydrous sodium sulphate and concentrated under reduced pressure. The residue was purified by silica gel column chromatography (ethyl acetate/petroleum ether (V/V) = 0/10-1/10) to obtain compound **11A** (0.62 g, yield: 82%).

Step 2: Preparation of **11B**

Compound **11A** (0.62 g, 2.05 mmol) was dissolved in a mixed solvent of ethanol (10 mL) and water (3 mL). Ammonium chloride (1.10 g, 20.56 mmol) and iron powder (1.14 g, 20.42 mmol) were added sequentially. After the addition, the mixture was warmed to 75°C, stirred for 1.5 h, cooled to room temperature and filtered. The filter cake was washed with 20 mL of dichloromethane. The filtrates were combined and washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 0/10-3/10) to obtain compound **11B** (0.48 g, yield: 86%).

LCMS m/z = 273.30 [M+H]+

Step 3: Preparation of **11C**

Compound **11B** (0.48 g, 1.76 mmol) was dissolved in THF (10 mL). N,N'-carbonyldiimidazole (0.43 g, 2.66 mmol) was added. After the addition, the mixture was stirred at room temperature for 16 h. 20 mL of dichloromethane was added. The organic layer was washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 1/10-1/2) to obtain compound **11C** (0.3 g, yield: 57%).

LCMS m/z = 299.1 [M+H]+

Step 4: Preparation of **11D**

Compound **11C** (0.14 g, 0.47 mmol), **8B** (0.29 g, 0.70 mmol) and potassium carbonate (0.13 g, 0.94 mmol) were mixed and dissolved in DMF (5 mL). The mixture was warmed to 100°C, stirred for 16 h, cooled to room temperature and extracted with 20 mL of ethyl acetate and 20 mL of water. The organic layer was washed with a saturated aqueous sodium chloride solution (10 mL x 3), dried over anhydrous sodium sulphate and concentrated under reduced pressure. The residue was purified by silica gel column chromatography (ethyl acetate/petroleum ether (V/V) = 1/5-1/1) to obtain compound **11D** (0.07 g, yield: 23%).

LCMS m/z = 642.3 [M+H]+

Step 5: Preparation of **compound 11**

Compound **11D** (0.070 g, 0.11 mmol) was dissolved in a mixed solvent of 1,4-dioxane (3 mL) and water (1 mL).

Lithium hydroxide (0.026 g, 1.09 mmol) was added. The mixture was warmed to 60°C, stirred for 1 h and cooled to room temperature. The reaction solution was adjusted to pH = 3-4 by adding a 1 *N* aqueous HCl solution. 20 mL of ethyl acetate was added, and the organic phase was washed with 10 mL of a saturated aqueous sodium chloride solution, dried over anhydrous sodium sulphate and concentrated under reduced pressure. The residue was purified by column chromatography (acetonitrile/water (containing 0.1% trifluoroacetic acid) (V/V) = 0/100-60/40) to obtain **compound 11** (20 mg, yield: 29%).

LCMS m/z = 628.2 $[M+H]^+$

$^1H$ NMR (400 MHz, DMSO-$d_6$) δ 12.50-11.80 (m, 1H), 11.49 (s, 1H), 7.87-7.79 (m, 1H), 7.75 (s, 1H), 7.39-7.30 (m, 1H), 6.98-6.87 (m, 1H), 6.87-6.60 (m, 2H), 4.22 (t, 2H), 2.80 - 2.60 (m, 4H), 1.81 (s, 3H), 1.11 (s, 3H), 1.09 (s, 3H).

**Example 12:**

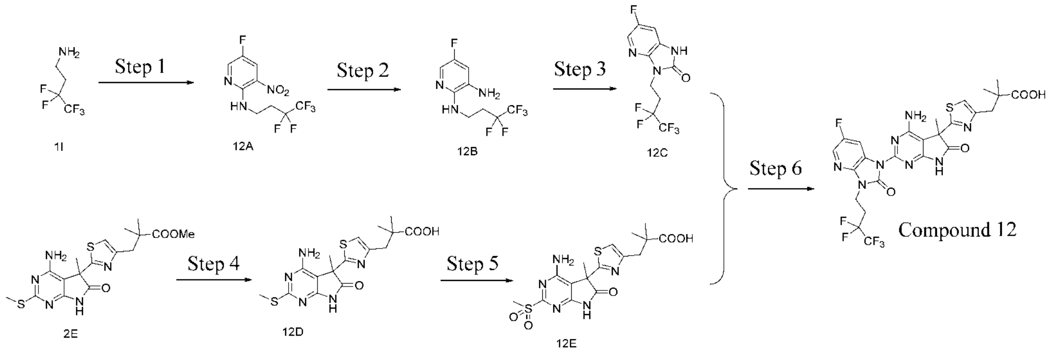


Step 1: Preparation of **12A**

Compound 2-chloro-3-nitro-5-fluoropyridine (0.33 g, 1.87 mmol) and compound **1I** hydrochloride (0.37 g, 1.87 mmol) were dissolved in DMSO (5 mL). Diisopropylethylamine (0.73 g, 5.61 mmol) was added, and the mixture was warmed to 60°C, stirred for 4 h and cooled to room temperature. 30 mL of MTBE and 30 mL of water were added, and the mixture was stirred for layer separation. The organic layer was washed with a saturated aqueous sodium chloride solution (20 mL x 3), dried over anhydrous sodium sulphate and concentrated under reduced pressure. The residue was purified by silica gel column chromatography (ethyl acetate/petroleum ether (V/V) = 0/10-1/10) to obtain compound **12A** (0.42 g, yield: 74%).

Step 2: Preparation of **12B**

Compound **12A** (0.4 g, 1.32 mmol) was dissolved in a mixed solvent of ethanol (6 mL) and water (2 mL). Ammonium chloride (0.73 g, 13.07 mmol) and iron powder (0.74 g, 13.25 mmol) were added sequentially. After the addition, the mixture was warmed to 75°C, stirred for 1.5 h, cooled to room temperature and filtered. The filter cake was washed with 20 mL of dichloromethane. The filtrates were combined and washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 0/10-3/10) to obtain compound **12B** (0.3 g, yield: 83%).

Step 3: Preparation of **12C**

Compound **12B** (0.3 g, 1.1 mmol) was dissolved in THF (5 mL). In an ice bath, N,N'-carbonyldiimidazole (0.54 g, 3.3 mmol) was added in portions. After the addition, the mixture was naturally warmed to room temperature and stirred for 2 h. 10 mL of a saturated aqueous sodium bicarbonate solution was added, and the mixture was stirred for 10 min and extracted with 20 mL of dichloromethane. The organic layer was washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 1/10-1/2) to obtain compound **12C** (0.2 g, yield: 61%).

LCMS m/z = 300.1$[M+H]^+$

Step 4: Preparation of **12D**

Compound **2E** (0.3 g, 0.74 mmol) was dissolved in a mixed solvent of 1,4-dioxane (5 mL) and water (0.8 mL). Lithium hydroxide hydrate (0.17 g, 7.16 mmol) was added. The mixture was warmed to 60°C, stirred for 3 h and cooled to room temperature. In an ice bath, the mixture was acidified by adding dilute hydrochloric acid, with white solids precipitated. The resulting mixture was extracted 3 times with dichloromethane. The organic phases were combined, dried over anhydrous sodium sulphate and concentrated under reduced pressure. The residue was purified by reverse-phase column chromatography (mobile phase: acetonitrile/0.1% TFA in water (V/V) = 5/95-50/50) to obtain compound **12D** (0.25 g, yield: 86%).

Step 5: Preparation of **12E**

Compound **12D** (0.25 g, 0.64 mmol) was dissolved in tetrahydrofuran (7 mL), and m-chloroperoxybenzoic acid (0.33 g, 1.92 mmol) was added at room temperature. After the addition, the mixture was further stirred for 2 h. 10 mL of a saturated aqueous sodium thiosulphate solution was added, and the mixture was stirred for 20 min. 10 mL of a saturated aqueous sodium bicarbonate solution was added, and the mixture was stirred for 10 min. 20 mL of ethyl acetate was added, and the mixture was stirred for layer separation. The organic layer was washed with 10 mL of a saturated aqueous sodium chloride solution and then dried over anhydrous sodium sulphate, filtered and concentrated to obtain compound **12E,** which was directly used in the next step.

Step 6: Preparation of **compound 12**

Compound **12C** (0.05 g, 0.17 mmol), **12E** (0.072 g, 0.17 mmol) and potassium carbonate (0.047 g, 0.34 mmol) were mixed and dissolved in DMF (3 mL). The mixture was warmed to 100°C and stirred for 16 h, further warmed to 120°C and stirred for 24 h, and cooled to room temperature. 20 mL of ethyl acetate and 20 mL of water were added, and the mixture was stirred for layer separation. The organic layer was washed with a saturated aqueous sodium chloride solution (10 mL x 3), dried over anhydrous sodium sulphate and concentrated under reduced pressure. The residue was purified by silica gel column chromatography (dichloromethane/methanol (V/V) = 100/0-100/3). The resulting residue was further purified by reversed-phase column chromatography (acetonitrile/water (containing 0.1% trifluoroacetic acid) (V/V) = 0/100-60/40) to obtain **compound 12** (10 mg, yield: 9%).

LCMS m/z = 645.2$[M+H]^+$

$^1H$ NMR (400 MHz, DMSO-$d_6$) δ 11.57 (s, 1H), 8.35 (dd, 1H), 8.17 - 8.13 (m, 1H), 7.26 (s, 1H), 7.22-7.02 (m, 2H), 4.22 (t, 2H), 2.93 (s, 2H), 2.88 - 2.72 (m, 2H), 1.81 (s, 3H), 1.16-1.04 (m, 6H).

Preparation of **compound 12-1 and compound 12-2:**

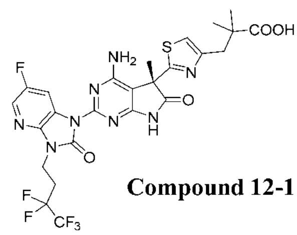

Compound 12-1

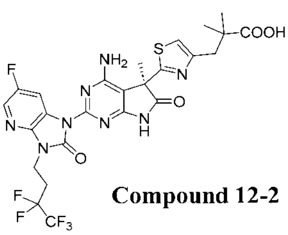

Compound 12-2

**Compound 12** (110 mg) was subjected to chiral resolution, and the chiral resolution method was as follows:

1. instrument: SFC Prep 150 AP; chromatographic column: IG (19 mm×250 mm)
2. The sample was dissolved in methanol and filtered with a 0.45 μm filter to prepare a sample solution.
3. Preparative chromatography conditions: a. composition of mobile phases A and B: mobile phase A: $CO_2$; mobile phase B: methanol (0.05% aqueous ammonia); b. isocratic elution, mobile phase B: 12%; c. flow rate: 40 ml/min.

After preparative separation, the components with the same retention time were combined and concentrated under reduced pressure to obtain **compound 12-1** (50 mg) and **compound 12-2** (50 mg).

**Compound 12-1:** retention time under chiral preparative conditions: 9.62 min, LCMS m/z = 645.2 [M+H]$^+$

$^1$H NMR (400 MHz, DMSO-$d_6$) δ 11.57 (s, 1H), 8.35 (dd, 1H), 8.18 - 8.10 (m, 1H), 7.26 (s, 1H), 7.23 - 7.01 (m, 2H), 4.22 (t, 2H), 2.93 (s, 2H), 2.88 - 2.71 (m, 2H), 1.80 (s, 3H), 1.16 - 1.03 (m, 6H).

**Compound 12-2:** retention time under chiral preparative conditions: 13.68 min, LCMS m/z = 645.2 [M+H]$^+$

$^1$H NMR (400 MHz, DMSO-$d_6$) δ 11.31 (s, 1H), 8.35 (dd, 1H), 8.19 - 8.12 (m, 1H), 7.26 (s, 1H), 7.22 - 7.05 (m, 2H), 4.22 (t, 2H), 2.93 (s, 2H), 2.88 - 2.71 (m, 2H), 1.81 (s, 3H), 1.16 - 1.02 (m, 6H).

**Example 13:**

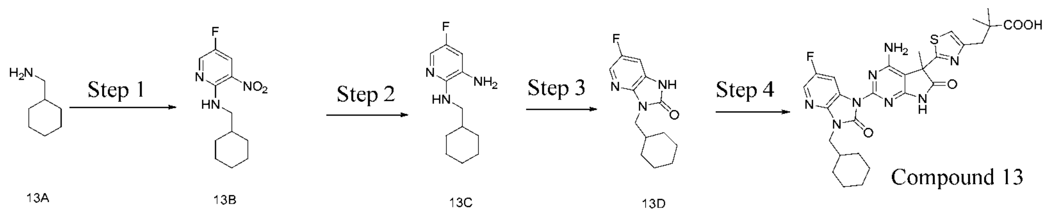


13A 13B 13C 13D Compound 13

Step 1: Preparation of **13B**

Compound 2-chloro-3-nitro-5-fluoropyridine (0.3 g, 1.87 mmol) and compound **13A** (0.21 g, 1.87 mmol) were dissolved in DMSO (5 mL). Diisopropylethylamine (0.73 g, 5.61 mmol) was added, and the mixture was warmed to 60°C, stirred for 4 h and cooled to room temperature. 30 mL of MTBE and 30 mL of water were added, and the mixture was stirred for layer separation. The organic layer was washed with a saturated aqueous sodium chloride solution (20 mL x 3), dried over anhydrous sodium sulphate and concentrated under reduced pressure. The residue was purified by silica gel column chromatography (ethyl acetate/petroleum ether (V/V) = 0/10-1/10) to obtain compound **13B** (0.4 g, yield: 84%).

Step 2: Preparation of **13C**

Compound **13B** (0.4 g, 1.58 mmol) was dissolved in a mixed solvent of ethanol (6 mL) and water (2 mL). Ammonium chloride (0.73 g, 13.64 mmol) and iron powder (0.76 g, 13.54 mmol) were added sequentially. After the addition, the mixture was warmed to 75°C, stirred for 1.5 h, cooled to room temperature and filtered. The filter cake was washed with 20 mL of dichloromethane. The filtrates were combined and washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 0/10-3/10) to obtain compound **13C** (0.3 g, yield: 85%).

Step 3: Preparation of **13D**

Compound **13C** (0.3 g, 1.34 mmol) was dissolved in THF (15 mL). In an ice bath, N,N'-carbonyldiimidazole (0.65 g, 4.01 mmol) was added in portions. After the addition, the mixture was naturally warmed to room temperature and stirred for 2 h. 10 mL of a saturated aqueous sodium bicarbonate solution was added, and the mixture was stirred for 10 min and extracted with 20 mL of dichloromethane. The organic layer was washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 1/10-1/2) to obtain compound **13D** (0.24 g, yield: 72%).
LCMS m/z = 250.2[M+H]$^+$

Step 4: Preparation of **compound 13**

Compound **13D** (40 mg, 0.16 mmol), **12E** (68 mg, 0.16 mmol) and potassium carbonate (44 mg, 0.32 mmol) were mixed and dissolved in DMF (3 mL). The mixture was warmed to 100°C and stirred for 16 h, further warmed to 120°C and stirred for 24 h, and cooled to room temperature. 20 mL of ethyl acetate and 20 mL of water were added, and the mixture was stirred for layer separation. The organic layer was washed with a saturated aqueous sodium chloride solution (10 mL x

3), dried over anhydrous sodium sulphate and concentrated under reduced pressure. The residue was purified by silica gel column chromatography (dichloromethane/methanol (V/V) = 100/0-100/3). The resulting residue was further purified by reversed-phase column chromatography (acetonitrile/water (containing 0.1% trifluoroacetic acid) (V/V) = 0/100-60/40) to obtain compound **13** (20 mg, yield: 21%).

LCMS m/z = 595.3$[M+H]^+$

$^1H$ NMR (400 MHz, DMSO-$d_6$) δ 11.57 (s, 1H), 8.28 (dd, 1H), 8.13 - 8.07 (m, 1H), 7.26 (s, 1H), 7.22-7.01 (m, 2H), 3.74 (d, 2H), 2.93 (s, 2H), 1.97- 1.86 (m, 1H), 1.81 (s, 3H), 1.74 - 1.52 (m, 5H), 1.26 - 0.92 (m, 11H).

**Example 14:**

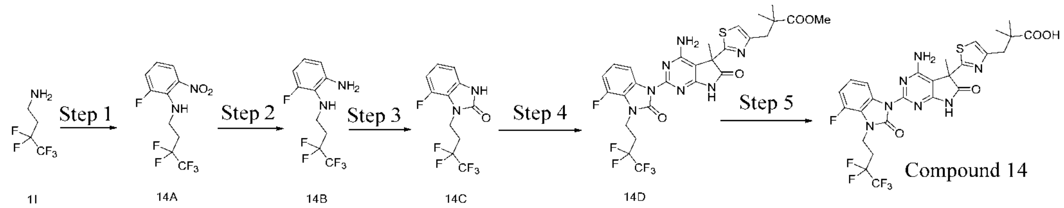


Step 1: Preparation of **14A**

Compound 2,3-difluoronitrobenzene (0.3 g, 1.87 mmol) and compound **1l** hydrochloride (0.37 g, 1.87 mmol) were dissolved in DMSO (5 mL). Diisopropylethylamine (0.73 g, 5.61 mmol) was added, and the mixture was warmed to 60°C, stirred for 4 h and cooled to room temperature. 30 mL of MTBE and 30 mL of water were added, and the mixture was stirred for layer separation. The organic layer was washed with a saturated aqueous sodium chloride solution (20 mL x 3), dried over anhydrous sodium sulphate and concentrated under reduced pressure. The residue was purified by silica gel column chromatography (ethyl acetate/petroleum ether (V/V) = 0/10-1/10) to obtain compound **14A** (0.4 g, yield: 71%).

Step 2: Preparation of **14B**

Compound **14A** (0.4 g, 1.35 mmol) was dissolved in a mixed solvent of ethanol (6 mL) and water (2 mL). Ammonium chloride (0.7 g, 13.07 mmol) and iron powder (0.74 g, 13.25 mmol) were added sequentially. After the addition, the mixture was warmed to 75°C, stirred for 1.5 h, cooled to room temperature and filtered. The filter cake was washed with 20 mL of dichloromethane. The filtrates were combined and washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 0/10-3/10) to obtain compound **14B** (0.3 g, yield: 84%).

Step 3: Preparation of **14C**

Compound **14B** (0.3 g, 1.1 mmol) was dissolved in THF (10 mL). In an ice bath, N,N'-carbonyldiimidazole (0.54 g, 3.3 mmol) was added in portions. After the addition, the mixture was naturally warmed to room temperature and stirred for 2 h. 10 mL of a saturated aqueous sodium bicarbonate solution was added, and the mixture was stirred for 10 min and extracted with 20 mL of dichloromethane. The organic layer was washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 1/10-1/2) to obtain compound **14C** (0.2 g, yield: 61%).

LCMS m/z = 299.1$[M+H]^+$

Step 4: Preparation of **14D**

Compound **14C** (0.1 g, 0.34 mmol), **2F** (0.15 g) and potassium carbonate (0.094 g, 0.68 mmol) were mixed and dissolved in DMF (3 mL). The mixture was warmed to 100°C and stirred for 16 h, further warmed to 120°C and stirred for 24 h, and cooled to room temperature. 20 mL of ethyl acetate and 20 mL of water were added, and the mixture was stirred for layer separation. The organic layer was washed with a saturated aqueous sodium chloride solution (10 mL x 3), dried over anhydrous sodium sulphate and concentrated under reduced pressure. The residue was purified by silica gel column chromatography (dichloromethane/methanol (V/V) = 100/0-100/3). The resulting residue was further purified by reversed-phase column chromatography (acetonitrile/water (containing 0.1% trifluoroacetic acid) (V/V) = 0/100-60/40) to obtain compound **14D** (20 mg, yield: 9%).

Step 5: Preparation of trifluoroacetate of **compound 14**

Compound **14D** (20 mg, 0.03 mmol) was dissolved in a mixed solvent of 1,4-dioxane (0.5 mL) and water (0.1 mL). Lithium hydroxide hydrate (12.6 mg, 0.3 mmol) was added. The mixture was warmed to 60°C, stirred for 3 h and cooled to room temperature. In an ice bath, the mixture was acidified by adding dilute hydrochloric acid, with the solids precipitated. The resulting mixture was extracted 3 times with dichloromethane. The organic phases were combined, dried over anhydrous sodium sulphate and concentrated under reduced pressure. The residue was purified by reverse-phase column chromatography (mobile phase: acetonitrile/0.1% TFA in water (V/V) = 5/95-50/50). The resulting product was treated with a saturated aqueous sodium bicarbonate solution, extracted with dichloromethane and concentrated under reduced pressure. An appropriate amount of water and acetonitrile was added, and the mixture was subjected to lyophilisation to obtain the trifluoroacetate of **compound 14** (10 mg)

LCMS m/z = 644.2[M+H]$^+$

$^1$H NMR (400 MHz, DMSO-$d_6$) δ 11.57 (s, 1H), 7.64-7.56 (m, 1H), 7.26 (s, 1H), 7.14-7.04 (m, 4H), 4.29 (t, 2H), 2.93 (s, 2H), 2.82-2.62 (m, 2H), 1.82 (s, 3H), 1.15-1.05 (m, 6H).

Preparation of **compound 14-1 and compound 14-2**

**Compound 14** (100 mg) was subjected to chiral resolution, and the chiral resolution method was as follows:

1. instrument: SFC Prep 150 AP; chromatographic column: IG (19 mm×250 mm)
2. The sample was dissolved in methanol and filtered with a 0.45 μm filter to prepare a sample solution.
3. Preparative chromatography conditions: a. composition of mobile phases A and B: mobile phase A: $CO_2$; mobile phase B: methanol (0.05% aqueous ammonia); b. isocratic elution, mobile phase B: 20%; c. flow rate: 40 ml/min.

After preparative separation, the components with the same retention time were combined and concentrated under reduced pressure to obtain **compound 14-1** (40 mg) and **compound 14-2** (40 mg).

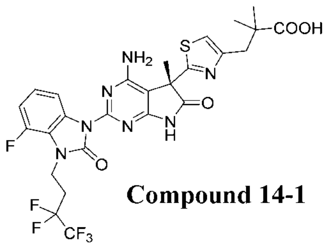

**Compound 14-1**

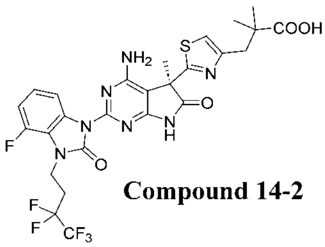

**Compound 14-2**

**Compound 14-1:** retention time under chiral preparative conditions: 5.40 min, LCMS m/z = 644.2 [M+H]$^+$

$^1$H NMR (400 MHz, DMSO-$d_6$) δ 11.52 (s, 1H), 7.64 - 7.56 (m, 1H), 7.26 (s, 1H), 7.16 - 6.99 (m, 4H), 4.29 (t, 2H), 2.93 (s, 2H), 2.81-2.64 (m, 2H), 1.82 (s, 3H), 1.15 - 1.05 (m, 6H).

**Compound 14-2:** retention time under chiral preparative conditions: 8.53 min, LCMS m/z = 644.2 [M+H]$^+$

$^1$H NMR (400 MHz, DMSO-$d_6$) δ 11.48 (s, 1H), 7.64 - 7.55 (m, 1H), 7.26 (s, 1H), 7.20 - 6.95 (m, 4H), 4.29 (t, 2H), 2.93 (s, 2H), 2.82 - 2.62 (m, 2H), 1.82 (s, 3H), 1.18 - 1.01 (m, 6H).

**Example 15:**

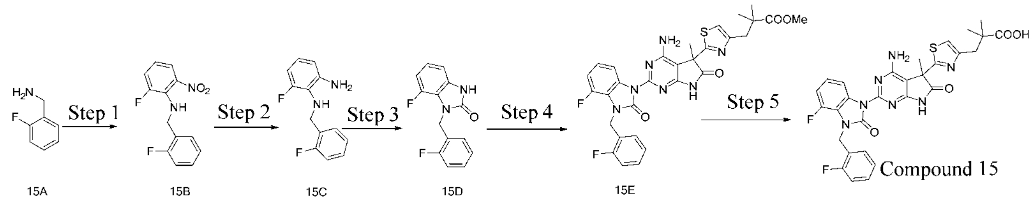


Step 1: Preparation of **15A**

Compound 2,3-difluoronitrobenzene (0.23 g, 1.87 mmol) and compound **15A** (0.3 g, 1.87 mmol) were dissolved in DMSO (5 mL). Diisopropylethylamine (0.71 g, 5.49 mmol) was added, and the mixture was warmed to 60°C, stirred for 4 h and cooled to room temperature. 30 mL of MTBE and 30 mL of water were added, and the mixture was stirred for layer separation. The organic layer was washed with a saturated aqueous sodium chloride solution (20 mL x 3), dried over anhydrous sodium sulphate and concentrated under reduced pressure. The residue was purified by silica gel column chromatography (ethyl acetate/petroleum ether (V/V) = 0/10-1/10) to obtain compound **15B** (0.4 g, yield: 81%).

Step 2: Preparation of **15C**

Compound **15B** (0.4 g, 1.51 mmol) was dissolved in a mixed solvent of ethanol (6 mL) and water (2 mL). Ammonium chloride (0.81 g, 15.1 mmol) and iron powder (0.84 g, 15.1 mmol) were added sequentially. After the addition, the mixture was warmed to 75°C, stirred for 1.5 h, cooled to room temperature and filtered. The filter cake was washed with 20 mL of dichloromethane. The filtrates were combined and washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 0/10-3/10) to obtain compound **15C** (0.3 g, yield: 85%).

Step 3: Preparation of **15D**

Compound **15C** (0.3 g, 1.28 mmol) was dissolved in THF (10 mL). In an ice bath, N,N'-carbonyldiimidazole (0.62 g, 3.84 mmol) was added in portions. After the addition, the mixture was naturally warmed to room temperature and stirred for 2 h. 10 mL of a saturated aqueous sodium bicarbonate solution was added, and the mixture was stirred for 10 min and extracted with 20 mL of dichloromethane. The organic layer was washed with 20 mL of a saturated aqueous sodium chloride solution. The organic phase was dried over anhydrous sodium sulphate and concentrated under reduced pressure. The residue was purified by silica gel column chromatography (ethyl acetate/petroleum ether (V/V) = 1/10-1/2) to obtain compound **15D** (0.2 g, yield: 60%).

LCMS m/z = 261.2$[M+H]^+$

Step 4: Preparation of **15E**

Compound **15D** (0.15 g, 0.58 mmol), **2F** (0.25 g) and potassium carbonate (0.16 g, 1.16 mmol) were mixed and dissolved in DMF (5 mL). The mixture was warmed to 100°C and stirred for 16 h, further warmed to 120°C and stirred for 24 h, and cooled to room temperature. 20 mL of ethyl acetate and 20 mL of water were added, and the mixture was stirred for layer separation. The organic layer was washed with a saturated aqueous sodium chloride solution (10 mL x 3), dried over anhydrous sodium sulphate and concentrated under reduced pressure. The residue was purified by silica gel column chromatography (dichloromethane/methanol (V/V) = 100/0-100/3). The resulting residue was further purified by reversed-phase column chromatography (acetonitrile/water (containing 0.1% trifluoroacetic acid) (V/V) = 0/100-60/40) to obtain compound **15E** (0.1 g, yield: 28%).

Step 5: Preparation of **compound 15**

Compound **15E** (100 mg, 0.16 mmol) was dissolved in a mixed solvent of 1,4-dioxane (2 mL) and water (1 mL). Lithium hydroxide hydrate (38 mg, 1.6 mmol) was added. The mixture was warmed to 60°C, stirred for 3 h and cooled to room temperature. In an ice bath, the mixture was acidified by adding dilute hydrochloric acid, with the solids precipitated. The resulting mixture was extracted 3 times with dichloromethane. The organic phases were combined, dried over anhydrous sodium sulphate and concentrated under reduced pressure. The residue was purified by reverse-phase column chromatography (mobile phase: acetonitrile/0.1% TFA in water (V/V) = 5/95-50/50). The resulting product was treated with a saturated aqueous sodium bicarbonate solution, extracted with dichloromethane and concentrated under

reduced pressure. An appropriate amount of water and acetonitrile was added, and the mixture was subjected to lyophilisation to obtain the trifluoroacetate of compound **15** (20 mg)

LCMS m/z = 606.2[M+H]+

1H NMR (400 MHz, DMSO-$d_6$) δ 11.59 (s, 1H), 7.61 (d, 1H), 7.40-7.31 (m, 1H), 7.29 - 6.91 (m, 8H), 5.23 (s, 2H), 2.93 (s, 2H), 1.82 (s, 3H), 1.10 (s, 6H).

Preparation of **compound 15-1** and **compound 15-2:**

**Compound 15** (200 mg) was subjected to chiral resolution, and the chiral resolution method was as follows:

1. Instrument: SFC Prep 150 AP; chromatographic column: IG (19 mm × 250 mm).
2. The sample was dissolved in methanol and filtered with a 0.45 μm filter to prepare a sample solution.
3. Preparative chromatography conditions: a. the mobile phase consists of systems A and B: mobile phase A: $CO_2$; mobile phase B: methanol (aqueous ammonia 0.05%); b. isocratic elution, mobile phase B: 40%; c. flow rate: 40 mL/min.

After preparative separation, the components with the same retention time were combined and concentrated under reduced pressure to obtain **compound 15-1** (70 mg) and **compound 15-2** (80 mg).

**Compound 15-1:** retention time under chiral preparative conditions: 7.38 min, LCMS m/z = 606.2 [M+1]+

**Compound 15-2:** retention time under chiral preparative conditions: 11.93 min, LCMS m/z = 606.2 [M+1]+

**Example 16:**

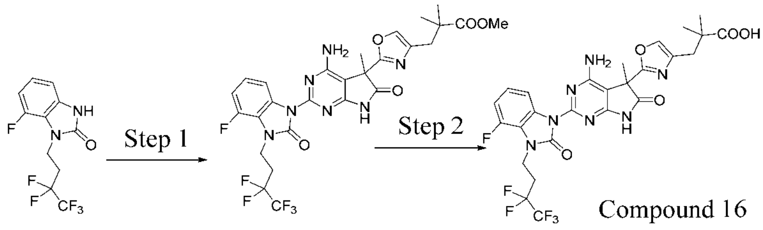

14C 16A Compound 16

With reference to the synthesis for **Example 8, compound 16** (20 mg) was obtained.

LCMS m/z = 628.2[M+H]+

1H NMR (400 MHz, DMSO-$d_6$) δ 11.52 (s, 1H), 7.75 (s, 1H), 7.63 - 7.56 (m, 1H), 7.13-7.05 (m, 2H), 6.96-6.68 (m, 2H), 4.29 (t, 2H), 2.82 - 2.60 (m, 4H), 1.82 (s, 3H), 1.10 (s, 3H), 1.09 (s, 3H).

**Example 17:**

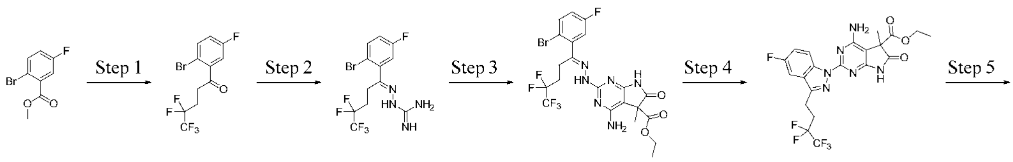


17A 17B 17C 17D

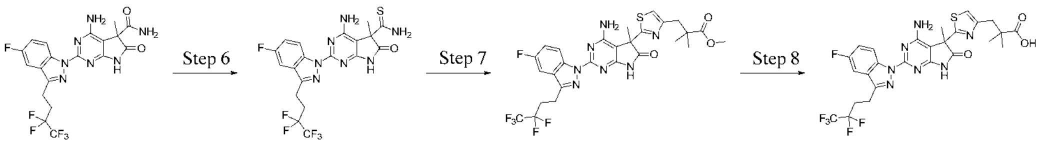


17E 17F 17G **Compound 17**

Step 1: Preparation of **17A**

Compound methyl 2-bromo-5-fluorobenzoate (5 g, 21.43 mmol) and 4,4,5,5,5-pentafluoropentanoic acid (5.66 g, 23.57 mmol) were dissolved in THF (100 mL). At -78°C, sodium bis(trimethylsilyl)amide (32.1 mL, 2 M, 64.28 mmol) was added, and the mixture was stirred and reacted at this temperature for 15 min, and then warmed to 0°C and reacted for 2 h. The reaction was quenched with 1 *N* hydrochloric acid (160 mL), and the mixture was stirred overnight at room temperature and extracted with ethyl acetate. The organic phase was washed twice with a saturated aqueous sodium bicarbonate solution, washed once with a saturated aqueous sodium chloride solution, dried over anhydrous sodium sulphate and concentrated under reduced pressure. The residue was purified by column chromatography (ethyl acetate/petroleum ether (V/V) = 0/1-1/20) to obtain compound **17A** (2.9 g, yield: 38%).

Step 2: Preparation of **17B**

Compound **17A** (2 g, 5.73 mmol) was dissolved in methanol (20 mL). Aminoguanidine hydrochloride (0.95 g, 8.59 mmol) and boron trifluoride diethyl etherate (1.41 mL, 11.46 mmol) were added. The tube was sealed, warmed to 100°C, reacted for 3 h and cooled to room temperature. A 1 *N* aqueous sodium hydroxide solution and ethyl acetate were added, and the mixture was stirred for layer separation. The organic phase was washed with a saturated aqueous sodium chloride solution, dried over anhydrous sodium sulphate and then concentrated under reduced pressure to obtain compound **17B,** which was directly used in the next step.

LCMS m/z = 405.10 $[M+H]^+$

Step 3: Preparation of **17C**

Compound **17B** (1 g, 2.47 mmol), **3H** (1.18 g, 4.94 mmol) and potassium tert-butoxide (0.28 g, 2.47 mmol) were mixed and dissolved in tert-butanol (10 mL). The tube was sealed, warmed to 130°C, stirred for 3 h and cooled to room temperature. A 1 N aqueous sodium hydroxide solution and ethyl acetate were added, and the mixture was stirred for layer separation. The organic phase was washed with a saturated aqueous sodium chloride solution, dried over anhydrous sodium sulphate and concentrated under reduced pressure. The residue was purified by column chromatography (mobile phase: dichloromethane/methanol (V/V) = 1/0-10/1) to obtain compound **17C** (320 mg, yield: 21%).

Step 4: Preparation of **17D**

Compound **17C** (300 mg, 0.50 mmol) was dissolved in DMF (5 mL). N,N-dimethylethylenediamine (66.11 mg, 0.75 mmol) and cuprous iodide (19.04 mg, 0.10 mmol) were added, and the mixture was purged three times with nitrogen. Under nitrogen atmosphere, the mixture was reacted for 2 h. Ethyl acetate and water were added, and the mixture was stirred for layer separation. The organic layer was washed three times with a saturated aqueous sodium chloride solution, dried over anhydrous sodium sulphate and concentrated under reduced pressure. The residue was purified by column chromatography (dichloromethane/methanol (V/V) = 1/0-10/1) to obtain compound **17D** (190 mg, yield: 73%).

Step 5: Preparation of **17E**

Compound **17D** (190 mg, 0.37 mmol) was dissolved in methanol (3 mL). A solution of ammonia in methanol (3 mL, 7 M) was added, and the mixture was reacted overnight at 40°C and concentrated under reduced pressure to obtain

compound **17E** (180 mg).

LCMS m/z = 488.30 [M+H]+

Step 6: Preparation of **17F**

Compound **17E** (180 mg, 0.37 mmol) was dissolved in toluene (5 mL). Lawesson's Reagent (224.48 mg, 0.55 mmol) was added. After the addition, the mixture was warmed to 80°C, stirred overnight, cooled to room temperature and concentrated under reduced pressure. The residue was purified by silica gel column chromatography (mobile phase: dichloromethane/methanol (V/V) = 1/0-5/1) to obtain compound **17F** (170 mg, yield: 91%).

LCMS m/z = 504.20 [M+H]+

Step 7: Preparation of**17G**

Compound **17F** (170 mg, 0.34 mmol) was dissolved in ethanol (5 mL). **2D** (110.25 mg, 0.46 mmol) was added. After the addition, the mixture was warmed to 80°C, stirred overnight, cooled to room temperature and concentrated under reduced pressure. The residue was purified by silica gel column chromatography (mobile phase: dichloromethane/-methanol (V/V) = 1/0-5/1) to obtain compound **17G** (150 mg, yield: 68%).

LCMS m/z = 642.40 [M+H]+

Step 8: Preparation of **compound 17**

Compound **17G** (150 mg, 0.23 mmol) was dissolved in a mixed solvent of 1,4-dioxane (3 mL) and water (3 mL). Lithium hydroxide hydrate (96.51 mg, 2.3 mmol) was added. The mixture was warmed to 60°C, stirred for 3 h and cooled to room temperature. In an ice bath, the mixture was acidified by adding dilute hydrochloric acid, with the solids precipitated. The resulting mixture was extracted 3 times with dichloromethane. The organic phases were combined, dried over anhydrous sodium sulphate and concentrated under reduced pressure. The resulting crude was further subjected to preparative HPLC (instrument: Waters AutoP (preparative liquid phase); chromatographic column: SunFire@ Prep C18 (19 mm×250 mm); composition of mobile phase: Mobile phase A: acetonitrile, mobile phase B: water (containing 5 mM ammonium acetate) to obtain **compound 17** (65 mg, yield: 45%).

LCMS m/z = 628.20 [M+H]+

$^1H$ NMR (400 MHz, DMSO-$d_6$) δ 12.26-12.02 (m, 1H), 11.52 (s, 1H), 8.87 (dd,1H), 7.81 (dd, 1H), 7.47-7.39 (m, 1H), 7.25 (s, 1H), 7.20-7.00 (m, 2H), 3.30 - 3.24 (m, 2H), 2.93 (s, 2H), 2.88 - 2.66 (m, 2H), 1.81 (s, 3H), 1.10 (s, 6H).

Preparation of **compound 17-1** and **compound 17-2:**

**Compound 17** (60 mg) was subjected to chiral resolution, and the chiral resolution method was as follows:

1. Instrument: SFC Prep 150 AP; chromatographic column: IG (19 mm × 250 mm).
2. The sample was dissolved in methanol and filtered with a 0.45 μm filter to prepare a sample solution.
3. Preparative chromatography conditions: a. the mobile phase consists of systems A and B: mobile phase A: $CO_2$; mobile phase B: methanol (aqueous ammonia 0.05%); b. isocratic elution, mobile phase B: 30%; c. flow rate: 45 mL/min.

After preparative separation, the components with the same retention time were combined and concentrated under reduced pressure to obtain **compound 17-1** (15 mg) and **compound 17-2** (20 mg).

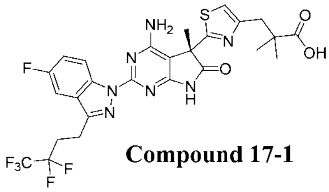

**Compound 17-1**

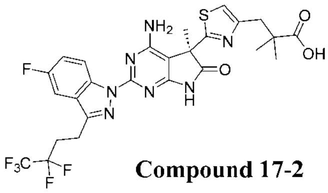

**Compound 17-2**

**Compound 17-1:** retention time under chiral preparative conditions: 3.33 min, LCMS m/z = 628.0 [M+1]+

$^1H$ NMR (400 MHz, DMSO-$d_6$) δ 12.24 - 12.11 (m, 1H), 11.52 (s, 1H), 8.87 (dd, 1H), 7.81 (dd, 1H), 7.49 - 7.38 (m, 1H), 7.25 (s, 1H), 7.21 - 7.01 (m, 2H), 3.30 - 3.24 (m, 2H), 2.93 (s, 2H), 2.87 - 2.69 (m, 2H), 1.82 (s, 3H), 1.11 (s, 6H).

**Compound 17-2:** retention time under chiral preparative conditions: 7.02 min, LCMS m/z = 628.0 [M+1]$^+$

**Example 18:**

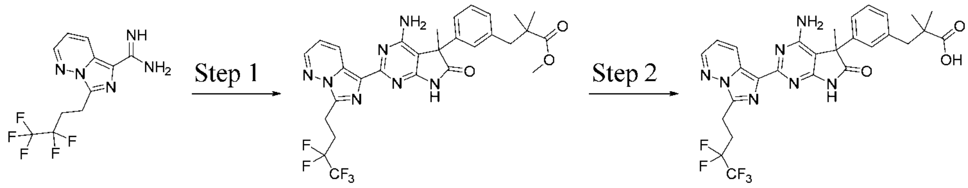


3G 18A **Compound 18**

With reference to the synthesis for **Example 3** and **Example 6,** compound **18** (100 mg) was obtained.

LCMS m/z = 604.2 [M+H]$^+$

$^1$H NMR (400 MHz, DMSO-$d_6$) δ 12.55-11.75 (m, 1H), 11.14 (s, 1H), 8.97 (d, 1H), 8.50 (s, 1H), 7.28-7.21 (m, 1H), 7.18 - 7.00 (m, 4H), 6.72-6.35 (m, 2H), 3.46 - 3.35 (m, 2H), 2.95-2.69 (m, 4H), 1.79 (s, 3H), 1.06 (s, 3H), 1.02 (s, 3H).

**Example 19:**

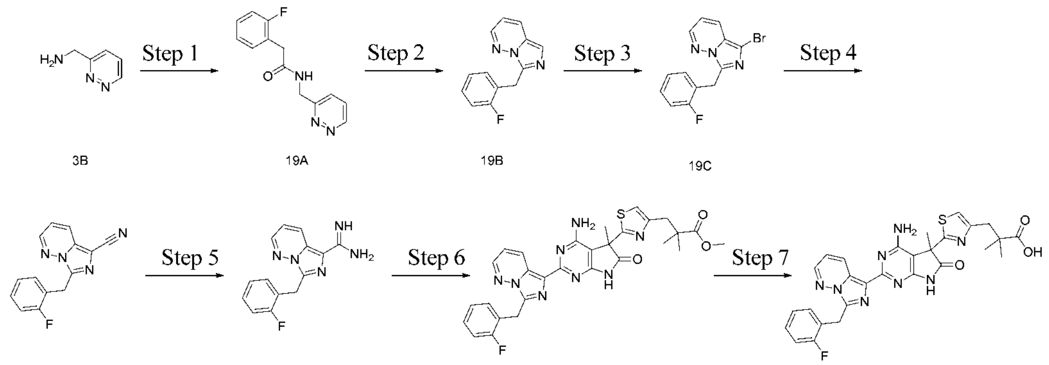


3B 19A 19B 19C

19D 19E 19F **Compound 19**

Step 1: Preparation of **19A**

Compound **3B hydrochloride** (2.83 g, 19.41 mmol) and o-fluorophenylacetic acid (2 g, 12.94 mmol) were dissolved in DMF (20 mL). HATU (7.38 g, 19.41 mmol) was added, and the mixture was stirred at room temperature for 30 min. DIPEA (5.02 g, 38.81 mmol) was added, and the mixture was reacted at room temperature for 1 h and extracted with water and ethyl acetate. The organic phase was washed with a saturated aqueous sodium chloride solution, dried over anhydrous sodium sulphate and concentrated under reduced pressure. The residue was purified by column chromatography (dichloromethane/methanol (V/V) = 100/0-100/10) to obtain compound **19A** (420 mg, yield: 13%).

LCMS m/z = 246.2 [M+H]$^+$

Step 2: Preparation of **19B**

Compound **19A** (420 mg, 1.71 mmol) was dissolved in DCE (20 mL). Phosphorus oxychloride (0.91 mL, 9.94 mmol) was added, and the mixture was warmed to reflux, reacted overnight, cooled to room temperature and concentrated under reduced pressure. The residue was carefully and slowly added to water, and the mixture was stirred for 5 min. Ethyl acetate was added, and the mixture was stirred for layer separation. The aqueous layer was treated with a saturated aqueous sodium bicarbonate solution and then extracted three times with ethyl acetate. The organic layers were combined, washed with a saturated aqueous sodium chloride solution, dried over anhydrous sodium sulphate and concentrated under reduced pressure. The residue was directly purified by column chromatography (ethyl acetate/petroleum ether (V/V) = 0/100-20/100) to obtain compound **19B** (250 mg, yield: 64%).

LCMS m/z = 228.1 $[M+H]^+$

Step 3: Preparation of **19C**

Compound **19B** (250 mg, 1.10 mmol) was dissolved in DCM (10 mL). NBS (215.36 mg, 1.21 mmol) was added, and the mixture was reacted at room temperature for 30 min. Water was added, and the mixture was stirred for layer separation. The organic layer was washed with a saturated aqueous sodium chloride solution, dried over anhydrous sodium sulphate and concentrated under reduced pressure. The residue was purified by column chromatography (ethyl acetate/petroleum ether (V/V) = 0/100-50/100) to obtain compound **19C** (280 mg, yield: 83%).

LCMS m/z = 305.90 $[M+H]^+$

Step 4: Preparation of **19D**

Compound **19C** (280 mg, 0.91 mmol) and zinc cyanide (220 mg, 2.06 mmol) were added to a 50 mL single-necked flask, and then zinc powder (150 mg, 2.29 mmol), 1,1'-bis(diphenylphosphino)ferrocene (302.69 mg, 0.55 mmol), tris(dibenzylidene-BASE acetone)dipalladium (250 mg, 0.27 mmol) and N,N-dimethylacetamide (10 mL) were added. Under nitrogen atmosphere, the mixture was warmed to 120°C and stirred for 2 h. The reaction solution was diluted in 50 mL of ethyl acetate, washed 3 times with water and washed once with saturated sodium chloride. The organic phase was dried over anhydrous sodium sulphate and then concentrated under reduced pressure. The residue was subjected to flash column chromatography (mobile phase: petroleum ether/ethyl acetate (V/V) = 2/1) to obtain compound **19D** (210 mg, yield: 91%).

LCMS m/z = 253.10 $[M+H]^+$

Step 5: Preparation of **19E**

In an ice bath, ammonium chloride (221.98 mg, 4.15 mmol) was added to a 50 mL single-necked flask. Toluene (10 mL) was added, and then trimethylaluminium (2.07 mL, 2 M in toluene) was slowly added dropwise. The mixture was warmed to room temperature, stirred and reacted for 3 h. Compound **19D** (210 mg, 0.83 mmol) was dissolved in toluene and added dropwise to the reaction solution. The mixture was warmed to 110°C, stirred overnight and cooled to room temperature. In an ice bath, silica gel and 20 mL of methanol were added. The mixture was further stirred for 30 min and subjected to suction filtration. The filter cake was washed 3 times with methanol. The organic phases were combined and concentrated under reduced pressure. The residue was purified by column chromatography (mobile phase: dichloromethane/methanol (V/V) = 8/1) to obtain compound **19E** (200 mg, yield: 89%).

LCMS m/z = 270.2 $[M+H]^+$

Step 6: Preparation of **19F**

Compound **19E** (100 mg, 0.37 mmol), ethyl 3,3-dicyano-2-(4-(3-methoxy-2,2-dimethyl-3-oxopropyl)thiazo-2-yl)-2-methylpropanoate (CAS: 2101649-65-2, synthesised with reference to US20170174693) (201.70 mg, 0.55 mmol) and potassium bicarbonate (111.13 mg, 1.11 mmol) were mixed and dissolved in tert-butanol (5 mL). The mixture was warmed to 80°C, stirred overnight, cooled to room temperature, and directly concentrated under reduced pressure. The residue was purified by column chromatography (mobile phase: dichloromethane/methanol (V/V) = 15/1) to obtain compound **19F** (190 mg, yield: 87%).

LCMS m/z = 587.2 $[M+H]^+$

Step 7: Preparation of **compound 19**

Compound **19F** (190 mg, 0.32 mmol) was dissolved in a mixed solvent of 1,4-dioxane (3 mL) and water (3 mL). Lithium hydroxide hydrate (134.27 mg, 3.2 mmol) was added. The mixture was warmed to 60°C, stirred for 3 h and cooled to room temperature. In an ice bath, the mixture was acidified by adding dilute hydrochloric acid. The resulting mixture was extracted 3 times with dichloromethane. The organic phases were combined, dried over anhydrous sodium sulphate and concentrated under reduced pressure. The residue was purified by reverse-phase column chromatography (mobile phase: acetonitrile/0.1% TFA in water (V/V) = 5/95-50/50) to obtain the trifluoroacetate of compound **19** (160 mg).

LCMS m/z = 573.1 $[M+H]^+$

$^1H$ NMR (400 MHz, DMSO-$d_6$) δ 12.50-11.80 (m, 1H), 11.41 (s, 1H), 8.97 (d, 1H), 8.46 (d, 1H), 7.36 - 7.10 (m, 5H), 7.04 (dd, 1H), 6.98-6.79 (m, 2H), 4.51 (s, 2H), 2.93 (s, 2H), 1.79 (s, 3H), 1.10 (s, 6H).

Preparation of **compound 19-1** and **compound 19-2:**

**Compound 19** (140 mg) was subjected to chiral separation and purification. Preparative conditions: instrument: Waters 150 Prep-SFC; chromatographic column: AD; column temperature: 35°C; mobile phase A: $CO_2$, and mobile phase B: methanol/acetonitrile (aqueous ammonia 0.1%); gradient: B: 55%; back pressure: 100 bar; cycle: 5.3 min; detection wavelength: 220 nm; flow rate: 100 ml/min.

Analysis conditions: instrument: SHIMADZU LC-30AD sf; chromatographic column: AD; mobile phase A: $CO_2$, and mobile phase B: methanol and acetonitrile (0.05% DEA); flow rate: 3 ml/min; column temperature: 35°C; detection wavelength: 220 nm.

After preparative separation, the components with the same retention time were combined and concentrated under reduced pressure to obtain **compound 19-1** (56 mg) and **compound 19-2** (61 mg).

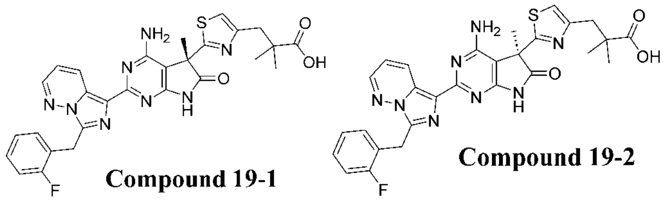

Compound 19-1 Compound 19-2

**Compound 19-1:** retention time under analysis conditions: 0.519 min, LCMS m/z = 573.2 $[M+H]^+$

$^1H$ NMR (400 MHz, DMSO-$d_6$) δ 10.96 - 9.69 (m, 1H), 9.00 (dd, 1H), 8.43 (dd, 1H), 7.38 - 7.06 (m, 5H), 6.98 (dd, 1H), 6.89 - 6.70 (m, 2H), 4.49 (s, 2H), 2.93 (s, 2H), 1.78 (s, 3H), 1.09 (s, 6H).

**Compound 19-2:** retention time under analysis conditions: 1.553 min, LCMS m/z = 573.1 $[M+H]^+$

$^1H$ NMR (400 MHz, DMSO-$d_6$) δ 11.85 - 11.05 (m, 1H), 9.00 (dd, 1H), 8.43 (dd, 1H), 7.37 - 7.09 (m, 5H), 6.98 (dd, 1H), 6.87 - 6.71 (m, 2H), 4.49 (s, 2H), 2.93 (s, 2H), 1.78 (s, 3H), 1.09 (s, 6H).

**Example 20:**

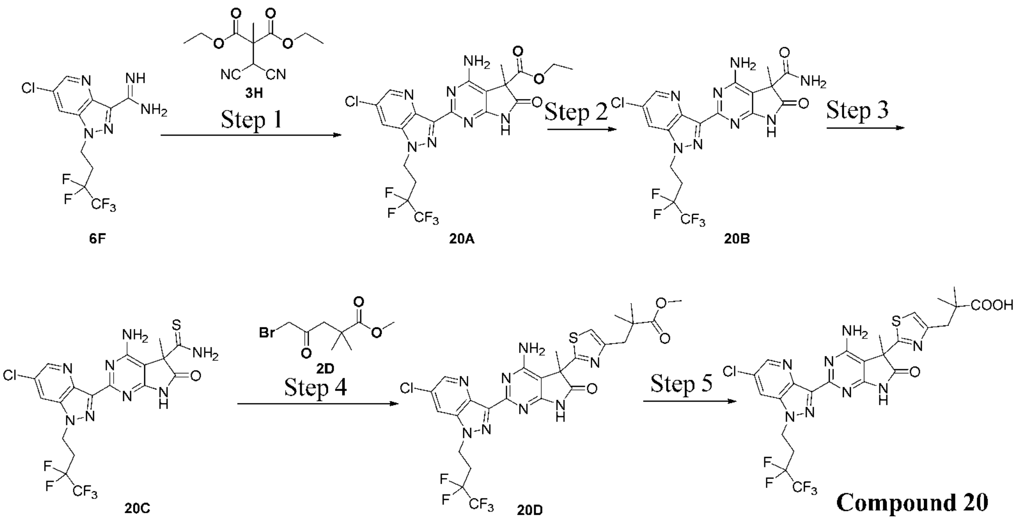


6F 3H 20A 20B 20C 2D 20D Compound 20

With reference to the synthesis for **Example 6** and **Example 21,** compound **20** (0.035 g) was obtained.

LCMS m/z = 645.1 $[M+H]^+$

$^1H$ NMR (400 MHz, DMSO-$d_6$) δ 12.50-11.80 (m, 1H), 11.55 (s, 1H), 8.66-8.61 (m, 2H), 7.26 (s, 1H), 6.90 *(br.s,* 2H), 4.86 (t, 2H), 3.04 - 2.86 (m, 4H), 1.81 (s, 3H), 1.10 (s, 6H).

**Example 21:**

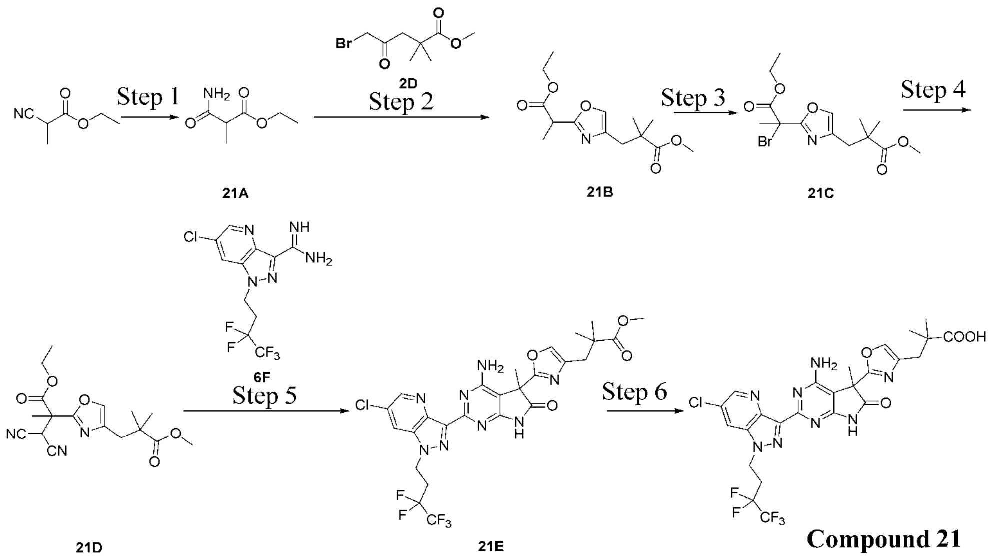


2D, 21A, 21B, 21C, 6F, 21D, 21E, Compound 21

Step 1: Preparation of **21A**

Ethyl 2-cyanopropionate (5.00 g, 39.32 mmol) was added to a 100 mL reaction flask. In an ice bath, trimethylchlorosilane (8.54 g, 78.64 mmol) was added dropwise, and then water (1.42 g, 78.64 mmol) was added dropwise. After the addition, the mixture was stirred at room temperature for 4 h. 50 mL of water was added to the reaction solution. The mixture was extracted with n-hexane, and the aqueous phase was separated, collected, basified with a saturated aqueous sodium bicarbonate solution, and extracted 3 times with dichloromethane. The organic phase was collected, dried over anhydrous sodium sulphate and concentrated under reduced pressure to obtain compound **21A** (1.46 g, yield: 25%).

LCMS m/z = 146.1 $[M+H]^+$

Step 2: Preparation of **21B**

Compound **2D** (0.50 g, 2.11 mmol) and **21A** (0.31 g, 2.15 mmol) were dissolved in ethyl acetate (10 mL), and then silver trifluoromethanesulphonate (0.54 g, 2.11 mmol) was added. After the addition, the mixture was warmed to 90°C and stirred for 2 h in the dark. The reaction solution was cooled to room temperature and subjected to suction filtration to remove solids. The filter cake was washed 3 times with dichloromethane. The organic phases were combined and concentrated under reduced pressure. The residue was subjected to flash column chromatography (mobile phase: petroleum ether/ethyl acetate (V/V) = 3/1) to obtain compound **21B** (0.30 g, yield: 31%).

LCMS m/z = 284.40 $[M+H]^+$

Step 3: Preparation of**21C**

Compound **21B** (0.30 g, 1.06 mmol) was dissolved in tetrahydrofuran (10 mL). Under nitrogen atmosphere, lithium bis(trimethylsilyl)amide (0.59 mL, 1.17 mmol, 2 M) was added dropwise in an ice bath, and the mixture was reacted at the same temperature for 0.5 hours. Then, N-bromosuccinimide (0.21 g, 1.17 mmol) was added. After the addition, the mixture was reacted for 0.5 hours in an ice bath. The reaction was quenched with saturated ammonium chloride and extracted 3 times with ethyl acetate. The organic phases were combined, dried over anhydrous sodium sulphate and concentrated under reduced pressure. The residue was subjected to flash column chromatography (mobile phase: petroleum ether/ethyl acetate (V/V) = 3/1) to obtain compound **21C** (0.217 g, yield: 56%).

LCMS m/z = 362.40 $[M+H]^+$

Step 4: Preparation of **21D**

Compound **21C** (0.217 g, 0.60 mmol) and malononitrile (0.079 g, 1.20 mmol) were dissolved in tetrahydrofuran (10 mL). In an ice bath, DBU (0.18 g, 1.20 mmol) was added dropwise. After the addition, the mixture was reacted in an ice

bath for 0.5 hours. The reaction was quenched with saturated ammonium chloride and extracted 3 times with ethyl acetate. The organic phases were combined, dried over anhydrous sodium sulphate and concentrated under reduced pressure. The residue was subjected to flash column chromatography (mobile phase: petroleum ether/ethyl acetate (V/V) = 3/1) to obtain compound **21D** (0.16 g, yield: 76%).

LCMS m/z = 348.1 $[M+H]^+$

Step 5: Preparation of**21E**

Compound **6F** (0.10 g, 0.29 mmol), **21D** (0.10 g, 0.29 mmol) and potassium bicarbonate (0.087 g, 0.87 mmol) were mixed and dissolved in tert-butanol (10 mL). The mixture was warmed to 80°C, stirred and reacted for 16 hours, cooled to room temperature, and directly concentrated under reduced pressure. The residue was purified by column chromatography (mobile phase: dichloromethane/methanol (V/V) = 15/1) to obtain compound **21E** (0.142 g, yield: 76%).

LCMS m/z = 643.2$[M+H]^+$

Step 6: Preparation of compound **21**

Compound **21E** (0.142 g, 0.22 mmol) was dissolved in a mixed solvent of 1,4-dioxane (5 mL) and water (5 mL). Lithium hydroxide hydrate (0.092 g, 2.2 mmol) was added. The mixture was warmed to 60°C, stirred for 3 h and cooled to room temperature. In an ice bath, the mixture was acidified by adding dilute hydrochloric acid, with white solids precipitated. The resulting mixture was extracted 3 times with dichloromethane. The organic phases were combined, dried over anhydrous sodium sulphate and concentrated under reduced pressure. The residue was purified by reverse-phase column chromatography (mobile phase: acetonitrile/0.1% TFA in water (V/V) = 5/95-50/50) to obtain compound **21** (0.095 g, yield: 67%).

LCMS m/z = 629.1 $[M+H]^+$

$^1H$ NMR (400 MHz, DMSO-$d_6$) δ 12.50-11.80 (m, 1H), 11.47 (s, 1H), 8.65-8.61 (m, 2H), 7.75 (s, 1H), 6.79 - 6.41 (m, 2H), 4.86 (t, 2H), 3.08-2.87 (m, 2H), 2.73-2.61 (m, 2H), 1.82 (s, 3H), 1.11 (s, 3H), 1.09 (s, 3H).

**Example 22:**

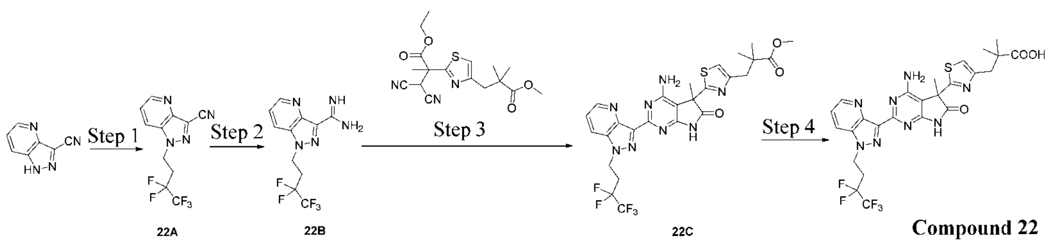


22A 22B 22C Compound 22

With reference to the synthesis for **Example 6, Example 20** and **Example 21,** the trifluoroacetate of **compound 22** (0.031 g) was obtained.

LCMS m/z = 611.2 $[M+H]^+$

$^1H$ NMR (400 MHz, DMSO-$d_6$) δ 11.59 (s, 1H), 8.76 (d, 1H), 8.52 (d, 1H), 7.67-7.59 (m, 1H), 7.27 (s, 1H), 7.13-6.90 (m, 2H), 4.92 (t, 2H), 3.05 - 2.89 (m, 4H), 1.83 (s, 3H), 1.11 (s, 6H).

**Example 23:**

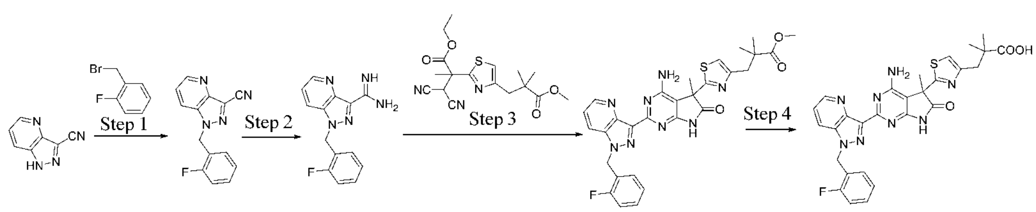


23A 23B 23C Compound 23

With reference to the synthesis for **Example 6, Example 20** and **Example 21,** the trifluoroacetate of **compound 23** (0.021 g) was obtained.

LCMS m/z = 573.2 $[M+H]^+$

$^1$H NMR (400 MHz, DMSO-$d_6$) δ 11.54 (s, 1H), 8.71 (d, 1H), 8.40 (d, 1H), 7.62-7.51 (m, 1H), 7.44-7.32 (m, 1H), 7.30 - 7.12 (m, 4H), 7.05-6.80 (m, 2H), 5.86 (s, 2H), 2.93 (s, 2H), 1.81 (s, 3H), 1.10 (s, 6H).

**Example 24:**

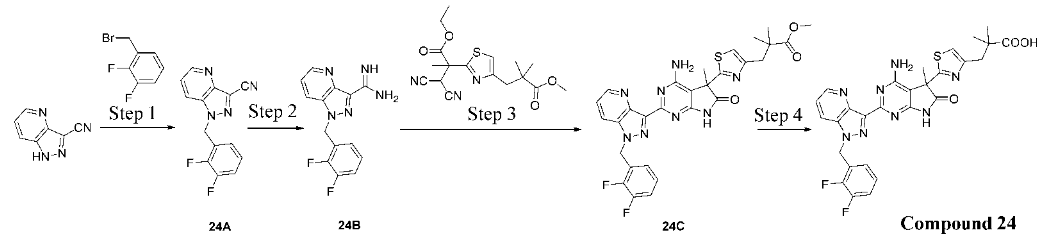


With reference to the synthesis for **Example 6, Example 20** and **Example 21,** compound **24** (0.117 g) was obtained.

LCMS m/z = 591.2 $[M+H]^+$

$^1$H NMR (400 MHz, DMSO-$d_6$) δ 13.00-11.60 (m, 1H), 11.53 (s, 1H), 8.70 (d, 1H), 8.39 (d, 1H), 7.63-7.51 (m, 1H), 7.46-7.35 (m, 1H), 7.26 (s, 1H), 7.23-7.13 (m, 1H), 7.08-6.98 (m, 1H), 6.98-6.82 (m, 2H), 5.91 (s, 2H), 2.94 (s, 2H), 1.82 (s, 3H), 1.10 (s, 6H).

**Example 25:**

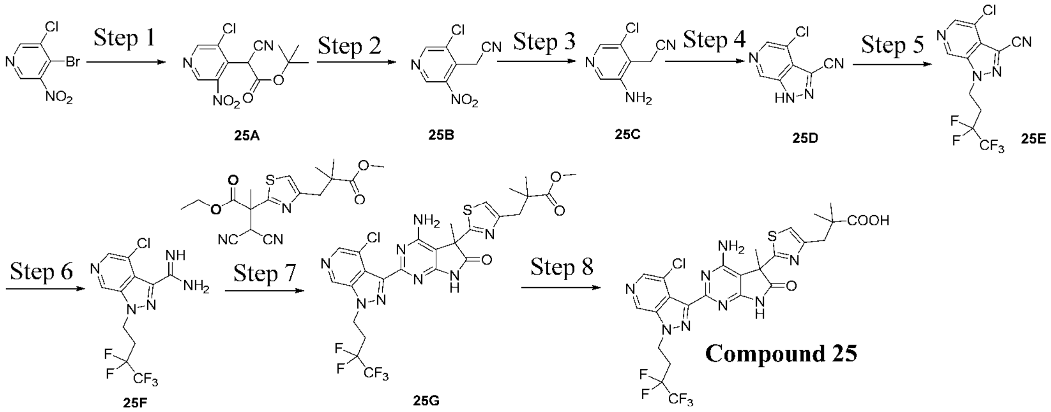


With reference to the synthesis for Example 6, Example 20 and Example 21, compound 25 (250 mg) was obtained.

LCMS m/z = 645.1 $[M+H]^+$

$^1$H NMR (400 MHz, DMSO-$d_6$) δ 12.35-11.95 (m, 1H), 11.43 (s, 1H), 9.31 (s, 1H), 8.35 (s, 1H), 7.28 (s, 1H), 6.89 *(br.s,* 2H), 4.98 (t, 2H), 3.09 - 2.88 (m, 4H), 1.83 (s, 3H), 1.10 (s, 6H).

**Example 26:**

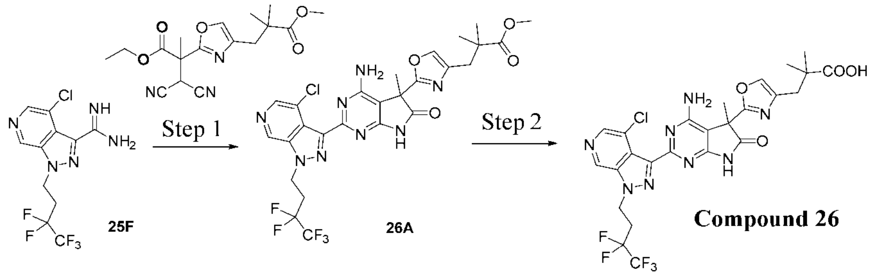


25F 26A Compound 26

With reference to the synthesis for Example 6, Example 20 and Example 21, compound 26 (100 mg) was obtained.

LCMS m/z = 629.1 $[M+H]^+$

$^1H$ NMR (400 MHz, DMSO-$d_6$) δ 12.16 (*br.s,* 1H), 11.37 (s, 1H), 9.31 (s, 1H), 8.35 (s, 1H), 7.76 (s, 1H), 6.73 - 6.45 (m, 2H), 4.98 (t, 2H), 3.08 - 2.91 (m, 2H), 2.75-2.61 (m, 2H), 1.84 (s, 3H), 1.12 (s, 3H), 1.10 (s, 3H).

**Example 27:**

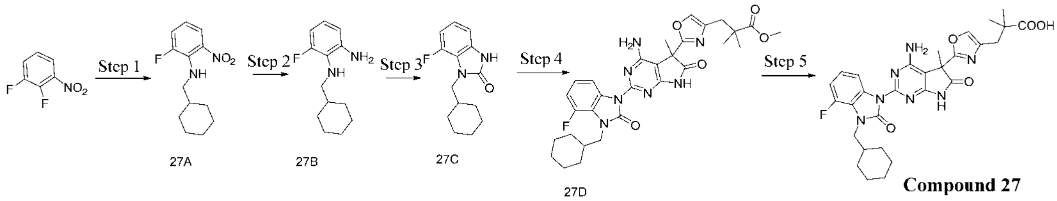


27A 27B 27C 27D Compound 27

With reference to the synthesis for **Example 16,** the trifluoroacetate of **compound 27** (10 mg) was obtained.

LCMS m/z = 578.3$[M+H]^+$

**Example 28:**

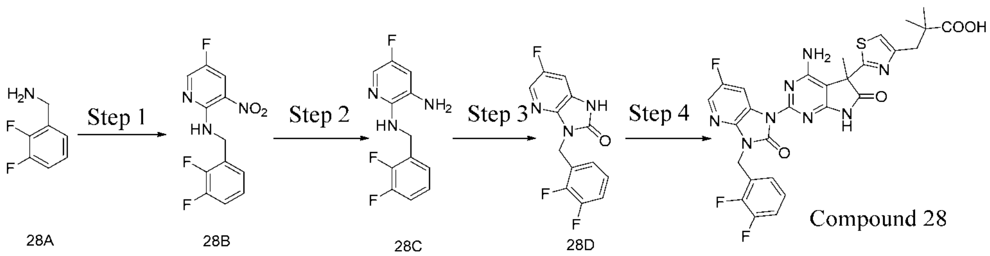


28A 28B 28C 28D Compound 28

With reference to the synthesis for **Example 15,** the trifluoroacetate of **compound 28** (20 mg) was obtained.

LCMS m/z = 623.5$[M-H]^-$

$^1H$ NMR (400 MHz, DMSO-$d_6$) δ 11.56 (s, 1H), 8.37 (dd, 1H), 8.15 - 8.05 (m, 1H), 7.44 - 7.30 (m, 1H), 7.25 (s, 1H), 7.21 - 7.00 (m, 4H), 5.18 (s, 2H), 2.93 (s, 2H), 1.81 (s, 3H), 1.10 (s, 6H).

**Example 29:**

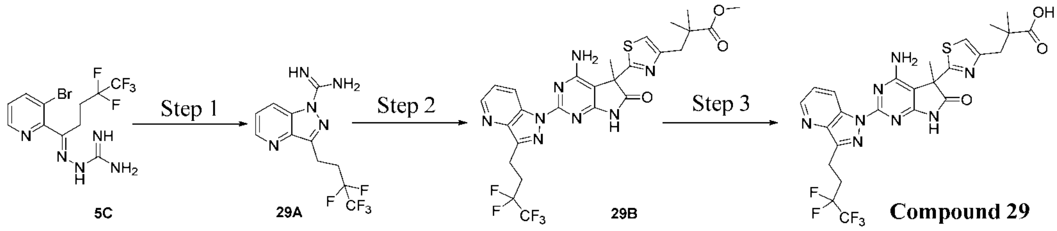


5C 29A 29B Compound 29

With reference to the synthesis for **Example 5, compound 29** (6 mg) was obtained.
LCMS m/z = 611.1[M+H]$^+$
$^1$H NMR (400 MHz, DMSO-$d_6$) δ 11.87 - 11.06 (m, 1H), 9.17 (d, 1H), 8.67 (d, 1H), 7.56 (dd, 1H), 7.32 - 7.07 (m, 3H), 3.41 - 3.33 (m, 2H), 2.99 - 2.81 (m, 4H), 1.81 (s, 3H), 1.10 (s, 6H).

**Example 30:**

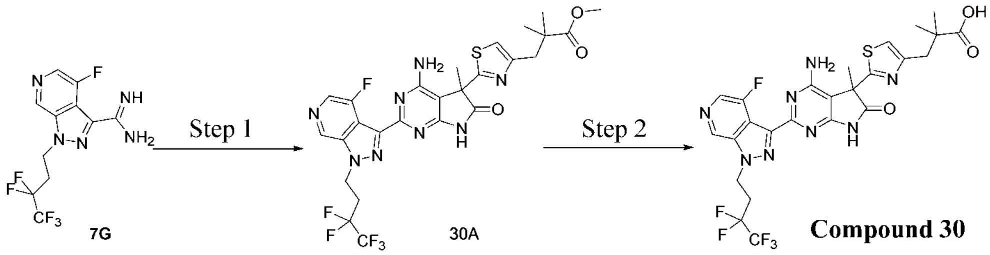


7G 30A Compound 30

With reference to the synthesis for **Example 6, Example 20** and **Example 21,** the trifluoroacetate of **compound 30** (170 mg) was obtained.
LCMS m/z = 629.1 [M+H]$^+$
$^1$H NMR (400 MHz, DMSO-$d_6$)δ 11.47 (s, 1H), 9.23 (s, 1H), 8.31 (s, 1H), 7.26 (s, 1H), 6.88 (s, 2H), 5.00 (t, 2H), 3.10 - 2.86 (m, 4H), 1.82 (s, 3H), 1.10 (s, 6H).

**Example 31:**

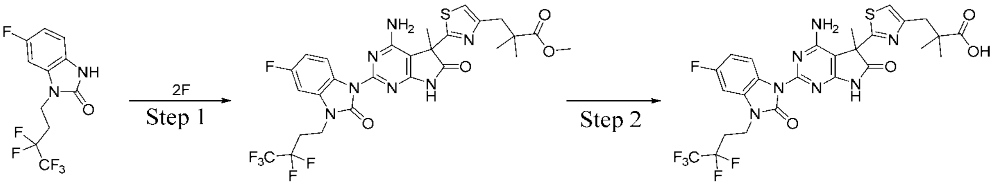


11C 31A Compound 31

Step 1: Preparation of**31A**

Compound **11C** (0.14 g, 0.47 mmol), **2F** (0.30 g) and potassium carbonate (0.13 g, 0.94 mmol) were mixed and dissolved in DMF (5 mL). The mixture was warmed to 100°C, stirred for 16 h, cooled to room temperature and extracted with 20 mL of ethyl acetate and 20 mL of water. The organic layer was washed with a saturated aqueous sodium chloride solution (10 mL × 3), dried over anhydrous sodium sulphate and concentrated under reduced pressure. The residue was purified by silica gel column chromatography (ethyl acetate/petroleum ether (V/V) = 1/5-1/1) to obtain compound **31A** (70 mg, yield: 23%).
LCMS m/z = 658.3 [M+H]$^+$

Step 2: Preparation of **compound 31**

Compound **31A** (0.070 g, 0.11 mmol) was dissolved in a mixed solvent of 1,4-dioxane (10 mL) and water (3 mL).

Lithium hydroxide (0.026 g, 1.09 mmol) was added. The mixture was warmed to 60°C, stirred for 1 h and cooled to room temperature. The reaction solution was adjusted to pH = 3-4 by adding a 1 *N* aqueous HCl solution. 20 mL of ethyl acetate was added, and the organic phase was washed with 10 mL of a saturated aqueous sodium chloride solution, dried over anhydrous sodium sulphate and concentrated under reduced pressure. The residue was purified by column chromatography (acetonitrile/water (containing 0.1% trifluoroacetic acid) (V/V) = 0/100-60/40) to obtain **compound 31** (20 mg, yield: 28%).

LCMS m/z = 644.2 $[M+H]^+$

$^1H$ NMR (400 MHz, DMSO-$d_6$) δ 11.56 (s, 1H), 7.83 (dd, 1H), 7.35 (dd, 1H), 7.26 (s, 1H), 7.17 - 6.99 (m, 2H), 6.96 - 6.87 (m, 1H), 4.22 (t, 2H), 2.93 (s, 2H), 2.83 - 2.63 (m, 2H), 1.81 (s, 3H), 1.10 (s, 6H).

Preparation of **compound 31-1** and **compound 31-2:**

**Compound 31** (180 mg) was subjected to chiral resolution, and the chiral resolution method was as follows:

1. Instrument: SFC Prep 150 AP; chromatographic column: IG (19 mm × 250 mm).
2. The sample was dissolved in methanol and filtered with a 0.45 μm filter to prepare a sample solution.
3. Preparative chromatography conditions: a. the mobile phase consists of systems A and B: mobile phase A: $CO_2$; mobile phase B: methanol /isopropanol = 1/1 (aqueous ammonia 0.05%); b. isocratic elution, mobile phase B: 25%; c. flow rate: 40 mL/min.

After preparative separation, the components with the same retention time were combined and concentrated under reduced pressure to obtain **compound 31-1** (75 mg) and **compound 31-2** (80 mg).

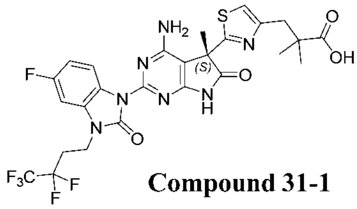
Compound 31-1

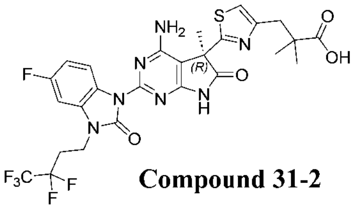
Compound 31-2

**Compound 31-1:** retention time under chiral preparative conditions: 4.37 min, LCMS m/z = 644.2 $[M+1]^+$

$^1H$ NMR (400 MHz, DMSO-$d_6$) δ 11.55 (s, 1H), 7.83 (dd, 1H), 7.35 (dd, 1H), 7.26 (s, 1H), 7.14 - 6.98 (m, 2H), 6.96 - 6.86 (m, 1H), 4.21 (t, 2H), 2.93 (s, 2H), 2.84 - 2.64 (m, 2H), 1.81 (s, 3H), 1.10 (s, 6H).

**Compound 31-2:** retention time under chiral preparative conditions: 6.45 min, LCMS m/z = 644.2 $[M+1]^+$

$^1H$ NMR (400 MHz, DMSO-$d_6$) δ 7.83 (dd, 1H), 7.35 (dd, 1H), 7.25 (s, 1H), 7.12 - 7.00 (m, 2H), 6.95 - 6.87 (m, 1H), 4.22 (t, 2H), 2.93 (s, 2H), 2.82 - 2.63 (m, 2H), 1.81 (s, 3H), 1.10 (s, 6H).

**Example 32:**

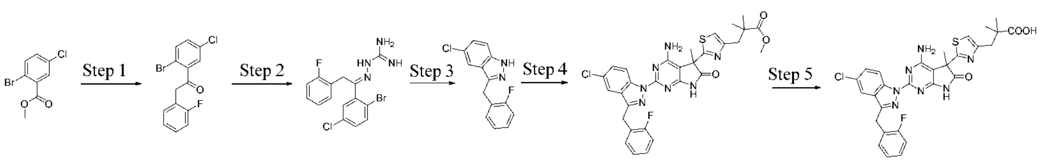

32A 32B 32C 32D Compound 32

Step 1: Preparation of **32A**

Compound methyl 2-bromo-5-chlorobenzoate (5 g, 20.07 mmol) and o-fluorophenylacetic acid (3.40 g, 22.08 mmol) were dissolved in THF (100 mL). At -78°C, sodium bis(trimethylsilyl)amide (25.09 mL, 2 M, 50.17 mmol) was added, and the mixture was stirred and reacted at this temperature for 15 min, and then warmed to 0°C and reacted for 2 h. The reaction was quenched with 1 *N* hydrochloric acid (150 mL), and the mixture was stirred overnight at room temperature and

extracted with ethyl acetate. The organic phase was washed twice with a saturated sodium bicarbonate solution, washed once with a saturated aqueous sodium chloride solution, dried over anhydrous sodium sulphate and concentrated under reduced pressure. The residue was purified by column chromatography (ethyl acetate/petroleum ether (V/V) = 0/1-1/20) to obtain compound **32A** (5.7 g, yield: 86%).

LCMS m/z = 327.0 $[M+H]^+$

Step 2: Preparation of **32B**

Compound **32A** (3 g, 9.16 mmol) was dissolved in methanol (30 mL). Aminoguanidine hydrochloride (1.52 g, 13.75 mmol) and boron trifluoride diethyl etherate (2.26 mL, 18.32 mmol) were added. The tube was sealed, warmed to 100°C, reacted for 3 h and cooled to room temperature. A 1 N aqueous sodium hydroxide solution and ethyl acetate were added, and the mixture was stirred for layer separation. The organic phase was washed with a saturated aqueous sodium chloride solution, dried over anhydrous sodium sulphate and then concentrated under reduced pressure to obtain compound **32B,** which was directly used in the next step.

LCMS m/z = 383.3 $[M+H]^+$

Step 3: Preparation of**32C**

Compound **32B** (600 mg, 1.56 mmol) was dissolved in DMF (10 mL). N,N-dimethylethylenediamine (206.27 mg, 2.34 mmol) and cuprous iodide (148.55 mg, 0.78 mmol) were added, and the mixture was purged three times with nitrogen. Under nitrogen atmosphere, the mixture was reacted for 4 h. Ethyl acetate and water were added, and the mixture was stirred for layer separation. The organic layer was washed three times with a saturated aqueous sodium chloride solution, dried over anhydrous sodium sulphate and concentrated under reduced pressure. The residue was purified by column chromatography (dichloromethane/methanol (V/V) = 1/0-10/1) to obtain compound **32C** (200 mg, yield: 49%).

LCMS m/z = 261.2 $[M+H]^+$

Step 4: Preparation of **32D**

Compound **32C** (60 mg, 0.23 mmol), **2F** (107.15 mg) and caesium carbonate (149.88 mg, 0.46 mmol) were mixed and dissolved in DMF (3 mL). The mixture was warmed to 100°C, stirred for 40 h, cooled to room temperature and extracted with 20 mL of ethyl acetate and 20 mL of water. The organic layer was washed with a saturated aqueous sodium chloride solution (10 mL × 3), dried over anhydrous sodium sulphate and concentrated under reduced pressure. The residue was purified by silica gel column chromatography (ethyl acetate/petroleum ether (V/V) = 115-111) to obtain compound **32D** (60 mg, yield: 42%).

LCMS m/z = 620.2$[M+H]^+$

Step 5: Preparation of **compound 32**

Compound **32D** (60 mg, 0.097 mmol) was dissolved in a mixed solvent of 1,4-dioxane (3 mL) and water (1.5 mL). Lithium hydroxide (40.70 mg, 0.97 mmol) was added. The mixture was warmed to 60°C, stirred for 1 h and cooled to room temperature. The reaction solution was adjusted to pH = 3-4 by adding a 1 *N* aqueous hydrochloric acid solution. 20 mL of ethyl acetate was added, and the organic phase was washed with 10 mL of a saturated aqueous sodium chloride solution, dried over anhydrous sodium sulphate and concentrated under reduced pressure. The residue was purified by column chromatography (acetonitrile/water (containing 0.1% trifluoroacetic acid) (V/V) = 0/100-60/40) to obtain the trifluoroacetate of **compound 32** (15 mg).

LCMS m/z = 606.2$[M+H]^+$

$^1H$ NMR (400 MHz, DMSO-$d_6$) δ 11.54 (s, 1H), 8.85 (d, 1H), 7.82 (d, 1H), 7.53 (dd, 1H), 7.39 (t, 1H), 7.35 - 7.02 (m, 6H), 4.40 (s, 2H), 2.93 (s, 2H), 1.81 (s, 3H), 1.10 (s, 6H).

Preparation of **compound 32-1** and **compound 32-2:**

**Compound 32** (65 mg) was subjected to chiral resolution, and the chiral resolution method was as follows:

1. Instrument: SFC Prep 150 AP; chromatographic column: IG (19 mm × 250 mm).
2. The sample was dissolved in methanol and filtered with a 0.45 μm filter to prepare a sample solution.
3. Preparative chromatography conditions: a. the mobile phase consists of systems A and B: mobile phase A: $CO_2$; mobile phase B: isopropanol (aqueous ammonia 0.05%); b. isocratic elution, mobile phase B: 40%; c. flow rate: 40 mL/min.

After preparative separation, the components with the same retention time were combined and concentrated under reduced pressure to obtain **compound 32-1** (20 mg) and **compound 32-2** (25 mg).

**Compound 32-1:** retention time under chiral preparative conditions: 7.40 min, LCMS m/z = 606.0 $[M+1]^+$

**Compound 32-2:** retention time under chiral preparative conditions: 11.97 min, LCMS m/z = 606.0 $[M+1]^+$

**Example 33:**

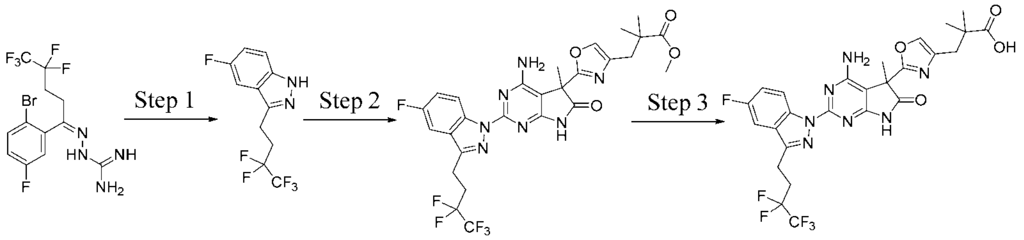


17B 33A 33B **Compound 33**

With reference to the synthesis for **Example 17,** the trifluoroacetate of **compound 33** (15 mg) was obtained.
LCMS m/z = 612.3 $[M+H]^+$
$^1$H NMR (400 MHz, DMSO-$d_6$) δ 11.47 (s, 1H), 8.86 (dd, 1H), 7.81 (dd, 1H), 7.75 (s, 1H), 7.47 - 7.37 (m, 1H), 7.20 - 6.46 (m, 2H), 3.35 - 3.23 (m, 2H), 2.87 - 2.72 (m, 2H), 2.71 - 2.61 (m, 2H), 1.81 (s, 3H), 1.11 (s, 3H), 1.09 (s, 3H).

**Example 34:**

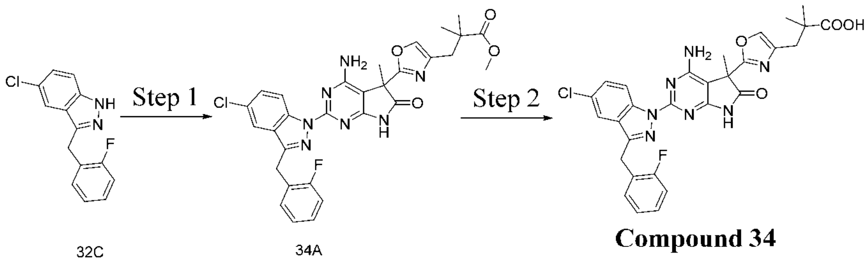


32C 34A **Compound 34**

With reference to the synthesis for **Example 4,** the trifluoroacetate of **compound 34** (8 mg) was obtained.
LCMS m/z = 590.2 $[M+H]^+$
$^1$H NMR (400 MHz, DMSO-$d_6$) δ 11.49 (s, 1H), 8.84 (d, 1H), 7.82 (d, 1H), 7.74 (s, 1H), 7.52 (dd, 1H), 7.42 - 7.27 (m, 2H), 7.24 - 7.13 (m, 2H), 7.03 - 6.65 (m, 2H), 4.40 (s, 2H), 2.71 - 2.58 (m, 2H), 1.81 (s, 3H), 1.11 (s, 3H), 1.08 (s, 3H).

**Example 35:**

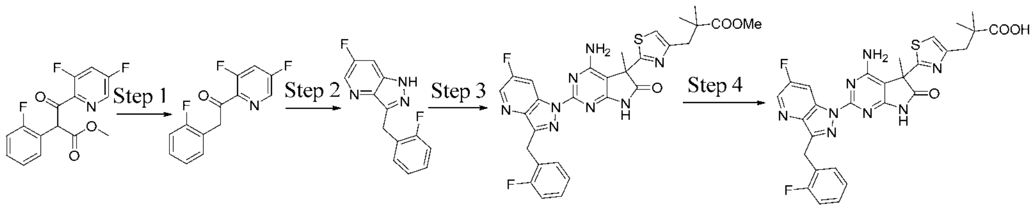


35A 35B 35C 35D **Compound 35**

Step 1: Preparation of **35B**

Compound **35A** (CAS: 1361570-31-1, synthesised with reference to US 2013210824) (1 g, 3.23 mmol) was dissolved in DMSO (5 mL). Sodium chloride (0.29 g, 4.93 mmol) and water (0.5 mL) were added sequentially. The mixture was warmed to 160°C, stirred for 30 min, cooled to room temperature and extracted with 10 mL of water and 20 mL of ethyl acetate. The organic phase was washed with a saturated aqueous sodium chloride solution (10 mL×1), dried over anhydrous sodium sulphate and concentrated under reduced pressure. The residue was purified by silica gel column chromatography (ethyl acetate/petroleum ether (V/V) = 1/20) to obtain compound **35B** (0.7 g, yield: 86%).

Step 2: Preparation of**35C**

Compound **35B** (0.7 g, 2.79 mmol) was dissolved in ethylene glycol (7 mL). Hydrazine hydrate (1.24 g, 19.75 mmol, wt% = 80%) was added. The mixture was warmed to 100°C and stirred for 30 min, then warmed to 120°C and stirred for 2 h, and cooled to room temperature. With stirring, 14 mL of water was slowly added dropwise, with the solids precipitated. The resulting mixture was filtered. The filter cake was dried under reduced pressure to obtain compound **35C** (0.35 g, yield: 51%).

LCMS m/z = 246.1 $[M+H]^+$

Step 3: Preparation of**35D**

Compound **35C** (0.1 g, 0.41 mmol), **2F** (0.21 g) and potassium carbonate (0.057 g, 0.41 mmol) were mixed and dissolved in DMF (5 mL). The mixture was warmed to 80°C, stirred for 16 h, cooled to room temperature and extracted with 20 mL of ethyl acetate and 20 mL of water. The organic layer was washed with a saturated aqueous sodium chloride solution (10 mL × 3), dried over anhydrous sodium sulphate and concentrated under reduced pressure. The residue was purified by silica gel column chromatography (ethyl acetate/petroleum ether (V/V) = 1/5-1/1) to obtain compound **35D** (0.22 g, yield: 88%).

LCMS m/z = 605.1 $[M+H]^+$

Step 4: Preparation of **compound 35**

Compound **35D** (0.22 g, 0.36 mmol) was dissolved in a mixed solvent of 1,4-dioxane (3 mL) and water (2 mL). Lithium hydroxide (0.086 g, 3.59 mmol) was added. The mixture was warmed to 60°C, stirred for 1 h and cooled to room temperature. The reaction solution was adjusted to pH = 3-4 by adding a 1 N aqueous HCl solution. 20 mL of ethyl acetate was added, and the organic phase was washed with 10 mL of a saturated aqueous sodium chloride solution, dried over anhydrous sodium sulphate and concentrated under reduced pressure. The residue was purified by silica gel column chromatography (dichloromethane/methanol (V/V) = 100/0-97/3) to obtain **compound 35** (0.15 g, yield: 70%).

LCMS m/z = 591.2 $[M+H]^+$

$^1$H NMR (400 MHz, DMSO-$d_6$) δ 12.19 (s, 1H), 11.55 (s, 1H), 9.04 (dd, 1H), 8.68 (d, 1H), 7.42 - 7.35 (m, 1H), 7.33 - 7.05 (m, 6H), 4.43 (s, 2H), 2.93 (s, 2H), 1.81 (s, 3H), 1.11 (s, 6H).

Preparation of **compound 35-1** and **compound 35-2:**

**Compound 35** (140 mg) was subjected to chiral separation and purification. Preparative conditions: instrument: SFC Prep 150 AP; chromatographic column: IG (19 mm×250 mm). The sample was dissolved in DMF and filtered with a 0.45 μm filter to prepare a sample solution. Preparative chromatography conditions: mobile phase A: $CO_2$, and mobile phase B: methanol (containing 0.5% aqueous ammonia); isocratic elution, mobile phase B: 30%; flow rate: 54 ml/min.

Analysis conditions: instrument: UPC2; chromatographic column: IG (3 mm×50 mm). The sample was dissolved in methanol and filtered with a 0.45 μm filter to prepare a sample solution. Preparative chromatography conditions: mobile phase A: $CO_2$, and mobile phase B: methanol (containing 0.5% aqueous ammonia); gradient elution, mobile phase B: 10%-40%, time: 5 min; isocratic elution, mobile phase B: 40%; flow rate: 1.5 ml/min.

After preparative separation, the components with the same retention time were combined and concentrated under reduced pressure to obtain **compound 35-1** (60 mg) and **compound 35-2** (70 mg).

**Compound 35-1:** retention time under analysis conditions: 3.4 min, LCMS m/z = 591.1 $[M+H]^+$

**Compound 35-2:** retention time under analysis conditions: 5.2 min, LCMS m/z = 591.1$[M+H]^+$

**Example 36:**

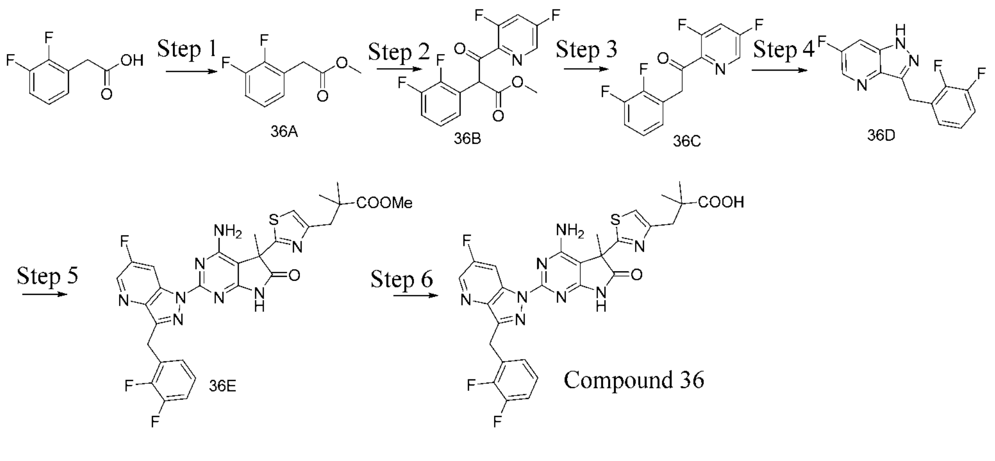


Step 1: Preparation of**36A**

Compound 2,3-difluorophenylacetic acid (1 g, 5.81 mmol) was dissolved in methanol (10 mL), and p-toluenesulphonic acid monohydrate (0.11 g, 0.58 mmol) was added. The mixture was warmed to reflux and reacted for 3 h. The reaction solution was cooled to room temperature and concentrated under reduced pressure. The residue was dissolved in 20 mL of ethyl acetate. The organic layer was washed sequentially with a saturated aqueous sodium bicarbonate solution (10 mL×2) and a saturated aqueous sodium chloride solution (10 mL), dried over anhydrous sodium sulphate and concentrated under reduced pressure, and the residue was directly used in the next step.

Step 2: Preparation of**36B**

Compound **36A** (1.11 g, 5.95 mmol) was dissolved in THF (10 mL). LiHMDS (7.14 mmol, 7.14 mL, 1 N) was slowly added dropwise in a dry ice-ethanol bath. After the addition, the mixture was stirred at -70°C for 1 h. At this temperature, a solution of 3,5-difluoropicolinoyl chloride (CAS: 1048340-35-7, synthesised with reference to WO 2011158149) (1.27 g, 7.14 mmol) in THF (10 mL) was slowly added dropwise. After the addition, the mixture was naturally warmed to room temperature, and the reaction was quenched with 10 mL of a saturated aqueous ammonium chloride solution and extracted with 20 mL of ethyl acetate. The organic phase was washed with a saturated aqueous sodium chloride solution (10 mL×1), dried over anhydrous sodium sulphate and concentrated under reduced pressure. The residue was purified by silica gel column chromatography (ethyl acetate/petroleum ether (V/V) = 1/20) to obtain compound **36B** (0.5 g, yield: 26%).

Step 3: Preparation of**36C**

Compound **36B** (0.5 g, 1.53 mmol) was dissolved in DMSO (5 mL). Sodium chloride (0.13 g, 2.29 mmol) and water (0.5 mL) were added sequentially. The mixture was warmed to 160°C, stirred for 30 min, cooled to room temperature and extracted with 10 mL of water and 20 mL of ethyl acetate. The organic phase was washed with a saturated aqueous sodium chloride solution (10 mL×1), dried over anhydrous sodium sulphate and concentrated under reduced pressure. The residue was purified by silica gel column chromatography (ethyl acetate/petroleum ether (V/V) = 1/20) to obtain compound **36C** (0.3 g, yield: 73%).

Step 4: Preparation of **36D**

Compound **36C** was dissolved in ethylene glycol (4 mL). Hydrazine hydrate (0.49 g, 7.86 mmol, wt% = 80%) was added. The mixture was warmed to 100°C and stirred for 30 min, then warmed to 120°C and stirred for 2 h, and cooled to room temperature. With stirring, 8 mL of water was slowly added dropwise, with the solids precipitated. The resulting mixture was filtered. The filter cake was dried under reduced pressure to obtain compound **36D** (0.14 g, yield: 48%).

LCMS m/z = 264.3 $[M+H]^+$

Step 5: Preparation of**36E**

Compound **36D** (0.1 g, 0.38 mmol), **2F** (0.19 g) and potassium carbonate (0.10 g, 0.76 mmol) were mixed and dissolved in DMF (5 mL). The mixture was warmed to 80°C, stirred for 16 h, cooled to room temperature and extracted with 20 mL of ethyl acetate and 20 mL of water. The organic layer was washed with a saturated aqueous sodium chloride solution (10 mL × 3), dried over anhydrous sodium sulphate and concentrated under reduced pressure. The residue was purified by silica gel column chromatography (ethyl acetate/petroleum ether (V/V) = 1/5-1/1) to obtain compound **36E** (0.2 g, yield: 84%).

LCMS m/z = 623.2 $[M+H]^+$

Step 6: Preparation of **compound 36**

Compound **36E** (0.2 g, 0.32 mmol) was dissolved in a mixed solvent of 1,4-dioxane (3 mL) and water (2 mL). Lithium hydroxide (0.077 g, 3.2 mmol) was added. The mixture was warmed to 60°C, stirred for 1 h and cooled to room temperature. The reaction solution was adjusted to pH = 3-4 by adding a 1 *N* aqueous HCl solution. 20 mL of ethyl acetate was added, and the organic phase was washed with 10 mL of a saturated aqueous sodium chloride solution, dried over anhydrous sodium sulphate and concentrated under reduced pressure. The residue was purified by silica gel column chromatography (dichloromethane/methanol (V/V) = 100/0-97/3) to obtain **compound 36** (0.12 g, yield: 61%).

LCMS m/z = 609.2 $[M+H]^+$

$^1$H NMR (400 MHz, DMSO-$d_6$) δ 12.19 (s, 1H), 11.54 (s, 1H), 9.05 (dd, 1H), 8.69 (d, 1H), 7.42 - 7.02 (m, 6H), 4.49 (s, 2H), 2.93 (s, 2H), 1.81 (s, 3H), 1.11 (s, 6H).

Preparation of **compound 36-1** and **compound 36-2:**

**Compound 36** (100 mg) was subjected to chiral separation and purification. Preparative conditions: instrument: SFC Prep 150 AP; chromatographic column: IG (19 mm×250 mm). The sample was dissolved in DMF and filtered with a 0.45 μm filter to prepare a sample solution. Preparative chromatography conditions: mobile phase A: $CO_2$, and mobile phase B: methanol (containing 0.5% aqueous ammonia); isocratic elution, mobile phase B: 30%; flow rate: 54 ml/min.

Analysis conditions: instrument: UPC2; chromatographic column: IG (3 mm×50 mm). The sample was dissolved in methanol and filtered with a 0.45 μm filter to prepare a sample solution. Preparative chromatography conditions: mobile phase A: $CO_2$, and mobile phase B: methanol (containing 0.5% aqueous ammonia); gradient elution, mobile phase B: 10%-40%, time: 5 min; isocratic elution, mobile phase B: 40%; flow rate: 1.5 ml/min.

After preparative separation, the components with the same retention time were combined and concentrated under reduced pressure to obtain **compound 36-1** (39 mg) and **compound 36-2** (35 mg).

**Compound 36-1:** retention time under analysis conditions: 3.3 min, LCMS m/z = 609.2 $[M+H]^+$

**Compound 36-2:** retention time under analysis conditions: 5.4 min, LCMS m/z = 609.2 $[M+H]^+$

**Example 37:**

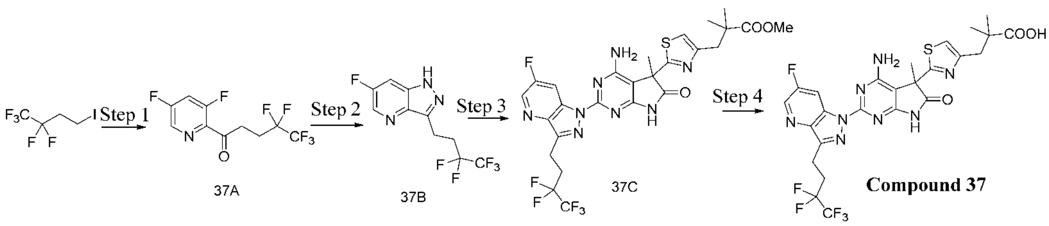


37A 37B 37C **Compound 37**

Step 1: Preparation of**37A**

THF (10 mL) was placed in a three-necked flask. High-purity zinc powder (0.74 g, 11.31 mmol) was added. The mixture was purged three times with nitrogen, and 1,2-dibromoethane (0.11 g, 0.56 mmol) was added. After the addition, the mixture was warmed to 70°C, stirred for 10 min and cooled to room temperature. Trimethylchlorosilane (0.061 g, 0.56 mmol) was slowly added. After the addition, the mixture was stirred vigorously at room temperature for 10 min. A solution of

1,1,1,2,2-pentafluoro-4-iodobutane (1.55 g, 5.66 mmol) in THF (5 mL) was slowly added dropwise. After the addition, the mixture was further stirred at room temperature for 2 h, and the resulting reagent was set aside for subsequent use. In another reaction flask, 3,5-difluoropicolinoyl chloride (CAS: 1048340-35-7, synthesised with reference to WO 2011158149) (0.5 g, 2.82 mmol) was dissolved in THF (10 mL). Bis(triphenylphosphine)palladium dichloride (0.20 g, 0.28 mmol) was added, and the mixture was purged three times with nitrogen. At room temperature, the above-mentioned reagent was slowly added dropwise at room temperature. After the addition, the mixture was stirred at room temperature for 2 h. The reaction was quenched with 20 mL of saturated ammonium chloride and extracted with 20 mL of ethyl acetate and 10 mL of water. The organic phase was washed with a saturated aqueous sodium chloride solution (10 mL), dried over anhydrous sodium sulphate and concentrated under reduced pressure. The residue was purified by silica gel column chromatography (ethyl acetate/petroleum ether (V/V) = 0/1-1/20) to obtain compound **37A** (0.22 g, yield: 27%).

LCMS m/z = 290.1 $[M+H]^+$

Step 2: Preparation of**37B**

Compound **37A** (0.22 g, 0.76 mmol) was dissolved in ethylene glycol (3 mL). Hydrazine hydrate (0.38 g, 7.59 mmol, wt% = 80%) was added. The mixture was warmed to 100°C and stirred for 30 min, then warmed to 120°C and stirred for 2 h, and cooled to room temperature. With stirring, 6 mL of water was slowly added dropwise, with the solids precipitated. The resulting mixture was filtered. The filter cake was dried under reduced pressure to obtain compound **37B** (0.1 g, yield: 46%).

LCMS m/z = 284.2 $[M+H]^+$

Step 3: Preparation of**37C**

Compound **37B** (0.1 g, 0.35 mmol), **2F** (0.18 g) and potassium carbonate (0.097 g, 0.70 mmol) were mixed and dissolved in DMF (5 mL). The mixture was warmed to 80°C, stirred for 16 h, cooled to room temperature and extracted with 20 mL of ethyl acetate and 20 mL of water. The organic layer was washed with a saturated aqueous sodium chloride solution (10 mL × 3), dried over anhydrous sodium sulphate and concentrated under reduced pressure. The residue was purified by silica gel column chromatography (ethyl acetate/petroleum ether (V/V) = 1/5-1/1) to obtain compound **37C** (0.15 g, yield: 66%).

LCMS m/z = 643.1 $[M+H]^+$

Step 4: Preparation of **compound 37**

Compound **37C** (0.15 g, 0.23 mmol) was dissolved in a mixed solvent of 1,4-dioxane (3 mL) and water (2 mL). Lithium hydroxide (0.056 g, 2.33 mmol) was added. The mixture was warmed to 60°C, stirred for 1 h and cooled to room temperature. The reaction solution was adjusted to pH = 3-4 by adding a 1 N aqueous HCl solution. 20 mL of ethyl acetate was added, and the organic phase was washed with 10 mL of a saturated aqueous sodium chloride solution, dried over anhydrous sodium sulphate and concentrated under reduced pressure. The residue was purified by silica gel column chromatography (dichloromethane/methanol (V/V) = 100/0-97/3) to obtain **compound 37** (90 mg, yield: 62%).

LCMS m/z = 629.2 $[M+H]^+$

$^1$H NMR (400 MHz, DMSO-$d_6$) δ 12.19 (s, 1H), 11.55 (s, 1H), 9.06 (dd, 1H), 8.72 (dd, 1H), 7.56 - 6.90 (m, 3H), 3.40-3.32 (m, 2H), 2.97 - 2.81 (m, 4H), 1.82 (s, 3H), 1.11 (s, 6H).

Preparation of **compound 37-1** and **compound 37-2:**

**Compound 37** (80 mg) was subjected to chiral separation and purification. Preparative conditions: instrument: SFC Prep 150 AP; chromatographic column: IG (19 mm×250 mm). The sample was dissolved in DMF and filtered with a 0.45 μm filter to prepare a sample solution. Preparative chromatography conditions: mobile phase A: $CO_2$, and mobile phase B: methanol (containing 0.5% aqueous ammonia); isocratic elution, mobile phase B: 15%; flow rate: 42 ml/min.

Analysis conditions: instrument: UPC2; chromatographic column: IG (3 mm×50 mm). The sample was dissolved in methanol and filtered with a 0.45 μm filter to prepare a sample solution. Preparative chromatography conditions: mobile phase A: $CO_2$, and mobile phase B: methanol (containing 0.5% aqueous ammonia); gradient elution, mobile phase B: 10%-40%, time: 5 min; isocratic elution, mobile phase B: 40%; flow rate: 1.5 ml/min.

After preparative separation, the components with the same retention time were combined and concentrated under reduced pressure to obtain **compound 37-1** (38 mg) and **compound 37-2** (35 mg).

**Compound 37-1:** retention time under analysis conditions: 1.9 min, LCMS m/z = 629.2 $[M+H]^+$

**Compound 37-2:** retention time under analysis conditions: 2.7 min, LCMS m/z = 629.2 $[M+H]^+$

**Example 38:**

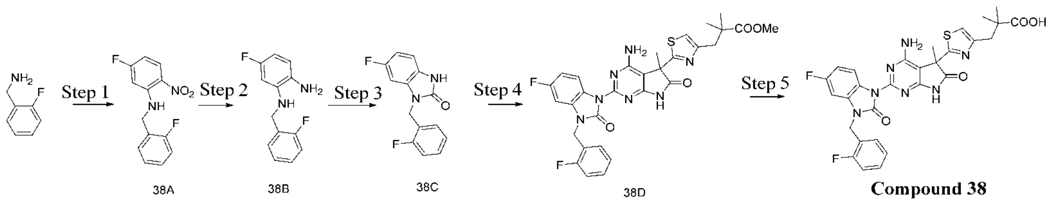


38A 38B 38C 38D **Compound 38**

Step 1: Preparation of **38A**

Compound 2,4-difluoronitrobenzene (1 g, 6.29 mmol) and 2-fluorobenzylamine (0.86 g, 6.88 mmol) were dissolved in DMSO (5 mL). Diisopropylethylamine (1.22 g, 9.44 mmol) was added, and the mixture was stirred at room temperature for 4 h and extracted with 30 mL of methyl tert-butyl ether and 30 mL of water. The organic layer was washed with a saturated aqueous sodium chloride solution (20 mL×3), dried over anhydrous sodium sulphate and concentrated under reduced pressure. The residue was slurried with 20 mL of methyl tert-butyl ether/petroleum ether (V/V = 1/5) to obtain compound **38A** (1.1 g, yield: 66%).

Step 2: Preparation of **38B**

Compound **38A** (1.1 g, 4.16 mmol) was dissolved in a mixed solvent of ethanol (10 mL) and water (3 mL). Ammonium chloride (2.22 g, 41.49 mmol) and iron powder (2.32 g, 41.56 mmol) were added sequentially. After the addition, the mixture was warmed to 75°C, stirred for 1.5 h, cooled to room temperature and filtered. The filter cake was washed with 20 mL of dichloromethane. The filtrates were combined and washed with 20 mL of a saturated aqueous sodium chloride solution. The organic phase was dried over anhydrous sodium sulphate and concentrated under reduced pressure. The residue was slurried with 20 mL of methyl tert-butyl ether/petroleum ether (V/V = 1/4) to obtain compound **38B** (0.8 g, yield: 82%).

Step 3: Preparation of**38C**

Compound **38B** (0.8 g, 3.42 mmol) was dissolved in THF (10 mL). N,N'-carbonyldiimidazole (0.83 g, 5.13 mmol) was added. After the addition, the mixture was stirred at room temperature for 16 h. 20 mL of dichloromethane was added. The organic layer was washed with 20 mL of a saturated aqueous sodium chloride solution. The organic phase was dried over anhydrous sodium sulphate and concentrated under reduced pressure. The residue was slurried with 20 mL of methyl tert-butyl ether/petroleum ether (V/V = 1/1) to obtain compound **38C** (0.45 g, yield: 50%).
LCMS m/z = 261.1 $[M+H]^+$

Step 4: Preparation of **38D**

Compound **38C** (0.10 g, 0.40 mmol), **2F** (0.20 g) and potassium carbonate (0.11 g, 0.80 mmol) were mixed and dissolved in DMF (5 mL). The mixture was warmed to 100°C, stirred for 16 h, cooled to room temperature and extracted with 20 mL of ethyl acetate and 20 mL of water. The organic layer was washed with a saturated aqueous sodium chloride solution (10 mL × 3), dried over anhydrous sodium sulphate and concentrated under reduced pressure. The residue was purified by silica gel column chromatography (ethyl acetate/petroleum ether (V/V) = 1/5-1/1) to obtain compound **38D** (70 mg, yield: 28%).
LCMS m/z = 620.3 $[M+H]^+$

Step 5: Preparation of **compound 38**

Compound **38D** (0.07 g, 0.11 mmol) was dissolved in a mixed solvent of 1,4-dioxane (3 mL) and water (2 mL). Lithium hydroxide (0.026 g, 1.1 mmol) was added. The mixture was warmed to 60°C, stirred for 1 h and cooled to room temperature. The reaction solution was adjusted to pH = 3-4 by adding a 1 *N* aqueous HCl solution. 20 mL of ethyl acetate was added, and the organic phase was washed with 10 mL of a saturated aqueous sodium chloride solution, dried over

anhydrous sodium sulphate and concentrated under reduced pressure. The residue was purified by column chromatography (acetonitrile/water (containing 0.1% trifluoroacetic acid) (V/V) = 0/100-50/50) to obtain the trifluoroacetate of **compound 38** (30 mg).

LCMS m/z = 606.0 $[M+H]^+$

$^1H$ NMR (400 MHz, DMSO-$d_6$) δ 11.55 (s, 1H), 7.84 (dd, 1H), 7.41 - 7.32 (m, 1H), 7.31 - 6.98 (m, 7H), 6.94 - 6.87 (m, 1H), 5.15 (s, 2H), 2.93 (s, 2H), 1.81 (s, 3H), 1.15 - 1.01 (m, 6H).

Preparation of **compound 38-1** and **compound 38-2:**

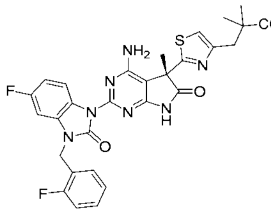

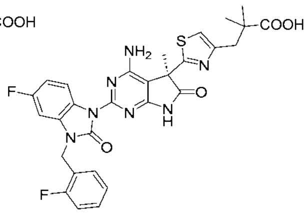

**Compound 38-1 and Compound 38-2**

**Compound 38** (300 mg) was subjected to chiral resolution, and the chiral resolution method was as follows:

1. Instrument: SFC Prep 150 AP; chromatographic column: IG (19 mm × 250 mm).
2. The sample was dissolved in methanol and filtered with a 0.45 μm filter to prepare a sample solution.
3. Preparative chromatography conditions: a. the mobile phase consists of systems A and B: mobile phase A: $CO_2$; mobile phase B: methanol (aqueous ammonia 0.05%); b. isocratic elution, mobile phase B: 40%; c. flow rate: 40 mL/min.

After preparative separation, the components with the same retention time were combined and concentrated under reduced pressure to obtain **compound 38-1** (110 mg) and **compound 38-2** (120 mg).

**Compound 38-1:** retention time under chiral preparative conditions: 7.78 min, LCMS m/z = 606.0 $[M+1]^+$

**Compound 38-2:** retention time under chiral preparative conditions: 12.85 min, LCMS m/z = 606.2 $[M+1]^+$

**Example 39:**

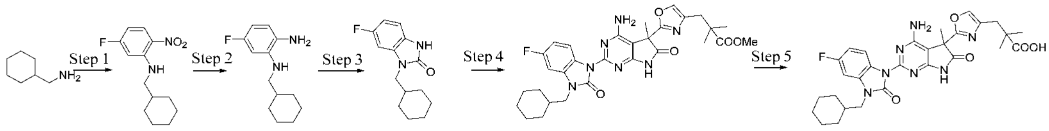


39A 39B 39C 39D Compound 39

With reference to the synthesis for **Example 38,** the trifluoroacetate of **compound 39** (25 mg) was obtained.

LCMS m/z = 578.2 $[M+H]^+$

**Example 40:**

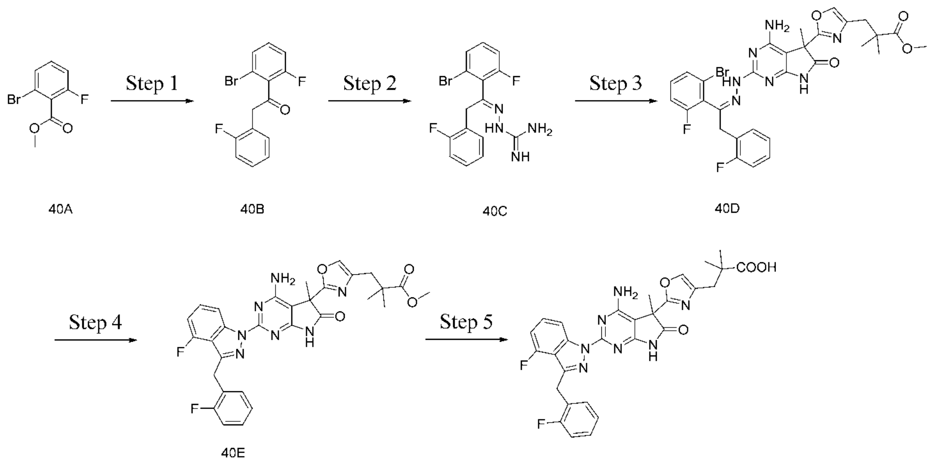


40A 40B 40C 40D

40E

Compound 40

Step 1: Preparation of **40B**

Compound methyl 2-bromo-6-fluorobenzoate (2 g, 8.58 mmol) and 2-fluorophenylacetic acid (1.45 g, 90.48 mmol) were dissolved in THF (40 mL). At - 78°C, sodium bis(trimethylsilyl)amide (12 mL, 2 M, 24.06 mmol) was added, and the mixture was stirred and reacted at this temperature for 15 min, and then warmed to 0°C and reacted for 2 h. The reaction was quenched with 1 *N* hydrochloric acid (60 mL), and the mixture was stirred overnight at room temperature and extracted with ethyl acetate. The organic phase was washed twice with a saturated sodium bicarbonate solution, washed once with a saturated aqueous sodium chloride solution, dried over anhydrous sodium sulphate and concentrated under reduced pressure. The residue was purified by column chromatography (ethyl acetate/petroleum ether (V/V) = 0/1-1/20) to obtain compound **40B** (1.8 g, yield: 67%).

Step 2: Preparation of **40C**

Compound **40B** (1.6 g, 5.14 mmol) was dissolved in methanol (10 mL). Aminoguanidine hydrochloride (0.57 g, 7.71 mmol) and boron trifluoride diethyl etherate (1.1 mL, 8.91 mmol) were added. The tube was sealed, warmed to 100°C, reacted for 3 h and cooled to room temperature. A 1 N aqueous sodium hydroxide solution and ethyl acetate were added, and the mixture was stirred for layer separation. The organic phase was washed with a saturated aqueous sodium chloride solution, dried over anhydrous sodium sulphate and then concentrated under reduced pressure to obtain compound **40C,** which was directly used in the next step.

LCMS m/z = 367.0[M+H]$^+$

Step 3: Preparation of **40D**

Compound **40C** (0.1 g, 0.27 mmol), **21D** (0.094 g, 0.27 mmol) and potassium tert-butoxide (0.03 g, 0.27 mmol) were mixed and dissolved in tert-butanol (15 mL). The tube was sealed, warmed to 130°C, stirred for 3 h and cooled to room temperature. A 1 *N* aqueous sodium hydroxide solution and ethyl acetate were added, and the mixture was stirred for layer separation. The organic phase was washed with a saturated aqueous sodium chloride solution, dried over anhydrous sodium sulphate and concentrated under reduced pressure. The residue was purified by column chromatography (mobile phase: dichloromethane/methanol (V/V) = 1/0-10/1) to obtain compound **40D** (90 mg, yield: 50%).

Step 4: Preparation of **40E**

Compound **40D** (90 mg, 0.13 mmol) was dissolved in DMF (3 mL). N,N-dimethylethylenediamine (17 mg, 0.19 mmol) and cuprous iodide (25 mg, 0.13 mmol) were added, and the mixture was purged three times with nitrogen. Under nitrogen atmosphere, the mixture was reacted for 2 h. Ethyl acetate and water were added, and the mixture was stirred for layer separation. The organic layer was washed three times with a saturated aqueous sodium chloride solution, dried over anhydrous sodium sulphate and concentrated under reduced pressure. The residue was purified by column chromatography (dichloromethane/methanol (V/V) = 1/0-10/1) to obtain compound **40E** (30 mg, yield: 39%).

Step 5: Preparation of **compound 40**

Compound **40E** (0.03 g, 0.051 mmol) was dissolved in a mixed solvent of 1,4-dioxane (3 mL) and water (1 mL). Lithium hydroxide (0.012 g, 0.51 mmol) was added. The mixture was warmed to 60°C, stirred for 1 h and cooled to room temperature. The reaction solution was adjusted to pH = 3-4 by adding a 1 *N* aqueous HCl solution. 20 mL of ethyl acetate was added, and the organic phase was washed with 10 mL of a saturated aqueous sodium chloride solution, dried over anhydrous sodium sulphate and concentrated under reduced pressure. The residue was purified by column chromatography (acetonitrile/water (containing 0.1% trifluoroacetic acid) (V/V) = 0/100-60/40) to obtain **compound 40** (10 mg, yield: 34%).
LCMS m/z = 572.5[M-H]$^-$
$^1$H NMR (400 MHz, DMSO-$d_6$) δ 11.50 (s, 1H), 8.67 (d, 1H), 7.75 (s, 1H), 7.56 - 7.46 (m, 1H), 7.33 - 7.25 (m, 1H), 7.24 - 7.16 (m, 2H), 7.14 - 7.00 (m, 2H), 6.96 - 6.74 (m, 2H), 4.43 (s, 2H), 2.71 - 2.61 (m, 2H), 1.81 (s, 3H), 1.11 (s, 3H), 1.08 (s, 3H).

**Example 41:**

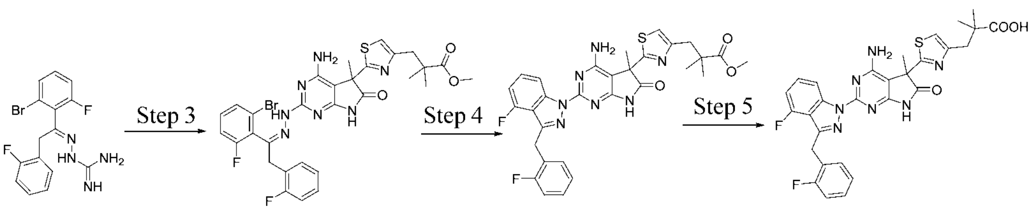


40C 41B 41C Compound 41

Step 1: Preparation of **41B**

Compound **40C** (0.1 g, 0.27 mmol), ethyl 3,3-dicyano-2-(4-(3-methoxy-2,2-dimethyl-3-oxopropyl)thiazo-2-yl)-2-methylpropanoate (CAS: 2101649-65-2, synthesised with reference to US20170174693) (98 mg, 0.27 mmol) and potassium tert-butoxide (0.03 g, 0.27 mmol) were mixed and dissolved in tert-butanol (5 mL). The tube was sealed, warmed to 130°C, stirred for 3 h and cooled to room temperature. A 1 *N* aqueous sodium hydroxide solution and ethyl acetate were added, and the mixture was stirred for layer separation. The organic phase was washed with a saturated aqueous sodium chloride solution, dried over anhydrous sodium sulphate and concentrated under reduced pressure. The residue was purified by column chromatography (mobile phase: dichloromethane/methanol (V/V) = 1/0-10/1) to obtain compound **41B** (90 mg, yield: 49%).

Step 2: Preparation of **41C**

Compound **41B** (90 mg, 0.90 mmol) was dissolved in DMF (3 mL). N,N-dimethylethylenediamine (11 mg, 0.13 mmol) and cuprous iodide (25 mg, 0.13 mmol) were added, and the mixture was purged three times with nitrogen. Under nitrogen atmosphere, the mixture was reacted for 2 h. Ethyl acetate and water were added, and the mixture was stirred for layer separation. The organic layer was washed three times with a saturated aqueous sodium chloride solution, dried over anhydrous sodium sulphate and concentrated under reduced pressure. The residue was purified by column chromatography (dichloromethane/methanol (V/V) = 1/0-10/1) to obtain compound **41C** (28 mg, yield: 36%).

Step 5: Preparation of **compound 41**

Compound **41C** (0.028 g, 0.046 mmol) was dissolved in a mixed solvent of 1,4-dioxane (3 mL) and water (1 mL). Lithium hydroxide (0.011 g, 0.46 mmol) was added. The mixture was warmed to 60°C, stirred for 1 h and cooled to room temperature. The reaction solution was adjusted to pH = 3-4 by adding a 1 *N* aqueous HCl solution. 20 mL of ethyl acetate was added, and the organic phase was washed with 10 mL of a saturated aqueous sodium chloride solution, dried over anhydrous sodium sulphate and concentrated under reduced pressure. The residue was purified by column chromatography (acetonitrile/water (containing 0.1% trifluoroacetic acid) (V/V) = 0/100-60/40) to obtain **compound 41** (10 mg, yield: 37%).
LCMS m/z = 588.5[M-H]$^-$

$^1H$ NMR (400 MHz, DMSO-$d_6$) δ 11.55 (s, 1H), 8.68 (d, 1H), 7.56 - 7.48 (m, 1H), 7.33 - 7.02 (m, 8H), 4.43 (s, 2H), 2.93 (s, 2H), 1.81 (s, 3H), 1.10 (s, 6H).

Preparation of **compound 41-1 and compound 41-2:**

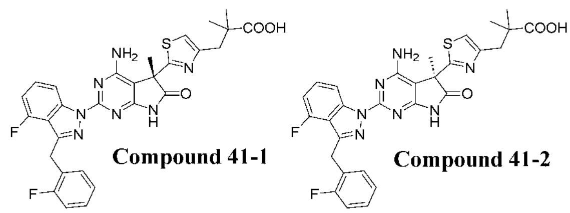

Compound 41-1 Compound 41-2

**Compound 41** (90 mg) was subjected to chiral resolution, and the chiral resolution method was as follows:

1. instrument: SFC Prep 150 AP; chromatographic column: IG (19 mm×250 mm)
2. The sample was dissolved in methanol and filtered with a 0.45 μm filter to prepare a sample solution.
3. Preparative chromatography conditions: a. composition of mobile phases A and B: mobile phase A: $CO_2$; mobile phase B: methanol/isopropanol = 8/2 (0.05% aqueous ammonia); b. isocratic elution, mobile phase B: 45%; c. flow rate: 44 ml/min.

After preparative separation, the components with the same retention time were combined and concentrated under reduced pressure to obtain **compound 41-1** (40 mg) and **compound 41-2** (40 mg).

**Compound 41-1:** retention time under chiral preparative conditions: 4.53 min, LCMS m/z = 590.5 [M+H]$^+$

$^1H$ NMR (400 MHz, DMSO-$d_6$) δ 11.52 (s, 1H), 8.68 (d, 1H), 7.57 - 7.43 (m, 1H), 7.35 - 6.98 (m, 8H), 4.43 (s, 2H), 2.93 (s, 2H), 1.81 (s, 3H), 1.10 (s, 6H).

**Compound 41-2:** retention time under chiral preparative conditions: 23.08 min, LCMS m/z = 590.5 [M+H]$^+$

$^1H$ NMR (400 MHz, DMSO-$d_6$) δ 11.55 (s, 1H), 8.68 (d, 1H), 7.59 - 7.43 (m, 1H), 7.36 - 6.93 (m, 8H), 4.43 (s, 2H), 2.93 (s, 2H), 1.81 (s, 3H), 1.10 (s, 6H).

**Example 42:**

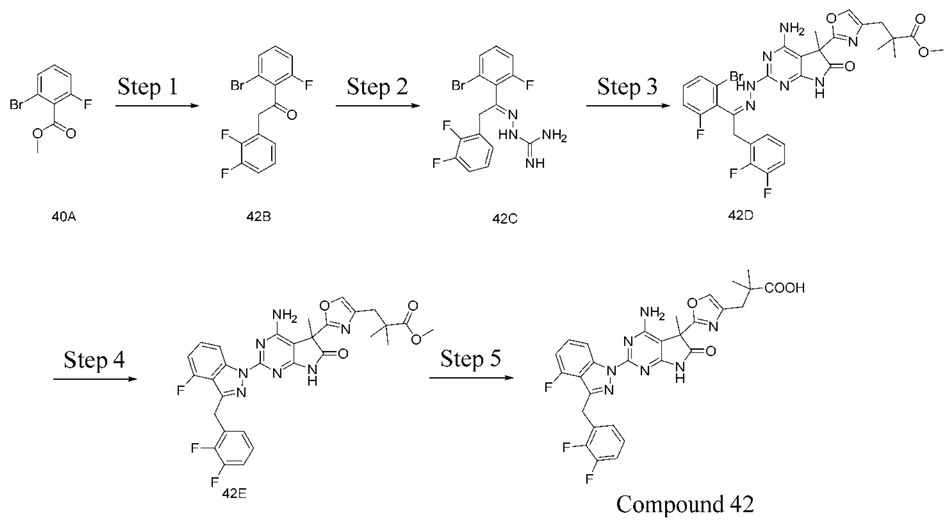


40A 42B 42C 42D 42E Compound 42

With reference to the synthesis for **Example 40, compound 42** (10 mg) was obtained.

LCMS m/z = 592.2$[M+H]^+$

$^1H$ NMR (400 MHz, DMSO-$d_6$) δ 11.50 (s, 1H), 8.67 (d, 1H), 7.75 (s, 1H), 7.57 - 7.48 (m, 1H), 7.37 - 7.25 (m, 1H), 7.16 - 6.99 (m, 3H), 6.99 - 6.68 (m, 2H), 4.48 (s, 2H), 2.71 - 2.61 (m, 2H), 1.82 (s, 3H), 1.11 (s, 3H), 1.09 (s, 3H).

**Example 43:**

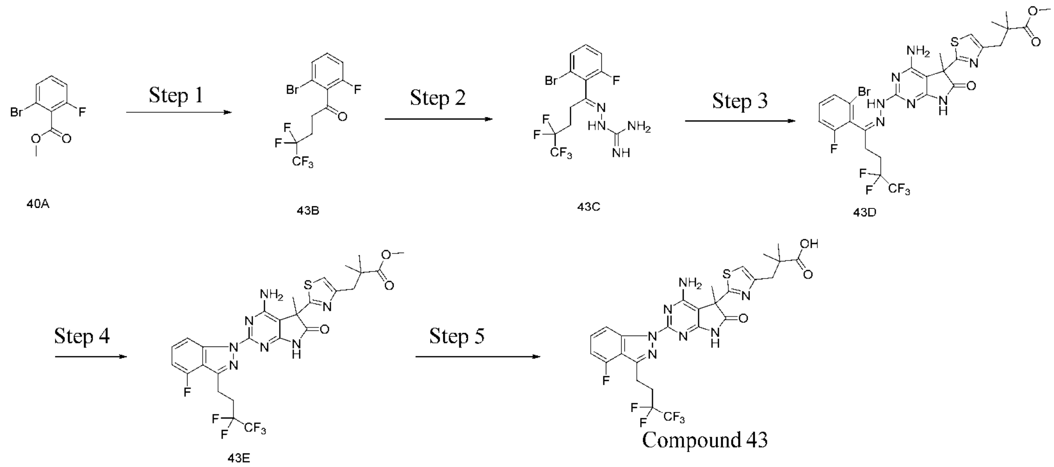


40A 43B 43C 43D 43E Compound 43

Step 1: Preparation of **43B**

Compound **40A** (2 g, 8.58 mmol) and 4,4,5,5,5-pentafluoropentanoic acid (3.3 g, 17 mmol) were dissolved in THF (100 mL). At -78°C, sodium bis(trimethylsilyl)amide (18 mL, 2 M, 36 mmol) was added, and the mixture was stirred and reacted at this temperature for 15 min, and then warmed to 0°C and reacted for 2 h. The reaction was quenched with 1 *N* hydrochloric acid (160 mL), and the mixture was stirred overnight at room temperature and extracted with ethyl acetate. The organic phase was washed twice with a saturated aqueous sodium bicarbonate solution, washed once with a saturated aqueous sodium chloride solution, dried over anhydrous sodium sulphate and concentrated under reduced pressure. The residue was purified by column chromatography (ethyl acetate/petroleum ether (V/V) = 0/1-1/20) to obtain compound **43B** (0.6 g, yield: 20%).

Step 2: Preparation of **43C**

Compound **43B** (0.6 g, 1.72 mmol) was dissolved in methanol (20 mL). Aminoguanidine hydrochloride (0.38 g, 3.44 mmol) and boron trifluoride diethyl etherate (0.49 g, 3.44 mmol) were added. The tube was sealed, warmed to 100°C, reacted for 3 h and cooled to room temperature. A 1 *N* aqueous sodium hydroxide solution and ethyl acetate were added, and the mixture was stirred for layer separation. The organic phase was washed with a saturated aqueous sodium chloride solution, dried over anhydrous sodium sulphate and concentrated under reduced pressure. The residue was purified by column chromatography (mobile phase: dichloromethane/methanol (V/V) = 1/0-10/1) to obtain compound **43C** (0.3 g, yield: 43%).

Step 3: Preparation of **43D**

Compound **43C** (0.34 g, 0.83 mmol), ethyl 3,3-dicyano-2-(4-(3-methoxy-2,2-dimethyl-3-oxopropyl)thiazo-2-yl)-2-methylpropanoate (CAS: 2101649-65-2, synthesised with reference to US20170174693) (300 mg, 0.83 mmol) and potassium bicarbonate (83 mg, 0.83 mmol) were mixed and dissolved in tert-butanol (5 mL). The tube was sealed, warmed to 130°C, stirred for 3 h and cooled to room temperature. A 1 *N* aqueous sodium hydroxide solution and ethyl acetate were added, and the mixture was stirred for layer separation. The organic phase was washed with a saturated aqueous sodium chloride solution, dried over anhydrous sodium sulphate and concentrated under reduced pressure. The residue was purified by column chromatography (mobile phase: dichloromethane/methanol (V/V) = 1/0-10/1) to obtain compound **43D** (200 mg, yield: 34%).

LCMS m/z = 722.1$[M+H]^+$

Step 4: Preparation of **43E**

Compound **43D** (200 mg, 0.28 mmol) was dissolved in DMF (5 mL). N,N-dimethylethylenediamine (37 mg, 0.42 mmol) and cuprous iodide (53 mg, 0.28 mmol) were added, and the mixture was purged three times with nitrogen. Under nitrogen atmosphere, the mixture was reacted for 2 h. Ethyl acetate and water were added, and the mixture was stirred for layer separation. The organic layer was washed three times with a saturated aqueous sodium chloride solution, dried over anhydrous sodium sulphate and concentrated under reduced pressure. The residue was purified by column chromatography (dichloromethane/methanol (V/V) = 1/0-10/1) to obtain compound **43E** (120 mg, yield: 68%).
LCMS m/z = 642.2$[M+H]^+$

Step 5: Preparation of **compound 43**

Compound **43E** (0.12 g, 0.19 mmol) was dissolved in a mixed solvent of 1,4-dioxane (3 mL) and water (1 mL). Lithium hydroxide (0.011 g, 0.46 mmol) was added. The mixture was warmed to 60°C, stirred for 1 h and cooled to room temperature. The reaction solution was adjusted to pH = 3-4 by adding a 1 *N* aqueous HCl solution. 20 mL of ethyl acetate was added, and the organic phase was washed with 10 mL of a saturated aqueous sodium chloride solution, dried over anhydrous sodium sulphate and concentrated under reduced pressure. The residue was purified by column chromatography (acetonitrile/water (containing 0.1% trifluoroacetic acid) (V/V) = 0/100-60/40) to obtain the trifluoroacetate of **compound 43** (40 mg).
LCMS m/z = 628.2$[M+H]^+$
$^1$H NMR (400 MHz, DMSO-$d_6$) δ 11.54 (s, 1H), 8.68 (d, 1H), 7.61 - 7.46 (m, 1H), 7.25 (s, 1H), 7.22 - 6.96 (m, 3H), 3.42 - 3.28 (m, 2H), 2.93 (s, 2H), 2.85 - 2.69 (m, 2H), 1.82 (s, 3H), 1.11 (s, 6H).
The racemate of **compound 43** (130 mg) was subjected to chiral resolution, and the chiral resolution method was as follows:

1. instrument: SFC Prep 150 AP; chromatographic column: IG (19 mm×250 mm)
2. The sample was dissolved in methanol and filtered with a 0.45 μm filter to prepare a sample solution.
3. Preparative chromatography conditions: a. composition of mobile phases A and B: mobile phase A: CO2; mobile phase B: methanol (0.05% aqueous ammonia); b. isocratic elution, mobile phase B: 22%; c. flow rate: 43 ml/min.

After preparative separation, the components with the same retention time were combined and concentrated under reduced pressure to obtain **compound 43-1** (60 mg) and **compound 43-2** (60 mg).

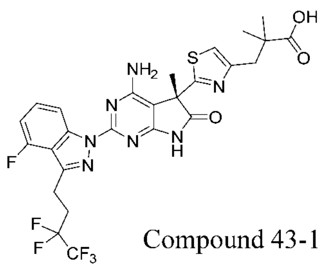
Compound 43-1

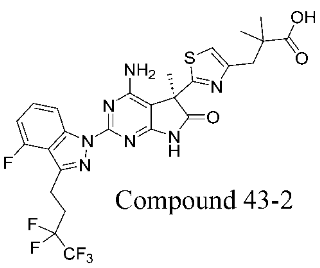
Compound 43-2

**Compound 43-1:** retention time under chiral preparative conditions: 4.05 min, LCMS m/z = 628.2 $[M+H]^+$

$^1$H NMR (400 MHz, DMSO-$d_6$) δ 11.53 (s, 1H), 8.68 (d, 1H), 7.58 - 7.49 (m, 1H), 7.25 (s, 1H), 7.22 - 6.98 (m, 3H), 3.39 - 3.32 (m, 2H), 2.94 (s, 2H), 2.88 - 2.64 (m, 2H), 1.82 (s, 3H), 1.11 (s, 6H).

**Compound 43-2:** retention time under chiral preparative conditions: 9.87 min, LCMS m/z = 628.2 $[M+H]^+$

$^1$H NMR (400 MHz, DMSO-$d_6$) δ 11.54 (s, 1H), 8.68 (d, 1H), 7.58 - 7.48 (m, 1H), 7.25 (s, 1H), 7.22 - 6.95 (m, 3H), 3.40 - 3.32 (m, 2H), 2.94 (s, 2H), 2.86 - 2.67 (m, 2H), 1.82 (s, 3H), 1.11 (s, 6H).

**Example 44:**

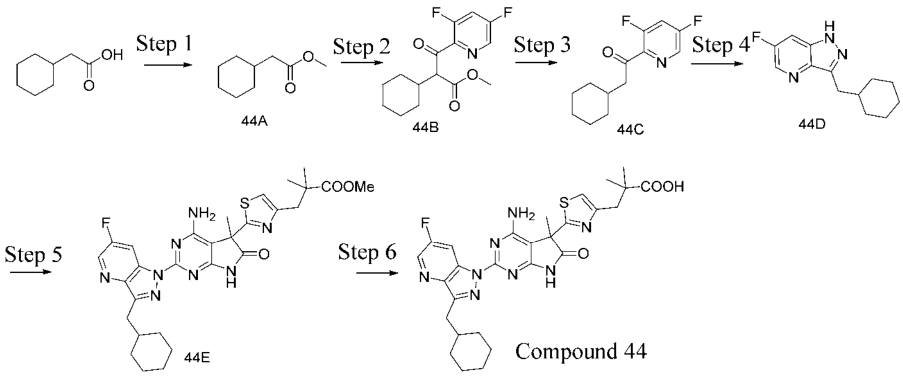


44A 44B 44C 44D 44E Compound 44

With reference to the synthesis for **Example 35, compound 44** (50 mg) was obtained.

LCMS m/z = 579.3 $[M+H]^+$

**Example 45:**

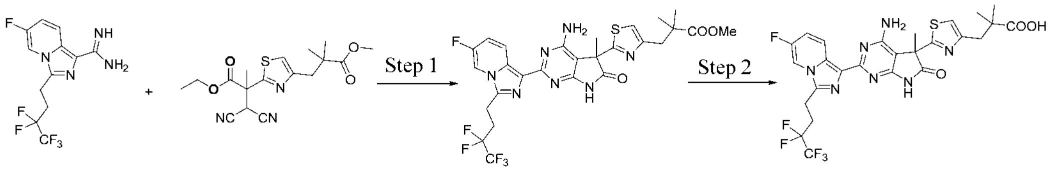


45A Compound 45

Step 1: Preparation of **45A**

To a reaction flask were added 6-fluoro-3-(3,3,4,4-pentafluorobutyl)imidazo[1,5-a]pyridine-1-carboxamide (CAS: 1407815-26-2, 0.2 g, 0.62 mmol), ethyl 3,3-dicyano-2-(4-(3-methoxy-2,2-dimethyl-3-oxopropyl)thiazo-2-yl)-2-methylpropanoate (CAS: 2101649-65-2,0.23 g, 0.62 mmol), sodium bicarbonate (0.21 g, 2.48 mmol) and tert-butanol (10 mL). The mixture was heated and reacted overnight in an oil bath at 85°. To the reaction solution was added an appropriate amount of silica gel, and the mixture was concentrated under reduced pressure. The residue was purified by silica gel column chromatography (eluent: PE-EA = 100-0 to 40-60) to obtain **45A** (0.23 g, yield: 58%).

Step 2: Preparation of **compound 45**

To a reaction flask were added **45A** (0.23 g, 0.36 mmol), lithium hydroxide monohydrate (0.15 g, 3.60 mmol), 1,4-dioxane (5 mL) and water (3 mL). The mixture was stirred overnight at room temperature reaction. Ethyl acetate and water were added, and the mixture was adjusted to pH 2-3 with 1 *N* hydrochloric acid. The organic layer was concentrated under reduced pressure to dryness. The residue was purified by silica gel column chromatography (eluent: DCM-$CH_3OH$ = 100-0 to 90-10) to obtain **compound 45** (0.19 g, yield: 84%).

LCMS m/z = 628.0$[M+H]^+$

**Compound 45** (180 mg) was subjected to chiral resolution, and the chiral resolution method was as follows:

1. instrument: SFC Prep 150 AP; chromatographic column: IG (19 mm × 250 mm)
2. The sample was dissolved in methanol and filtered with a 0.45 μm filter to prepare a sample solution.
3. Preparative chromatography conditions: a. composition of mobile phases A and B: mobile phase A: CO2; mobile phase B: methanol/isopropanol = 8/2 (0.05% aqueous ammonia); b. isocratic elution, mobile phase B: 25%; c. flow rate: 40 ml/min.

After preparative separation, the components with the same retention time were combined and concentrated under reduced pressure to obtain **compound 45-1** (72 mg) and **compound 45-2** (70 mg).

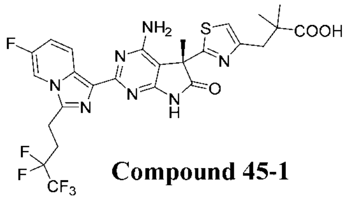

Compound 45-1

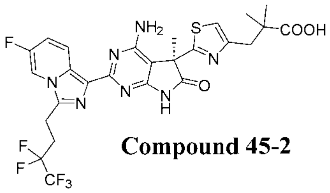

Compound 45-2

**Compound 45-1:** retention time under chiral preparative conditions: 8.1 min, LCMS m/z = 628.3[M+H]$^+$

$^1$H NMR (400 MHz, $CDCl_3/CD_3OD$(v/v) = 1/1) δ 8.68 (dd, 1H), 8.16 - 8.05 (m, 1H), 7.14 - 6.91 (m, 2H), 3.33 - 3.28 (m, 2H), 3.13 - 2.98 (m, 2H), 2.94 - 2.76 (m, 2H), 1.88 (s, 3H), 1.24 (s, 3H), 1.21 (s, 3H).

**Compound 45-2:** retention time under chiral preparative conditions: 27.1 min, LCMS m/z = 628.3[M+H]$^+$

$^1$H NMR (400 MHz, $CDCl_3/CD_3OD$(v/v) = 1/1) δ 8.68 (dd, 1H), 8.09 - 7.93 (m, 1H), 7.12 - 6.90 (m, 2H), 3.34 - 3.25 (m, 2H), 3.14 - 2.98 (m, 2H), 2.94 - 2.74 (m, 2H), 1.88 (s, 3H), 1.24 (s, 3H), 1.22 (s, 3H).

**Example 46:**

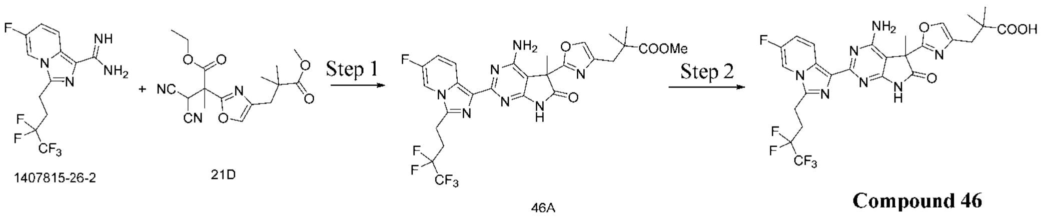


1407815-26-2 21D 46A Compound 46

Step 1: Preparation of **46A**

To a reaction flask were added 6-fluoro-3-(3,3,4,4-pentafluorobutyl)imidazo[1,5-a]pyridine-1-carboxamide (CAS: 1407815-26-2, 0.16 g, 0.49 mmol), **21D** (0.17 g, 0.49 mmol), sodium bicarbonate (0.16 g, 1.96 mmol) and tert-butanol (10 mL). The mixture was heated and reacted overnight in an oil bath at 85°C. To the reaction solution was added an appropriate amount of silica gel, and the mixture was concentrated under reduced pressure. The residue was purified by silica gel column chromatography (eluent: PE-EA = 100-0 to 40-60) to obtain **46A** (0.23 g, yield: 75%).

Step 2: Preparation of **compound 46**

To a reaction flask were added **46A** (0.21 g, 0.34 mmol), lithium hydroxide monohydrate (0.14 g, 3.40 mmol), 1,4-dioxane (5 mL) and water (3 mL). The mixture was stirred overnight at room temperature reaction. Ethyl acetate and water were added, and the mixture was adjusted to pH 2-3 with 1 *N* hydrochloric acid. The organic layer was concentrated under reduced pressure to dryness. The residue was purified by silica gel column chromatography (eluent: DCM-$CH_3OH$ = 100-0 to 90-10) to obtain **compound 46** (0.18 g, yield: 88%).

LCMS m/z = 612.2[M+H]$^+$

**Example 47:**

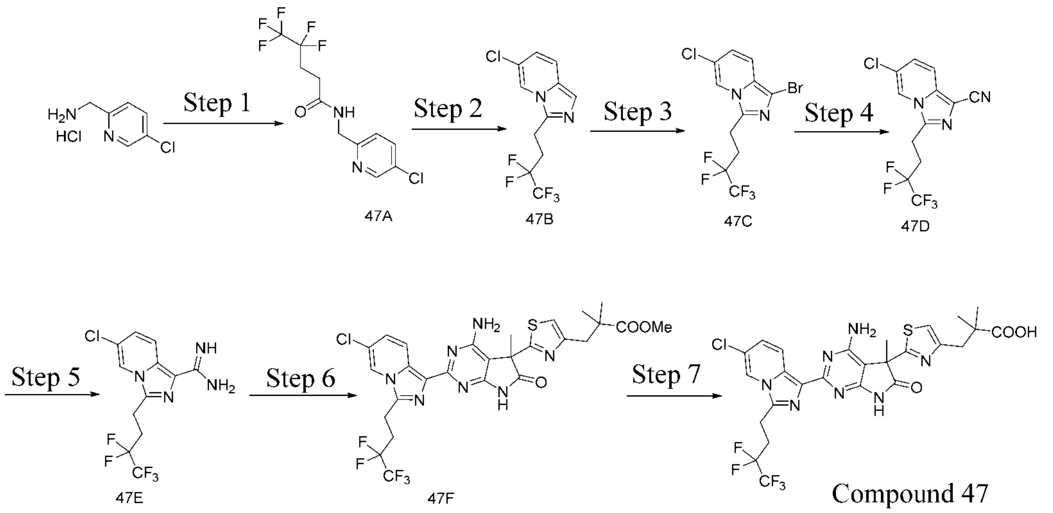


Step 1: Preparation of **47A**

Compound 2-(aminomethyl)-5-chloropyridine hydrochloride (1.00 g, 5.59 mmol) and 4,4,5,5,5-pentafluoropentanoic acid (1.61 g, 8.38 mmol) were dissolved in DMF (20 mL). HATU (4.25 g, 11.18 mmol) was added, and the mixture was stirred at room temperature for 30 min. DIPEA (3.61 g, 27.95 mmol) was added, and the mixture was reacted at room temperature for 1 h and extracted with water and ethyl acetate. The organic phase was washed with a saturated aqueous sodium chloride solution, dried over anhydrous sodium sulphate and concentrated under reduced pressure. The residue was purified by column chromatography (petroleum ether/ethyl acetate (V/V) = 100/0-100/30) to obtain compound **47A** (1.7 g, yield: 96%).

Step 2: Preparation of **47B**

Compound **47A** (1.7 g, 5.37 mmol) was dissolved in phosphorus oxychloride (20 mL), and the mixture was warmed to reflux and reacted overnight, cooled to room temperature and concentrated under reduced pressure. The residue was carefully and slowly added to water, and the mixture was stirred for 5 min. Ethyl acetate was added, and the mixture was stirred for layer separation. The aqueous layer was treated with a saturated aqueous sodium bicarbonate solution and then extracted three times with ethyl acetate. The organic layers were combined, washed with a saturated aqueous sodium chloride solution, dried over anhydrous sodium sulphate and concentrated under reduced pressure. The residue was purified by flash silica gel column chromatography (ethyl acetate/petroleum ether (V/V) = 0/100-20/100) to obtain compound **47B** (1.6 g).

LCMS m/z = 299.1 $[M+H]^+$

Step 3: Preparation of **47C**

Compound **47B** (1.7 g, 5.69 mmol) was dissolved in DCM (20 mL). NBS (1.14 g, 6.26 mmol) was added, and the mixture was reacted at room temperature for 30 min. Water was added, and the mixture was stirred for layer separation. The organic layer was washed with a saturated aqueous sodium chloride solution, dried over anhydrous sodium sulphate and concentrated under reduced pressure. The residue was separated and purified by flash silica gel column chromatography (ethyl acetate/petroleum ether (V/V) = 0/100-10/100) to obtain compound **47C** (2.1 g).

Step 4: Preparation of **47D**

Compound **47C** (2 g, 5.30 mmol) and zinc cyanide (1.24 g, 10.6 mmol) were added to a 50 mL single-necked flask, and then zinc powder (693.35 mg, 10.6 mmol), 1,1'-bis(diphenylphosphino)ferrocene (1.76 g, 3.18 mmol), tris(dibenzylidene-BASE acetone)dipalladium (1.46 g, 1.59 mmol) and N,N-dimethylacetamide (30 mL) were added. Under nitrogen atmosphere, the mixture was warmed to 120°C and stirred for 2 h. The reaction solution was diluted in 100 mL of ethyl acetate, washed 3 times with water and washed once with saturated sodium chloride. The organic phase was dried over anhydrous sodium sulphate and then concentrated under reduced pressure. The residue was subjected to flash silica gel column chromatography (mobile phase: petroleum ether/ethyl acetate (V/V) = 10/1) to obtain compound **47D** (400 mg, yield: 23%).

LCMS m/z = 324.0 $[M+H]^+$

Step 5: Preparation of **47E**

In an ice bath, ammonium chloride (190 mg, 3.55 mmol) was added to a 50 mL single-necked flask. Toluene (10 mL) was added, and then trimethylaluminium (1.77 mL, 2 M in toluene) was slowly added dropwise. The mixture was warmed to room temperature, stirred and reacted for 3 h. Compound **47D** (230 mg, 0.71 mmol) was dissolved in toluene and added dropwise to the reaction solution. The mixture was warmed to 110°C, stirred overnight and cooled to room temperature. In an ice bath, silica gel and 20 mL of methanol were added. The mixture was further stirred for 30 min and subjected to suction filtration. The filter cake was washed 3 times with methanol. The organic phases were combined and concentrated under reduced pressure. The residue was purified by silica gel column chromatography (mobile phase: dichloromethane/methanol (V/V) = 8/1) to obtain compound **47E** (145 mg, yield: 60%).

Step 6: Preparation of **47F**

Compound **47E** (140 mg, 0.41 mmol), ethyl 3,3-dicyano-2-(4-(3-methoxy-2,2-dimethyl-3-oxopropyl)thiazo-2-yl)-2-methylpropanoate (CAS: 2101649-65-2, synthesised with reference to US20170174693) (149 mg, 0.41 mmol) and sodium bicarbonate (137.78 mg, 1.64 mmol) were mixed and dissolved in tert-butanol (5 mL). The mixture was warmed to 80°C, stirred overnight, cooled to room temperature, and directly concentrated under reduced pressure. The residue was purified by silica gel column chromatography (mobile phase: dichloromethane/methanol (V/V) = 20/1) to obtain compound **47F** (120 mg, yield: 44%).

LCMS m/z = 658.2 $[M+H]^+$

Step 7: Preparation of **compound 47**

Compound **47F** (120 mg, 0.18 mmol) was dissolved in a mixed solvent of 1,4-dioxane (4 mL) and water (2 mL). Lithium hydroxide hydrate (76 mg, 1.8 mmol) was added. The mixture was warmed to 60°C, stirred for 3 h and cooled to room temperature. In an ice bath, the mixture was acidified by adding dilute hydrochloric acid. The resulting mixture was extracted 3 times with dichloromethane. The organic phases were combined, dried over anhydrous sodium sulphate and concentrated under reduced pressure. The residue was purified by reverse-phase column chromatography (mobile phase: acetonitrile /0.1% TFA in water (V/V) = 5/95-50/50) to obtain compound **47** (68 mg, yield: 58%)

LCMS m/z = 644.1 $[M+H]^+$

**Compounds 47-1 and 47-2**

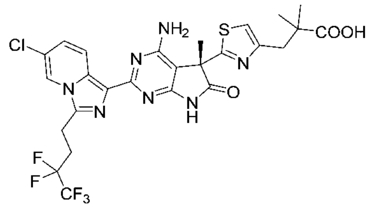
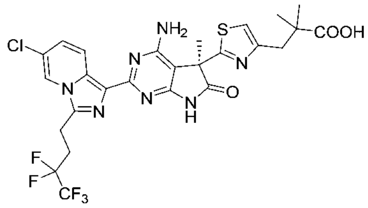

Compound 47-1 and Compound 47-2

The racemate of **compound 47** (68 mg) was subjected to chiral resolution, and the chiral resolution method was as follows:

1. instrument: SFC Prep 150 AP; chromatographic column: IG (19 mm×250 mm)
2. The sample was dissolved in methanol and filtered with a 0.45 μm filter to prepare a sample solution.
3. Preparative chromatography conditions: a. composition of mobile phases A and B: mobile phase A: CO2; mobile phase B: methanol/isopropanol = 8/2 (0.05% aqueous ammonia); b. isocratic elution, mobile phase B: 40%; c. flow rate: 41 ml/min.

After preparative separation, the components with the same retention time were combined and concentrated under reduced pressure to obtain **compound 47-1** (30 mg) and **compound 47-2** (30 mg).

**Compound 47-1:** retention time under chiral preparative conditions: 4.85 min, LCMS m/z = 644.5 [M+H]+

$^1$H NMR (400 MHz, DMSO-$d_6$) δ 12.30 - 11.96 (m, 1H), 11.31 (s, 1H), 8.72 - 8.62 (m, 2H), 7.23 (s, 1H), 7.12 - 7.02 (m, 1H), 6.83 - 6.67 (m, 2H), 3.39 - 3.32 (m, 2H), 2.99 - 2.78 (m, 4H), 1.78 (s, 3H), 1.11 (s, 6H).

**Compound 47-2:** retention time under chiral preparative conditions: 19.15 min, LCMS m/z = 644.5 [M+H]+

$^1$H NMR (400 MHz, DMSO -$d_6$) δ 12.36 - 12.01 (m, 1H), 11.31 (s, 1H), 8.78 - 8.58 (m, 2H), 7.23 (s, 1H), 7.11 - 7.01 (m, 1H), 6.82 - 6.66 (m, 2H), 3.41 - 3.31 (m, 2H), 3.00 - 2.76 (m, 4H), 1.79 (s, 3H), 1.11 (s, 6H).

| **Control compound 1**: compound **77B** in WO 2017112617 |
| --- |
| 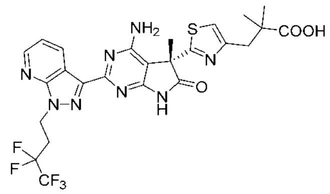 |

## Biological test examples

### 1. cGMP detection experiment in CHO-KI/sGC cells

Stably-transfected CHO-K1 cells stably expressing sGC α1/β1 heterodimers were constructed and named CHO-KI/sGC. The CHO-KI/sGC cells were cultured in a complete medium (FK12+10% FBS+1% penicillin-streptomycin+0.5 mg/mL hygromycin+ 0.25 mg/mL G418). On the day of the detection, the cells were reselected in EAB assay buffer (EBSS assay buffer+5 mL of MgCl2+10 mM HEPES+0.05% BSA) at a density of 2.25*10$^5$/mL. 0.5 mM IBMX was added to prevent cGMP degradation.

The cells were pre-incubated with 1 pM diethylenetriamine/nitric oxide (EDTA-NO) at room temperature for 30 minutes, and then different concentrations of the compounds were added. The mixture was further incubated at 37°C for 1 h. After the incubation was completed, the reaction was terminated, and intracellular cGMP levels were measured according to the instructions for the Cisbio kit (CisBio, 62GM2PEC). The maximum cGMP production relative to the positive compound was calculated according to Equation (1-1), where $RLU_{compound}$ represents the reading of the test compound, and $RLU_{reference}$ represents the maximum reading of the positive compound.

$$\text{Activion \%} = RLU_{compound}/RUL_{reference}*100\% \quad \text{Equation (1-1)}$$

Conclusion: The compounds of the present invention, such as the example compounds, have good stimulatory effects on cGMP production in CHO-KI/sGC cells.

### 2. In vitro detection experiment for soluble guanylate cyclase (sGC) activity

First, 100 nL of the compounds at different concentrations were transferred to a 384-well reaction plate (Greiner, Cat. No. 784075) using Echo 655 (LABCYTE, Cat # 655), with the final DMSO concentration of 1% in the reaction mixture; 2 μL of sGC (ICE, Cat. No. S2304F-H07SH2) was added to a 384-well reaction plate, and the mixture was centrifuged at 1000 rpm for 1 min; and then 1 μL of DETA NONOate was added, and the mixture was incubated at 37°C for10 min. After the incubation was completed, 2 μL of GTP was added, and the mixture was centrifuged at 1000 rpm for 1 min and reacted at 37°C for 60 min. In the reaction system, the final concentrations of sGC, GTP, and DETA NONOate were 1.5 nM, 5 μM, and 100 μM, respectively. After the reaction was completed, 5 μL of the test mixture (PerkinElmer, Cat. No. 62GM2PEG) was added and incubated at room temperature for 60 min. The TR-FRET signal (Ratio: 665/620 nm) was read using a microplate reader (BMG, Cat. No. PHERAstar FSX). A nonlinear regression curve was fitted using GraphPad Prism software, and $EC_{50}$ values were calculated. Compound A (Example 1 in WO 2010065275) was used as a positive reference compound. The activation rate was calculated according to Equation 2-1, where Low control represents the TR-FRET signal value of 1 μM compound A, and High control represents the TR-FRET signal value in the DMSO well.

Stimulation% = (ave High control - cpd well)/(ave High control - ave Low control)*100% Equation 2-1

Table 2-1 Results of enzymatic agonism and/or activation of test compounds on soluble guanylate cyclase (sGC).

| Compound No. | $EC_{50}$ (nM) | Compound No. | $EC_{50}$ (nM) |
|---|---|---|---|
| Compound 1-2 | A | Trifluoroacetate of compound 23 | A |
| Compound 3 | A | Compound 24 | A |
| Trifluoroacetate of compound 4 | A | Trifluoroacetate of compound 27 | A |
| Trifluoroacetate of compound 5 | A | Trifluoroacetate of compound 28 | A |
| Compound 8 | A | Compound 29 | A |
| Compound 9 | A | Compound 31 | A |
| Trifluoroacetate of compound 10 | A | Trifluoroacetate of compound 32 | A |
| Compound 11 | A | Trifluoroacetate of compound 33 | A |
| Compound 12 | A | Trifluoroacetate of compound 34 | A |
| Compound 13 | A | Compound 35 | A |
| Trifluoroacetate of compound 14 | A | Compound 36 | A |
| Trifluoroacetate of compound 15 | A | Compound 37 | A |
| Compound 16 | A | Trifluoroacetate of compound 38 | A |
| Compound 17 | A | Trifluoroacetate of compound 39 | A |
| Compound 18 | A | Compound 40 | A |
| Compound 19 | A | Compound 41 | A |
| Compound 20 | A | Compound 42 | A |
| Compound 21 | A | Compound 44 | A |
| Compound 46 | A | Compound 45 | A |
| Note: A < 20 nM, 20 nM ≤ B < 50 nM, 50 nM ≤ C < 200 nM | | | |

Conclusion: The compounds of the present invention, such as the example compounds, have good agonistic and/or activating effects on the enzyme activity of soluble guanylate cyclase (sGC).

### 3. cGMP detection experiment in LNCap cells

LNCap is a human prostate cancer cell line that expresses sGC protein. LNCap cells were purchased from ATCC and cultured in a complete medium (RPMI-1640+10% FBS+1% PS). On the day of the detection, the cells were reselected in assay buffer (EBSS assay buffer+5 mL of MgCl2+10 mM HEPES+0.05% BSA) at a density of $2 \times 10^5$/mL. 0.5 mM IBMX was added to prevent cGMP degradation.

The cells were pre-incubated with 20 μM diethylenetriamine/nitric oxide (DETA-NO) at 37°C for 30 minutes, and then different concentrations of the compounds were added. The mixture was further incubated at room temperature for 1 h. After the incubation was completed, the reaction was terminated, and intracellular cGMP levels were measured according to the instructions for the CisBio kit (Cisbio, 62GM2PEC). The cGMP production relative to compound A (Example 1 in WO 2010065275) was calculated according to Equation (3-1), and $EC_{50}$ values were calculated. Sample cGMP represents the reading of the test compound, Low control GMP represents the 1% DMSO control, and High control cGMP represents the maximum cGMP reading of compound A.

%Activity = (Sample cGMP - Low control GMP)/(High control cGMP - Low control cGMP)*100% Equation (3-1)

Table 3-1. Results of stimulatory effects of test compounds on cGMP production in LNCap cells

| Compound No. | $EC_{50}$ (nM) |
|---|---|
| Compound 4-1 | <500 |
| Compound 14-1 | <500 |
| Compound 15-1 | <500 |
| Compound 17-1 | <500 |
| Trifluoroacetate of compound 27 | <500 |
| Compound 31-1 | <500 |
| Compound 32-1 | <500 |
| Compound 35-1 | <500 |
| Compound 41-1 | <500 |
| Compound 43-1 | <500 |
| Control compound 1 | 3220 |

Conclusion: The compounds of the present invention, such as the example compounds, have good stimulatory effects on cGMP production in LNCap cells.

**4: Pharmacokinetic test in rats (inhalation administration)**

**Experimental objective:** In this experiment, the test compound was administered as a single dose via inhalation to SD rats, and the concentrations of the test compound in rat plasma and lungs were determined to evaluate the pharmacokinetic characteristics and bioavailability of the test compound in the rats.

**Experimental animals:** Male SD rats, 180-200 g, purchased from Chengdu Dossy Experimental Animals Co., Ltd.

**Experimental method:** On the day of the experiment, SD rats were randomly assigned into 6 groups according to body weight (for each compound), with 2 rats in each group. The rats were fasted with water available for 12 to 16 hours one day before the administration, and were fed 4 hours after the administration.

| Administration information | | | | | | |
|---|---|---|---|---|---|---|
| Test compound | Administrati on dosage* (mg/kg) | Administrati on concentration (mg/mL) | Administrati on volume (mL/kg) | Collected sample | Mode of administra tion | Vehicle |
| Compound of the present invention | 0.03 | 0.03 | 1 | Plasma or lung tissue | Inhalation | DMA+H-S-15(Solu-tol) +Saline |
| *Dosage is calculated based on free base. (DMA: dimethylacetamide; HS-15(Solutol): polyethylene glycol-15-hydroxystearate; saline: physiological saline) | | | | | | |

**Plasma sampling:** before and after the administration, blood was taken from the orbits of the rats under isoflurane anaesthesia, and placed in an $EDTAK_2$ centrifuge tube. Centrifugation was performed at 6000 rpm at 4°C for 10 min, and the plasma was collected. Time points for plasma collection comprise 0, 0.0833, 0.25, 0.5, 1, 2, 4, 7 and 24 h.

**Lung tissue sampling:** Animals from each group were euthanised by $CO_2$ inhalation at 0.25, 0.5, 1, 4, 7 and 24 hours after the administration. Then, the animal was dissected to collect lung tissue, which was rinsed with physiological saline, dried with filter paper, weighed in the balance, kept on wet ice and homogenised within 2 hours using a 6-fold volume (i.e., 6 mL per 1 g of tissue) of homogenisation buffer (50% methanol-water).

Before analysis and detection, all samples were stored at -60°C. The samples were analysed quantitatively by LC-MS/MS.

Table 4-1 Pharmacokinetic parameters of test compounds in rats (inhalation administration)

| Test compound | Lung $AUC_{0-t}$ (h.ng. $mL^{-1}$) | Lung $AUC_{0-t}$/plasma $AUC_{0-t}$ | Lung Cmax (ng/mL) |
|---|---|---|---|
| Compound 3-1 | 3626 | 65 | 2979 |
| Compound 4-1 | 2462 | 77.1 | 2352 |
| Compound 6-1 | 5173 | 659 | 2825 |
| Compound 17-1 | 2021 | 48.4 | 1754 |
| Compound 19-1 | 12248 | 245 | 2842 |
| Compound 31-1 | 608 | 97.9 | 1183 |
| Compound 35-1 | 5432 | / | 2545 |
| Compound 36-1 | 10133 | 49.1 | 4067 |
| Compound 37-1 | 3281 | / | 1729 |
| Compound 38-1 | / | 83.6 | 1743 |
| Compound 45-1 | 4500 | 43.4 | 2846 |
| Control compound 1 | 220 | 29.1 | 273 |

Conclusion: The compounds of the present invention, such as the example compounds, have good exposure levels and/or lung-to-plasma ratios in the lungs of rats after inhalation administration.

**5. Test for hERG potassium ion channel**

**Experimental platform:** Electrophysiological manual patch-clamp system

**Cell line:** Chinese hamster ovary (CHO) cell lines stably expressing hERG potassium ion channel

**Experimental method:** In CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarised from -80 mV to +20 mV for 2 s, then repolarised to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells ($n \geq 2$) were tested at each concentration.

**Data processing:** Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition\%} = [1 - (I / Io)] \times 100\%$$

where Inhibition% represents the percentage of inhibition of hERG potassium current by the compound, and *I* and *I*o represent the amplitude of hERG potassium current after and before the administration, respectively.

Compound $IC_{50}$ was calculated using GraphPad Prism 5 software by fitting according to the following equation:

$$Y = \text{Bottom} + (\text{Top-Bottom})/(1+10\verb|^|((\text{LogIC}_{50}\text{-X})^*\text{HillSlope}))$$

where X represents the Log value of the tested concentration of the test compound, Y represents the percentage inhibition at the corresponding concentration, and Bottom and Top represent the minimum and maximum percentage inhibitions, respectively.

Conclusion: The compounds of the present invention have no obvious inhibitory effects on the hERG potassium

ion channel.

### 6. CYP450 enzyme inhibition test

The objective of this study was to evaluate the effects of test compounds on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were incubated with human liver microsomes and test compounds of different concentrations, and reduced nicotinamide adenine dinucleotide phosphate (NADPH) was added to initiate the reaction. After the reaction was completed, the sample was treated and liquid chromatography-tandem mass spectrometry (LC-MS/MS) was used to quantitatively detect metabolites produced by specific substrates, changes in CYP enzyme activity were determined, and $IC_{50}$ value was calculated to evaluate the inhibitory potential of the test compound on each CYP enzyme subtype.

Conclusion: The compounds of the present invention have no significant or only weak inhibitory effects on CYP450 enzymes.

### 7. Pharmacodynamic study on relaxation of thoracic aortic vascular rings in SD rats

Male SD rats, 8 weeks old and of SPF grade, were purchased from Beijing Vital River Laboratory Animal Technology Co., Ltd. The animals were acclimatised for at least one week upon arrival at the animal facility. Prior to the experiment, the rats were weighed and randomly assigned to groups based on body weight. On the day of the experiment, the rats were deeply anesthetised with Zoletil 50 (20 mg/kg, i.p.) and Xylazine (8 mg/kg, i.p.). The thoracic cavity was quickly opened, and the aorta was carefully isolated. A segment of the descending aorta was excised and placed in a petri dish containing a K-H solution saturated with carbogen (95% $O_2$+5% $CO_2$). After removing connective tissue surrounding the vessel, vascular rings of approximately 3-5 mm in length were prepared. The vascular rings were suspended using custom-made hooks in an isolated tissue perfusion bath containing a 37°C K-H solution. The bath was aerated with carbogen, and connected to a force transducer (Chengdu Instrument Factory, JZ101H). The force transducer was connected to a multichannel electrophysiological signal recording system (Chengdu Instrument Factory, RM6240E). The vascular rings were rinsed with a K-H solution and equilibrated for 90 minutes under a resting tension of 3 g. Subsequently, the vascular rings were pre-contracted with $10^{-6}$ M norepinephrine in the bath until a stable tension was achieved. The test compound was then added to the bath in a cumulative concentration-gradient manner (from low to high) to induce vasodilation, with a 5-minute interval between each addition. The changes in tension of the thoracic aortic vascular rings were observed. The percentage of relaxation was calculated for each concentration.

Conclusion: The compounds of the present invention, such as the example compounds, have significant vasodilatory effects in rat thoracic aortic vascular rings.

### 8. SHR rat telemetric blood pressure monitoring

Surgical implantation of blood pressure telemetry transmitter: one day prior to surgery, the DSI transmitter was sterilised by immersion in a 2% glutaraldehyde solution for 8-10 h; animals were weighed, and anesthetised with Xylazine (8 mg/kg, i.p.) and Zoletil 50 (20 mg/kg, i.p.); and the animals were fasted overnight before the procedure. The implantation procedure was performed on Day 1 of the study, as detailed below. The abdominal skin was aseptically disinfected, and a longitudinal incision was made. The abdominal organs were dissected to expose the abdominal aorta. The pressure-sensing catheter of the transmitter was inserted into the abdominal aorta. Haemostasis and closure of the surgical site were achieved with bioadhesives, and then the transmitter body was secured to the abdominal wall. The muscle and skin layers were sutured, the surgical site was disinfected, and meloxicam was administered subcutaneously for analgesia. Following surgery, the animals were recovered in a 37°C incubator until resumption of voluntary motor activity, and then singly housed in their cages. For the first three postoperative days, the animals received daily subcutaneous injections of gentamicin sulphate (4-8 mg/kg) for infection prophylaxis and meloxicam for analgesia.

Blood pressure monitoring: After approximately 10 days of postoperative recovery, baseline blood pressure was recorded over a 24-hour period. Rats were assigned to groups based on baseline blood pressure. Following grouping, the rats were administered (either orally or via IT). After a single administration, blood pressure was monitored for 24 hours, during which changes in systolic blood pressure, diastolic blood pressure, mean arterial pressure, and heart rate were recorded. Raw data were used to calculate the mean systolic blood pressure, mean diastolic blood pressure, mean arterial pressure, and mean heart rate at regular intervals (conventionally set at 30 minutes). A p-value of less than 0.05 was considered statistically significant.

Conclusion: The compounds of the present invention, such as the example compounds, have significant blood pressure-lowering effects in rats, while no significant reduction in abdominal aortic pressure was observed following the IT administration.

## Claims

1. A compound or a stereoisomer, tautomer, deuterated compound, solvate, prodrug, metabolite, pharmaceutically acceptable salt or co-crystal thereof, **characterised in that** the compound is a compound represented by general formula (I), wherein

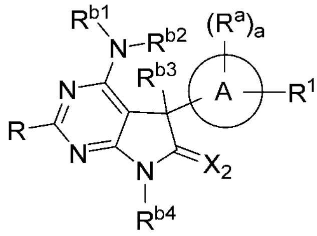

(I)

R is selected from

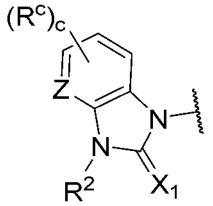
,
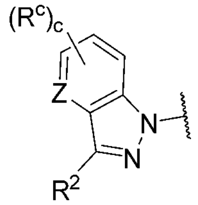
,
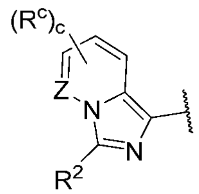
,

or

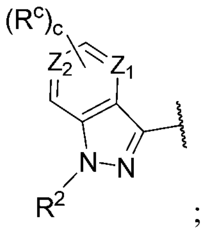
;

Z is selected from CH or N;
$Z_1$ or $Z_2$ is each independently selected from CH or N, and at least one of $Z_1$ and $Z_2$ is N;
$X_1$ or $X_2$ is each independently selected from O or S;
A is selected from $C_{3-12}$ carbocyclyl or 4- to 12-membered heterocyclyl;
$R^1$ is $-M-(CR^{1a}R^{1b})_r-(CR^{1c}R^{1d})_s-COOH$;
M is selected from a bond, $C_{3-12}$ carbocyclyl or 4- to 12-membered heterocyclyl, wherein the carbocyclyl or heterocyclyl is optionally substituted with 1 to 4 $R^m$;
$R^2$ is selected from $C_{1-6}$ alkyl or $C_{1-6}$ alkylene-Q, wherein the alkyl or alkylene is optionally substituted with 1 to 10 $R^k$;
Q is selected from $C_{3-12}$ carbocyclyl or 4- to 12-membered heterocyclyl, wherein the carbocyclyl or heterocyclyl is optionally substituted with 1 to 4 $R^q$;
$R^a$, $R^c$, $R^m$, $R^q$, $R^{b3}$, $R^{1a}$, $R^{1b}$, $R^{1c}$, and $R^{1d}$ are each independently selected from H, deuterium, halogen, OH, CN, $NH_2$, $C_{1-6}$ alkyl, $OC_{1-6}$ alkyl, $SC_{1-6}$ alkyl, $C_{2-6}$ alkenyl, $C_{2-6}$ alkynyl, $NHC_{1-6}$ alkyl, $N(C_{1-6}$ alkyl$)_2$, $-O-C_{3-6}$ carbocyclyl, -O-3- to 7-membered heterocyclyl, $-NH-C_{3-6}$ carbocyclyl, -NH-3- to 7-membered heterocyclyl, $-C_{0-4}$ alkylene-$C_{3-6}$ carbocyclyl, or $-C_{0-4}$ alkylene-3- to 7-membered heterocyclyl, wherein the alkyl, alkylene, alkenyl, alkynyl, carbocyclyl or heterocyclyl is optionally substituted with 1 to 4 $R^k$;
alternatively, $R^{1a}$ and $R^{1b}$ or $R^{1c}$ and $R^{1d}$ respectively taken together with the carbon atom to which they are attached form $C_{3-12}$ carbocyclyl or 4- to 12-membered heterocyclyl, wherein the carbocyclyl or heterocyclyl is optionally substituted with 1 to 4 $R^k$;
$R^{b1}$, $R^{b2}$, and $R^{b4}$ are each independently selected from H, deuterium, OH, CN, $NH_2$, $C_{1-6}$ alkyl, $C_{2-6}$ alkenyl, $C_{2-6}$ alkynyl, $NHC_{1-6}$ alkyl, $N(C_{1-6}$ alkyl$)_2$, $-NH-C_{3-6}$ carbocyclyl, -NH-3- to 7-membered heterocyclyl, $-C_{0-4}$ alkylene-$C_{3-6}$ carbocyclyl, or $-C_{0-4}$ alkylene-3- to 7-membered heterocyclyl, wherein the alkyl, alkylene, alkenyl, alkynyl,

carbocyclyl or heterocyclyl is optionally substituted with 1 to 4 $R^k$;
each $R^k$ is independently selected from H, deuterium, halogen, OH, =O, CN, $NH_2$, COOH, $CONH_2$, $C_{1-6}$ alkyl, $OC_{1-6}$ alkyl, $SC_{1-6}$ alkyl, $C_{2-6}$ alkenyl, $C_{2-6}$ alkynyl, $NHC_{1-6}$ alkyl, $N(C_{1-6}$ alkyl$)_2$, -O-$C_{3-6}$ carbocyclyl, -O-3- to 7-membered heterocyclyl, -NH-$C_{3-6}$ carbocyclyl, -NH-3- to 7-membered heterocyclyl, -$C_{0-4}$ alkylene-$C_{3-6}$ carbocyclyl, or -$C_{0-4}$ alkylene-3- to 7-membered heterocyclyl, wherein the alkyl, alkylene, alkenyl, alkynyl, carbocyclyl or heterocyclyl is optionally substituted with 1 to 4 substituents selected from deuterium, halogen, =O, CN, OH, $NH_2$, $C_{1-6}$ alkyl, or $C_{1-6}$ alkoxy;
a, c, r, and s are each independently selected from 0, 1, 2, 3, or 4.

2. The compound or the stereoisomer, tautomer, deuterated compound, solvate, prodrug, metabolite, pharmaceutically acceptable salt or co-crystal thereof according to claim 1, **characterised in that**

A is selected from $C_{3-11}$ cycloalkyl, 4- to 11-membered heterocycloalkyl, $C_{6-10}$ aryl or 5- to 10-membered heteroaryl;
M is selected from a bond, $C_{3-11}$ cycloalkyl, 4- to 11-membered heterocycloalkyl, $C_{6-10}$ aryl or 5- to 10-membered heteroaryl, wherein the cycloalkyl, heterocycloalkyl, aryl, or heteroaryl is optionally substituted with 1 to 4 $R^m$;
$R^2$ is selected from $C_{1-5}$ alkyl or $C_{1-4}$ alkylene-Q, wherein the alkyl or alkylene is optionally substituted with 1 to 8 $R^k$;
Q is selected from $C_{3-11}$ cycloalkyl, 4- to 11-membered heterocycloalkyl, $C_{6-10}$ aryl or 5- to 10-membered heteroaryl, wherein the cycloalkyl, heterocycloalkyl, aryl, or heteroaryl is optionally substituted with 1 to 4 $R^q$;
$R^a$, $R^c$, $R^m$, $R^q$, $R^{b3}$, $R^{1a}$, $R^{1b}$, $R^{1c}$, and $R^{1d}$ are each independently selected from H, deuterium, halogen, OH, CN, $NH_2$, $C_{1-4}$ alkyl, $OC_{1-4}$ alkyl, $SC_{1-4}$ alkyl, $C_{2-4}$ alkenyl, $C_{2-4}$ alkynyl, $NHC_{1-4}$ alkyl, $N(C_{1-4}$ alkyl$)_2$, -O-$C_{3-6}$ carbocyclyl, -O-3- to 6-membered heterocyclyl, -NH-$C_{3-6}$ carbocyclyl, -NH-3- to 6-membered heterocyclyl, -$C_{0-2}$ alkylene-$C_{3-6}$ carbocyclyl, or -$C_{0-2}$ alkylene-3- to 6-membered heterocyclyl, wherein the alkyl, alkylene, alkenyl, alkynyl, carbocyclyl or heterocyclyl is optionally substituted with 1 to 4 $R^k$;
alternatively, $R^{1a}$ and $R^{1b}$ or $R^{1c}$ and $R^{1d}$ respectively taken together with the carbon atom to which they are attached form $C_{3-11}$ cycloalkyl or 4- to 11-membered heterocycloalkyl, wherein the cycloalkyl or heterocycloalkyl is optionally substituted with 1 to 4 $R^k$;
$R^{b1}$, $R^{b2}$, and $R^{b4}$ are each independently selected from H, deuterium, OH, CN, $NH_2$, $C_{1-4}$ alkyl, $C_{2-4}$ alkenyl, $C_{2-4}$ alkynyl, $NHC_{1-4}$ alkyl, $N(C_{1-4}$ alkyl$)_2$, -NH-$C_{3-6}$ carbocyclyl, -NH-3- to 6-membered heterocyclyl, -$C_{0-2}$ alkylene-$C_{3-6}$ carbocyclyl, or -$C_{0-2}$ alkylene-3- to 6-membered heterocyclyl, wherein the alkyl, alkylene, alkenyl, alkynyl, carbocyclyl or heterocyclyl is optionally substituted with 1 to 4 $R^k$;
each $R^k$ is independently selected from H, deuterium, halogen, OH, =O, CN, $NH_2$, COOH, $CONH_2$, $C_{1-4}$ alkyl, $OC_{1-4}$ alkyl, $SC_{1-4}$ alkyl, $C_{2-4}$ alkenyl, $C_{2-4}$ alkynyl, $NHC_{1-4}$ alkyl, $N(C_{1-4}$ alkyl$)_2$, -O-$C_{3-6}$ carbocyclyl, -O-3- to 6-membered heterocyclyl, -NH-$C_{3-6}$ carbocyclyl, -NH-3- to 6-membered heterocyclyl, -C0-2 alkylene-C3-6 carbocyclyl, or -C0-2 alkylene-3- to 6-membered heterocyclyl, wherein the alkyl, alkylene, alkenyl, alkynyl, carbocyclyl or heterocyclyl is optionally substituted with 1 to 4 substituents selected from deuterium, halogen, =O, CN, OH, $NH_2$, $C_{1-4}$ alkyl, or $C_{1-4}$ alkoxy.

3. The compound or the stereoisomer, tautomer, deuterated compound, solvate, prodrug, metabolite, pharmaceutically acceptable salt or co-crystal thereof according to claim 2, **characterised in that**

A is selected from $C_{3-6}$ cycloalkyl, 4- to 6-membered heterocycloalkyl, $C_{6-10}$ aryl or 5- to 10-membered heteroaryl;
M is selected from a bond, $C_{3-6}$ cycloalkyl, 4- to 6-membered heterocycloalkyl, $C_{6-10}$ aryl or 5- to 10-membered heteroaryl, wherein the cycloalkyl, heterocycloalkyl, aryl, or heteroaryl is optionally substituted with 1 to 4 $R^m$;
$R^2$ is selected from $C_{1-4}$ alkyl or $C_{1-3}$ alkylene-Q, wherein the alkyl or alkylene is optionally substituted with 1 to 6 $R^k$;
Q is selected from $C_{3-6}$ cycloalkyl, 4- to 6-membered heterocycloalkyl, $C_{6-10}$ aryl or 5- to 10-membered heteroaryl, wherein the cycloalkyl, heterocycloalkyl, aryl, or heteroaryl is optionally substituted with 1 to 4 $R^q$;
$R^a$, $R^c$, $R^m$, $R^q$, $R^{b3}$, $R^{1a}$, $R^{1b}$, $R^{1c}$, and $R^{1d}$ are each independently selected from H, deuterium, F, Cl, Br, I, OH, CN, $NH_2$, $NHCH_3$, $N(CH_3)_2$, or the following groups optionally substituted with 1 to 4 $R^k$: methyl, ethyl, propyl, isopropyl, methoxy, ethoxy, isopropoxy, ethenyl, ethynyl, methylthio, cyclopropyl, cyclobutyl, cyclopentyl, cyclohexyl, azetidinyl, oxetanyl, pyrrolidyl, piperidyl, morpholinyl, or phenyl;
alternatively, $R^{1a}$ and $R^{1b}$ or $R^{1c}$ and $R^{1d}$ respectively taken together with the carbon atom to which they are attached form $C_{3-7}$ cycloalkyl or 4- to 7-membered heterocycloalkyl, wherein the cycloalkyl or heterocycloalkyl is optionally substituted with 1 to 4 $R^k$;
$R^{b1}$, $R^{b2}$, and $R^{b4}$ are each independently selected from H, deuterium, OH, CN, $NH_2$, or the following groups optionally substituted with 1 to 4 $R^k$; methyl, ethyl, propyl, isopropyl, ethenyl, ethynyl, cyclopropyl, cyclobutyl,

cyclopentyl, cyclohexyl, azetidinyl, oxetanyl, pyrrolidyl, piperidyl, morpholinyl, or phenyl;

each $R^k$ is independently selected from deuterium, F, Cl, Br, I, OH, =O, CN, $NH_2$, COOH, $CONH_2$, $NHCH_3$, $N(CH_3)_2$, methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl, wherein the methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl is optionally substituted with 1 to 4 substituents selected from deuterium, F, Cl, Br, I, =O, CN, OH, $NH_2$, $C_{1-4}$ alkyl, or $C_{1-4}$ alkoxy.

**4.** The compound or the stereoisomer, tautomer, deuterated compound, solvate, prodrug, metabolite, pharmaceutically acceptable salt or co-crystal thereof according to claim 3, **characterised in that**

A is selected from the following groups optionally substituted with 1 to 4 $R^a$: cyclopropyl, cyclobutyl, cyclopentyl, cyclohexyl, oxetanyl, azetidinyl, pyrrolidyl, piperidyl, piperazinyl, morpholinyl, tetrahydrofuryl, tetrahydropyranyl, phenyl, pyrrolyl, thienyl, furyl, pyrazolyl, imidazolyl, thiazolyl, isothiazolyl, oxazolyl, isoxazolyl, pyridyl, pyrimidyl, pyrazinyl, or pyridazinyl;

M is selected from a bond, or the following groups optionally substituted with 1 to 4 $R^m$: cyclopropyl, cyclobutyl, cyclopentyl, cyclohexyl, azetidinyl, pyrrolidyl, piperidyl, piperazinyl, phenyl, pyrrolyl, thienyl, furyl, pyrazolyl, imidazolyl, thiazolyl, isothiazolyl, oxazolyl, isoxazolyl, pyridyl, pyrimidyl, pyrazinyl, or pyridazinyl;

$R^2$ is selected from methyl, ethyl, propyl, butyl, -methylene-Q, -ethylene-Q, or -propylene-Q, wherein the methyl, ethyl, propyl, butyl, methylene, ethylene, or propylene is optionally substituted with 1 to 6 $R^k$;

Q is selected from the following groups optionally substituted with 1 to 4 $R^q$: cyclopropyl, cyclobutyl, cyclopentyl, cyclohexyl, oxetanyl, azetidinyl, pyrrolidyl, piperidyl, piperazinyl, morpholinyl, tetrahydrofuryl, tetrahydropyranyl, phenyl, pyrrolyl, thienyl, furyl, pyrazolyl, imidazolyl, thiazolyl, isothiazolyl, oxazolyl, isoxazolyl, pyridyl, pyrimidyl, pyrazinyl, or pyridazinyl;

$R^a$, $R^c$, $R^m$, $R^q$, $R^{b3}$, $R^{1a}$, $R^{1b}$, $R^{1c}$, and $R^{1d}$ are each independently selected from H, deuterium, F, Cl, Br, I, OH, CN, $NH_2$, $NHCH_3$, $N(CH_3)_2$, or methyl, ethyl, methoxy or cyclopropyl optionally substituted with 1 to 4 $R^k$;

alternatively, $R^{1a}$ and $R^{1b}$ or $R^{1c}$ and $R^{1d}$ respectively taken together with the carbon atom to which they are attached form the following groups optionally substituted with 1 to 4 $R^k$: cyclopropyl, cyclobutyl, cyclopentyl, cyclohexyl, oxetanyl, azetidinyl, pyrrolidyl, piperidyl, piperazinyl, morpholinyl, tetrahydrofuryl, or tetrahydropyranyl;

$R^{b1}$, $R^{b2}$, and $R^{b4}$ are each independently selected from H, deuterium, OH, CN, $NH_2$, or methyl, ethyl, methoxy or cyclopropyl optionally substituted with 1 to 4 $R^k$;

each $R^k$ is independently selected from deuterium, F, Cl, Br, I, OH, =O, CN, $NH_2$, COOH, $CONH_2$, $NHCH_3$, $N(CH_3)_2$, methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl, wherein the methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl is optionally substituted with 1 to 4 substituents selected from deuterium, F, Cl, Br, I, =O, CN, OH, $NH_2$, methyl, ethyl, methoxy, or ethoxy.

**5.** The compound or the stereoisomer, tautomer, deuterated compound, solvate, prodrug, metabolite, pharmaceutically acceptable salt or co-crystal thereof according to claim 4, **characterised in that**

A is selected from the following groups optionally substituted with 1 to 4 $R^a$: phenyl, thiazolyl, oxazolyl, pyridyl, pyrimidyl, pyrazinyl, or pyridazinyl;

$R^1$ is selected from

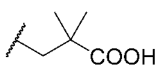 , 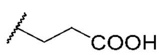 , 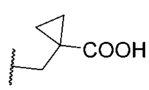 , 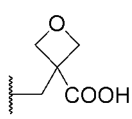 ,

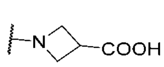 , 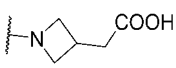 , or 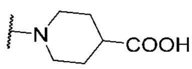 ;

R is selected from

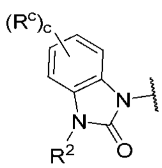, 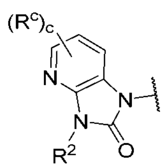, 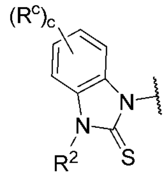, 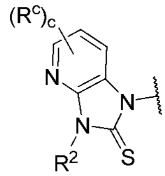,

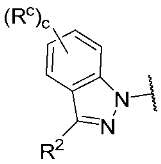, 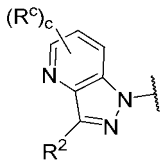, 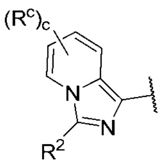, 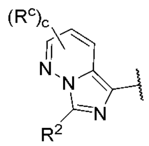, 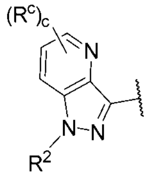, or 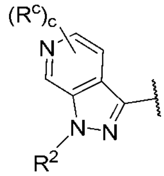;

$R^2$ is selected from

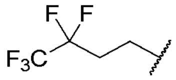, 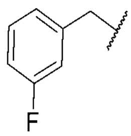, 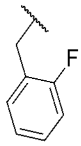, 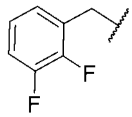, 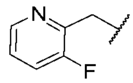,

or

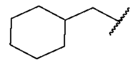;

$R^a$, $R^c$, and $R^{b3}$ are each independently selected from H, deuterium, F, Cl, Br, I, OH, CN, $NH_2$, $NHCH_3$, $N(CH_3)_2$, $CF_3$, $CHF_2$, $CH_2F$, methyl, ethyl, methoxy, or cyclopropyl;
$R^{b1}$, $R^{b2}$, and $R^{b4}$ are each independently selected from H, deuterium, OH, CN, $NH_2$, methyl, ethyl, methoxy, or cyclopropyl;
c is selected from 0, 1 or 2.

**6.** The compound or the stereoisomer, tautomer, deuterated compound, solvate, prodrug, metabolite, pharmaceutically acceptable salt or co-crystal thereof according to claim 1, **characterised in that** the compound has a structure selected from one of those in Table E-1.

**7.** A pharmaceutical composition, **characterised by** comprising the compound or the stereoisomer, tautomer, deuterated compound, solvate, prodrug, metabolite, pharmaceutically acceptable salt or co-crystal thereof according to any one of claims 1-6, and a pharmaceutically acceptable carrier, wherein preferably, the pharmaceutical composition comprises 0.01-1500 mg of the compound or the stereoisomer, tautomer, deuterated compound, solvate, prodrug, metabolite, pharmaceutically acceptable salt or co-crystal thereof according to any one of claims 1-6.

**8.** Use of the compound or the stereoisomer, tautomer, deuterated compound, solvate, prodrug, metabolite, pharmaceutically acceptable salt or co-crystal thereof according to any one of claims 1-6 or the pharmaceutical composition according to claim 7 in the preparation of a drug for treating sGC-related diseases.

**9.** The use according to claim 8, **characterised in that** the diseases are selected from cardiovascular diseases, kidney diseases or respiratory diseases, preferably pulmonary arterial hypertension, pulmonary hypertension or chronic obstructive pulmonary disease.

**10.** A method for treating a disease in a mammal, **characterised in that** the method comprises administering to a subject a therapeutically effective amount of the compound or the stereoisomer, deuterated compound, solvate, prodrug, metabolite, pharmaceutically acceptable salt or co-crystal thereof according to any one of claims 1-6 or the pharmaceutical composition according to claim 7, wherein the therapeutically effective amount is preferably 0.01-1500 mg, and the disease is preferably cardiovascular diseases, kidney diseases or respiratory diseases.

# INTERNATIONAL SEARCH REPORT

International application No.
**PCT/CN2024/098179**

## A. CLASSIFICATION OF SUBJECT MATTER

C07D487/04(2006.01)i; C07D471/04(2006.01)i; C07D519/00(2006.01)i; A61K31/519(2006.01)i; A61P9/00(2006.01)i; A61P11/00(2006.01)i

According to International Patent Classification (IPC) or to both national classification and IPC

## B. FIELDS SEARCHED

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXT, CNKI, ISI Web of science, Registry(STN), Caplus(STN), MARPAT(STN): 海思科, 可溶性鸟苷酸环化酶, 调节剂, 活化剂, 吡咯, 嘧啶, haisco, sGC, modulators, activator, pyrrole , pyrimidine, 结构式检索, structural formula search

## C. DOCUMENTS CONSIDERED TO BE RELEVANT

| Category* | Citation of document, with indication, where appropriate, of the relevant passages | Relevant to claim No. |
|---|---|---|
| X | Registry. "2101646-21-1" *STN*, 10 July 2017 (2017-07-10), entire document | 1-4 |
| Y | CN 103096718 A (MERCK SHARP & DOHME CORP.) 08 May 2013 (2013-05-08) claims 1 and 9-20, and description, paragraph [0008], and embodiment 1 | 1-10 |
| Y | CN 108738320 A (MERCK SHARP & DOHME CORP.) 02 November 2018 (2018-11-02) claims 1 and 14-25, and description, paragraphs [0006]-[0009], and embodiments 1A-263BA | 1-10 |
| A | WO 2022057836 A1 (MEDSHINE DISCOVERY INC.) 24 March 2022 (2022-03-24) claims 1 and 20-21, and description, page 2, paragraph 1, and embodiments 12-14 | 1-10 |
| A | CN 104321324 A (BAYER INTELLECTUAL PROPERTY GMBH et al.) 28 January 2015 (2015-01-28) claims 1-2 and 7-14, and description, paragraph [0010] | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 September 2024** | **09 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/098179**

**C. DOCUMENTS CONSIDERED TO BE RELEVANT**

| Category* | Citation of document, with indication, where appropriate, of the relevant passages | Relevant to claim No. |
|---|---|---|
| A | CN 104039784 A (BAYER INTELLECTUAL PROPERTY GMBH et al.) 10 September 2014 (2014-09-10) claims 1-2 and 7-14, and description, paragraph [0010], and embodiments 1-117 | 1-10 |
| A | CN 103649093 A (BAYER INTELLECTUAL PROPERTY GMBH et al.) 19 March 2014 (2014-03-19) claims 1 and 5-12, and description, paragraph [0008], and embodiments 1-12 | 1-10 |

Form PCT/ISA/210 (second sheet) (July 2022)

**INTERNATIONAL SEARCH REPORT**

International application No.
**PCT/CN2024/098179**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **10**
because they relate to subject matter not required to be searched by this Authority, namely:

Claim 10 falls within disease treatment methods as defined in PCT Rule 39.1(iv). The present search report is provided on the basis of the use of a compound in the preparation of a drug for treating related diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/098179**

| Patent document cited in search report | Publication date (day/month/year) | Patent family member(s) | Publication date (day/month/year) |
|---|---|---|---|
| CN 103096718 A | 08 May 2013 | UA 107112 C2 | 25 November 2014 |
| | | CR 20120599 A | 13 March 2013 |
| | | BR 112012030177 A2 | 24 November 2015 |
| | | BR 112012030177 B1 | 27 August 2019 |
| | | AR 081830 A1 | 24 October 2012 |
| | | JP 2013527196 A | 27 June 2013 |
| | | JP 5409961 B2 | 05 February 2014 |
| | | KR 20130032329 A | 01 April 2013 |
| | | KR 101499308 B1 | 05 March 2015 |
| | | NZ 603884 A | 27 June 2014 |
| | | MA 34330 B1 | 01 June 2013 |
| | | GT 201200320 A | 19 February 2015 |
| | | PE 20130222 A1 | 14 March 2013 |
| | | HN 2012002555 A | 07 September 2015 |
| | | MX 2012013774 A | 17 December 2012 |
| | | NI 201200172 A | 15 April 2013 |
| | | CL 2012003317 A1 | 08 March 2013 |
| | | DOP 2012000300 A | 30 April 2013 |
| | | EA 201291348 A1 | 28 June 2013 |
| | | EA 023254 B1 | 31 May 2016 |
| | | KR 20140019004 A | 13 February 2014 |
| | | WO 2011149921 A1 | 01 December 2011 |
| | | JP 2014055151 A | 27 March 2014 |
| | | JP 5789288 B2 | 07 October 2015 |
| | | SG 185777 A1 | 28 December 2012 |
| | | IL 223259 A0 | 03 February 2013 |
| | | AU 2011258436 A1 | 10 January 2013 |
| | | AU 2011258436 B2 | 12 June 2014 |
| | | CA 2800541 A1 | 01 December 2011 |
| | | CA 2800541 C | 22 March 2016 |
| | | EP 2575473 A1 | 10 April 2013 |
| | | EP 2575473 A4 | 27 November 2013 |
| | | EP 2575473 B1 | 20 January 2016 |
| | | ES 2564503 T3 | 23 March 2016 |
| | | TW 201202245 A | 16 January 2012 |
| | | CO 6640255 A2 | 22 March 2013 |
| | | TN 2012000544 A1 | 01 April 2014 |
| | | US 2013072492 A1 | 21 March 2013 |
| | | US 9365574 B2 | 14 June 2016 |
| CN 108738320 A | 02 November 2018 | ES 2794654 T3 | 18 November 2020 |
| | | HK 1255028 A1 | 02 August 2019 |
| | | SG 11201805272 XA | 30 July 2018 |
| | | EP 3394067 A1 | 31 October 2018 |
| | | EP 3394067 B1 | 01 April 2020 |
| | | CO 2018006300 A2 | 10 July 2018 |
| | | MX 2020011080 A | 06 November 2020 |
| | | PH 12018501342 A1 | 18 February 2019 |
| | | ECSP 18054606 A | 31 December 2018 |
| | | EA 201891303 A1 | 30 November 2018 |
| | | EA 036445 B1 | 11 November 2020 |

Form PCT/ISA/210 (patent family annex) (July 2022)

**INTERNATIONAL SEARCH REPORT**
**Information on patent family members**

International application No.
**PCT/CN2024/098179**

| Patent document cited in search report | Publication date (day/month/year) | Patent family member(s) | Publication date (day/month/year) |
|---|---|---|---|
| | | MX 2018007728 A | 15 August 2018 |
| | | RS 60344 B1 | 31 July 2020 |
| | | CR 20180330 A | 21 August 2018 |
| | | UA 122264 C2 | 12 October 2020 |
| | | BR 112018012579 A2 | 04 December 2018 |
| | | BR 112018012579 B8 | 24 October 2023 |
| | | SV 2018005715 A | 19 October 2018 |
| | | TN 2018000219 A1 | 04 October 2019 |
| | | US 2017174693 A1 | 22 June 2017 |
| | | US 10030027 B2 | 24 July 2018 |
| | | CA 3009193 A1 | 29 June 2017 |
| | | CA 3009193 C | 15 September 2020 |
| | | PE 20181802 A1 | 19 November 2018 |
| | | IL 260020 A | 31 July 2018 |
| | | IL 260020 B | 31 May 2021 |
| | | AR 107154 A1 | 28 March 2018 |
| | | CY 1123173 T1 | 29 October 2021 |
| | | NI 201800069 A | 05 October 2018 |
| | | CL 2018001671 A1 | 03 August 2018 |
| | | MY 194021 A | 08 November 2022 |
| | | WO 2017107052 A1 | 29 June 2017 |
| | | PT 3394067 T | 02 June 2020 |
| | | WO 2017112617 A1 | 29 June 2017 |
| | | LT 3394067 T | 10 June 2020 |
| | | TW 201734017 A | 01 October 2017 |
| | | TWI 724079 B | 11 April 2021 |
| | | JP 2018538333 A | 27 December 2018 |
| | | JP 6454448 B2 | 16 January 2019 |
| | | SI 3394067 T1 | 31 July 2020 |
| | | DOP 2018000153 A | 30 November 2018 |
| | | HUE 049941 T2 | 30 November 2020 |
| | | US 2018305366 A1 | 25 October 2018 |
| | | US 10428076 B2 | 01 October 2019 |
| | | HRP 20200857 T1 | 13 November 2020 |
| | | ME 03748 B | 20 April 2021 |
| | | PL 3394067 T3 | 24 August 2020 |
| | | AU 2016377364 A1 | 14 June 2018 |
| | | AU 2016377364 B2 | 14 February 2019 |
| | | KR 20180095904 A | 28 August 2018 |
| | | KR 102191312 B1 | 15 December 2020 |
| | | DK 3394067 T3 | 15 June 2020 |
| WO 2022057836 A1 | 24 March 2022 | WO 2022057836 A8 | 30 March 2023 |
| CN 104321324 A | 28 January 2015 | JP 2015505318 A | 19 February 2015 |
| | | JP 6157505 B2 | 05 July 2017 |
| | | UY 34568 A | 02 September 2013 |
| | | EP 2802587 A2 | 19 November 2014 |
| | | EP 2802587 B1 | 06 September 2017 |
| | | ES 2654297 T3 | 13 February 2018 |
| | | AR 089699 A1 | 10 September 2014 |
| | | TW 201341383 A | 16 October 2013 |

Form PCT/ISA/210 (patent family annex) (July 2022)

**INTERNATIONAL SEARCH REPORT**
**Information on patent family members**

International application No.
**PCT/CN2024/098179**

| Patent document cited in search report | Publication date (day/month/year) | Patent family member(s) | Publication date (day/month/year) |
|---|---|---|---|
| | | CA 2860847 A1 | 18 July 2013 |
| | | HK 1206336 A1 | 08 January 2016 |
| | | WO 2013104598 A2 | 18 July 2013 |
| | | WO 2013104598 A3 | 10 October 2013 |
| | | DE 102012200352 A1 | 11 July 2013 |
| | | US 2013210824 A1 | 15 August 2013 |
| | | US 9023849 B2 | 05 May 2015 |
| CN 104039784 A | 10 September 2014 | HK 1198698 A1 | 29 May 2015 |
| | | CO 6950471 A2 | 20 May 2014 |
| | | MX 2014002075 A | 12 January 2015 |
| | | MX 348470 B | 13 June 2017 |
| | | US 2015105374 A1 | 16 April 2015 |
| | | PE 20141582 A1 | 06 November 2014 |
| | | CR 20140096 A | 25 March 2014 |
| | | AU 2012300844 B2 | 30 March 2017 |
| | | CU 20140025 A7 | 27 May 2014 |
| | | AP 201407541 A0 | 31 March 2014 |
| | | US 2013338137 A1 | 19 December 2013 |
| | | US 8859569 B2 | 14 October 2014 |
| | | UY 34306 A | 05 April 2013 |
| | | TW 201326172 A | 01 July 2013 |
| | | TWI 565708 B | 11 January 2017 |
| | | EP 2751106 A1 | 09 July 2014 |
| | | EP 2751106 B1 | 18 October 2017 |
| | | KR 20140068112 A | 05 June 2014 |
| | | AR 087769 A1 | 16 April 2014 |
| | | SG 11201400083 VA | 27 June 2014 |
| | | ECSP 14013224 A | 31 March 2014 |
| | | IL 231244 A0 | 30 April 2014 |
| | | IL 231244 A | 28 September 2017 |
| | | MA 35365 B1 | 01 August 2014 |
| | | WO 2013030288 A1 | 07 March 2013 |
| | | CA 2847075 A1 | 07 March 2013 |
| | | EA 201490546 A1 | 29 August 2014 |
| | | EA 026701 B1 | 31 May 2017 |
| | | CL 2014000504 A1 | 03 October 2014 |
| | | GT 201400033 A | 15 January 2015 |
| | | NZ 621396 A | 24 June 2016 |
| | | DOP 2014000043 A | 15 April 2014 |
| | | BR 112014005110 A2 | 18 April 2017 |
| | | JP 2014527540 A | 16 October 2014 |
| | | JP 6054967 B2 | 27 December 2016 |
| CN 103649093 A | 19 March 2014 | WO 2012152629 A1 | 15 November 2012 |
| | | ES 2592267 T3 | 29 November 2016 |
| | | EP 2705037 A1 | 12 March 2014 |
| | | EP 2705037 B1 | 22 June 2016 |
| | | JP 2014513112 A | 29 May 2014 |
| | | JP 5976788 B2 | 24 August 2016 |
| | | CA 2834901 A1 | 15 November 2012 |
| | | US 2014171434 A1 | 19 June 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20170174693 A
- US 2013210824 A
- WO 2011158149 A
- WO 2010065275 A


### Non-patent literature cited in the description


- *Int. J. Mol. Sci.*, 2021, vol. 22, 6029
- *Molecules*, 2021, vol. 26, 3418
- *Nat. Rev. Cardiol.*, 2018, vol. 15, 292-316
- *Molecules*, 2023, vol. 28, 861
- *J. Med. Chem.*, 2017, vol. 60, 5146-5161
- *CHEMICAL ABSTRACTS*, 2101649-65-2
- *CHEMICAL ABSTRACTS*, 1361570-31-1
- *CHEMICAL ABSTRACTS*, 1048340-35-7
- *CHEMICAL ABSTRACTS*, 1407815-26-2